# EUROPEAN PATENT APPLICATION

(11) **EP 4 706 689 A2**
(43) Date of publication of application: **11.03.2026**
(21) Application number: 26150893.1
(22) Date of filing: 21.12.2020
(51) Int. Cl.: A61K 49/00

(54) **COMPOSITIONS FOR DELIVERY OF ANTISENSE COMPOUNDS**

(30) Priority: 19.12.2019 US 201962950639 P; 08.06.2020 US 202063036240 P; 15.07.2020 US 202063052286 P; 25.08.2020 US 202063069984 P
(62) Divisional of application: 20903078.2
(71) Applicant: Entrada Therapeutics, Inc., Boston, MA 02210 (US)
(72) Inventor: QIAN, Ziqing, Boston, 02210 (US); LI, Xiang, Boston, 02210 (US); KHEIRABADI, Mah, Boston, 02210 (US); DHANABAL, Mohanraj, Boston, 02210 (US); LIU, Haoming, Boston, 02210 (US); SHEN, Xiulong, Boston, 02210 (US); KAMER, Kimberli, Boston, 02210 (US); SETHURAMAN, Natarajan, Boston, 02210 (US)
(74) Representative: Secerna LLP

(57) **Abstract**

Provided herein are compounds comprising cyclic cell penetrating peptides and antisense compounds. Also provided herein are methods of modulating splicing, inhibiting or regulating translation, mediating degradation, blocking expansions of nucleotide repeats, and treating disease using the aforementioned compounds.

## Description

### CROSS-REFERENCE TO RELATED APPLICATIONS

This application claims priority to U.S. Application No. 62/950,639, filed December 19, 2019, U.S. Application No. 63/036,240, filed June 8, 2020, U.S. Application No. 63/052,286, filed July 15, 2020, and U.S. Application No. 63/069,984, filed August 25, 2020, each of which is incorporated by reference herein in its entirety.

### BACKGROUND

Therapeutic antisense compounds include e.g., antisense oligonucleotides, small interfering RNA (siRNA), microRNA (miRNA), ribozymes, immune stimulating nucleic acids, antagomir, antimir, microRNA mimic, supermir, Ul adaptors, CRISPR machinery and aptamers. These oligonucleotide containing compounds act via a variety of mechanisms. The therapeutic applications of antisense compounds are extremely broad, since these compounds can be synthesized with any nucleotide sequence directed against virtually any target gene or genomic segments.

A major problem for the use of antisense compounds in therapeutics is their limited ability to gain access to the intracellular compartment when administered systemically. Intracellular delivery of antisense compounds can be facilitated by use of carrier systems such as polymers, cationic liposomes or by chemical modification of the construct, for example by the covalent attachment of cholesterol molecules. However, intracellular delivery efficiency is low. Improved delivery systems are still required to increase the potency of these antisense compounds.

There is an unmet need for effective compositions to deliver antisense compounds to intracellular compartments so as to treat diseases caused by, e.g., aberrant gene transcription, splicing and/or translation.

### SUMMARY

Compositions for delivering nucleic acids are described herein.

In some embodiments, provided herein is a compound comprising: (a) a cyclic cell penetrating peptide (cCPP) sequence and (b) an antisense compound (AC) that is complementary to a target sequence in a pre-mRNA sequence.

In some embodiments, provided herein is a compound comprising a cCPP and an AC that is complementary to a target in a pre-mRNA sequence, wherein the AC comprises at least one modified nucleotide or nucleic acid selected from a phosphorothioate (PS) nucleotide, a phosphorodiamidate morpholino nucleotide, a locked nucleic acid (LNA), a peptide nucleic acid (PNA), a nucleotide comprising a 2'-O-methyl (2'-OMe) modified backbone, a 2'O-methoxyethyl (2'-MOE) nucleotide, a 2',4' constrained ethyl (cEt) nucleotide, and a 2'-deoxy-2'-fluoro-beta-D-arabinonucleic acid (2'F-ANA), and wherein hybridization of the AC with the target sequence reduces or prevents splicing, inhibits or regulates translation, mediates degradation, or blocks expansions of nucleotide repeats.

In some embodiments, provided herein is a compound comprising a cCPP and an AC that is complementary to a target in a pre-mRNA sequence, wherein the AC comprises small interfering RNA (siRNA), microRNA (miRNA), ribozymes, immune stimulating nucleic acids, antisense, antagomir, antimir, microRNA mimic, supermir, Ul adaptor, aptamer, or a CRISPR gene-editing machinery.

In some embodiments, provided herein is a compound comprising a cCPP and an AC that is complementary to a target in a pre-mRNA sequence, wherein the cCPP is conjugated to the 5' end or the 3' end of the AC.

In some embodiments, provided herein is a compound comprising a cCPP and an AC that is complementary to a target in a pre-mRNA sequence, further comprising a linker (L), which conjugates the cCPP to the AC.

In some embodiments, provided herein is a compound comprising a cCPP and an AC that is complementary to a target in a pre-mRNA sequence further comprising L, wherein the L is covalently bound to the side chain of an amino acid on the CPP.

In some embodiments, provided herein is a compound having a structure according to Formula I-A or Formula I-B:

cCPP-L-AC (I-A)

or

AC-L-cCPP (I-B),

wherein L of Formula I-A is covalently bound to the side chain of an amino acid on the CPP and to the 5' end of the AC, and L of Formula I-B is covalently bound to the side chain of an amino acid on the CPP and the 3' end of the AC.

In some embodiments, provided herein is a compound comprising a cCPP and an AC that is complementary to a target in a pre-mRNA sequence further comprising L, wherein L comprises one or more D or L amino acids, each of which is optionally substituted; alkylene, alkenylene, alkynylene, carbocyclyl, or heterocyclyl, each of which is optionally substituted; or -(R¹⁻X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is independently selected from H, alkyl, alkenyl, alkynyl, carbocyclyl, and heterocyclyl, each of which is optionally substituted, and z is an integer from 1 to 20; or combinations thereof.

In some embodiments, provided herein is a compound comprising a cCPP and an AC that is complementary to a target in a pre-mRNA sequence further comprising L, comprising one or more D or L amino acids; -(R¹⁻X-R²)z-, wherein each of R¹ and R², at each instance, are independently alkylene, each X is independently NR³, -NR³C(O)-, S, and O, wherein R³ is independently selected from H and alkyl, and z is an integer from 1 to 20; or combinations thereof.

In some embodiments, provided herein is a compound comprising a cCPP and an AC that is complementary to a target in a pre-mRNA sequence further comprising L, wherein the linker is conjugated to the AC through a bonding group (M).

In some embodiments, provided herein is a compound comprising a cCPP and an AC that is complementary to a target in a pre-mRNA sequence further comprising L, wherein the linker is conjugated to the AC through a bonding group (M), wherein M is selected from the group consisting of: 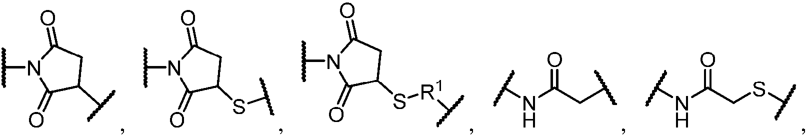 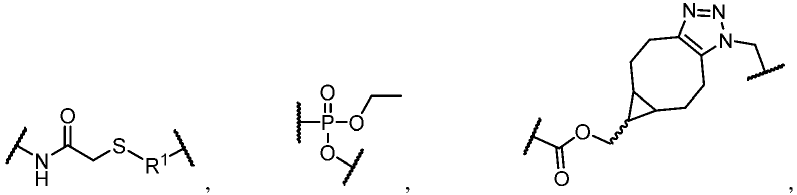 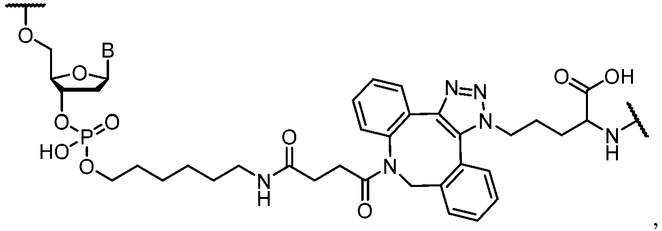 and 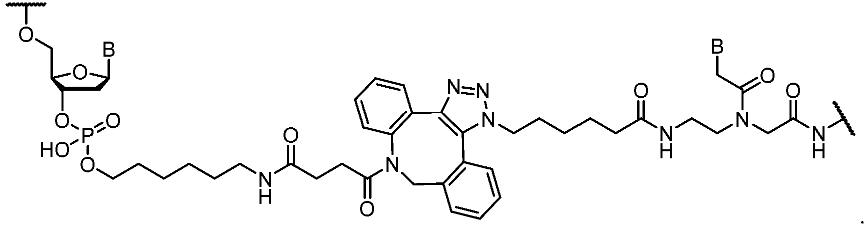 wherein: R¹ is alkylene, cycloalkyl, or 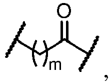 wherein m is 0 to 10 wherein each R is independently an alkyl, alkenyl, alkynyl, carbocyclyl, or heterocyclyl.

In some embodiments, provided herein is a compound comprising a cCPP and an AC that is complementary to a target in a pre-mRNA sequence further comprising L, wherein the linker is conjugated to the AC through a bonding group (M), wherein M is

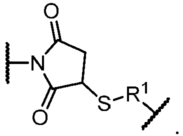

In some embodiments, provided herein is a compound comprising a cCPP and an AC that is complementary to a target in a pre-mRNA sequence further comprising L, wherein the linker is conjugated to the AC through a bonding group (M), wherein M is selected from the group consisting of: 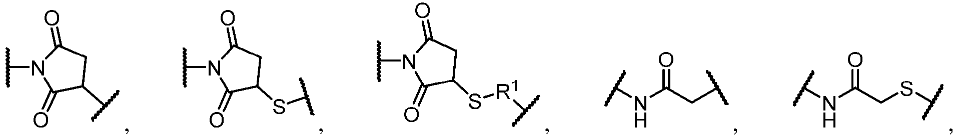 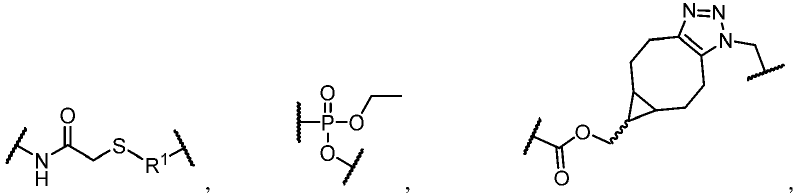 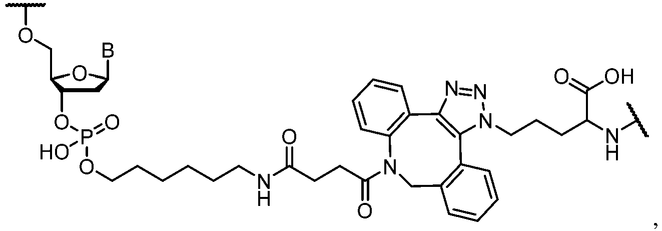 and 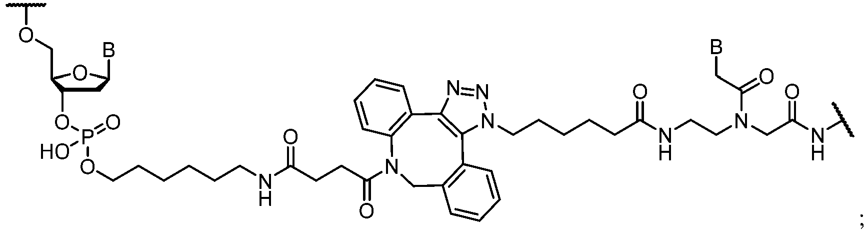 wherein: R¹ is alkylene, cycloalkyl, or 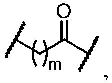 wherein m is 0 to 10 wherein each R is independently an alkyl, alkenyl, alkynyl, carbocyclyl, or heterocyclyl, wherein R¹ is 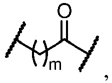 and m is 2.

In some embodiments, provided herein is a compound comprising a cCPP and an AC that is complementary to a target in a pre-mRNA sequence further comprising L, wherein L has the following structure: 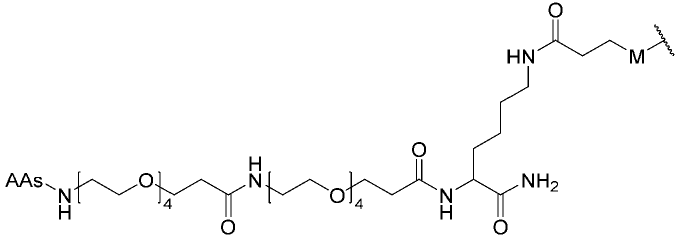 wherein AAₛ is a side chain or terminus of an amino acid on the CPP.

In some embodiments, provided herein is a compound having a following structure: 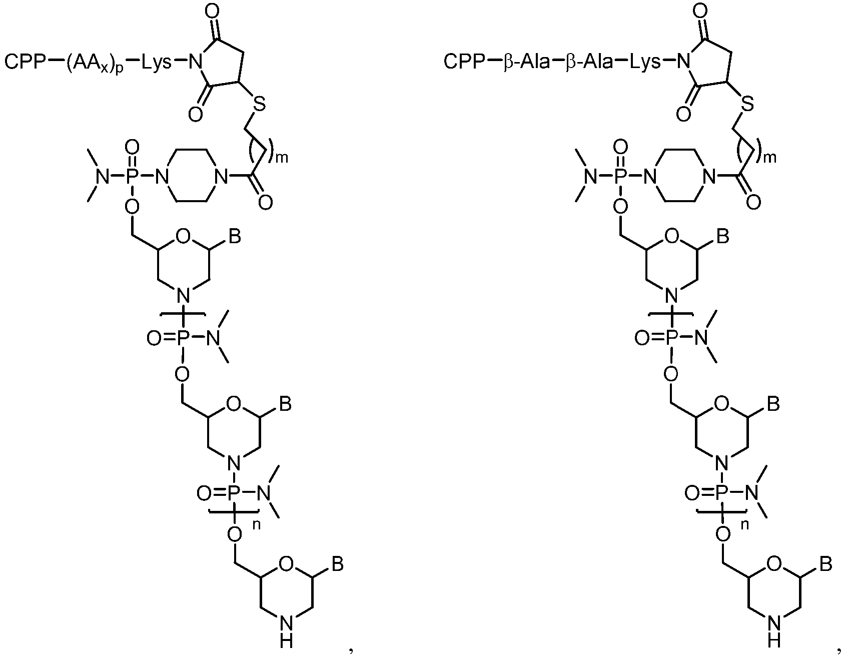 or 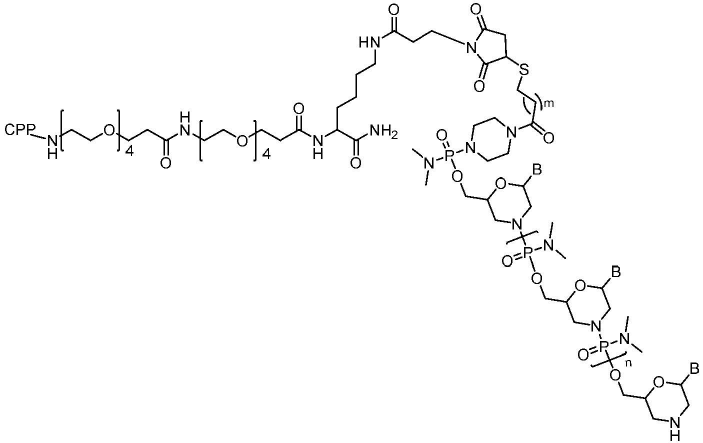 wherein each B is independently a nucleobase; each AAₓ is independently an amino acid; m is 1 to 10; and n is an integer from 1 to 50.

In some embodiments, provided herein is a compound comprising a cCPP and an AC that is complementary to a target in a pre-mRNA sequence, wherein the cCPP has a sequence comprising Formula III: 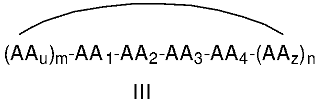 wherein: each of AA₁, AA₂, AA₃, and AA₄, are independently selected from a D or L amino acid, each of AAᵤ and AA_{z}, at each instance and when present, are independently selected from a D or L amino acid, and m and n are independently selected from a number from 0 to 6; and wherein: at least two amino acids are independently arginine, and at least two amino acids are independently a hydrophobic amino acid.

In some embodiments, provided herein is a compound comprising a cCPP and an AC that is complementary to a target in a pre-mRNA sequence, wherein the cCPP has a sequence comprising any one of Formula IV-A-D: 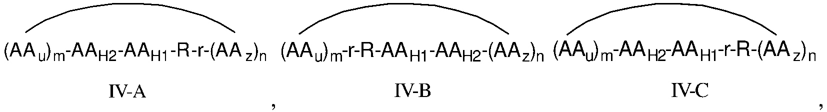 and 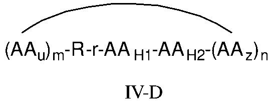
wherein: each of AA_{H1} and AA_{H2} are independently a D or L hydrophobic amino acid;
at each instance and when present, each of AA_{U} and AA_{Z} are independently a D or L amino acid; and m and n are independently selected from a number from 0 to 6.

In some embodiments, provided herein is a compound comprising a cCPP and an AC that is complementary to a target in a pre-mRNA sequence further comprising L, wherein the linker is conjugated to the AC through a bonding group (M), wherein M and covalently bound to the 5' end of the AC or the 3' end of the AC.

In some embodiments, provided herein is a compound comprising a cCPP and an AC that is complementary to a target in a pre-mRNA sequence, wherein the AC is complementary to a target sequence comprising an intron, comprising by an exon, or bridging an intron/exon junction.

In some embodiments, provided herein is a compound comprising a cCPP and an AC that is complementary to a target in a pre-mRNA sequence, wherein the AC is complementary to a target sequence comprising an intronic silencer sequence (ISS) or terminal stem loop (TSL) sequence of the target pre-mRNA.

In some embodiments, provided herein is a compound comprising a cCPP and an AC that is complementary to a target in a pre-mRNA sequence, wherein the AC is complementary to part or all of a splice site.

In some embodiments, provided herein is a compound comprising a cCPP and an AC that is complementary to a target in a pre-mRNA sequence, wherein the AC is complementary to part or all of a splice site, wherein the splice site is a splice donor site, a splice acceptor site, a cryptic splice site, or a mutation-induced aberrant splice site.

In some embodiments, provided herein is a compound comprising a cCPP and an AC that is complementary to a target in a pre-mRNA sequence, wherein hybridization of the AC with its target sequence results in exon skipping or exon inclusion.

In some embodiments, provided herein is a compound comprising a cCPP and an AC that is complementary to a target in a pre-mRNA sequence, wherein the AC is complementary to at least a portion of expended nucleotide repeats in target mRNA.

In some embodiments, provided herein is a compound comprising a cCPP and an AC that is complementary to a target in a pre-mRNA sequence, wherein hybridization of the AC with the target sequence blocks transcription of at least a portion of the nucleotide repeats.

In some embodiments, provided herein is a compound comprising a cCPP and an AC that is complementary to a target in a pre-mRNA sequence, wherein the AC is 5-50 nucleotides in length.

In some embodiments, provided herein is a compound comprising a cCPP and an AC that is complementary to a target in a pre-mRNA sequence, wherein the AC comprises one or more modified nucleotides or nucleic acids that affect one or more of nuclease resistance, pharmacokinetics, and affinity.

In some embodiments, provided herein is a compound comprising a cCPP and an AC that is complementary to a target in a pre-mRNA sequence, wherein the AC comprises one or more phosphorodiamidate morpholino nucleosides, 2'-O-methylated nucleosides, and/or locked nucleic acids (LNAs).

In some embodiments, provided herein is a compound comprising a cCPP and an AC that is complementary to a target in a pre-mRNA sequence, wherein the target gene is involved in the pathogenesis of a disease.

In some embodiments, provided herein is a compound comprising a cCPP and an AC that is complementary to a target in a pre-mRNA sequence, wherein the target gene is involved in the pathogenesis of a genetic disease.

In some embodiments, provided herein is a compound comprising a cCPP and an AC that is complementary to a target in a pre-mRNA sequence, wherein the target gene is involved in the pathogenesis of a cancer, an autoimmune disease, an inflammatory disease, or an infection.

In some embodiments, provided herein is a compound comprising a cCPP and an AC that is complementary to a target in a pre-mRNA sequence, wherein the target sequence in the pre-mRNA comprises a mutation-induced aberrant splice site.

In some embodiments, provided herein is a compound comprising a cCPP and an AC that is complementary to a target in a pre-mRNA sequence, wherein hybridization of the AC with the target sequence suppresses aberrant splicing of the target pre-mRNA.

In some embodiments, provided herein is a compound comprising a cCPP and an AC that is complementary to a target in a pre-mRNA sequence, wherein hybridization of the AC with the target sequence induces expression of one or more protein isomers encoded by the target gene.

In some embodiments, provided herein is a compound comprising a cCPP and an AC that is complementary to a target in a pre-mRNA sequence, wherein hybridization of the AC with the target sequence suppresses expression of one or more protein isomers encoded by the target gene.

In some embodiments, provided herein is a compound comprising a cCPP and an AC that is complementary to a target in a pre-mRNA sequence, wherein the target protein produced by splicing and translation of the target pre-mRNA is not functional or is less functional than a wild type target protein in the absence of AC hybridization to the target sequence.

In some embodiments, provided herein is a compound comprising a cCPP and an AC that is complementary to a target in a pre-mRNA sequence, wherein hybridization of the AC with the target sequence suppresses expression of the target protein that would have been expressed from the target pre-mRNA in the absence of AC hybridization.

In some embodiments, provided herein is a compound comprising a cCPP and an AC that is complementary to a target in a pre-mRNA sequence, wherein hybridization of the AC with the target sequence results in expression of a re-spliced target protein having one or more improved functions or characteristics compared to the expressed target protein in the absence of AC hybridization.

In some embodiments, provided herein is a compound comprising a cCPP and an AC that is complementary to a target in a pre-mRNA sequence, wherein the one or more improved characteristics comprise function and/or activity.

In some embodiments, provided herein is a compound comprising a cCPP and an AC that is complementary to a target in a pre-mRNA sequence, wherein the re-spliced target protein comprises an active fragment of a wild type target protein.

In some embodiments, provided herein is a compound comprising a cCPP and an AC that is complementary to a target in a pre-mRNA sequence, wherein hybridization of the AC with the target sequence results in expression of a re-spliced target protein that ameliorates or rescues aspects of a disease phenotype associated with the target gene.

In some embodiments, provided herein is a compound comprising a cCPP and an AC that is complementary to a target in a pre-mRNA sequence, further comprising a nuclear localization signal (NLS).

In some embodiments, provided herein is a compound comprising a cCPP and an AC that is complementary to a target in a pre-mRNA sequence further comprising an NLS, wherein the C-terminus of the NLS sequence is conjugated to the CPP.

In some embodiments, provided herein is a pharmaceutical composition comprising a compound comprising a cCPP and an AC that is complementary to a target in a pre-mRNA sequence.

In some embodiments, provided herein is a compound comprising a cCPP and an AC that is complementary to a target in a pre-mRNA sequence.

In some embodiments, provided herein is a method of modulating the splicing of a target pre-mRNA in a subject in need thereof, comprising administering a compound comprising a cCPP and an AC that is complementary to a target in a pre-mRNA sequence.

In some embodiments, provided herein is a method of modulating the splicing of a target pre-mRNA in a subject in need thereof, comprising administering a compound comprising a cCPP and an AC that is complementary to a target in a pre-mRNA sequence, wherein the compound suppresses expression of the target protein translated from the target pre-mRNA in the absence of the compound.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in the expression of a re-spliced target protein.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in the expression of a re-spliced target protein, wherein the re-spliced target protein has one or more improved characteristics compared to the target protein.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in the expression of a re-spliced target protein, wherein the re-spliced target protein has one or more improved characteristics compared to the target protein, wherein the one or more improved characteristics are selected from the list consisting of: function, activity, binding, and enzymatic activity.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in the increased expression of one or more protein isomers encoded by the target gene.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in the decreased expression of one or more protein isomers encoded by the target gene.

In some embodiments, provided herein is a method of modulating the splicing of a target pre-mRNA in a subject in need thereof, comprising administering a compound comprising a cCPP and an AC that is complementary to a target in a pre-mRNA sequence, wherein administration of the compound results in increased expression of a wild type target protein or an active fragment of a wild type target protein.

In some embodiments, provided herein is a method of modulating the splicing of a target pre-mRNA in a subject in need thereof, comprising administering a compound comprising a cCPP and an AC that is complementary to a target in a pre-mRNA sequence, wherein the compound modulates splicing of exon 2, 8, 11, 17, 19, 23, 29, 40, 41, 42, 43, 44, 45, 46, 48, 49, 50, 51, 52, 53, 55, and 59 of DMD.

In some embodiments, provided herein is a method of modulating the splicing of a target pre-mRNA in a subject in need thereof, comprising administering a compound comprising a cCPP and an AC that is complementary to a target in a pre-mRNA sequence, wherein the compound modulates splicing of exon 2, 8, 11, 23 43, 44, 45, 50, 51, 53, and 55 of DMD.

In some embodiments, provided herein is a method of modulating the splicing of a target pre-mRNA in a subject in need thereof, comprising administering a compound comprising a cCPP and an AC that is complementary to a target in a pre-mRNA sequence, wherein the compound modulates splicing of exon 2, 23, 44, or 51 of DMD.

In some embodiments, provided herein is a method of modulating the splicing of a target pre-mRNA in a subject in need thereof, comprising administering a compound comprising a cCPP and an AC that is complementary to a target in a pre-mRNA sequence, wherein the AC is selected from Table B1-B3.

In some embodiments, provided herein is a method of modulating the splicing of a target pre-mRNA in a subject in need thereof comprising administering a compound comprising a cCPP and an AC that is complementary to a target in a pre-mRNA sequence, wherein the compound modulates splicing of exon 1, exon 2, exon 3, exon 4, exon 5, exon 6, exon 7a, and exon 7b of CD33.

In some embodiments, provided herein is a method of modulating the splicing of a target pre-mRNA in a subject in need thereof comprising administering a compound comprising a cCPP and an AC that is complementary to a target in a pre-mRNA sequence, wherein the AC is selected from Table C.

In some embodiments, provided herein is a method of treating a genetic disease in a subject in need thereof, comprising administering a compound comprising a cCPP and an AC that is complementary to a target in a pre-mRNA sequence.

In some embodiments, provided herein is a method of treating a genetic disease in a subject in need thereof, comprising administering a compound comprising a cCPP and an AC that is complementary to a target in a pre-mRNA sequence, wherein administration of the compound modulates splicing or expression of a target gene, degrades mRNA, stabilizes mRNA, or sterically blocks mRNA.

In some embodiments, provided herein is a method of treating a genetic disease in a subject in need thereof, comprising administering a compound comprising a cCPP and an AC that is complementary to a target in a pre-mRNA sequence, wherein administration of the compound modulates splicing of the target pre-mRNA.

In some embodiments, provided herein is a method of treating a genetic disease in a subject in need thereof, comprising administering a compound comprising a cCPP and an AC that is complementary to a target in a pre-mRNA sequence, wherein administration of the compound results in an increase in the expression of a wild type target protein or an active fragment thereof.

In some embodiments, provided herein is a method of treating a genetic disease in a subject in need thereof, comprising administering a compound comprising a cCPP and an AC that is complementary to a target in a pre-mRNA sequence, wherein administration of the compound results in expression of a re-spliced target protein that is more highly expressed, functional, and/or active than the target protein expressed in the absence of the compound.

In some embodiments, provided herein is a method of treating a genetic disease in a subject in need thereof, comprising administering a compound comprising a cCPP and an AC that is complementary to a target in a pre-mRNA sequence, wherein administration of the compound results in expression of a re-spliced target protein that is more highly expressed, functional, and/or active than the target protein expressed in the absence of the compound, wherein the re-spliced target protein comprises an active fragment of a wild type target protein.

In some embodiments, provided herein is a method of treating a genetic disease in a subject in need thereof, comprising administering a compound comprising a cCPP and an AC that is complementary to a target in a pre-mRNA sequence, wherein the genetic disease is a central nervous system disorder, a neuromuscular disorder, or a musculoskeletal disorder.

In some embodiments, provided herein is a method of treating a genetic disease in a subject in need thereof, comprising administering a compound comprising a cCPP and an AC that is complementary to a target in a pre-mRNA sequence, wherein the genetic disease is Duchenne muscular dystrophy, β thalassemia, dystrophin Kobe, osteogenesis imperfect, cystic fibrosis, Merosin-deficient congenital muscular dystrophy type 1A, or spinal muscular atrophy.

In some embodiments, provided herein is a method of treating a genetic disease in a subject in need thereof, comprising administering a compound comprising a cCPP and an AC that is complementary to a target in a pre-mRNA sequence, wherein the disease is Duchenne muscular dystrophy.

In some embodiments, provided herein is a method of treating a genetic disease in a subject in need thereof, comprising administering a compound comprising a cCPP and an AC that is complementary to a target in a pre-mRNA sequence, wherein the AC is selected from Table B1-B3.

In some embodiments, provided herein is a method of treating a genetic disease in a subject in need thereof, comprising administering a compound comprising a cCPP and an AC that is complementary to a target in a pre-mRNA sequence, wherein the AC has a nucleic acid sequence of 5'- GCTATTACCTTAACCCA-3' (SEQ ID NO: 152).

In some embodiments, provided herein is a method of treating a genetic disease in a subject in need thereof, comprising administering a compound comprising a cCPP and an AC that is complementary to a target in a pre-mRNA sequence, wherein the AC is a phosphorodiamidate morpholino oligomer (PMO).

In some embodiments, provided herein is a method of treating a genetic disease in a subject in need thereof, comprising administering a compound comprising a cCPP and an AC that is complementary to a target in a pre-mRNA sequence, wherein the AC has a nucleic acid sequence of 5'- GTAACTGTATTTGGTACTTCC-3' (SEQ ID NO: 153).

In some embodiments, provided herein is a method of treating a genetic disease in a subject in need thereof, comprising administering a compound comprising a cCPP and an AC that is complementary to a target in a pre-mRNA sequence, wherein the AC is a phosphorodiamidate morpholino oligomer (PMO).

In some embodiments, provided herein is a method of treating a genetic disease in a subject in need thereof, comprising administering a compound comprising a cCPP and an AC that is complementary to a target in a pre-mRNA sequence, resulting in a splicing increase in the expression of a wild type target protein or an active fragment thereof in muscle tissue, diaphragm tissue, quadriceps, and/or heart tissue.

In some embodiments, provided herein is a method of treating a genetic disease in a subject in need thereof, comprising administering a compound comprising a cCPP and an AC that is complementary to a target in a pre-mRNA sequence, resulting in a splicing increase in the expression of a wild type target protein or an active fragment thereof in heat tissue.

In some embodiments, provided herein is a method of treating a genetic disease in a subject in need thereof, comprising administering a compound comprising a cCPP and an AC that is complementary to a target in a pre-mRNA sequence, wherein administration of the compound blocks transcription of at least a portion of the trinucleotide repeats.

In some embodiments, provided herein is a method of treating a genetic disease in a subject in need thereof, comprising administering a compound comprising a cCPP and an AC that is complementary to a target in a pre-mRNA sequence, wherein administration of the compound reduces the length and/or number of expanded nucleotide repeats an expressed protein.

In some embodiments, provided herein is a method of treating a genetic disease in a subject in need thereof, comprising administering a compound comprising a cCPP and an AC that is complementary to a target in a pre-mRNA sequence, wherein the genetic disease is Fragile X, Friedreich's ataxia (FRDA), Huntington's Disease (HD), Myotonic dystrophy type 1 (DM1), Myotonic dystrophy type 2 (DM2), Spinal and bulbar muscular atrophy (SBMA), Spinal cerebellar ataxia type 1 (SCA1), Spinal cerebellar ataxia type 2 (SCA2), or Spinal cerebellar ataxia type 3 (SCA3).

In some embodiments, provided herein is a method of treating a genetic disease in a subject in need thereof, comprising administering a compound comprising a cCPP and an AC that is complementary to a target in a pre-mRNA sequence, wherein the genetic disease is Fragile X, Friedreich's ataxia (FRDA), Huntington's Disease (HD), Myotonic dystrophy type 1 (DM1).

In some embodiments, provided herein is a method of treating a genetic disease in a subject in need thereof, comprising administering a compound comprising a cCPP and an AC that is complementary to a target in a pre-mRNA sequence, wherein the genetic disease is FRDA, and the AC is selected from Table 8.

In some embodiments, provided herein is a method of treating a genetic disease in a subject in need thereof, comprising administering a compound comprising a cCPP and an AC that is complementary to a target in a pre-mRNA sequence, wherein the disease is DM1, and the AC is selected from Table 7.

In some embodiments, provided herein is a method of treating a genetic disease in a subject in need thereof, comprising administering a compound comprising a cCPP and an AC that is complementary to a target in a pre-mRNA sequence, wherein the AC comprises a gapmer, which hybridizes with the target RNA and catalyzes degradation of the target.

In some embodiments, provided herein is a method of treating a genetic disease in a subject in need thereof, comprising administering a compound comprising a cCPP and an AC that is complementary to a target in a pre-mRNA sequence, wherein the AC comprises a gapmer, which hybridizes with the target RNA and catalyzes degradation of the target, wherein the AC is selected from Table 8.

In some embodiments, provided herein is a method of treating Fragile X, Friedreich's ataxia (FRDA), Huntington's Disease (HD), Myotonic dystrophy type 1 (DM1), Myotonic dystrophy type 2 (DM2), Spinal and bulbar muscular atrophy (SBMA), Spinal cerebellar ataxia type 1 (SCA1), Spinal cerebellar ataxia type 2 (SCA2), or Spinal cerebellar ataxia type 3 (SCA3), comprising administering a compound comprising a cCPP and an AC that is complementary to a target in a pre-mRNA sequence, wherein the AC comprises a sequence that is complementary to a trinucleotide repeat in a target mRNA sequence, and the AC hybridizes with the target mRNA sequence to blocks transcription of the trinucleotide repeat.

In some embodiments, provided herein is a method of treating a genetic disease in a subject in need thereof, comprising administering a compound comprising a cCPP and an AC that is complementary to a target in a pre-mRNA sequence, wherein the genetic disease is FRDA, and the AC is selected from Table 8.

In some embodiments, provided herein is a method of treating a genetic disease in a subject in need thereof, comprising administering a compound comprising a cCPP and an AC that is complementary to a target in a pre-mRNA sequence, wherein the disease is DM1, and the AC is selected from Table 7.

In some embodiments, provided herein is a method of blocking or degrading nucleotide repeat expansions in RNA comprising administering a compound comprising a cCPP and an AC that is complementary to a target in a pre-mRNA sequence, wherein the AC comprises an antisense oligonucleotide sequence that is complementary to at least a portion of the nucleotide repeats in the target RNA.

In some embodiments, provided herein is a method of blocking or degrading nucleotide repeat expansions in RNA comprising administering a compound comprising a cCPP and an AC that is complementary to a target in a pre-mRNA sequence, wherein the administration prevents or reduces translation of at least a portion of the nucleotide repeats.

In some embodiments, provided herein is a method of blocking or degrading nucleotide repeat expansions in RNA comprising administering a compound comprising a cCPP and an AC that is complementary to a target in a pre-mRNA sequence, wherein the nucleotide repeats expansions are trinucleotide repeat expansions, pentanucleotide repeat expansions, or hexanucleotide repeat expansions.

In some embodiments, provided herein is a method of blocking or degrading nucleotide repeat expansions in RNA comprising administering a compound comprising a cCPP and an AC that is complementary to a target in a pre-mRNA sequence, wherein the nucleotide repeat expansions comprises expansions of CAG repeats, CGG repeats, GCC repeats, GAA repeats, or CUG repeats.

In some embodiments, provided herein is a method of blocking or degrading nucleotide repeat expansions in RNA comprising administering a compound comprising a cCPP and an AC that is complementary to a target in a pre-mRNA sequence, wherein the nucleotide repeat expansions are located in the *FMR1* gene, *HTT* gene, *ATP7B* gene, *DMPK* gene, or *FXN* gene.

In some embodiments, provided herein is a method of blocking or degrading nucleotide repeat expansions in RNA comprising administering a compound comprising a cCPP and an AC that is complementary to a target in a pre-mRNA sequence, wherein the nucleotide repeat expansions are located in the *DMPK* gene or *FXN* gene.

In some embodiments, provided herein is a method of blocking or degrading nucleotide repeat expansions in RNA comprising administering a compound comprising a cCPP and an AC that is complementary to a target in a pre-mRNA sequence, wherein the gene is *FXN,* and the AC is selected from Table 8.

In some embodiments, provided herein is a method of blocking or degrading nucleotide repeat expansions in RNA comprising administering a compound comprising a cCPP and an AC that is complementary to a target in a pre-mRNA sequence, wherein the gene is *DMPK,* and the AC is selected from Table 9.

In some embodiments, provided herein is a compound comprising a cCPP is selected from Table 4 or a method comprising administering a cCPP selected from Table 4.

In some embodiments, provided herein is a compound and/or method comprising cCPP12.

### BRIEF DESCRIPTION OF THE DRAWINGS

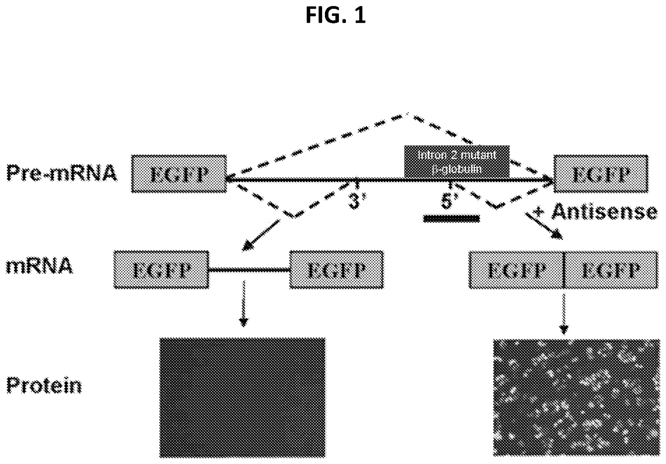

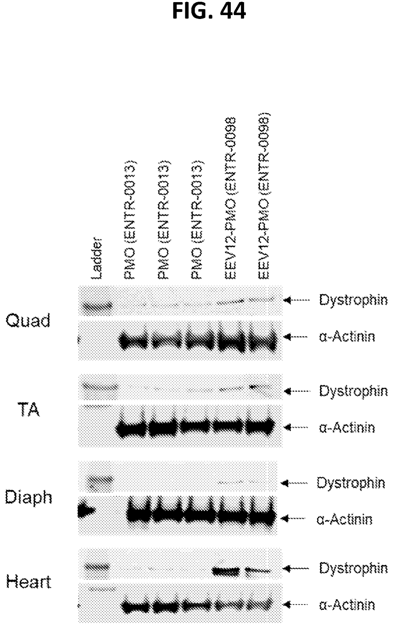

**FIG. 1** shows a schematic of the experimental design for Example 1. The pre-mRNA line shows the coding sequence of EGFP (boxes) interrupted by intron 2 of the human β globin gene (thin line between boxes). The mutation at position 654 of the intron is also illustrated. The dotted lines above the pre-mRNA schematic show proper splicing of the intron. The dotted lines below the pre-mRNA schematic show improper splicing of the intron as a result of the mutation at position 654. The black bar shows the binding location of the designed antisense compound (AC). The mature, spliced mRNA line shows the mRNA sequence resulting from improper splicing (left) and from proper splicing (right). The protein line shows the lack of fluorescence from the nonfunctional protein resulting from translation of the improperly spliced mRNA sequence (left) and the restored fluorescence of the functional EGFP protein resulting from translation of the properly spliced mRNA sequence (right).
**FIG. 2** shows the conjugation of an exemplary CPP and an exemplary AC, as described in Example 1.
**FIG. 3** shows the bright field microscopy (top) and fluorescence microscopy (bottom) imaging of untreated (mock-treated) control HeLa-654 cells (left) and 5 µM PMO-treated HeLa-654 cells (right), as described in Example 1.
**FIG. 4** shows the bright field microscopy (top) and fluorescence microscopy (bottom) imaging of HeLa-654 cells treated with 0.6 µM (left), 1.2 µM (middle), or 1.8 µM (right) CPP-PMO, as described in Example 1.
**FIG. 5** shows the results of FACS analysis of untreated, PMO-treated (ENTR-0070, PMO654, Table A)-treated, and CPP-PMO-treated (ENTR-0121, CPP12-PMO654, Table A)HeLa-654 cells, as described in Example 1.
**FIG. 6** shows the results of RT-PCR analysis of splicing correction of EGFP654 in mice tissue samples after PMO (ENTR-0070, PMO654, Table A) treatment or CPP-PMO (ENTR-0070, PMO654, Table 1) treatment, as described in Example 2.

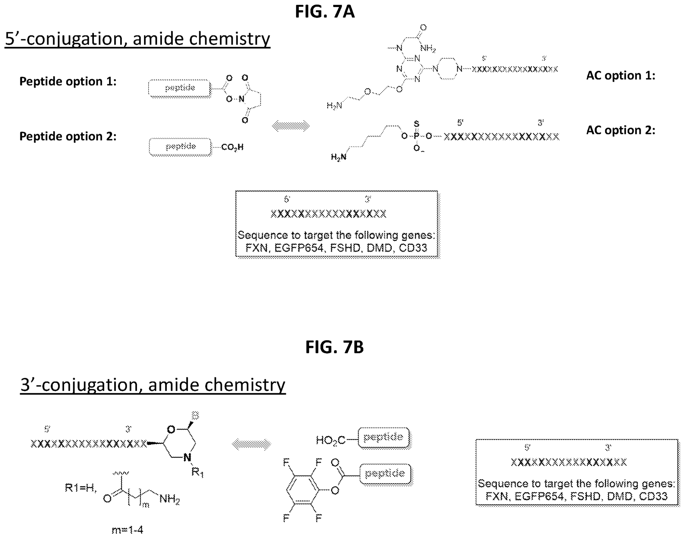

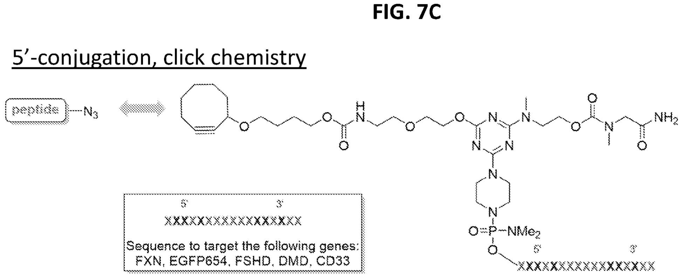

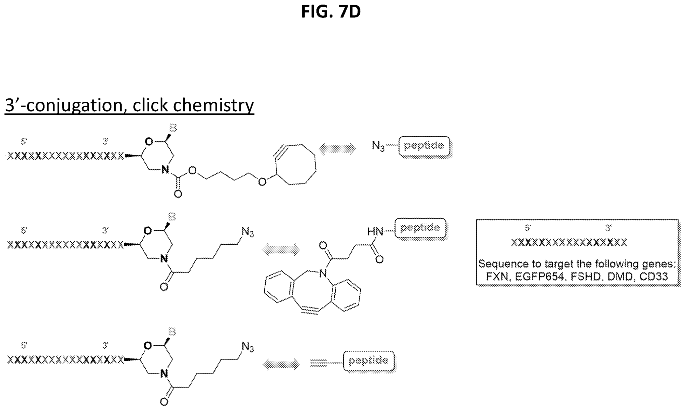

**FIGS. 7A-D** illustrate conjugation chemistries for connecting AC with peptides. **FIG. 7A** shows the amide bond formation between peptides with carboxylic acid group or with TFP activated ester and primary amine residues at the 5' end of AC. **FIG. 7B** shows the conjugation of secondary amine or primary amine modified AC at 3' and peptide-TFP ester through amide bond formation. **FIG. 7C** shows the conjugation of peptide-azide to the 5' cyclooctyne modified AC via cupper-free azide-alkyne cycloaddition. **FIG. 7D** demonstrates another exemplary conjugation between 3' modified cyclooctyne ACs or 3' modified azide ACs and CPP containing linker-azide or linker-alkyne/cyclooctyne moiety, via a cupper-free azide-alkyne cycloaddition or cupper catalyzed azide-alkyne cycloaddition, respectively (click reaction).
**FIG. 8** shows the conjugation chemistry for connecting AC and CPP with additional linker modality containing a polyethylene glycol (PEG) moiety.
**FIG. 9** shows an illustrative scheme for conjugating a CPPto an AC (SEQ ID NO: 214).

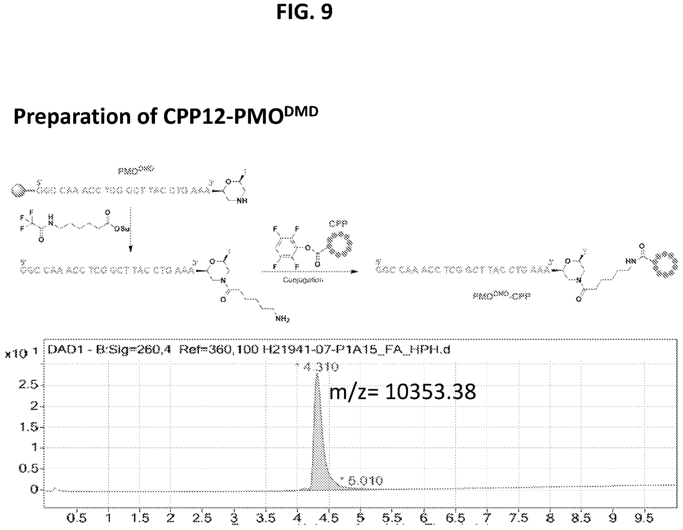

**FIG. 9** shows a schematic of preparation of CPP12-PMO^{DMD} (also called "PMO-DMD") and CPP12-PMO^{DMD} (also called "EEV12-PMO^{DMD}).

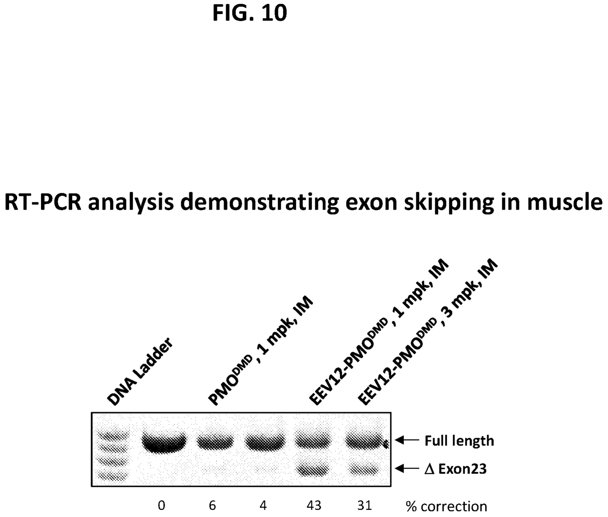

**FIG. 10** shows the RT-PCR result of dystrophin exon skipping products in local muscle group post intramuscular administration (IM) of PMO^{DMD} (ENTR-0013, Table B1) and CPP12-PMO^{DMD} (ENTR-0014, Table B2) in wild type mice.

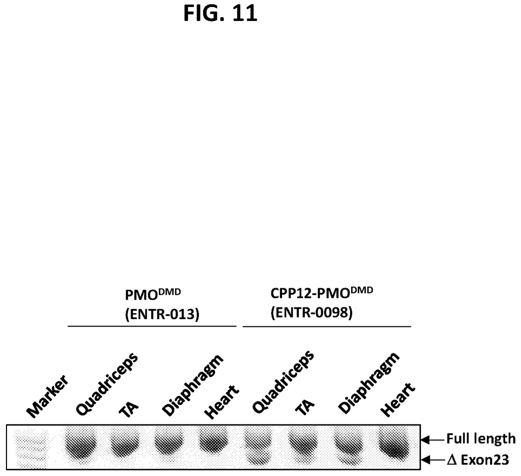

**FIG. 11** shows dystrophin exon skipping products in various treated muscle groups post intravenous administration of PMO^{DMD} (ENTR-0013, Table B1) and CPP12-PMO^{DMD} (ENTR-0014, Table B2) in wild type mice.

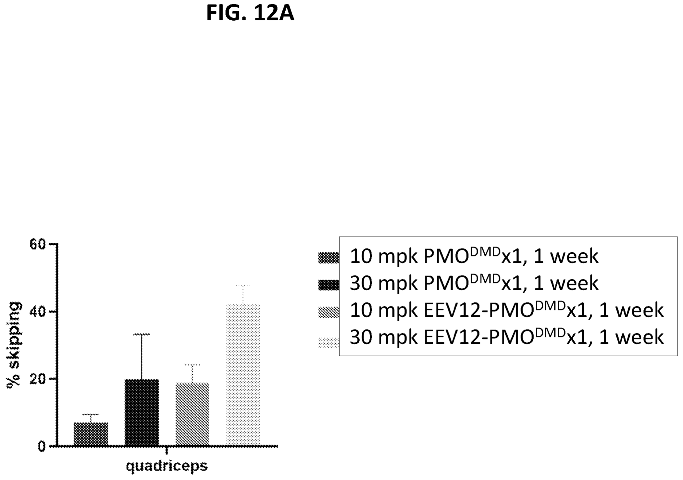

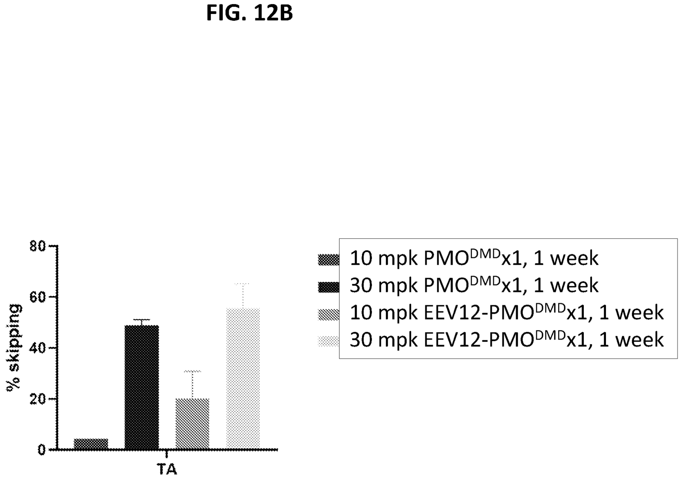

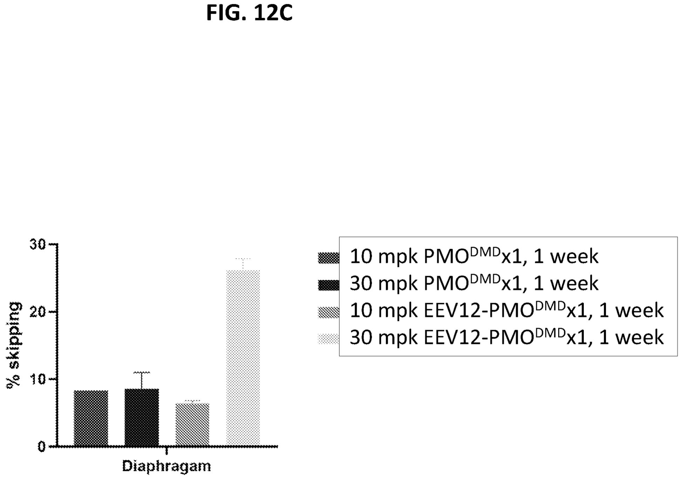

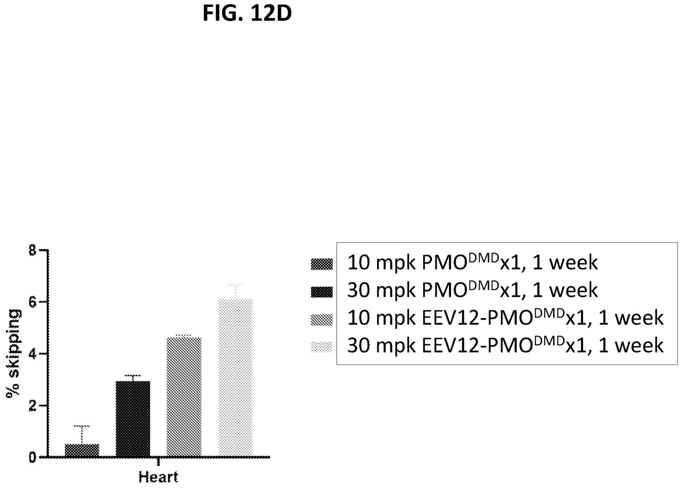

**FIGS. 12A-D** shows the percentage of exon skipping in MDX mice after delivery of PMO^{DMD} or EEV12-PMO^{DMD} in the quadriceps **(****FIG. 12A****),** transverse abdominus (TA) **(****FIG. 12B****),** diaphragm **(****FIG. 12C****),** and heart **(****FIG. 12D****).**

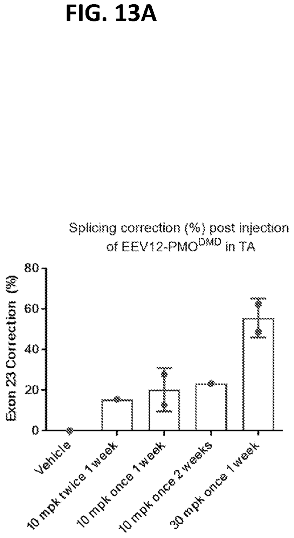

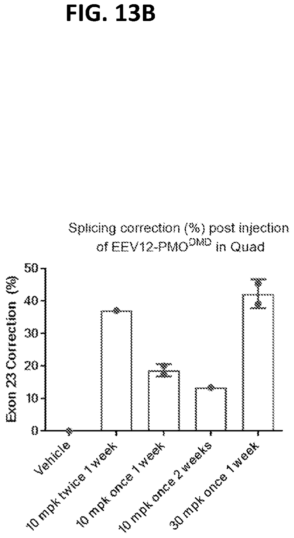

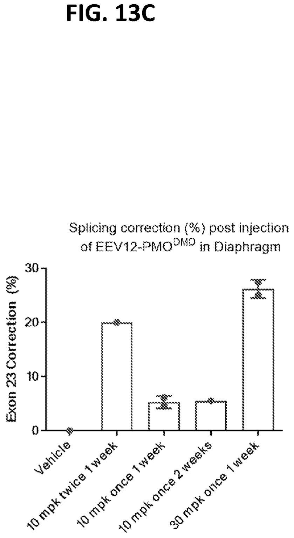

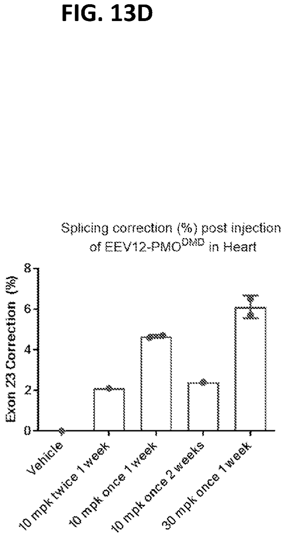

**FIGS. 13A-D** shows the percentage of exon 23 correction in MDX mice after delivery of EEV12-PMO^{DMD} in the transverse abdominus (TA) **(****FIG. 13A****),** quadriceps **(****FIG. 13B****),** diaphragm **(****FIG. 13C****),** and heart **(****FIG. 13D****).**

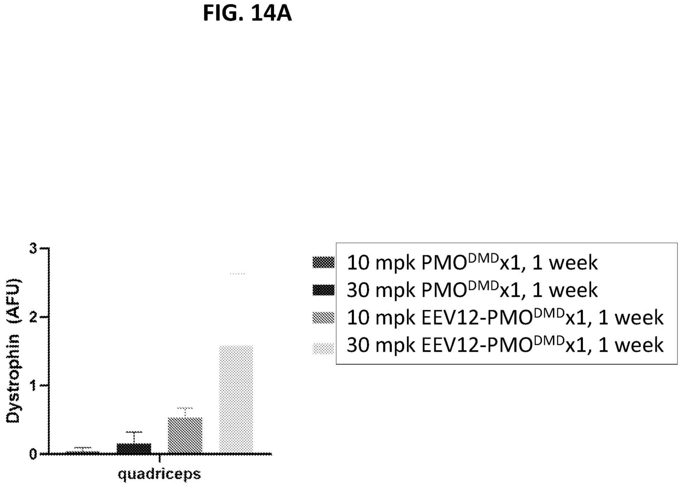

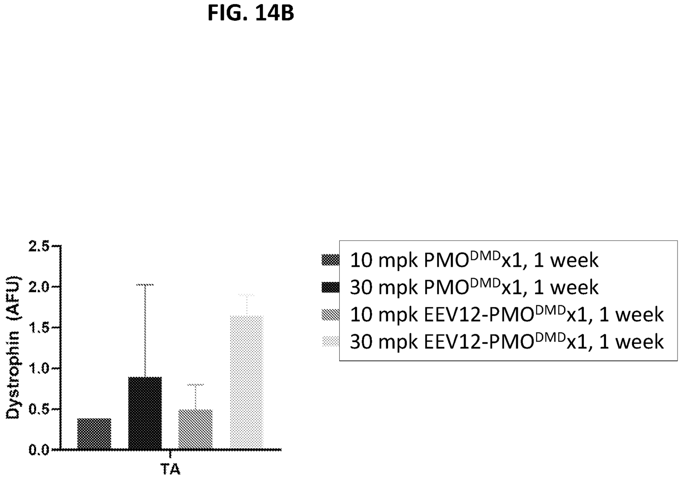

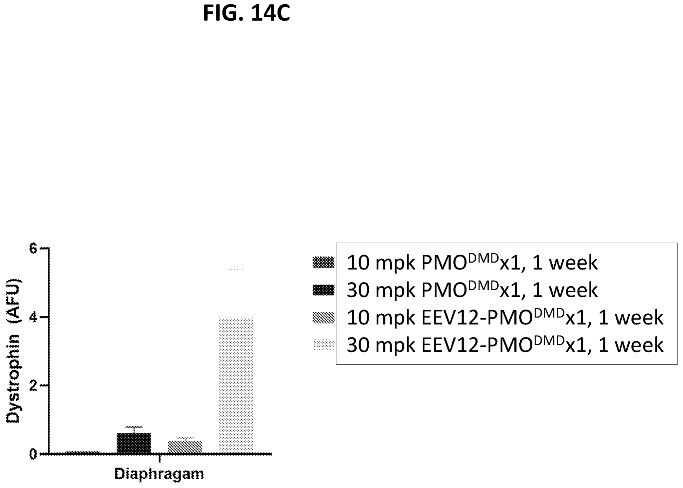

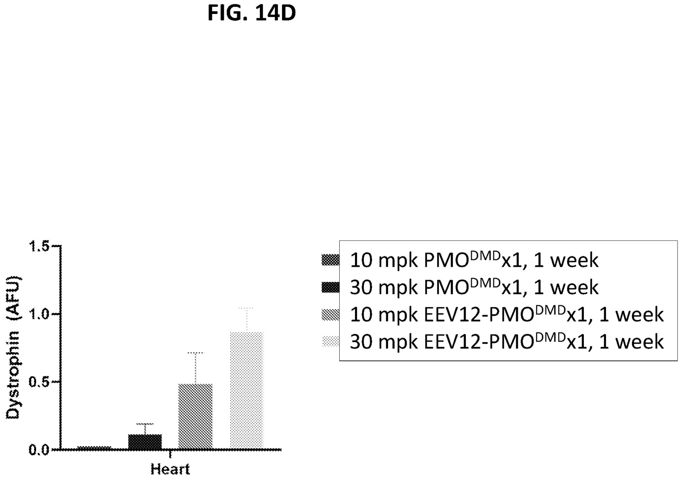

**FIGS. 14A-D** shows the amount of exon-23 corrected dystrophin detected after delivery of PMO^{DMD} or EEV12-PMO^{DMD} in the quadriceps **(****FIG. 14A****),** transverse abdominus (TA) **(****FIG. 14B****),** diaphragm **(****FIG. 14C****),** and heart **(****FIG. 14D****)** by Western Blot.

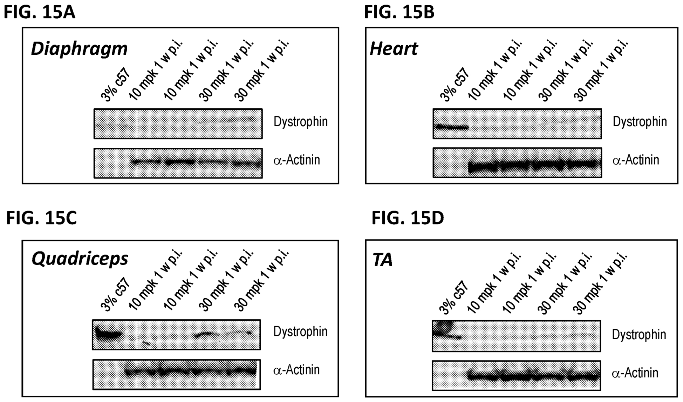

**FIGS. 15A-D** show Western Blots of exon-23 corrected dystrophin and α-actinin in the diaphragm **(****FIG. 15A****),** heart **(****FIG. 15B****),** quadriceps **(****FIG. 15C****),** and transverse abdominus **(****FIG. 15D****)** after intravenous delivery of 10 mpk or 30 mpk EEV12-PMO^{DMD}.

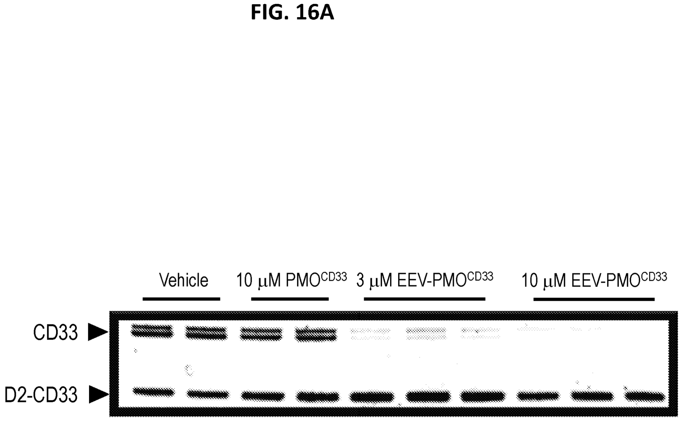

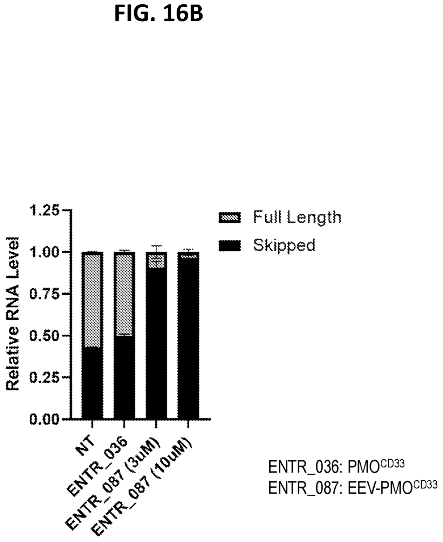

**FIGS. 16A-B** show a real-time PCR analysis of exon 2 skipping of CD33 **(****FIG. 16A****)** and quantification of full length CD33 versus D2-CD33 transcripts **(****FIG. 16B****)** after treatment with EEV-PMO^{CD33} (also called "ENTR_087") or PMO^{CD33} (also called "ENTR_036"). PMO^{CD33} is an antisense compound that targets exon 2 of CD33.

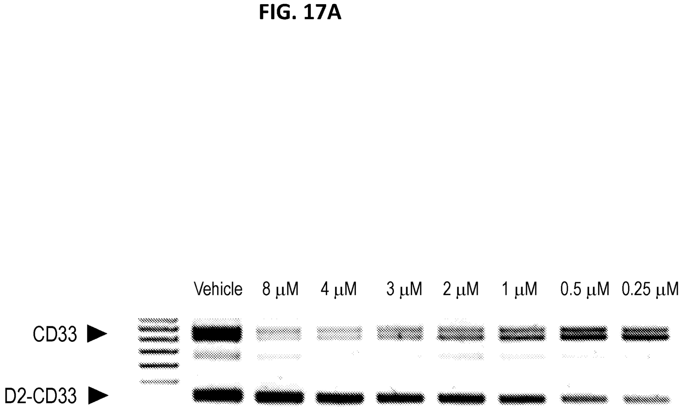

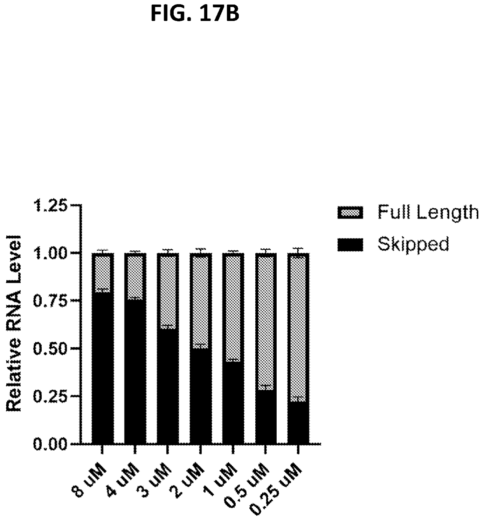

**FIGS. 17A-B** show the dose dependence of real-time PCR analysis of exon 2 skipping of CD33 **(****FIG. 17A****)** and quantification of full length CD33 versus D2-CD33 transcripts by dose **(****FIG. 17B****)** after treatment with EEV-PMO^{CD33}. The PMO targeted exon 2 of CD33.

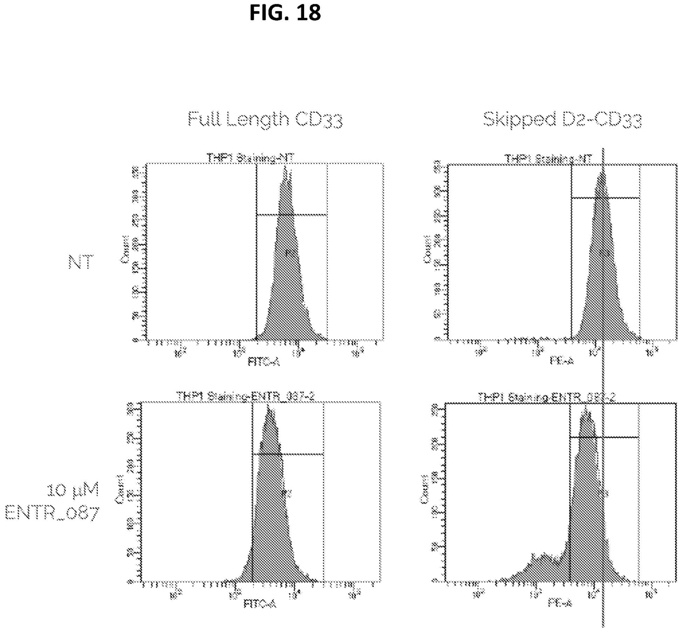

**FIG. 18** shows flow cytometry analysis of THP1 cells after treatment with EEV-PMO^{CD33} compared to untreated ("NT") cells.

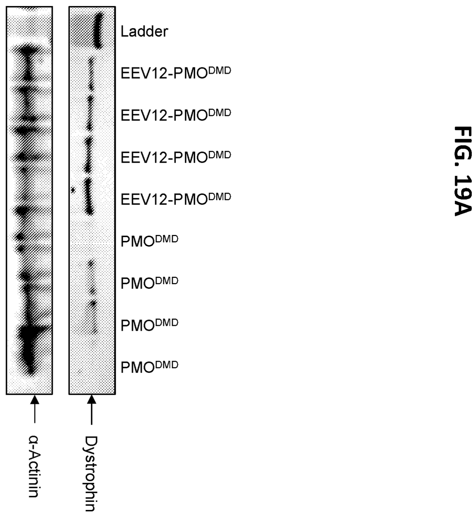

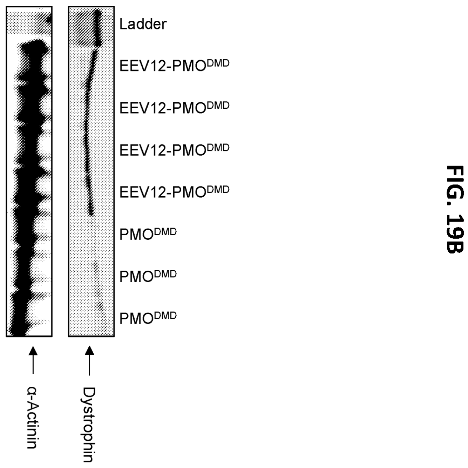

**FIGS. 19A-B** show the dystrophin levels in MDX mice two weeks **(****FIG. 19A****)** and four weeks **(****FIG. 19B****)** after treatment with 30 mpk EEV12-PMO^{DMD} or 30 mpk PMO^{DMD}.

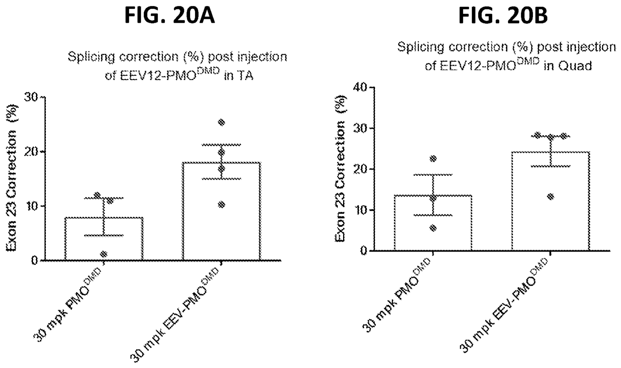

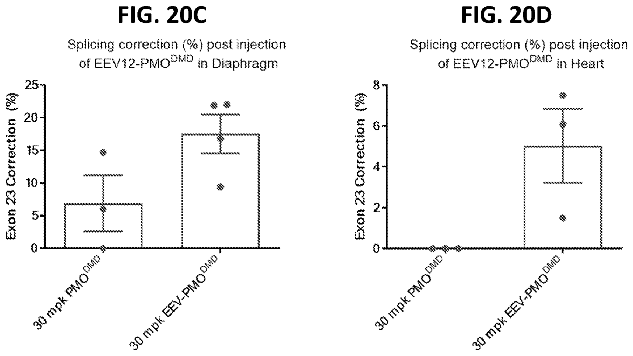

**FIGS. 20A-D** show the percentage of exon 23 corrected dystrophin products in transverse abdominus **(****FIG. 20A****),** quadriceps **(****FIG. 20B****),** diaphragm **(****FIG. 20C****),** and the heart **(****FIG. 20D****)** in MDX mice that were administered either 30 mpk of PMO^{DMD} or 30 mpk of EEV12-PMO^{DMD}. Mice administered EEV12-PMO^{DMD} exhibited enhanced splicing correction, compared to mice administered PMO^{DMD} alone.

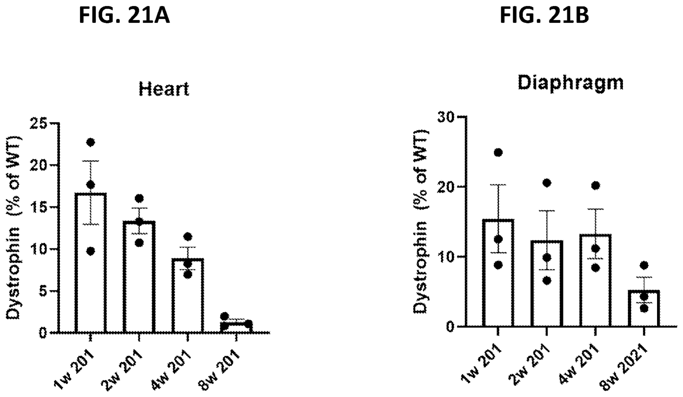

**FIGS. 21A-B** shows the effect of a composition called Oligo 201 comprising an AC, a nuclear localization sequence, and a CPP on exon 44 skipping in an MDX mouse model. The presence of an exon-skipped DMD product after treatment with Oligo 201 once a week is evaluated after one week, two weeks, four weeks, and eight weeks of treatment in the heart **(****FIG. 21A****)** and the diaphragm **(****FIG. 21B****).**

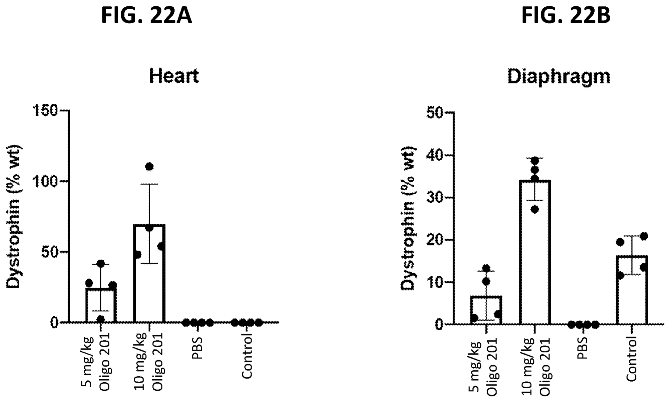

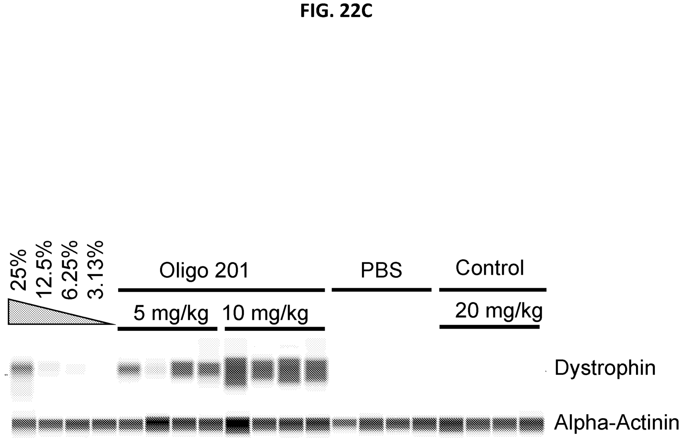

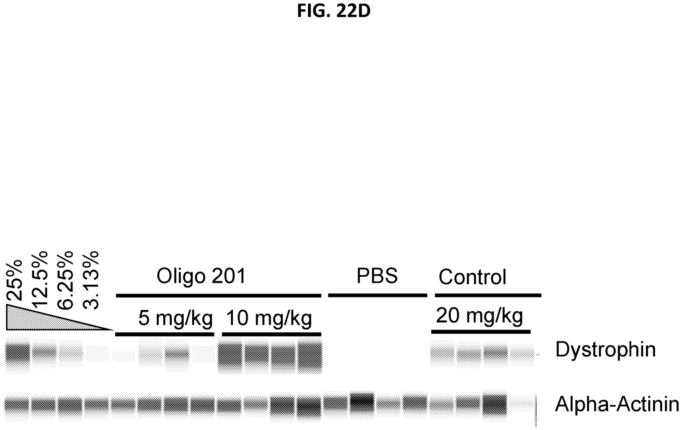

**FIGS. 22A-D** shows the effect of a composition called Oligo 201 comprising an AC, a nuclear localization sequence, and a CPP on exon 44 skipping in an MDX mouse model. The presence of an exon-skipped DMD product after treatment with 5 mg/kg Oligo 201 or 10 mg/kg Oligo 201 four times per week is evaluated in the heart **(****FIG. 22A****)** and diaphragm **(****FIG. 22B****).** **FIG. 22C** and **FIG. 22D** illustrate the percentage of splicing correction of DMD in the heart and diaphragm on electrophoresis gels. Alpha-actinin is included as a positive control.

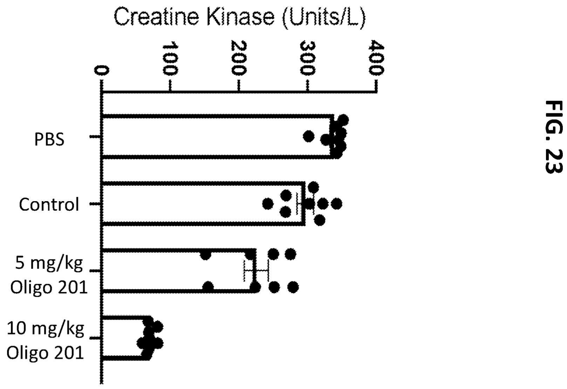

**FIG. 23** shows the serum levels of creatine kinase in MDX mice treated with 5 mg/kg Oligo 201 or 10 mg/kg Oligo 201 four times per week.

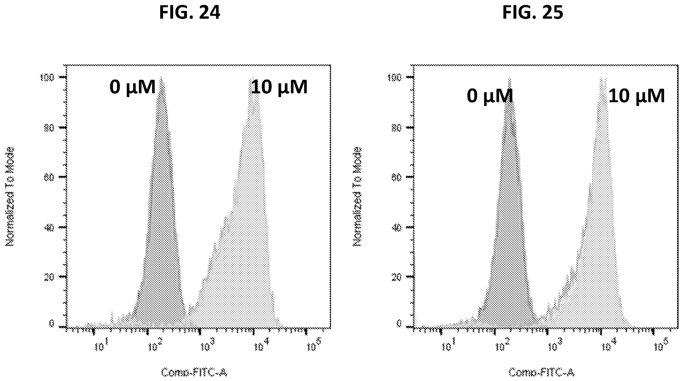

**FIG. 24** show splicing correction of eGFP in HeLA-654 cells, as demonstrated by the fluorescence of HeLA-654 cells treated with 0 µM or 10 µM of ENTR-0203.
**FIG. 25** shows splicing correction of eGFP in HeLA-654 cells, as demonstrated by the fluorescence of HeLA-654 cells treated with 0 µM or 10 µM of ENTR-0207.

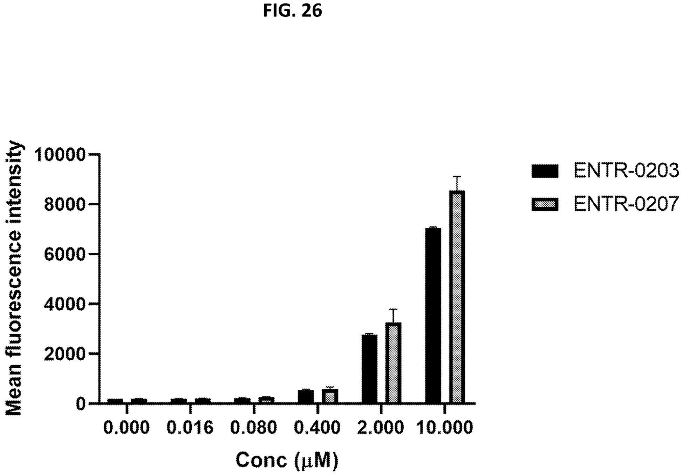

FIG. 26 shows the results of fluorescent activated cell sorting analysis of HeLa-654 cells treated for 24 hr with 0, 0.016 µM, 0.08 µM, 0.4 µM, 2 µM, 10 µM CPP-NLS-PMO constructs, including ENTR-0203 (non-cleavable linker, Table 1) and ENTR-0207 (cleavable linker, Table A), as described in Example 1.

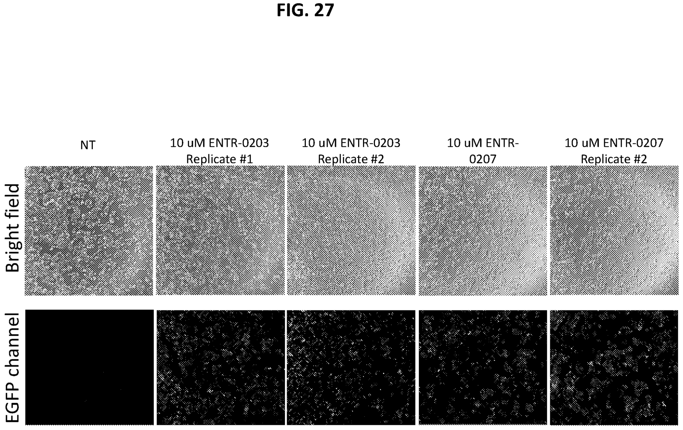

**FIG. 27** shows fluorescence microscopy images of HeLA-654 cells treated with either 10 µM of ENTR-0203 or 10 µM of ENTR-0207.

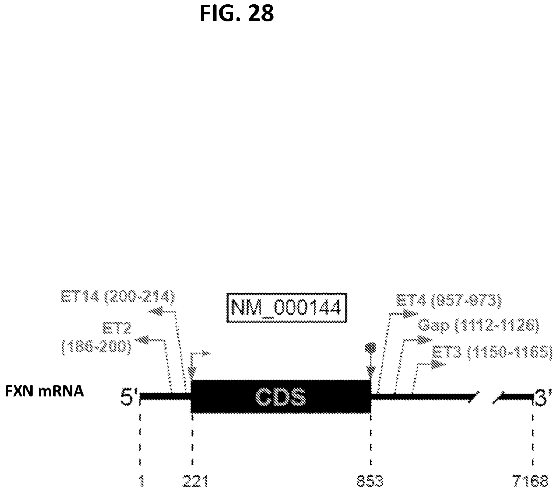

**FIG. 28** shows a schematic of FXN mRNA.

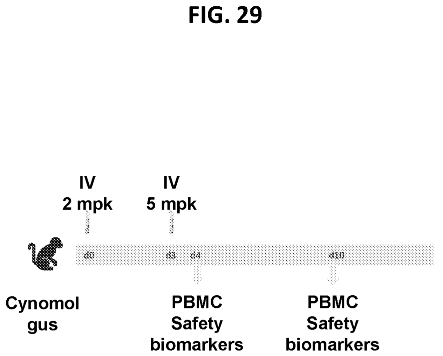

**FIG. 29** shows the study design of to evaluate pharmacodynamic effects of CPP-conjugated AC and unconjugated AC against CD33 in cyno monkeys.

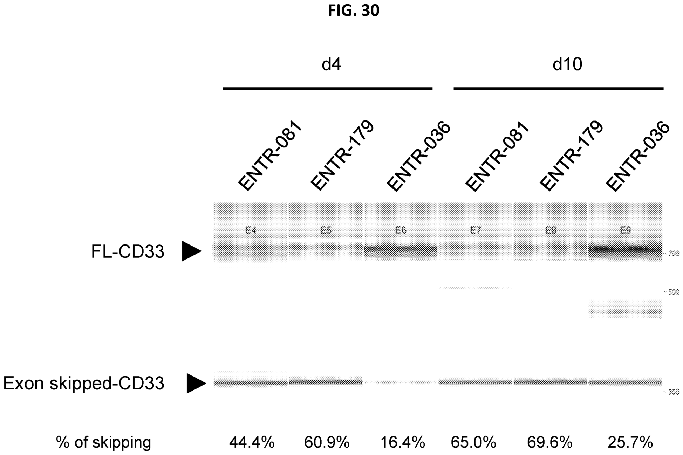

**FIG. 30** shows RT-PCR analysis of exon skipping of CD33 gene in peripheral mononuclear blood cells (PBMCs) of cyno monkey after intravenous infusion of CPP-PMO^{CD33} (also called "ENTR-081", TABLE 5), CPP-NLS-PMO^{CD33} (also called "ENTR-179, TABLE C), or PMO^{CD33} (also called "ENTR-036", TABLE 5).

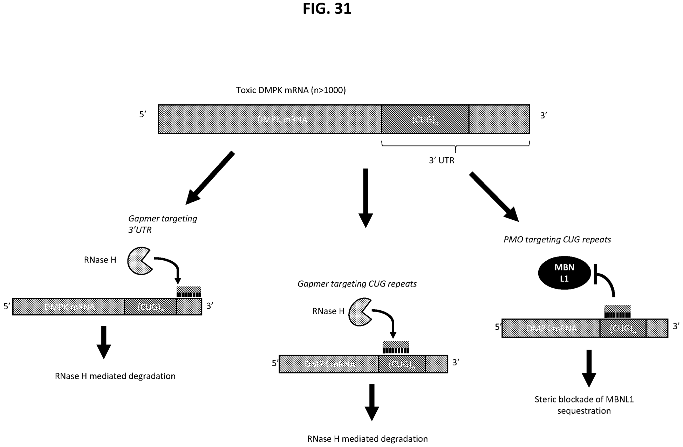

**FIG. 31** shows various mechanisms by which antisense compounds modulate expression of *DMPK.*

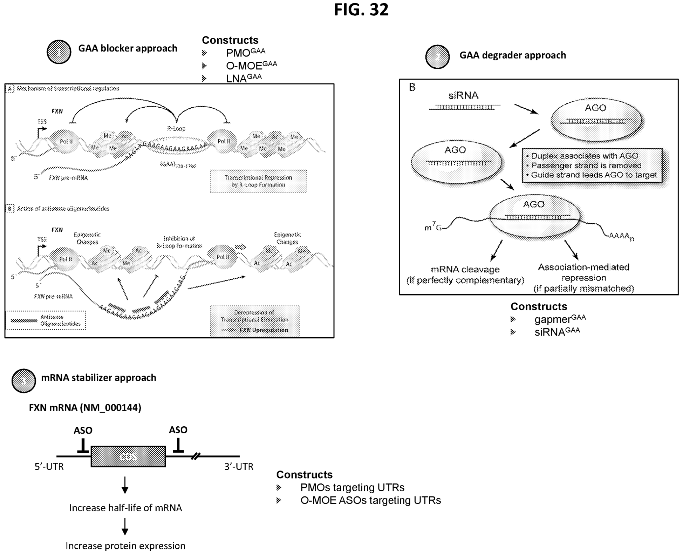

**FIG. 32** shows mechanisms by which antisense compounds modulate transcription, degrade mRNA, and stabilize mRNA (SEQ ID NOs: 215 and 216).

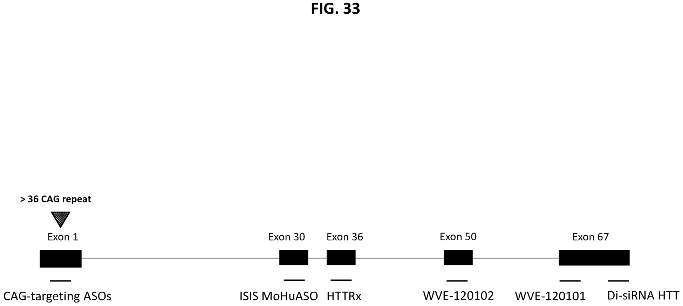

**FIG. 33** shows exemplary antisense oligonucleotides and their pre-mRNA *HTT* targets.

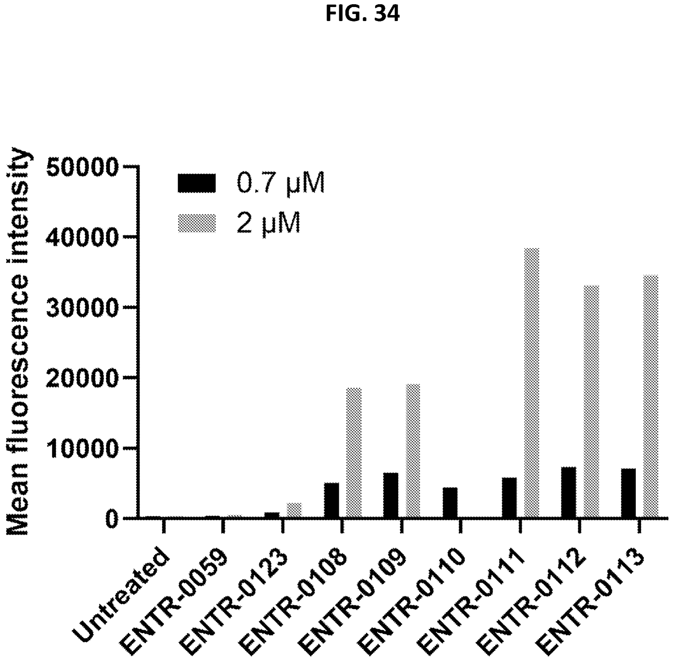

**FIG. 34** shows the cellular uptake results of rhodamine (LSR) labeled PMO and CPP-PMO conjugates quantified by the fluorescent activated cell sorting analysis using HeLa-654 cells were treated with 0.7 µM or 2 µM of PMO-LSR (ENTR-0059, Table 1), monovalent CPP-PMO-LSR (ENTR-0123), bivalent CPP-PMO-LSR (ENTR-0108, ENTR-0109, or ENTR-0110, Table 1), or trivalent CPP-PMO-LSR (ENTR-0111, ENTR-0112, or ENTR-0113) for 48 hours, as described in Example 1.

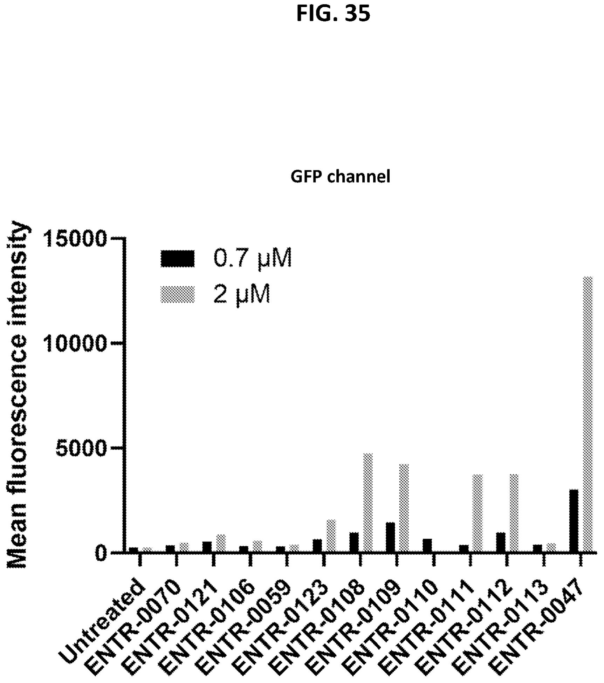

**FIG. 35** shows the results of cellular activity as quantified by EGFP correction by fluorescent activated cell sorting analysis using HeLa-654 cells after treatment with medium, 0.7 µM or 2 µM of PMO (ENTR-0059 or ENTR-0070, Table 1), monovalent CPP-PMO conjugates (ENTR-0121 or ENTR-0123, Table 1), bivalent CPP-PMO conjugates (ENTR-0106, ENTR-0108, ENTR-0109 or ENTR-0110, Table 1), trivalent CPP-PMO (ENTR-0111, ENTR-0112, or ENTR-0113, Table 1) or CPP-NLS-PMO (ENTR-0047) for 48 hours, as described in Example 1.

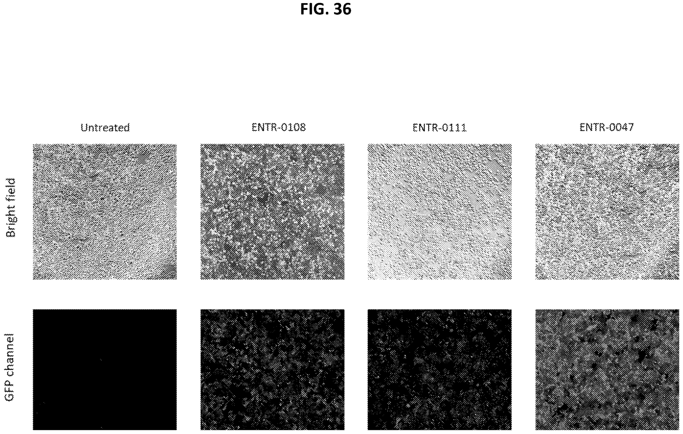

**FIG. 36** shows the cellular activity of various CPP-conjugated PMO constructs on HeLa-654 cells after 48 hr treatment of medium, 2 µM bidentate CPP-PMO (ENTR-0108, Table 1), tridentate CPP-PMO (ENTR-0111, Table 1) and CPP-NLS-PMO (ENTR-0047, Table 1). Bright field microscopy (top) and fluorescence microscopy (bottom, GFP channel) images were captured as described in Example 1.

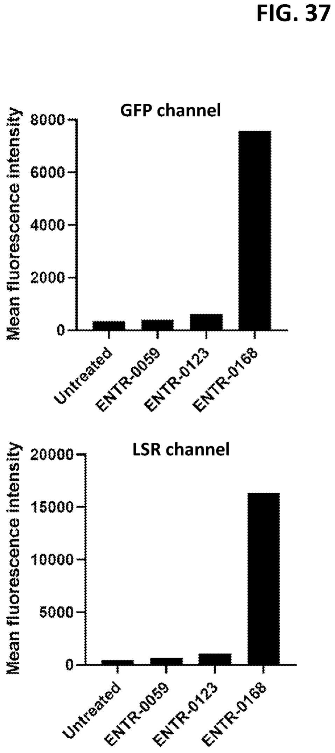

**FIG. 37** shows the results of fluorescent activated cell sorting analysis (Top: GFP channel; Bottom: LSR channel) of HeLa-654 cells treated for 24 hr with 2 µM of PMO-LSR (ENTR-0059, Table 1), CPP-PMO-LSR (ENTR-0123, Table 1), or CPP-NLS-PMO-LSR (ENTR-0168, Table 1), as described in Example 1.

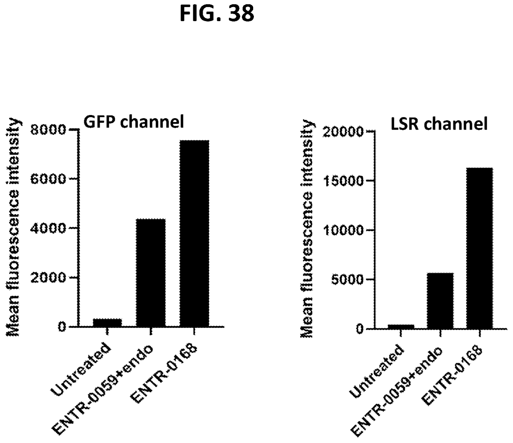

**FIG. 38** shows the results of fluorescent activated cell sorting analysis (Top: GFP channel; Bottom: LSR channel) of HeLa-654 cells treated for 24 hr with medium, 2 µM of PMO-LSR (ENTR-0059, Table 1) with transfection reagent, endoporter (6 µL/mL) or CPP-NLS-PMO-LSR (ENTR-0168, Table 1), as described in Example 1.

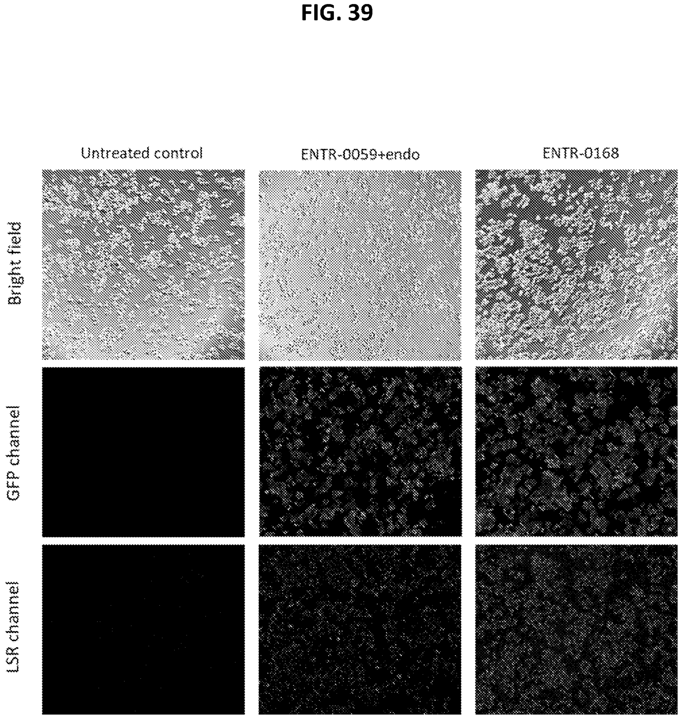

**FIG. 39** shows the bright field microscopy (top) and fluorescence microscopy (GFP channel, middle; LSR channel, bottom) imaging of HeLa-654 cells treated with 2 µM of PMO-LSR (ENTR-0059, Table 1) + endoporter (6 µL/mL) and CPP-NLS-PMO-LSR (ENTR-0168, Table 1) post 24 hours, as described in Example 1.

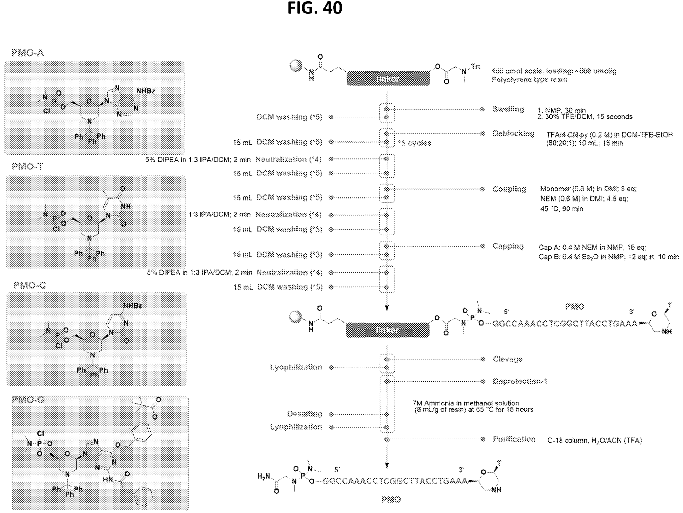

**FIG. 40** shows a schematic preparation of the PMO oligo and the monomers used for assembling the PMO oligo (SEQ ID NO: 214).

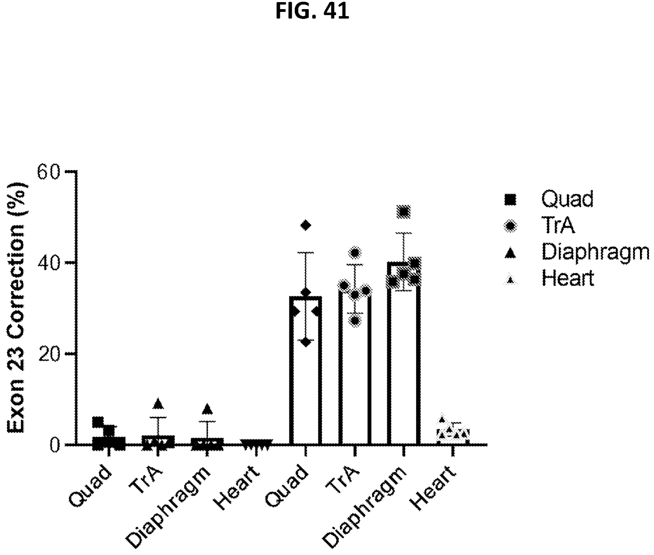

**FIG. 41** shows the RT-PCR result of exon skipping activities of PMO alone (ENTR-0013, Table B1) (24 mpk) and CPP12-PMO^{DMD} (ENTR-0098, Table B2) (30 mpk) in C57BL/10J mouse. Various muscle groups were analyzed at 1 week post intravenous injection (Quad=quadriceps, TrA=transverse abdominis, Diaphragm=Diaphragm muscles, Heart=cardiac muscle).

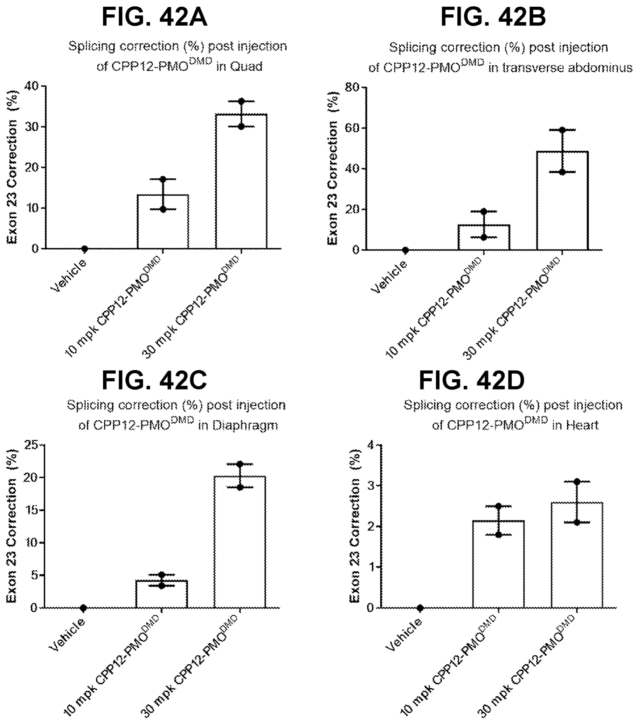

**FIGS. 42A-D** shows the RT-PCR result of exon skipping activities in quadriceps **(****FIG. 42A****),** transverse abdominis **(****FIG. 42B****),** diaphragm **(****FIG. 42C****),** and heart **(****FIG. 42D****)** in C57BL/10ScSn-Dmdmdx/J (MDX) mice after intravenous injection of CPP12-PMO^{DMD}(ENTR-0098, Table 3) at 10 and 30 mpk.

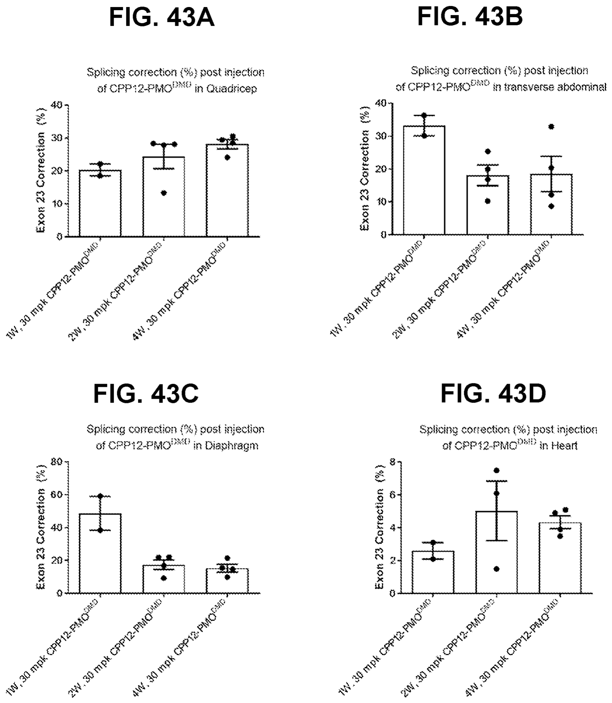

**FIGS. 43A-D** show the RT-PCR result of exon skipping activities in quadriceps **(****FIG. 43A****),** transverse abdominis **(****FIG. 43B****),** diaphragm **(****FIG. 43C****),** and heart **(****FIG. 43D****)** in MDX mice after a single 30 mpk intravenous injection of CPP12-PMO^{DMD} (ENTR-0098, Table B2) analyzed at 1 week, 2 weeks, and 4 weeks post injection.
. **FIG. 44** shows Western Blots of exon-23 corrected dystrophin and α-actinin in various muscle groups of MDX mice 1 week post single intravenous injection of PMO^{DMD} (8 mpk, ENTR-0013, Table 2) or CPP12-PMO^{DMD} (10 mpk, ENTR-0098, Table 3) in various muscle groups (quadriceps, Quad; transverse abdominis, TA; diaphragm, and heart.

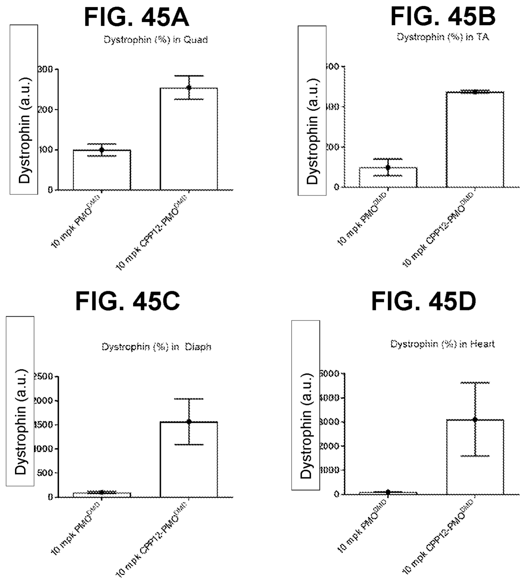

**FIGS. 45A-D** show the amount of exon-23 corrected dystrophin detected by Western Blot 1-week post single intravenous injection of PMO^{DMD} (ENTR-0013, Table 2) or CPP12-PMO^{DMD} (ENTR-0098, Table B2) in the quadriceps (Quad) **(****FIG. 45A****),** transverse abdominis (TA) **(****FIG. 45B****),** diaphragm **(****FIG. 45C****),** and heart **(****FIG. 45D****)** of MDX mice. The level of dystrophin correction after PMO injection is calibrated as 100%. Alpha-actinin is used as loading control.

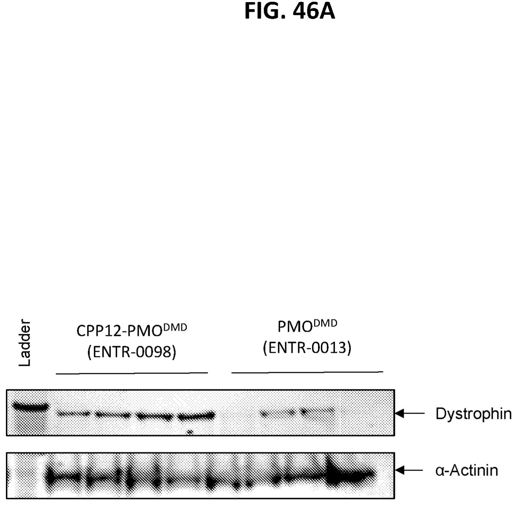

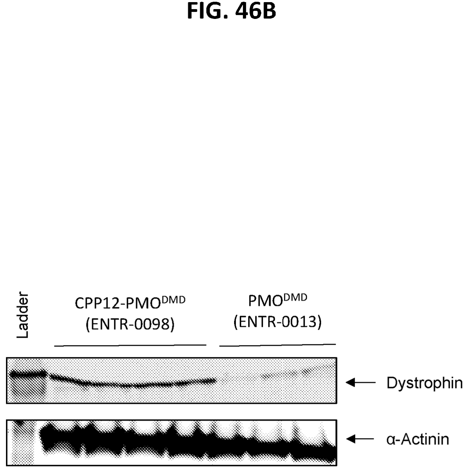

**FIGS. 46A-B** show Western Blots of exon-23 corrected dystrophin and α-actinin in the heart 2-week **(****FIG. 46A****)** or 4-week **(****FIG. 46B****)** after intravenous injection in MDX mice at 24 mpk of PMO^{DMD} (ENTR-0013, Table B1) or 30 mpk CPP12-PMO^{DMD} (ENTR-0098, Table B2).

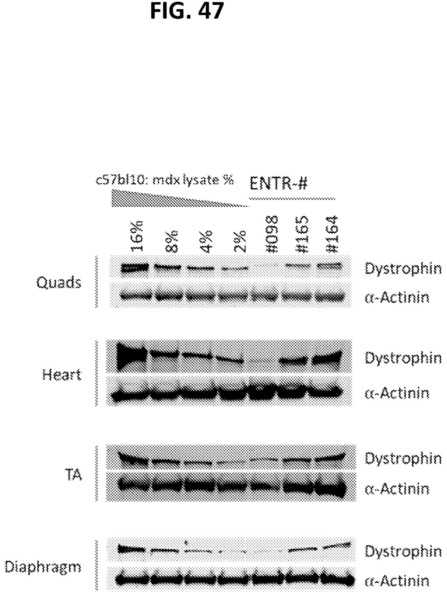

**FIG. 47** shows Western Blots of exon-23 corrected dystrophin and α-actinin in various muscle groups of MDX mice 1 week post single intravenous injection of CPP12-PMO^{DMD} (20 mpk, ENTR-0098, Table B2), or CPP12-NLS-PMO (20 mpk, construct ENTR-0164 or ENTR-0165, Table B3) in various muscle groups (quadriceps, Quad; heart; transverse abdominis, TA; and diaphragm). Alpha-actinin is used as loading control.

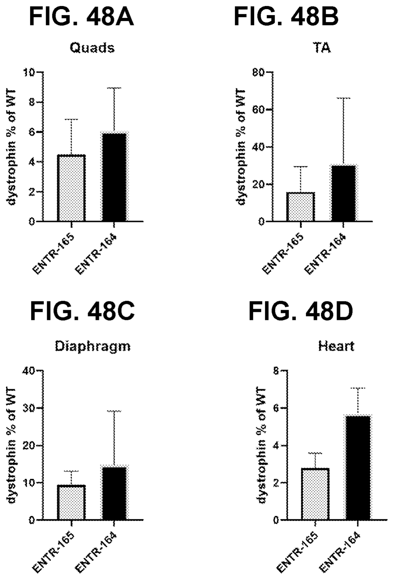

**FIGS. 48A-D** show the amount of exon-23 corrected dystrophin detected by Western Blot 1-week post single intravenous injection of CPP12-NLS-PMO constructs (20 mpk, construct ENTR-0164 or ENTR-0165, Table 4) in the quadriceps (Quad) **(****FIG. 48A****),** transverse abdominis (TA) **(****FIG. 48B****),** diaphragm **(****FIG. 48C****),** and heart **(****FIG. 48D****)** of MDX mice. Data is shown as % of dystrophin level in wild type animal (C57BL/10). Alpha-actinin as loading control.

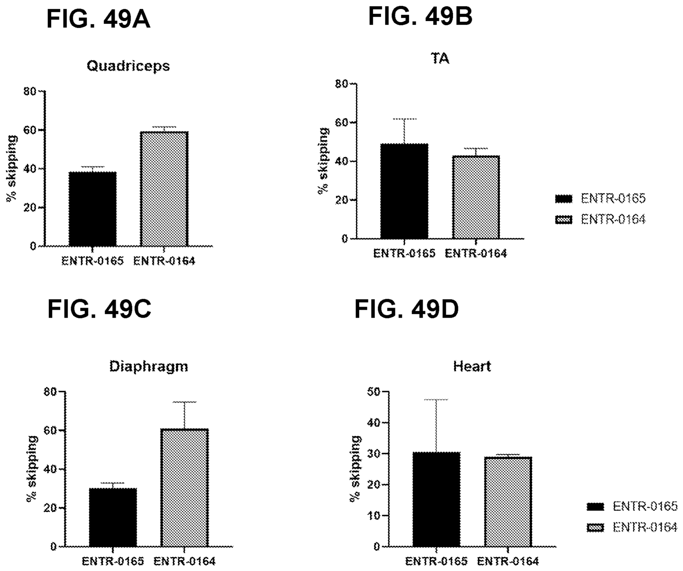

**FIGS. 49A-D** show the RT-PCR result of exon skipping activities in the quadriceps (Quad) **(****FIG. 49A****),** transverse abdominis (TA) **(****FIG. 49B****),** diaphragm **(****FIG. 49C****),** and heart **(****FIG. 49D****)** of MDX mice at 1-week post single intravenous injection of CPP12-NLS-PMO constructs (20 mpk, construct ENTR-0164 or ENTR-0165, Table B3)

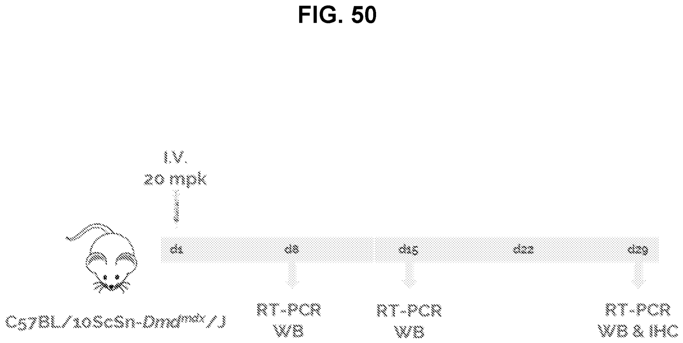

**FIG. 50** shows the study design of to evaluate the duration of effects of ENTR-201 in MDX (C57BL/10ScSn-*Dmd^{mdx}*/J) mice.

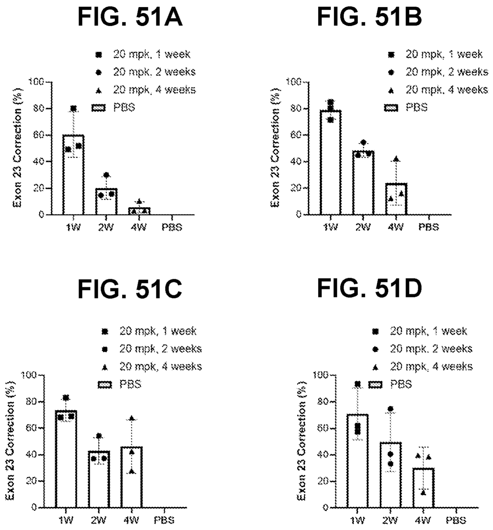

**FIGS. 51A-D** show the RT-PCR result of exon skipping activities of PBS vehicle or CPP12-NLS-PMO construct (ENTR-201, Table 4) in heart **(****FIG. 51A****),** diaphragm **(****FIG. 51B****),** quadricep **(****FIG. 51C****),** and transverse abdominis (TrA) (**FIG. 51D**) in MDX mice 1-week, 2-week, or 4-week post single IV injection at 20 mpk.

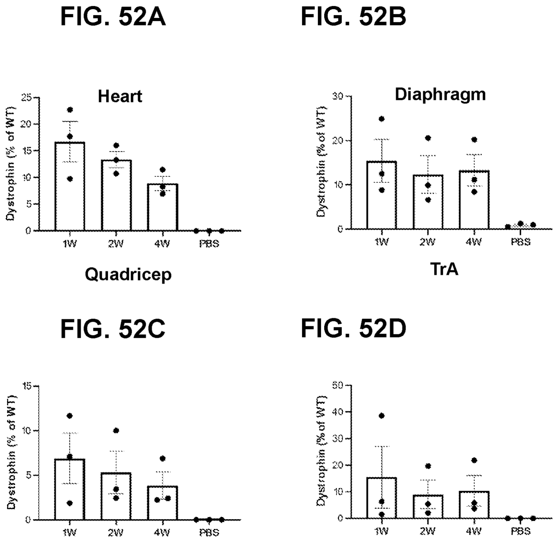

**FIGS. 52A-D** show the amount of exon-23 corrected dystrophin detected by Western Blot in the heart **(****FIG. 52A****),** diaphragm **(****FIG. 52B****),** quadricep **(****FIG. 52C****),** and transverse abdominis (TrA) **(****FIG. 52D****)** of MDX mice 1-week, 2-week, or 4-week post single intravenous injection of PBS vehicle or CPP12-NLS-PMO construct (ENTR-201, Table 4). Data is shown as % of dystrophin level in wild type animal (C57BL/10). Alpha-actinin as loading control.

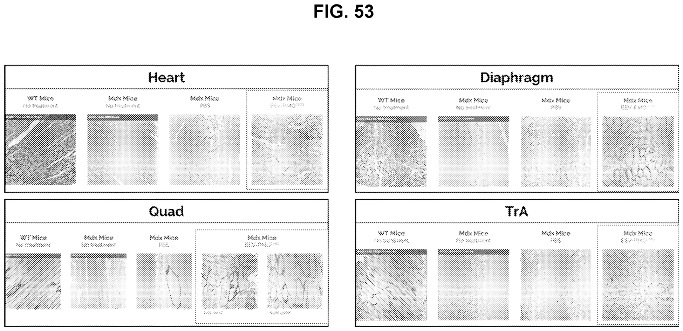

**FIG. 53** shows immunohistochemistry of muscle groups in mdx mice 4 weeks post single IV injection of ENTR-201 at 20 mpk and PBS vehicle.

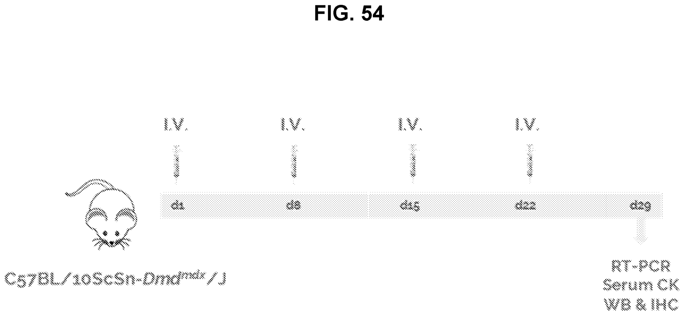

**FIG. 54** shows the study design to evaluate the efficacy of CPP12-NLS-PMO construct (ENTR-201, Table 4) at 10 mg/kg or PMO itself (ENTR-0013, Table B2) after repeated dose in MDX (C57BL/10ScSn-*Dmd^{mdx}*/J) mice. QW, weekly dosage.

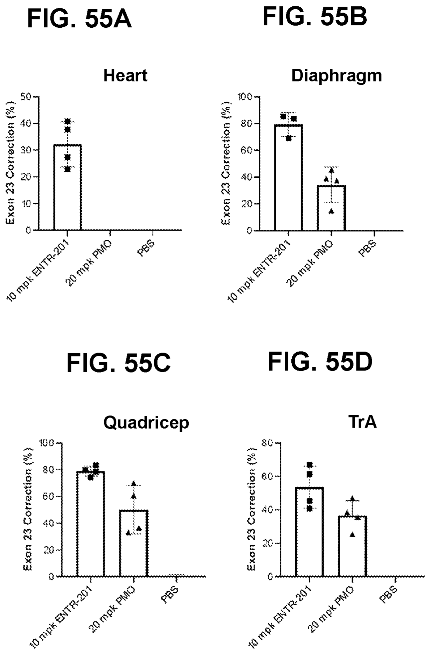

**FIGS. 55A-D** show the RT-PCR result of exon skipping activities of 4 weekly IV dosage of vehicle, CPP12-NLS-PMO construct (ENTR-201 at 10 mpk) or PMO alone (ENTR-013, at 20 mpk) in heart **(****FIG. 55A****),** diaphragm **(****FIG. 55B****),** quadricep **(****FIG. 55C****),** and transverse abdominis (TrA) **(****FIG. 55D****)** of MDX mice.

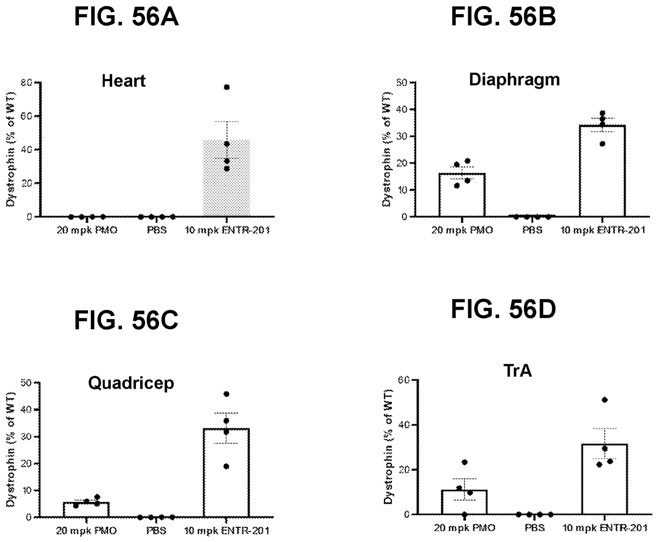

**FIGS. 56A-D** show the amount of exon-23 corrected dystrophin protein detected by Western Blot in the heart **(****FIG. 56A****),** diaphragm **(****FIG. 56B****),** quadricep (**FIG. 56C**), and transverse abdominis (TrA) **(****FIG. 56D****)** muscles of MDX mice after 4 weekly IV dosage of vehicle, CPP12-NLS-PMO construct (ENTR-201 at 10 mpk) or PMO alone (ENTR-013, at 20 mpk). Data is shown as % of dystrophin level in wild type animal (C57BL/10). Alpha-actinin as loading control.

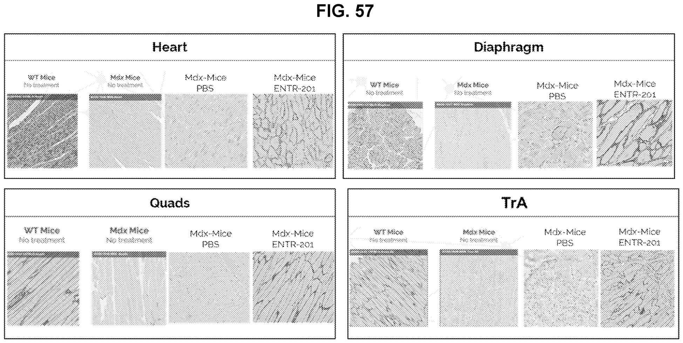

**FIG. 57** shows immunohistochemistry of muscle groups in mdx mice analyzed one week after 4 weekly dosages of ENTR-201 at 10 mpk and vehicle.

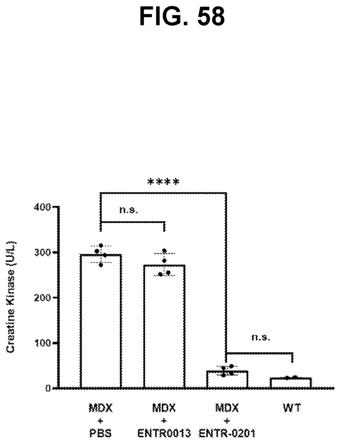

**FIG. 58** shows serum creatine kinase level in the serum of mdx mice analyzed one week after 4 weekly dosages of ENTR-201 at 10 mpk, PMO alone (ENTR-0013) at 20 mpk or vehicle. All data are reported as mean values +/- SEM. Statistical differences between treatment groups and control groups were evaluated by one-tailed t-test for statistical significance.

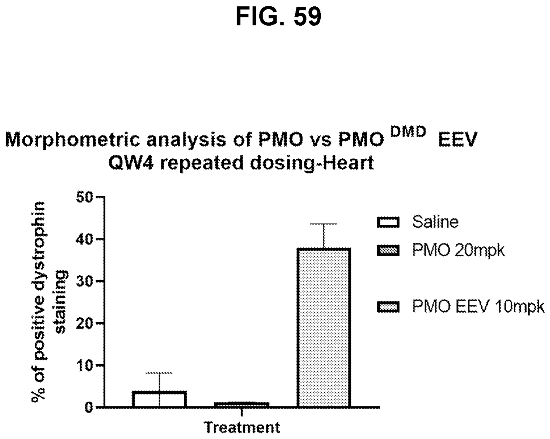

**FIG. 59** shows quantification of dystrophin staining intensity in the heart tissue using Halo membrane algorithm version 1.7 after 4 weekly dosages of ENTR-201 at 10 mpk, PMO alone at 20 mpk, or vehicle. Data is presented as % of dystrophin level among different treatment groups, CPP12-NLS-PMO construct (ENTR-201 at 10 mpk) or PMO alone (ENTR-013, at 20 mpk).

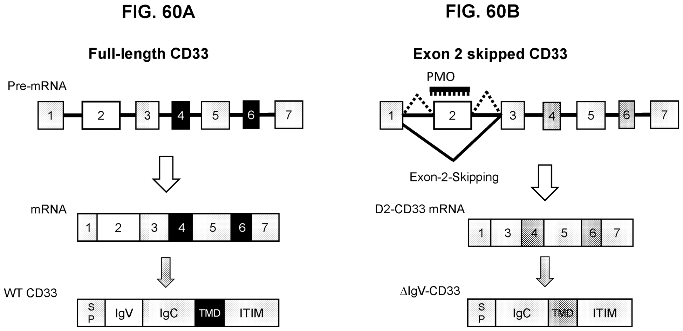

**FIGS. 60A-B** shows a schematic of CD33 PMO oligo design. PMO oligo targeting exon 2 of CD33 gene (also known as PMO^{CD33}) induces Exon-2-skipping of CD33 pre-mRNA, resulting in a truncated mature mRNA lacking exon 2 (D2-CD33) **(****FIG. 60B****).** The resulting protein translated from D2-CD33 mRNA lacks the extracellular IgV domain (ΔIgV-CD33), resulting in non-functional CD33 protein. **FIG. 60A** shows translation of wild-type CD33.

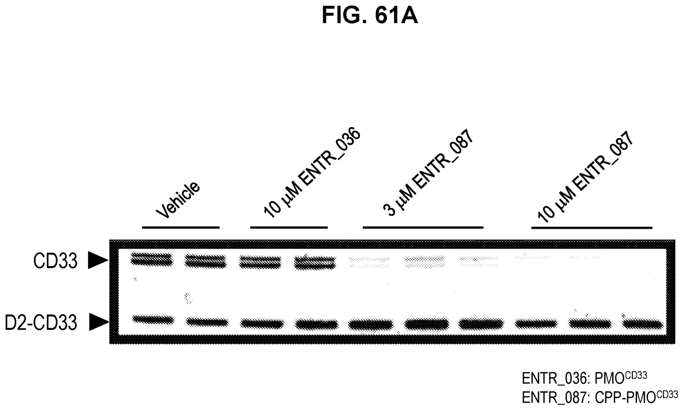

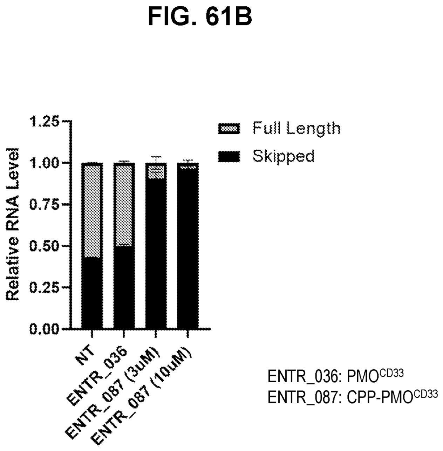

**FIGS. 61A-B** show a RT-PCR analysis of exon 2 skipping of CD33 **(****FIG. 61A****)** and quantification of full length CD33 versus D2-CD33 transcripts **(****FIG. 61B****)** after treatment with CPP-PMO^{CD33} (ENTR-087, TABLE 5) or PMO^{CD33} (ENTR-036, TABLE 5). PMO^{CD33} is an antisense compound that targets exon 2 of CD33.

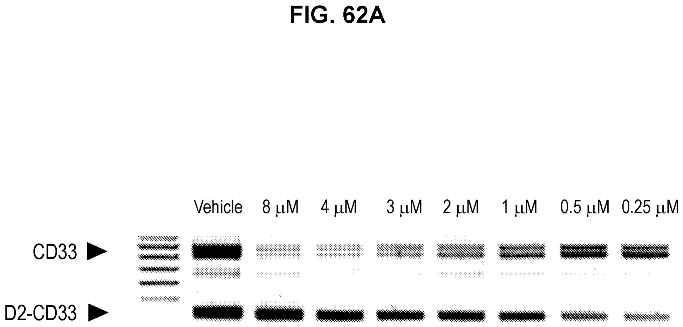

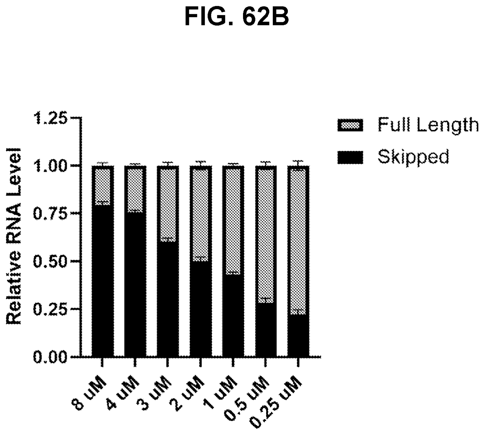

**FIGS. 62A-B** show the dose dependence of RT-PCR analysis of exon 2 skipping of CD33 **(****FIG. 62A****)** and quantification of full length CD33 versus D2-CD33 transcripts by dose **(****FIG. 62B****)** after treatment with CPP-PMO^{CD33} (ENTR-087, TABLE 5). The PMO targeted exon 2 of CD33.

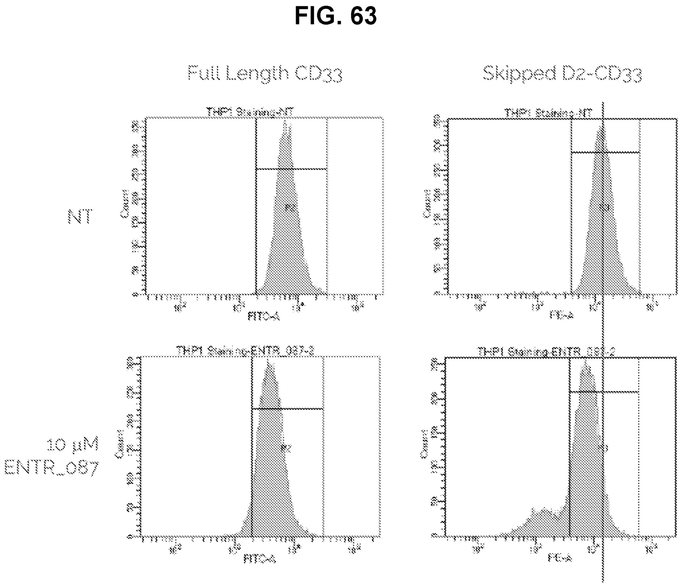

**FIG. 63** shows flow cytometry analysis of THP1 cells after treatment with CPP-PMO^{CD33} compared to untreated ("NT") cells.

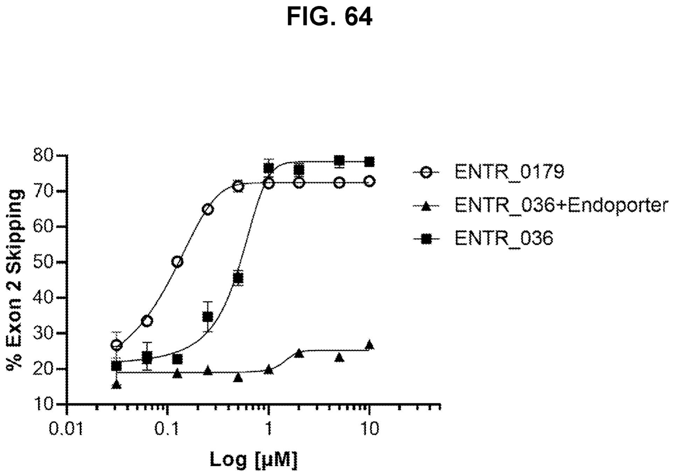

**FIG. 64** shows RT-PCR analysis of exon 2 skipping of CD33 gene. Differentiated THP1 cells were treated with CPP-NLS- PMO^{CD33} (ENTR-179, TABLE 5), PMO^{CD33} (ENTR-036, Table C) with and without Endoporter transfection reagent (6µL/mL).

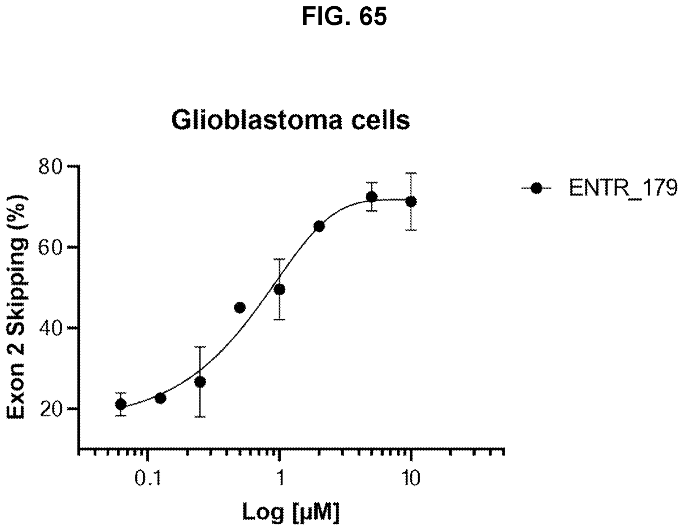

**FIG. 65** shows RT-PCR analysis of exon 2 skipping of CD33 gene in glioblastoma cells. Human glioblastoma cells were treated with various dose (from 0.0625 µM to 10 µM) of CPP-NLS-PMO^{CD33} (ENTR-179, Table C).

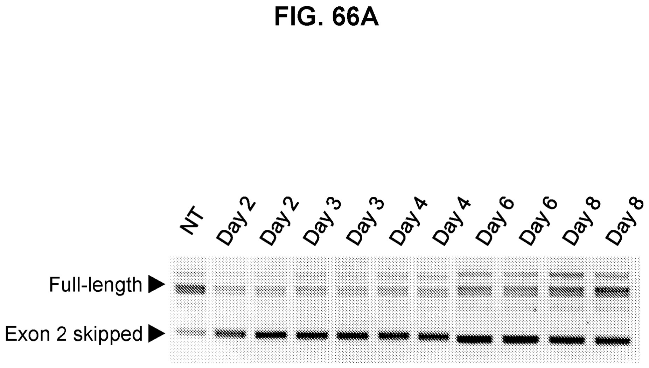

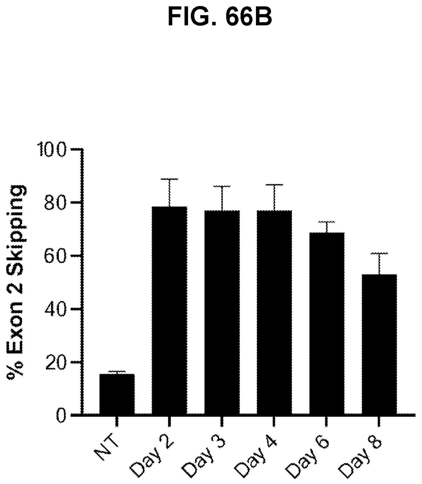

**FIGS. 66A-B** show RT-PCR analysis and quantification of exon 2 skipping of CD33 gene in THP1 cells in a time course study. Differentiated THP1 cells were treated with CPP- PMO^{CD33} (ENTR-087") for 24 hr before washed and cultured with compound-free media. Cells were harvested at Day 2, 3, 4, 6, and 8 post treatment.

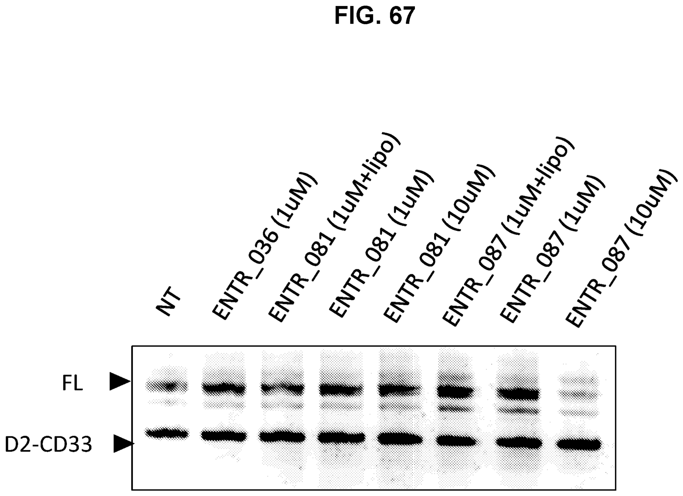

**FIG. 67** shows RT-PCR analysis of exon 2 skipping of CD33 gene in THP1 cells treated by CPP-PMO^{CD33} (ENTR-087, Table C), CPP-PMO^{CD33} (ENTR-081, TABLE 5), or PMO^{CD33} (ENTR-036, TABLE 5) with or without transfection reagent.

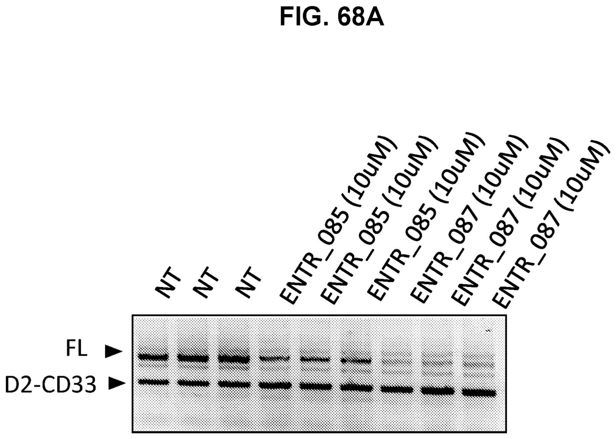

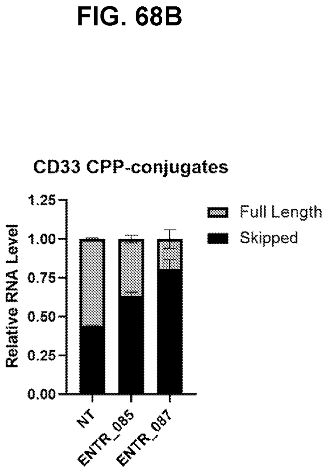

**FIGS. 68A-B** show RT-PCR analysis **(****FIG. 68A****)** and quantification of exon 2 skipping of CD33 gene **(****FIG. 68B****)** in THP1 cells. Differentiated THP1 cells were treated with CPP-PMO^{CD33} with two different PMO sequences (15-mer in ENTR-085 and 21-mer ENTR-087) for 24 hrs.

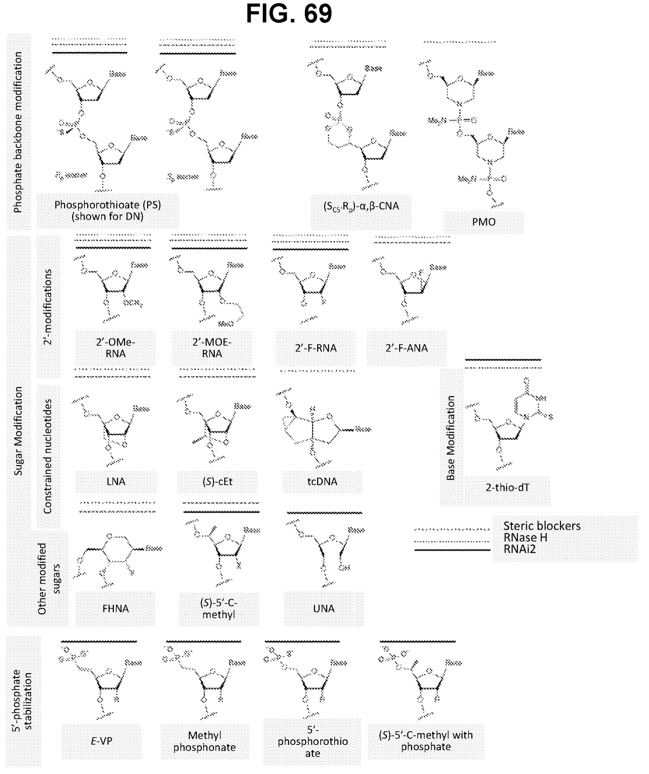

**FIG. 69** shows nucleosides used in the antisense oligonucleotides of the disclosure.

### DETAILED DESCRIPTION

### Definitions

The term "pharmaceutically acceptable" means suitable for use in contact with the tissues of humans and animals without undue toxicity, irritation, allergic response, and the like, commensurate with a reasonable benefit/risk ratio, and effective for their intended use within the scope of sound medical judgment.

The term "pharmaceutically acceptable salts" include those obtained by reacting the active compound functioning as a base, with an inorganic or organic acid to form a salt, for example, salts of hydrochloric acid, sulfuric acid, phosphoric acid, methanesulfonic acid, camphorsulfonic acid, oxalic acid, maleic acid, succinic acid, citric acid, formic acid, hydrobromic acid, benzoic acid, tartaric acid, fumaric acid, salicylic acid, mandelic acid, carbonic acid, etc. Those skilled in the art will further recognize that acid addition salts may be prepared by reaction of the compounds with the appropriate inorganic or organic acid via any of a number of known methods. The term "pharmaceutically acceptable salts" also includes those obtained by reacting the active compound functioning as an acid, with an inorganic or organic base to form a salt, for example salts of ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris-(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, and the like. Non limiting examples of inorganic or metal salts include lithium, sodium, calcium, potassium, magnesium salts and the like.

As used herein, "treat," "treating," "treatment" and variants thereof, refers to any administration of the disclosed compounds that partially or completely alleviates, ameliorates, relieves, inhibits, delays onset of, reduces severity of, and/or reduces incidence of one or more symptoms or features of a disease as described herein.

As used herein, "therapeutically effective" refers to an amount of a disclosed compound which confers a therapeutic effect on a patient. In some embodiments, the therapeutically effective amount is an amount sufficient to treat a disease in a subject in need thereof.

As used herein, "cell penetrating peptide" or "CPP" refers to any cyclic peptide which is capable of penetrating a cell membrane. Cell penetrating peptides may also be referred to as an endosomal escape vehicle or EEV. In some embodiments, the CPP is cyclic, and is represented as "cCPP". The cyclic cell penetrating peptide is also capable of directing a compound (e.g., AC) to penetrate the membrane of a cell. In some embodiments, the cCPP delivers the AC to the cytosol of the cell. In some embodiments, the cCPP delivers the AC to the cellular location where the target sequence on pre-mRNA is located.

As used herein, "linker" or "L" refers to a moiety which that covalently bonds two or more moieties (e.g., a cCPP and an AC or a cCPP and CRISPR gene-editing machinery). In some embodiments, the linker can be natural or non-natural amino acid or polypeptide. In other embodiments, the linker is a synthetic compound containing two or more appropriate functional groups suitable to bind a CPP and an AC, to thereby form the compounds disclosed herein. In yet another embodiment, the linker comprises an M moiety to thereby conjugate the CPP to the AC. For example, in some embodiments, the cCPP may be covalently bound to the AC via a linker. For example, in some embodiments, the cCPP may be covalently bound to the CRISPR gene-editing machinery via a linker.

As used herein, "polypeptide" refers to a string of at least two amino acids attached to one another by a peptide bond. There is no upper limit to the number of amino acids that can be included in a polypeptide. Further, polypeptides may include non-natural amino acids, amino acid analogs, or other synthetic molecules that are capable of integrating into a polypeptide.

As used herein, the term "sequence identity" refers to the percentage of amino acids between two polypeptide sequences that are the same and in the same relative position. As such one polypeptide sequence has a certain percentage of sequence identity compared to another polypeptide sequence. For sequence comparison, typically one sequence acts as a reference sequence, to which test sequences are compared. Those of ordinary skill in the art will appreciate that two sequences are generally considered to be "substantially identical" if they contain identical residues in corresponding positions. In some embodiments, the sequence identity between two amino acid sequences may be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), in the version that exists as of the date of filing. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues×100)/(Length of Alignment-Total Number of Gaps in Alignment)

In other embodiments, sequence identity may be determined using the Smith-Waterman algorithm, in the version that exists as of the date of filing.

As used herein, "sequence homology" refers to the percentage of amino acids between two polypeptide sequences that are homologous and in the same relative position. As such one polypeptide sequence has a certain percentage of sequence homology compared to another polypeptide sequence. As will be appreciated by those of ordinary skill in the art, two sequences are generally considered to be "substantially homologous" if they contain homologous residues in corresponding positions. Homologous residues may be identical residues. Alternatively, homologous residues may be non-identical residues with appropriately similar structural and/or functional characteristics. For example, as is well known by those of ordinary skill in the art, certain amino acids are typically classified as "hydrophobic" or "hydrophilic" amino acids, and/or as having "polar" or "non-polar" side chains, and substitution of one amino acid for another of the same type may often be considered a "homologous" substitution.

As is well known in this art, amino acid sequences may be compared using any of a variety of algorithms, including those available in commercial computer programs such as BLASTP, gapped BLAST, and PSI-BLAST, in existence as of the date of filing. Exemplary such programs are described in Altschul, et al., Basic local alignment search tool, J. Mol. Biol., 215(3): 403-410, 1990; Altschul, et al., Methods in Enzymology*;* Altschul, et al., "Gapped BLAST and PSI-BLAST: a new generation of protein database search programs", Nucleic Acids Res. 25:3389-3402, 1997; Baxevanis, et al., Bioinformatics A Practical Guide to the Analysis of Genes and Proteins, Wiley, 1998; and Misener, et al., (eds.), Bioinformatics Methods and Protocols (Methods in Molecular Biology, Vol. 132), Humana Press, 1999. In addition to identifying homologous sequences, the programs mentioned above typically provide an indication of the degree of homology.

As used herein, the term "nuclear localization sequence" (NLS) refers to an amino acid sequence which induces transport of molecules comprising such sequences or linked to such sequences into the nucleus of eukaryotic cells. Non-limiting examples of nuclear localization sequences include the nuclear localization sequence of the SV40 virus large T-antigen the minimal functional unit of which is the seven amino acid sequence PKKKRKV (SEQ ID NO: 131), the nucleoplasmin bipartite NLS with the sequence NLSKRPAAIKKAGQAKKKK (SEQ ID NO: 132), the c-myc nuclear localization sequence having the amino acid sequence PAAKRVKLD (SEQ ID NO: 133) or RQRRNELKRSF (SEQ ID NO: 134), the sequence RMRKFKNKGKDTAELRRRRVEVSVELRKAKKDEQILKRRNV (SEQ ID NO: 135) of the IBB domain from importin-alpha, the sequences VSRKRPRP (SEQ ID NO: 136) and PPKKARED (SEQ ID NO: 137) of the myoma T protein, the sequence PQPKKKPL (SEQ ID NO: 138) of human p53, the sequence SALIKKKKKMAP (SEQ ID NO: 139) of mouse c-abl IV, the sequences DRLRR (SEQ ID NO: 140) and PKQKKRK (SEQ ID NO: 141) of the influenza virus NS1, the sequence RKLKKKIKKL (SEQ ID NO: 142) of the Hepatitis virus delta antigen and the sequence REKKKFLKRR (SEQ ID NO: 143) of the mouse Mxl protein, the sequence KRKGDEVDGVDEVAKKKSKK (SEQ ID NO: 144) of the human poly(ADP-ribose) polymerase and the sequence RKCLQAGMNLEARKTKK (SEQ ID NO: 145) of the steroid hormone receptors (human) glucocorticoid. International Publication No. 2001/038547 describes additional examples of NLSs and is incorporated by reference herein in its entirety.

"Alkyl" or "alkyl group" refers to a fully saturated, straight or branched hydrocarbon chain having from one to twelve carbon atoms, and which is attached to the rest of the molecule by a single bond. Alkyls comprising any number of carbon atoms from 1 to 12 are included. An alkyl comprising up to 12 carbon atoms is a C₁-C₁₂ alkyl, an alkyl comprising up to 10 carbon atoms is a C₁-C₁₀ alkyl, an alkyl comprising up to 6 carbon atoms is a C₁-C₆ alkyl and an alkyl comprising up to 5 carbon atoms is a C₁-C₅ alkyl. A C₁-C₅ alkyl includes C₅ alkyls, C₄ alkyls, C₃ alkyls, C₂ alkyls and C₁ alkyl (*i.e.,* methyl). A C₁-C₆ alkyl includes all moieties described above for C₁-C₅ alkyls but also includes C₆ alkyls. A C₁-C₁₀ alkyl includes all moieties described above for C₁-C₅ alkyls and C₁-C₆ alkyls, but also includes C₇, Cs, C₉ and C₁₀ alkyls. Similarly, a C₁-C₁₂ alkyl includes all the foregoing moieties, but also includes C₁₁ and C₁₂ alkyls. Non-limiting examples of C₁-C₁₂ alkyl include methyl, ethyl, n-propyl, i-propyl, *sec*-propyl, n-butyl, i-butyl, sec-butyl, *t-*butyl, n-pentyl, t-amyl, n-hexyl, n-heptyl, n-octyl, n-nonyl, n-decyl, n-undecyl, and n-dodecyl. Unless stated otherwise specifically in the specification, an alkyl group can be optionally substituted.

"Alkylene" or "alkylene chain" refers to a fully saturated, straight or branched divalent hydrocarbon chain radical, having from one to forty carbon atoms. Non-limiting examples of C₂-C₄₀ alkylene include ethylene, propylene, n-butylene, ethenylene, propenylene, n-butenylene, propynylene, n-butynylene, and the like. The alkylene chain is attached, directly or indirectly, to the cCPP through a single bond and, directly or indirectly, to the AC through a single bond. Unless stated otherwise specifically in the specification, an alkylene chain can be optionally substituted as described herein.

"Alkenylene" or "alkenylene chain" refers to a straight or branched divalent hydrocarbon chain radical, having from two to forty carbon atoms, and having one or more carbon-carbon double bonds. Non-limiting examples of C₂-C₄₀ alkenylene include ethene, propene, butene, and the like. The alkenylene chain is attached, directly or indirectly, to the cCPP through a single bond and, directly or indirectly, to the AC through a single bond. Unless stated otherwise specifically in the specification, an alkenylene chain can be optionally substituted.

"Alkynyl" or "alkynyl group" refers to a straight or branched hydrocarbon chain having from two to twelve carbon atoms, and having one or more carbon-carbon triple bonds. Each alkynyl group is attached to the rest of the molecule by a single bond. Alkynyl group comprising any number of carbon atoms from 2 to 12 are included. An alkynyl group comprising up to 12 carbon atoms is a C₂-C₁₂ alkynyl, an alkynyl comprising up to 10 carbon atoms is a C₂-C₁₀ alkynyl, an alkynyl group comprising up to 6 carbon atoms is a C₂-C₆ alkynyl and an alkynyl comprising up to 5 carbon atoms is a C₂-C₅ alkynyl. A C₂-C₅ alkynyl includes C₅ alkynyls, C₄ alkynyls, C₃ alkynyls, and C₂ alkynyls. A C₂-C₆ alkynyl includes all moieties described above for C₂-C₅ alkynyls but also includes C₆ alkynyls. A C₂-C₁₀ alkynyl includes all moieties described above for C₂-C₅ alkynyls and C₂-C₆ alkynyls, but also includes C₇, Cs, C₉ and C₁₀ alkynyls. Similarly, a C₂-C₁₂ alkynyl includes all the foregoing moieties, but also includes C₁₁ and C₁₂ alkynyls. Non-limiting examples of C₂-C₁₂ alkenyl include ethynyl, propynyl, butynyl, pentynyl and the like. Unless stated otherwise specifically in the specification, an alkyl group can be optionally substituted.

"Alkynylene" or "alkynylene chain" refers to a straight or branched divalent hydrocarbon chain, having from two to forty carbon atoms, and having one or more carbon-carbon triple bonds. Non-limiting examples of C₂-C₄₀ alkynylene include ethynylene, propargylene and the like. The alkynylene chain is attached, directly or indirectly, to the CPP through a single bond and, directly or indirectly, to the AC through a single bond. Unless stated otherwise specifically in the specification, an alkynylene chain can be optionally substituted.

"Carbocyclyl," "carbocyclic ring" or "carbocycle" refers to a rings structure, wherein the atoms which form the ring are each carbon, and which is attached to the rest of the molecule by a single bond. Carbocyclic rings can comprise from 3 to 20 carbon atoms in the ring. Unless stated otherwise specifically in the specification, the carbocyclyl can be a monocyclic, bicyclic, tricyclic or tetracyclic ring system, which can include fused or bridged ring systems Carbocyclic rings include aryls and cycloalkyl, cycloalkenyl, and cycloalkynyl as defined herein. Unless stated otherwise specifically in the specification, a carbocyclyl group can be optionally substituted. In some embodiments, the carbocyclyl divalent, and is attached, directly or indirectly, to the CPP through a single bond and, directly or indirectly, to the AC through a single bond. Unless stated otherwise specifically in the specification, a heterocyclyl group can be optionally substituted.

"Cycloalkyl" refers to a stable non-aromatic monocyclic or polycyclic fully saturated hydrocarbon having from 3 to 40 carbon atoms and at least one ring, wherein the ring consists solely of carbon and hydrogen atoms, which can include fused or bridged ring systems. Monocyclic cycloalkyls include, for example, cyclopropyl, cyclobutyl, cyclopentyl, cyclohexyl, cycloheptyl, and cyclooctyl. Polycyclic cycloalkyls include, for example, adamantyl, norbornyl, decalinyl, 7,7-dimethyl-bicyclo[2.2.1]heptanyl, and the like. In some embodiments, the cycloalkyl divalent and is attached, directly or indirectly, to the CPP through a single bond and, directly or indirectly, to the AC through a single bond. Unless otherwise stated specifically in the specification, a cycloalkyl group can be optionally substituted.

"Cycloalkenyl" refers to a stable non-aromatic monocyclic or polycyclic hydrocarbon having from 3 to 40 carbon atoms, at least one ring having, and one or more carbon-carbon double bonds, wherein the ring consists solely of carbon and hydrogen atoms, which can include fused or bridged ring systems. Monocyclic cycloalkenyls include, for example, cyclopentenyl, cyclohexenyl, cycloheptenyl, cycloctenyl, and the like. Polycyclic cycloalkenyl radicals include, for example, bicyclo[2.2.1]hept-2-enyl and the like. In some embodiments, cycloalkenyl is divalent and is attached, directly or indirectly, to the CPP through a single bond and, directly or indirectly, to the AC through a single bond. Unless otherwise stated specifically in the specification, a cycloalkenyl group can be optionally substituted.

"Cycloalkynyl" refers to a stable non-aromatic monocyclic or polycyclic hydrocarbon having from 3 to 40 carbon atoms, at least one ring having, and one or more carbon-carbon triple bonds, wherein the ring consists solely of carbon and hydrogen atoms, which can include fused or bridged ring systems. Monocyclic cycloalkynyls include, for example, cycloheptynyl, cyclooctynyl, and the like. The cycloalkynyl is attached, directly or indirectly, to the CPP through a single bond and, directly or indirectly, to the AC through a single bond. Unless otherwise stated specifically in the specification, a cycloalkynyl group can be optionally substituted.

"Aryl" refers to a hydrocarbon ring system comprising hydrogen, 6 to 40 carbon atoms and at least one aromatic ring. For purposes of this disclosure, the aryl can be a monocyclic, bicyclic, tricyclic or tetracyclic ring system, which can include fused or bridged ring systems. Aryls include, but are not limited to, aryl divalent radicals derived from aceanthrylene, acenaphthylene, acephenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. In some embodiments, the aryl divalent and is attached, directly or indirectly, to the CPP through a single bond and, directly or indirectly, to the AC through a single bond. Unless stated otherwise specifically in the specification, an aryl group can be optionally substituted.

"Heterocyclyl," "heterocyclic ring" or "heterocycle" refers to a stable 3- to 22-membered ring system which consists of two to fourteen carbon atoms and from one to eight heteroatoms selected from the group consisting of nitrogen, oxygen and sulfur. Heterocyclyl or heterocyclic rings include heteroaryls as defined below. Unless stated otherwise specifically in the specification, the heterocyclyl can be a monocyclic, bicyclic, tricyclic or tetracyclic ring system, which can include fused or bridged ring systems; and the nitrogen, carbon or sulfur atoms in the heterocyclyl can be optionally oxidized; the nitrogen atom can be optionally quaternized; and the heterocyclyl can be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, succinimidyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. In some embodiments, the heterocyclyl is divalent and is attached, directly or indirectly, to the CPP through a single bond and, directly or indirectly, to the AC through a single bond. Unless stated otherwise specifically in the specification, a heterocyclyl group can be optionally substituted.

"Heteroaryl" refers to a 5- to 22-membered aromatic ring comprising hydrogen atoms, one to fourteen carbon atoms, one to eight heteroatoms selected from the group consisting of nitrogen, oxygen and sulfur, and at least one aromatic ring. For purposes of this disclosure, the heteroaryl can be a monocyclic, bicyclic, tricyclic or tetracyclic ring system, which can include fused or bridged ring systems; and the nitrogen, carbon or sulfur atoms in the heteroaryl can be optionally oxidized; the nitrogen atom can be optionally quaternized. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e. thienyl). In some embodiments, the heteroaryl is divalent and is attached, directly or indirectly, to the CPP through a single bond and, directly or indirectly, to the AC through a single bond. Unless stated otherwise specifically in the specification, a heteroaryl group can be optionally substituted.

The term "ether" used herein refers to a divalent moiety having a formula -[(R₁)ₘ-O-(R₂)ₙ]_{z}- wherein each of m, n, and z are independently selected from 1 to 40, and R1 and R2 are independently selected from an alkylene. Examples include polyethylene glycol. The ether is attached, directly or indirectly, to the CPP through a single bond and, directly or indirectly, to the AC through a single bond. Unless stated otherwise specifically in the specification, the ether can be optionally substituted.

The term "substituted" used herein means any of the above groups (*i.e.,* alkylene, alkenylene, alkynylene, aryl, carbocyclyl, cycloalkyl, cycloalkenyl, cycloalkynyl, heterocyclyl, heteroaryl, and/or ether) wherein at least one hydrogen atom is replaced by a bond to a non-hydrogen atoms such as, but not limited to: a deuterium atom; a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (e.g., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with -NR_{g}Rₕ, -NR_{g}C(=O)Rₕ, -NR_{g}C(=O)NR_{g}Rₕ, -NR_{g}C(=O)ORₕ, -NR_{g}SO₂Rₕ, -OC(=O)NR_{g}Rₕ, - OR_{g}, -SR_{g}, -SOR_{g}, -SO₂R_{g}, -OSO₂R_{g}, -SO₂OR_{g}, =NSO₂R_{g}, and -SO₂NR_{g}Rₕ. "Substituted also means any of the above groups in which one or more hydrogen atoms are replaced with -C(=O)R_{g}, -C(=O)OR_{g}, -C(=O)NR_{g}Rₕ, -CH₂SO₂R_{g}, -CH₂SO₂NR_{g}Rₕ. In the foregoing, R_{g} and Rₕ are the same or different and independently hydrogen, alkyl, alkenyl, alkynyl, alkoxy, alkylamino, thioalkyl, aryl, aralkyl, cycloalkyl, cycloalkenyl, cycloalkynyl, cycloalkylalkyl, haloalkyl, haloalkenyl, haloalkynyl, heterocyclyl, N-heterocyclyl, heterocyclylalkyl, heteroaryl, N-heteroaryl and/or heteroarylalkyl. "Substituted" further means any of the above groups in which one or more hydrogen atoms are replaced by a bond to an amino, cyano, hydroxyl, imino, nitro, oxo, thioxo, halo, alkyl, alkenyl, alkynyl, alkoxy, alkylamino, thioalkyl, aryl, aralkyl, cycloalkyl, cycloalkenyl, cycloalkynyl, cycloalkylalkyl, haloalkyl, haloalkenyl, haloalkynyl, heterocyclyl, N-heterocyclyl, heterocyclylalkyl, heteroaryl, N-heteroaryl and/or heteroarylalkyl group. In addition, each of the foregoing substituents can also be optionally substituted with one or more of the above substituents. Further, those skilled in the art will recognize that "substituted" also encompasses instances in which one or more hydrogen atoms on any of the above groups are replaced by a substituent listed in this paragraph, and the substituent then forms a covalent bond with the CPP or AC. The resulting bonding group can be considered a "substituent." For example, In some embodiments, any of the above groups can be substituted at a first position with a carboxylic acid (i.e., -C(=O)OH) which forms an amide bond with an appropriate amino acid CPP (e.g., lysine), and also substituted at a second position with either an electrophilic group (e.g., -C(=O)H, -CO₂R_{g}, -halide, etc.) or a nucleophilic group (-NH₂, -NHR_{g}, -OH, etc.) which forms a bond with the 5' end of an AC or alternatively which forms a bond with the 3' end of AC. The resulting bond, e.g., amide bond, can be considered a "substituent." In some embodiments, the second position is substituted with a thiol group which forms a disulfide bond with a thiol group attached to the AC. The resulting disulfide is encompassed by the term substituent.

As used herein, the symbol " 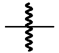 " (hereinafter can be referred to as "a point of attachment bond") denotes a bond that is a point of attachment between two chemical entities, one of which is depicted as being attached to the point of attachment bond and the other of which is not depicted as being attached to the point of attachment bond. For example, " 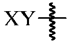 " indicates that the chemical entity "XY" is bonded to another chemical entity via the point of attachment bond. Furthermore, the specific point of attachment to the non-depicted chemical entity can be specified by inference. For example, the compound CH₃-R³, wherein R³ is H or " 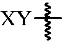 " infers that when R³ is "XY", the point of attachment bond is the same bond as the bond by which R³ is depicted as being bonded to CH₃.

As used herein, the terms "antisense compound" and "AC" are used interchangeably to refer to a polymeric nucleic acid structure which is at least partially complementary to a target nucleic acid molecule to which it (the AC) hybridizes. The AC may be a short (in some embodiments, less than 50 base pair) polynucleotide or polynucleotide homologue comprising a sequence complimentary to a target sequence in a target pre-mRNA strand. The AC may be formed of natural nucleic acids, synthetic nucleic acids, nucleic acid homologues, or any combination thereof. In some embodiments, the AC comprises oligonucleosides. In some embodiments, AC comprises antisense oligonucleotides. In some embodiments, the AC comprises conjugate groups. Nonlimiting examples of ACs include, but are not limited to, primers, probes, antisense oligonucleotides, external guide sequence (EGS) oligonucleotides, alternate splicers, siRNAs, oligonucleotides, oligonucleosides, oligonucleotide analogs, oligonucleotide mimetics, and chimeric combinations of these. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound. In some embodiments, an AC modulates (increases, decreases, or changes) expression of a target nucleic acid.

The terms "pre-mRNA" and "primary transcript" as used herein refer to a newly synthesized eukaryotic mRNA molecule directly after DNA transcription. A pre-mRNA must be capped with a 5' cap, modified with a 3' poly-A tail, and spliced to produce a mature mRNA sequence.

As used herein, the terms "target nucleic acid" and "target sequence" refer to the nucleic acid sequence to which the antisense compound binds or hybridizes. Target nucleic acids include, but are not limited to, RNA (including, but not limited to pre-mRNA and mRNA or portions thereof), cDNA derived from such RNA, as well as non-translated RNA, such as miRNA. For example, in some embodiments, a target nucleic acid can be a cellular gene (or mRNA transcribed from such gene) whose expression is associated with a particular disorder or disease state, or a nucleic acid molecule from an infectious agent.

As used herein, the terms "splicing" and "processing" refer to the modification of a pre-mRNA following transcription, in which introns are removed and exons are joined. Splicing occurs in a series of reactions that are catalyzed by a large RNA-protein complex composed of five small nuclear ribonucleoproteins (snRNPs) referred to as a spliceosome. Within an intron, a 3' splice site, a 5' splice site, and a branch site are required for splicing. The RNA components of snRNPs interact with the intron and may be involved in catalysis

The "target pre-mRNA" is the pre-mRNA comprising the target sequence to which the AC hybridizes.

The "target mRNA" is the mRNA sequence resulting from splicing of the target pre-mRNA sequence. In some embodiments, the target mRNA does not encode a functional protein. In some embodiments, the target mRNA retains one or more intron sequences.

The "target gene" of the present disclosure refers to the gene that encodes the target pre-mRNA.

The "target protein" refers to the amino acid sequence encoded by the target mRNA. In some embodiments, the target protein may not be a functional protein.

"Wild type target protein" refers to a native, functional protein isomer produced by a wild type, normal, or unmutated version of the target gene. The wild type target protein also refers to the protein resulting from a target pre-mRNA that has been properly spliced.

A "re-spliced target protein", as used herein, refers to the protein encoded by the mRNA resulting from the splicing of the target pre-mRNA to which the AC hybridizes. Re-spliced target protein may be identical to a wild type target protein, may be homologous to a wild type target protein, may be a functional variant of a wild type target protein, or may be an active fragment of a wild type target protein.

As used herein, "functional fragment" or "active fragment" refers to a portion of a eukaryotic wild type target protein that exhibits an activity, such as one or more activities of a full-length wild type target protein, or that possesses another activity. In some embodiments, a re-spliced target protein that shares at least one biological activity of wild type target protein is considered to be an active fragment of the wild type target protein. Activity can be any percentage of activity (*i.e.*, more or less) of the full-length wild type target protein, including but not limited to, about 1% of the activity, about 2%, about 3%, about 4%, about 5%, about 10%, about 20%, about 30%, about 40%, about 50%, about 60%, about 70%, about 80%, about 90%, about 95%, about 96%, about 97%, about 98%, about 99%, about 100%, about 200%, about 300%, about 400%, about 500%, or more (including all values and ranges inbetween these values) activity compared to the wild type target protein. Thus, in some embodiments, the active fragment may retain at least a portion of one or more biological activities of wild type target protein. In other embodiments, the active fragment may enhance one or more biological activities of wild type target protein.

As used herein, the term "nucleoside" means a glycosylamine comprising a nucleobase and a sugar. Nucleosides includes, but are not limited to, natural nucleosides, abasic nucleosides, modified nucleosides, and nucleosides having mimetic bases and/or sugar groups. A "natural nucleoside" or "unmodified nucleoside" is a nucleoside comprising a natural nucleobase and a natural sugar. Natural nucleosides include RNA and DNA nucleosides.

As used herein, the term "natural sugar" refers to a sugar of a nucleoside that is unmodified from its naturally occurring form in RNA (2'-OH) or DNA (2'-H).

As used herein, the term "nucleotide" refers to a nucleoside having a phosphate group covalently linked to the sugar. Nucleotides may be modified with any of a variety of substituents.

As used herein, the term "nucleobase" refers to the base portion of a nucleoside or nucleotide. A nucleobase may comprise any atom or group of atoms capable of hydrogen bonding to a base of another nucleic acid. A natural nucleobase is a nucleobase that is unmodified from its naturally occurring form in RNA or DNA.

As used herein, the term "heterocyclic base moiety" refers to a nucleobase comprising a heterocycle.

As used herein "oligonucleoside" refers to an oligonucleotide in which the internucleoside linkages do not contain a phosphorus atom.

As used herein, the term "oligonucleotide" refers to an oligomeric compound comprising a plurality of linked nucleotides or nucleosides. In certain embodiment, one or more nucleotides of an oligonucleotide is modified. In some embodiments, an oligonucleotide comprises ribonucleic acid (RNA) or deoxyribonucleic acid (DNA). In some embodiments, oligonucleotides are composed of natural and/or modified nucleobases, sugars and covalent internucleoside linkages, and may further include non-nucleic acid conjugates.

As used herein "internucleoside linkage" refers to a covalent linkage between adjacent nucleosides.

As used herein "natural internucleotide linkage" refers to a 3' to 5' phosphodiester linkage.

As used herein, the term "modified internucleoside linkage" refers to any linkage between nucleosides or nucleotides other than a naturally occurring internucleoside linkage.

As used herein the term "chimeric antisense compound" refers to an antisense compound, having at least one sugar, nucleobase and/or internucleoside linkage that is differentially modified as compared to the other sugars, nucleobases and internucleoside linkages within the same oligomeric compound. The remainder of the sugars, nucleobases and internucleoside linkages can be independently modified or unmodified. In general a chimeric oligomeric compound will have modified nucleosides that can be in isolated positions or grouped together in regions that will define a particular motif. Any combination of modifications and or mimetic groups can comprise a chimeric oligomeric compound as described herein.

As used herein, the term "mixed-backbone antisense oligonucleotide" refers to an antisense oligonucleotide wherein at least one internucleoside linkage of the antisense oligonucleotide is different from at least one other internucleotide linkage of the antisense oligonucleotide.

As used herein, the term "nucleobase complementarity" refers to a nucleobase that is capable of base pairing with another nucleobase. For example, in DNA, adenine (A) is complementary to thymine (T). For example, in RNA, adenine (A) is complementary to uracil (U). In some embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

As used herein, the term "non-complementary nucleobase" refers to a pair of nucleobases that do not form hydrogen bonds with one another or otherwise support hybridization.

As used herein, the term "complementary" refers to the capacity of an oligomeric compound to hybridize to another oligomeric compound or nucleic acid through nucleobase complementarity. In some embodiments, an antisense compound and its target are complementary to each other when a sufficient number of corresponding positions in each molecule are occupied by nucleobases that can bond with each other to allow stable association between the antisense compound and the target. One skilled in the art recognizes that the inclusion of mismatches is possible without eliminating the ability of the oligomeric compounds to remain in association. Therefore, described herein are antisense compounds that may comprise up to about 20% nucleotides that are mismatched (i.e., are not nucleobase complementary to the corresponding nucleotides of the target). Preferably the antisense compounds contain no more than about 15%, more preferably not more than about 10%, most preferably not more than 5% or no mismatches. The remaining nucleotides are nucleobase complementary or otherwise do not disrupt hybridization (e.g., universal bases). One of ordinary skill in the art would recognize the compounds provided herein are at least 80%, at least 85%, at least 90%, at least 95%, at least 96%, at least 97%, at least 98%, at least 99% or 100% nucleobase complementary to a target nucleic acid.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymidine and uracil which pair through the formation of hydrogen bonds. The natural base guanine is nucleobase complementary to the natural bases cytosine and 5-methyl cytosine. Hybridization can occur under varying circumstances.

As used herein, the term "specifically hybridizes" refers to the ability of an oligomeric compound to hybridize to one nucleic acid site with greater affinity than it hybridizes to another nucleic acid site. In some embodiments, an antisense oligonucleotide specifically hybridizes to more than one target site. In some embodiments, an oligomeric compound specifically hybridizes with its target under stringent hybridization conditions.

As used herein, the term "modulation" refers to a perturbation of function or activity when compared to the level of the function or activity prior to modulation. For example, modulation includes the change, either an increase (stimulation or induction) or a decrease (inhibition or reduction) in gene expression. As further example, modulation of expression can include perturbing splice site selection of pre-mRNA processing.

As used herein, the term "expression" refers to all the functions and steps by which a gene's coded information is converted into structures present and operating in a cell. Such structures include, but are not limited to the products of transcription and translation.

As used herein, the term "2'-modified" or "2'-substituted" means a sugar comprising substituent at the 2' position other than H or OH. 2'-modified monomers, include, but are not limited to, BNA's and monomers (e.g., nucleosides and nucleotides) with 2'- substituents, such as allyl, amino, azido, thio, O-allyl, O-C1-C10 alkyl, -OCF3, O-(CH2)2-O-CH3, 2'-O(CH2)2SCH3, O-(CH2)2-O-N(Rm)(Rn), or O-CH2-C(=O)-N(Rm)(Rn), where each Rm and Rn is, independently, H or substituted or unsubstituted C1-C10 alkyl.

As used herein, the term "MOE" refers to a 2'-O-methoxyethyl substituent.

As used herein, the term "high-affinity modified nucleotide" refers to a nucleotide having at least one modified nucleobase, internucleoside linkage or sugar moiety, such that the modification increases the affinity of an antisense compound comprising the modified nucleotide to a target nucleic acid. High-affinity modifications include, but are not limited to, BNAs, LNAs and 2'-MOE.

As used herein the term "mimetic" refers to groups that are substituted for a sugar, a nucleobase, and/ or internucleoside linkage in an AC. Generally, a mimetic is used in place of the sugar or sugar-internucleoside linkage combination, and the nucleobase is maintained for hybridization to a selected target. Representative examples of a sugar mimetic include, but are not limited to, cyclohexenyl or morpholino. Representative examples of a mimetic for a sugar-internucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14, incorporated herein by reference). Methods of synthesis of sugar, nucleoside and nucleobase mimetics are well known to those skilled in the art.

As used herein, the term "bicyclic nucleoside" or "BNA" refers to a nucleoside wherein the furanose portion of the nucleoside includes a bridge connecting two atoms on the furanose ring, thereby forming a bicyclic ring system. BNAs include, but are not limited to, α-L-LNA, β-D-LNA, ENA, Oxyamino BNA (2'-O-N(CH3)-CH2-4') and Aminooxy BNA (2'-N(CH3)-O-CH2-4').

As used herein, the term "4' to 2' bicyclic nucleoside" refers to a BNA wherin the bridge connecting two atoms of the furanose ring bridges the 4' carbon atom and the 2' carbon atom of the furanose ring, thereby forming a bicyclic ring system.

As used herein, a "locked nucleic acid" or "LNA" refers to a nucleotide modified such that the 2'-hydroxyl group of the ribosyl sugar ring is linked to the 4' carbon atom of the sugar ring via a methylene groups, thereby forming a 2'-C,4'-C-oxymethylene linkage. LNAs include, but are not limited to, α-L-LNA, and β-D-LNA.

As used herein, the term "cap structure" or "terminal cap moiety" refers to chemical modifications, which have been incorporated at either end of an AC.

As used herein, the term "parenteral administration," refers to administration through injection or infusion. Parenteral administration includes, but is not limited to, subcutaneous administration, intravenous administration, or intramuscular administration.

As used herein, the term "subcutaneous administration" refers to administration just below the skin. "Intravenous administration" means administration into a vein.

As used herein, the term "dose" refers to a specified quantity of a pharmaceutical agent provided in a single administration. In some embodiments, a dose may be administered in two or more boluses, tablets, or injections. For example, In some embodiments, where subcutaneous administration is desired, the desired dose requires a volume not easily accommodated by a single injection. In such embodiments, two or more injections may be used to achieve the desired dose. In some embodiments, a dose may be administered in two or more injections to minimize injection site reaction in an individual.

As used herein, the term "dosage unit" refers to a form in which a pharmaceutical agent is provided. In some embodiments, a dosage unit is a vial comprising lyophilized antisense oligonucleotide. In some embodiments, a dosage unit is a vial comprising reconstituted antisense oligonucleotide.

Below is a list of abbrevations found herein: TFP refers to tetrafluorophenyl; Dap refers to diammonium phosphate; BCN refers to Nbenzyloxycarbonyloxy-5-norbornene-2,3-dicarboximide; PYAOP is tripyrrolidinophosphonium hexafluorophosphate; DIPEA is N,N-Diisopropylethylamine. Pip6a refers to a peptide with a sequence of:

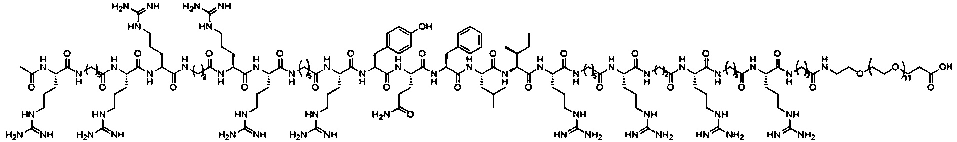

### Compounds

Disclosed herein, in various embodiments, are compounds for treating disease. The compounds described herein comprise a cell-penetrating peptide and an antisense compound (AC). The compounds are designed to deliver an antisense compound (AC) intracellularly to subjects in need thereof. Upon cell entry, the AC binds to the target mRNA or pre-mRNA. By doing so, the compounds disclosed herein reduce or prevent splicing, inhibit or regulate translation, mediate degradation, or block expansions of nucleotide repeats.

When the compounds of the disclosure reduce or prevent splicing, the resulting re-spliced target protein may be more functional than the target protein produced by the splicing of the target pre-mRNA in the absence of the AC. In some embodiments, the re-spliced target protein increases target protein function by about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, about 150%, about 200%, about 250%, about 300%, about 350%, about 400%, about 450%, about 500%, or more, compared to the function of the target protein produced by splicing, inclusive of all values and ranges therebetween. In some embodiments, the re-spliced target protein restores function to about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 100% of the function of a wild type target protein, inclusive of all values and ranges therebetween.

Similarly, the compounds of the disclosure can inhibit or regulate translation, mediate degradation, or block expansions of nucleotide repeats of a target by about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 100% of the function of a wild type target protein, inclusive of all values and ranges therebetween.

In various embodiments, the compounds disclosed herein have an AC moiety and/or CRISPR gene-editing machinery and cell penetrating activity (e.g., a cCPP), such that the compounds are able to traverse the cell membrane and bind to target pre-mRNA *in vivo.* In some embodiments, the compounds comprise: a) at least one CPP moiety; and b) at least one AC and/or at least one CRISPR gene-editing machinery, wherein the CPP is coupled, directly or indirectly, to the AC and/or CRISPR gene-editing machinery. In some embodiments, the compounds comprise 1, 2, 3, 4, 5, 6, 7, 8, 9, 10, or more AC moieties. In some embodiments, the compounds comprise 1, 2, 3, 4, 5, 6, 7, 8, 9, 10, or more CPP moieties. In some embodiments, the compounds comprise 1, 2, 3, 4, 5, 6, 7, 8, 9, 10, or more CRISPR gene-editing machinery. In some embodiments, the compounds comprise one AC moiety. In some embodiments, the compounds comprise two AC moieties. As used herein, "coupled" can refer to a covalent or non-covalent association between the CPP to the AC, including fusion of the CPP to the AC and chemical conjugation of the CPP to the AC. A non-limiting example of a means to non-covalently attach the CPP to the AC is through the streptavidin/biotin interaction, e.g., by conjugating biotin to CPP and fusing AC to streptavidin. In the resulting compound, the CPP is coupled to the AC via non-covalent association between biotin and streptavidin.

In some embodiments, the CPP is conjugated, directly or indirectly, to the AC to thereby form a CPP-AC conjugate. Conjugation of the AC to the CPP may occur at any appropriate site on these moieties. For example, in some embodiments, the 5' or the 3' end of the AC may be conjugated to the C-terminus, the N-terminus, or a side chain of an amino acid in the CPP.

In some embodiments, the AC is covalently linked to the CPP. Covalent linkage, as used herein, refer to constructs where a linear CPP moiety is covalently linked to the 5' and/or 3' end of the AC moiety. Such conjugates may alternatively be described as having a cell penetrating moiety and an oligonucleotide moiety. Methods of covalent linkage are well-known in the art. A covalently-linked AC-CPP conjugate, in accordance with certain embodiments of the disclosure, includes the AC component and the linear CPP component associated with one another by linkers.

In other embodiments, the AC may be chemically conjugated to the CPP through a moiety on the 5' or 3' end of the AC. In still other embodiments, the AC may be conjugated to the CPP through a side chain of an amino acid on the CPP. Any amino acid side chain on the CPP which is capable of forming a covalent bond, or which may be so modified, can be used to link AC to the CPP. The amino acid on the CPP can be a natural or non-natural amino acid. In some embodiments, the amino acid on the CPP used to conjugate the AC is aspartic acid, glutamic acid, glutamine, asparagine, lysine, ornithine, 2,3-diaminopropionic acid, or analogs thereof, wherein the side chain is substituted with a bond to the AC or linker. In particular embodiments, the amino acid is lysine, or an analog thereof. In other embodiments, the amino acid is glutamic acid, or an analog thereof. In further embodiments, the amino acid is aspartic acid, or an analog thereof.

In some embodiments of the present disclosure, the compounds further comprise a linker (L), which conjugates the CPP to AC. In some embodiments, L conjugates the CPP to the 5' or the 3' end of the AC. In some embodiments of the present disclosure, the compounds further comprise a linker (L), which conjugates a CPP to CRISPR gene-editing machinery. In some embodiments, L conjugates the CPP to the gRNA. In some embodiments, L conjugates the CPP to the nuclease.

In some embodiments, compounds comprising an AC moiety and CPP comprise a nuclear localization sequence (NLS). In some embodiments, the NLS is coupled to the AC. In some embodiments, the NLS is coupled to the CPP. In some embodiments, the NLS is coupled to the AC and the CPP. Coupling between the NLS, AC, CPP, or combinations thereof, may be non-covalent or covalent. In some embodiments, the NLS is attached through a peptide bond to the N-terminus of the CPP. In some embodiments, the NLS is attached through a peptide bond to the C-terminus of the CPP. In some embodiments, the NLS is attached to the CPP through a side chain of an amino acid in the CPP. In some embodiments, the NLS is attached to the CPP through a side chain of a lysine which is conjugated to the side chain of a glutamine in the CPP. In some embodiments, the NLS is conjugated to the 5' or 3' end of an AC. In some embodiments, the NLS is coupled to a linker. In some embodiments, the NLS is coupled to a linker via the C-terminus of an NLS and a CPP through a side chain on the CPP and/or NLS. For example, an NLS may comprise a terminal lysine which is then coupled to a CPP containing a glutamine through an amide bond. When the NLS contains a terminal lysine, and the side chain of the lysine is used to attach the CPP, the C- or N-terminus may be attached to the linker on the AC.

In some embodiments, the CPP is cyclic (as described herein), and referred to herein as a cCPP. There are numerous possible configurations for the compounds disclosed herein. In some embodiments, the compounds of the disclosure are exocyclic compounds wherein AC is conjugated to the side chain of an amino acid in the cCPP. In some embodiments, the compounds disclosed herein have structure (i.e., exocyclic) according to Formula I-A or Formula I-A1:

CPP-L-AC (I-A)

or

AC-L-CPP (I-B),

wherein L is covalently bound to the side chain of an amino acid on the CPP and to the 5' end of the AC, the backbone of the AC, or the 3' end of the AC.

In some embodiments, the compounds (e.g., exocyclic compounds) disclosed herein have a structure according to Formula I-A:

CPP-L-AC (I-A),

wherein L is covalently bound to the side chain of an amino acid on the CPP and to the 5' or 3' end of the AC.

In some embodiments, the compound of the present disclosure is a compound of Formula (I) having the structure: 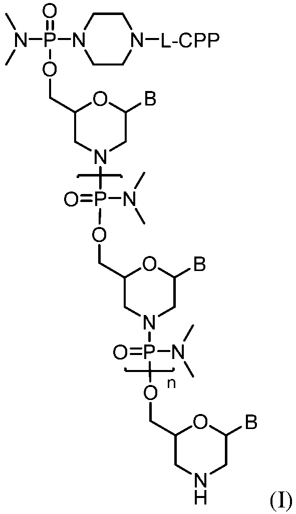 wherein:
B is each independently a nucleobase that is complementary to a base in the target sequence;
n is 1 to 50; and
L is a linker.

In some embodiments, n is an integer from 5 to 50, e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50, inclusive of all subranges therebetween. In some embodiments, n is an integer from 1 to 40. In some embodiments, n is an integer from 1 to 30. In some embodiments, n is an integer from 1 to 20. In some embodiments, n is an integer from 1 to 10.

L may be any appropriate moiety which conjugates CPP (e.g., as described herein) to a AC moiety. Thus, prior to conjugation to the CPP and AC, the linker has two or more functional groups, each of which are independently capable of forming a covalent bond to the CPP moiety and the AC moiety. In various embodiments of the present disclosure, L is covalently bound to the 5' end of the AC or the 3' end of the AC. In some embodiments, L is covalently bound to the 5' end of the AC. In other embodiments, L is covalently bound to the 3' end of the AC. In still other embodiments, L is covalently bound to the backbone of the AC.

L may be any appropriate moiety which conjugates CPP (e.g., as described herein) to an oligonucleotide. Thus, prior to conjugation to the CPP and the AC, the linker has two or more functional groups, each of which are independently capable of forming a covalent bond to the CPP moiety and the AC. In various embodiments of the present disclosure, L is covalently bound to a nucleophilic moiety on the AC. In some embodiments, the nucleophilic moiety is conjugated to the AC so that the AC can be attached to the CPP through L. In some embodiments, L is covalently bound to a piperazine moiety on the AC. In some embodiments, L is covalently bound to a side chain or terminus of an amino acid on the CPP. In certain embodiments, L is covalently bound to the side chain of an amino acid on the CPP.

In various embodiments of the present disclosure, L comprises (i) one or more D or L amino acids, each of which is optionally substituted; (ii) alkylene, alkenylene, alkynylene, carbocyclyl, or heterocyclyl, each of which is optionally substituted; or (iii) -(R¹-X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is H, alkyl, alkenyl, alkynyl, carbocyclyl, or heterocyclyl, each of which is optionally substituted, and z is an integer from 1 to 50; or (iv) combinations thereof. In some embodiments, L comprises one or more D or L amino acids, each of which is optionally substituted. In other embodiments, L comprises alkylene, alkenylene, alkynylene, carbocyclyl, or heterocyclyl, each of which is optionally substituted. In still other embodiments, L comprises -(R¹-X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is H, alkyl, alkenyl, alkynyl, carbocyclyl, or heterocyclyl, each of which is optionally substituted, and z is an integer from 1 to 50; or combinations thereof. In certain embodiments, L is an ether, which is optionally substituted. In more specific embodiments, L comprises -(CH₂-O-CH₂)z-, wherein Z is an integer from 1-50. In more specific embodiments, L comprises -(CH₂-O-CH₂)z-, wherein Z is an integer from 1-25 (e.g., 12), and one or more D or L amino acids, such as and lysine. For example, in various embodiments, L comprises a polyethylene glycol moiety, having from 1 to 50 ethylene glycol units, and a lysine residue. In other specific embodiments, L comprises -(CH₂-S-CH₂)z-, wherein z is an integer from 1-50. In still other specific embodiments, L comprises -(CH₂-NR³-CH₂)z-, wherein R³ is H, -C(O), alkyl, alkenyl, alkynyl, carbocyclyl, or heterocyclyl, each of which is optionally substituted, and z is an integer from 1-50, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50, inclusive of all subranges therebetween. In some embodiments, z is an integer from 10-15. In a specific embodiment, z is 12.

In some embodiments, the CPP is attached to the AC through a linker ("L"). In some embodiments, the linker is conjugated to the AC through a bonding group ("M").

As discussed above, L or M may be covalently bound to AC at any suitable location on AC. In various embodiments of the present disclosure, L or M is covalently bound to the 3' end of AC or the 5' end of AC. In some embodiments, L or M is covalently bound to the backbone of AC.

In some embodiments, L is bound to the side chain of aspartic acid, glutamic acid, glutamine, asparagine, or lysine, or a modified side chain of glutamine or asparagine (e.g., a reduced side chain having an amino group), on the CPP. In particular embodiments, the L is bound to the side chain of lysine on the CPP.

In some embodiments, L has a structure according to Formula (II): 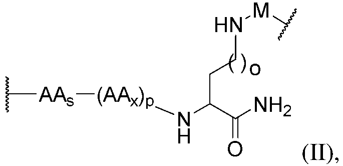 wherein
M is a group that conjugates L to an oligonucleotide;
AAₛ is a side chain or terminus of an amino acid on the CPP;
AAₓ is an amino acid;
o is an integer from 0 to 10; and
p is an integer from 0 to 5.

In some embodiments, L has a structure according to Formula (III): 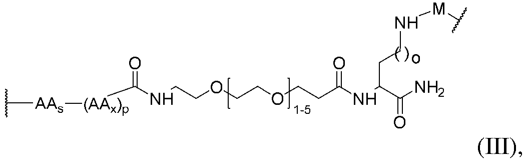 wherein
M is a group that conjugates L to an oligonucleotide;
AAₛ is a side chain or terminus of an amino acid on the CPP;
AAₓ is an amino acid;
o is an integer from 0 to 10; and
p is an integer from 0 to 5.

L or M may be covalently bound to the AC at any suitable location on the AC (e.g., the 3' or 5' end). In various embodiments of the present disclosure, M is covalently bound to a nucleophilic moiety on the AC. In some embodiments, the nucleophilic moiety is a nitrogen-containing moiety. In some embodiments, M is covalently bound to a piperazine moiety of the AC.

In some embodiments of Formula (II), M comprises an alkylene, alkenylene, alkynylene, carbocyclyl, or heterocyclyl, each of which is optionally substituted. In some embodiments, M is selected from the group consisting of: 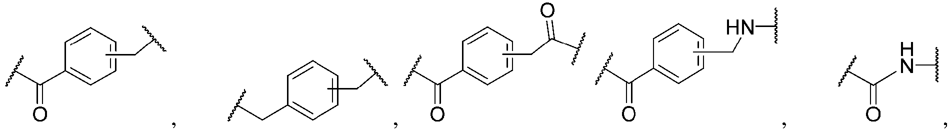 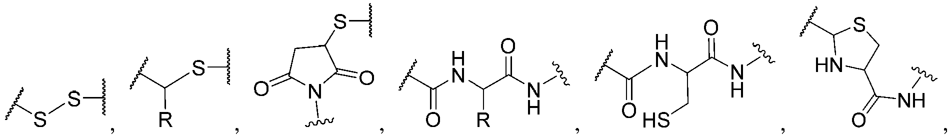 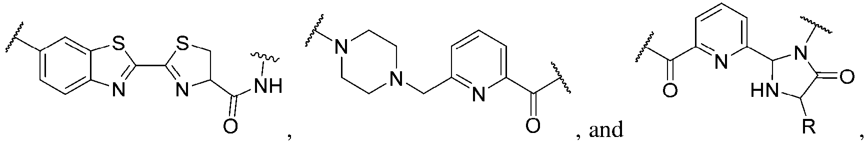 wherein R is alkyl, alkenyl, alkynyl, carbocyclyl, or heterocyclyl.

In some embodiments, M is selected from the group consisting of: 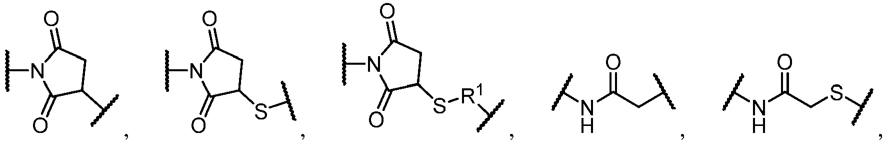 and 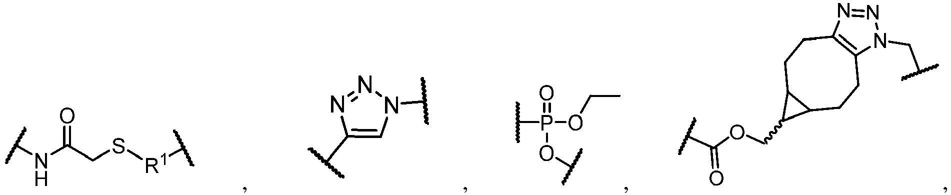 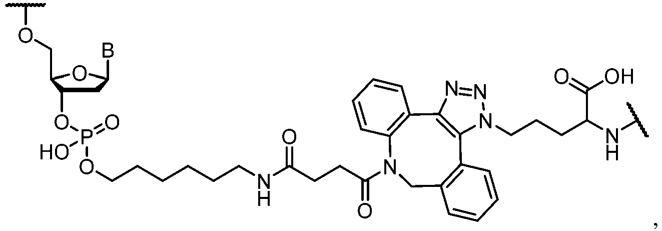 and 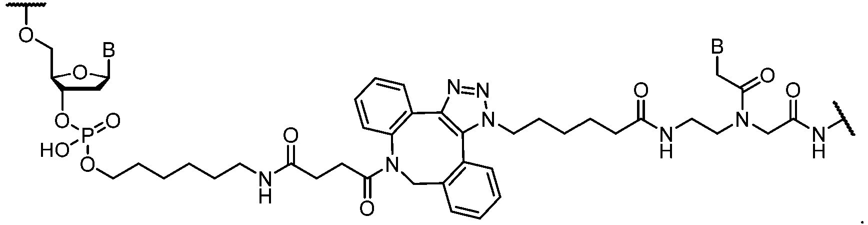 wherein: R¹ is alkylene, cycloalkyl, or 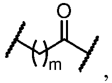 wherein m is 0 to 10. In some embodiments, M is 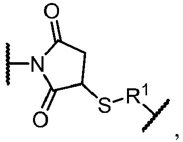 R¹ is 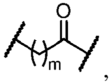 and m is 0 to 10.

In some embodiments, M is a heterobifunctional crosslinker, e.g., 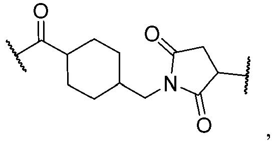 which is disclosed in Williams et al. *Curr. Protoc Nucleic Acid Chem.* **2010,** *42*, 4.41.1-4.41.20, incorporated herein by reference its entirety.

In some embodiments, m is an integer from 0 to 10, e.g., 0, 1, 2, 3, 4, 5, 6, 7, 8, 9, and 10. In some embodiments, m is an integer from 1 to 5. In some embodiments, m is an integer from 1 to 3. In some embodiments, m is 1. In some embodiments, m is 2. In some embodiments, m is 3. In some embodiments, m is 4. In some embodiments, m is 5.

In some embodiments, AAₛ is a side chain or terminus of an amino acid on the CPP. Non-limiting examples of AAₛ include aspartic acid, glutamic acid, glutamine, asparagine, or lysine, or a modified side chain of glutamine or asparagine (e.g., a reduced side chain having an amino group).

In some embodiments, each AAₓ is independently a natural or non-natural amino acid. In some embodiments, one or more AAₓ is a natural amino acid. In some embodiments, one or more AAₓ is a non-natural amino acid. In some embodiments, one or more AAₓ is a β-amino acid. In some embodiments, the β-amino acid is β-alanine.

In some embodiments, o is an integer from 0 to 10, e.g., 0, 1, 2, 3, 4, 5, 6, 7, 8, 9, and 10. In some embodiments, o is 0, 1, 2, or 3. In some embodiments, o is 0. In other embodiments, o is 1. In still other embodiments, o is 2. In yet another embodiment, o is 3.

In some embodiments, p is 0 to 5, e.g., 0, 1, 2, 3, 4, or 5. In some embodiments, p is 0. In other embodiments, p is 1. In still other embodiments p is 2. In yet other embodiments, p is 3. In another embodiment, p is 4. In still another embodiment, p is 5.

In some embodiments, L has a structure according to Formula II-A or Formula II-B: 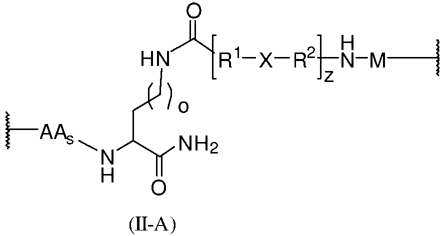 or 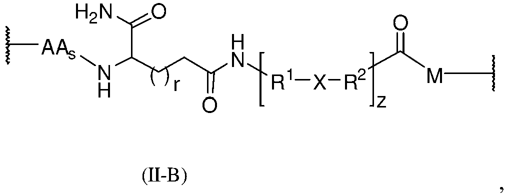 wherein M, AAₛ, each -(R¹-X-R²)z-, and o are defined as above for Formula (II); and r is 0 or 1.

In some embodiments, r is 0. In some embodiments, r is 1.

In some embodiments, each of R¹ and R², at each instance, are independently selected from alkylene, alkenylene, alkynylene, carbocyclyl, and heterocyclyl, each of which is optionally substituted.

In some embodiments, each X is independently NR³, -NR³C(O)-, S, and O, and wherein R³ is independently selected from H, alkyl, alkenyl, alkynyl, carbocyclyl, and heterocyclyl, each of which is optionally substituted.

In some embodiments, L has a structure according to Formula II-A' or II-B': 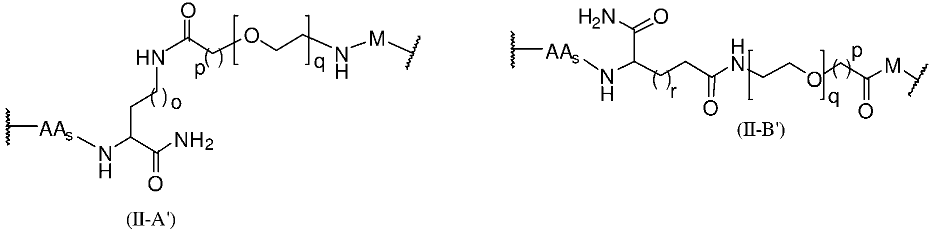 wherein each of M, AAₛ, o, p, and r are defined above.

In some embodiments, q is an integer from 1 to 50, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50, inclusive of all ranges and values therebetween. In other embodiments, q is an integer from 5-20. In other embodiments, q is an integer from 10-15.

In some embodiments, L has a structure according to Formula (IIc): 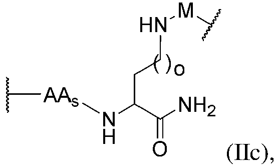 wherein:
M, AAₛ and o are as defined above for Formula I.

Other non-limiting examples of suitable L groups include: 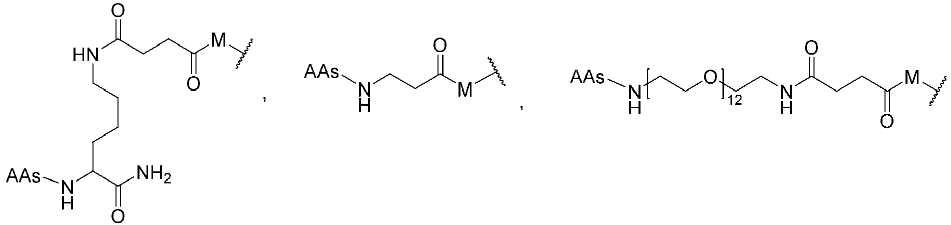 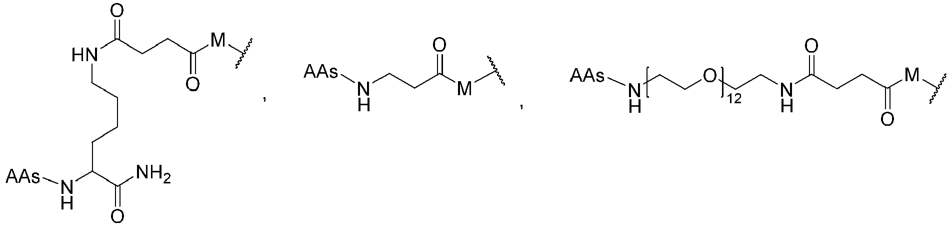 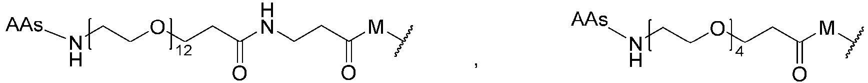 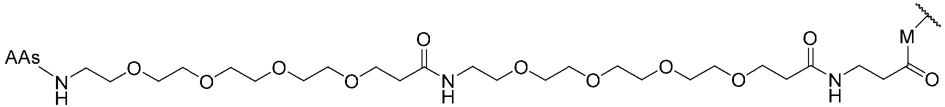 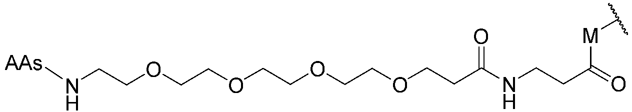 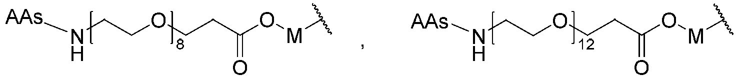 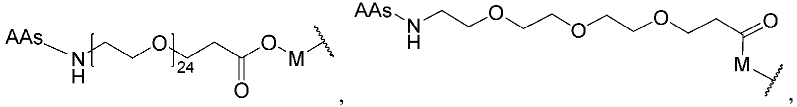 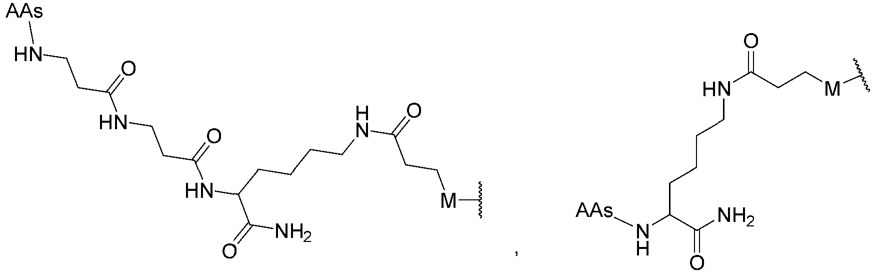 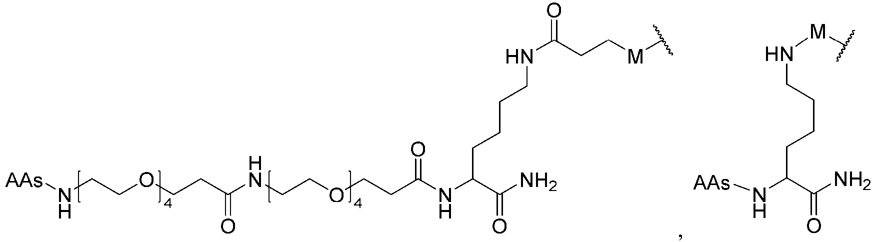 and

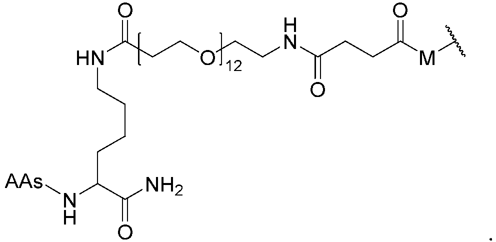

In some embodiments, L and M have the following structure :

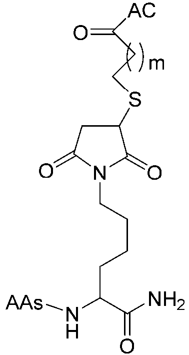

In some embodiments, the present disclosure provides a compound of Formula (Ia) having the structure: 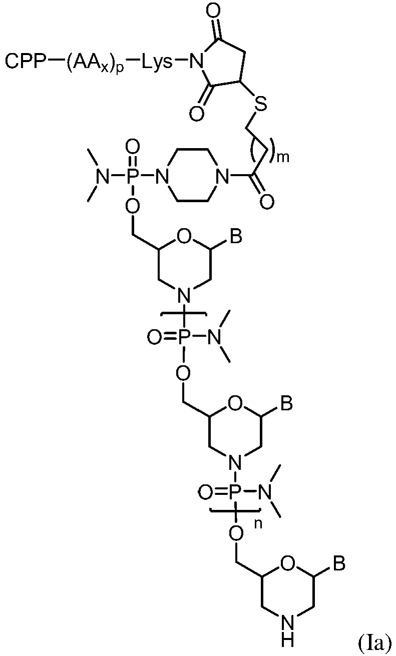 wherein:
m, n, p, AAₓ, and B are as defined above.

In some embodiments, the present disclosure provides a compound of Formula (Ib) having the structure: 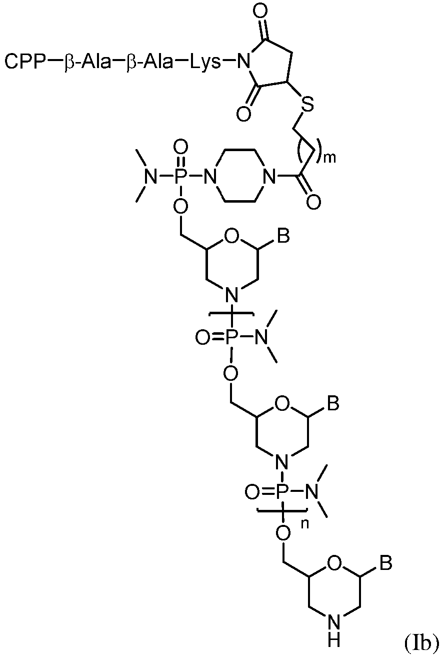 wherein:
m, n, and B are as defined above.

In some embodiments, the present disclosure provides a compound of Formula (Ic) having the structure: 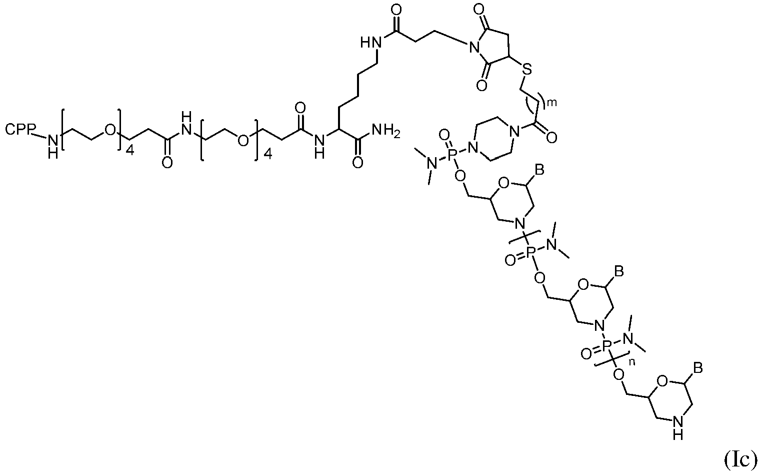 wherein:
m, n, and B are as defined above.

In some embodiments, the L contains a group which may be cleaved after cytosolic uptake of the compounds of the disclosure to release the AC. Non-limiting examples of physiologically cleavable linking group include carbonate, thiocarbonate, thioester, disulfide, sulfoxide, hydrazine, protease-cleavable dipeptide linker, and the like.

In some embodiments, a precursor to L also contains a thiol group, which forms a disulfide bond with the side chain of cysteine or cysteine in the CPP or attached to the 5' or 3' end of the AC.

Accordingly, in various embodiments, the compounds disclosed herein (e.g., the compounds for Formula (I-A) have the following structure:

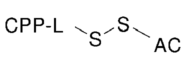

In some embodiments, the disulfide bond is formed between a thiol group on L, and the side chain of cysteine or an amino acid analog having a thiol group on CPP or attached to the 5' or 3' end of the AC. Non-limiting examples of amino acid analogs having a thiol group which can be used with the compounds disclosed herein include: 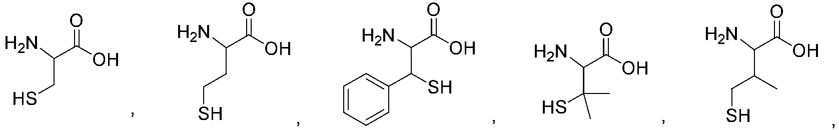 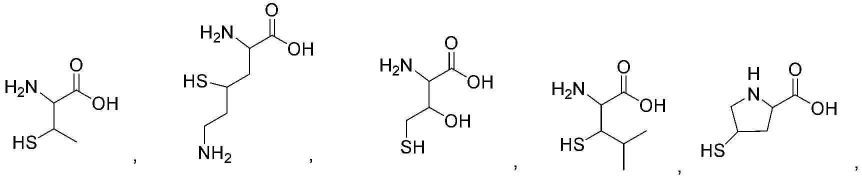 or

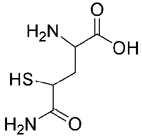

One skilled in the art will recognize that the amino acid analogs depicted above are shown as precursors, i.e., prior to incorporation into the compounds. When incorporated in the compounds of the present disclosure, the N- and C-termini are independently substituted to form peptide bonds, and the hydrogen on the thiol group is replaced with a bond to another sulfur atom to thereby form a disulfide.

Non-limiting examples of unconjugated AC structures (i.e. prior to conjugation to the CPP) are provided below. Underlining represents the antisense oligonucleotide (SEQ ID NO: 217). The antisense oligonucleotide sequences shown below are for illustrative purposes only, and can be substituted for another antisense oligonucleotide sequence depending on the target of interest.

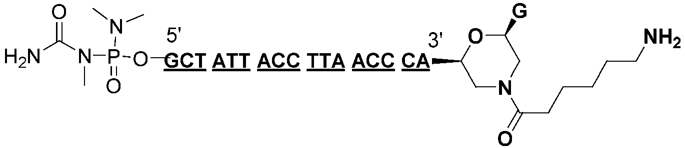

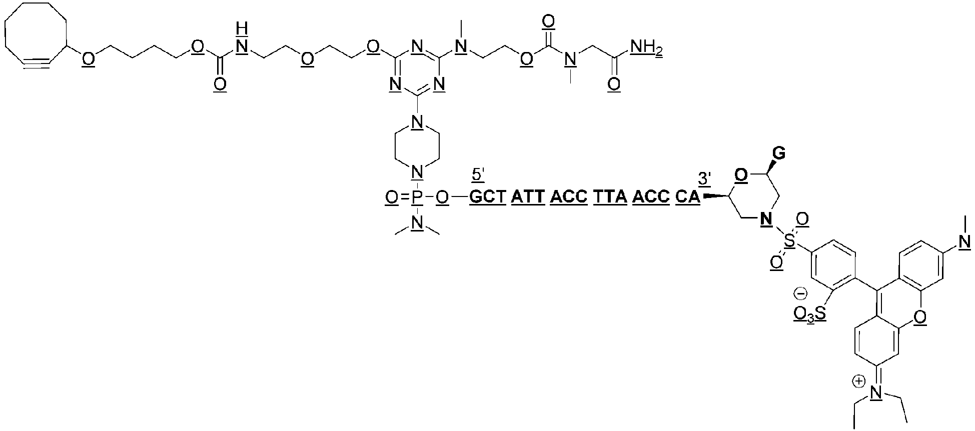

### Cell-Penetrating Peptides

As discussed above, the compounds disclosed herein comprise cell-penetrating peptides (CPPs).

The CPP may be or include any amino sequence which facilitates cellular uptake of the compounds disclosed herein. Suitable CPPs for use in the compounds and methods described herein can include naturally occurring sequences, modified sequences, and synthetic sequences. In embodiments, the total number of amino acids in the CPP may be in the range of from 4 to about 20 amino acids, e.g., about 5, about 6, about 7, about 8, about 9, about 10, about 11, about 12, about 13, about 14, about 15, about 16, about 17, about 18, and about 19 amino acids, inclusive of all ranges and subranges therebetween. In some embodiments, the CPPs disclosed herein comprise about 4 to about to about 13 amino acids. In particular embodiments, the CPPs disclosed herein comprise about 6 to about 10 amino acids, or about 6 to about 8 amino acids.

Each amino acid in the CPP may be a natural or non-natural amino acid. The term "non-natural amino acid" refers to an organic compound that is a congener of a natural amino acid in that it has a structure similar to a natural amino acid so that it mimics the structure and reactivity of a natural amino acid. The non-natural amino acid can be a modified amino acid, and/or amino acid analog, that is not one of the 20 common naturally occurring amino acids or the rare natural amino acids selenocysteine or pyrrolysine. Non-natural amino acids can also be the D-isomer of the natural amino acids. Examples of suitable amino acids include, but are not limited to, alanine, allosoleucine, arginine, asparagine, aspartic acid, cysteine, glutamine, glutamic acid, glycine, histidine, isoleucine, leucine, lysine, methionine, napthylalanine, phenylalanine, proline, pyroglutamic acid, serine, threonine, tryptophan, tyrosine, valine, a derivative, or combinations thereof. These, and others, are listed in the Table 1 along with their abbreviations used herein.

**Table 1. Amino Acid Abbreviations**

| **Amino Acid** | **Abbreviations* L-amino acid** | **Abbreviations* D-amino acid** |
|---|---|---|
| Alanine | Ala (A) | ala (a) |
| Allo-isoleucine | AIle | aile |
| Arginine | Arg (R) | arg (r) |
| Asparagine | Asn (N) | asn (n) |
| aspartic acid | Asp (D) | asp (d) |
| Cysteine | Cys (C) | cys (c) |
| Cyclohexylalanine | Cha | cha |
| 2,3-diaminopropionic acid | Dap | dap |
| 4-fluorophenylalanine | Fpa (∑) | pfa |
| glutamic acid | Glu (E) | glu (e) |
| glutamine | Gln (Q) | gln (q) |
| glycine | Gly (G) | gly (g) |
| histidine | His (H) | his (h) |
| Homoproline (aka pipecolic acid) | Pip (Θ) | pip (θ) |
| isoleucine | Ile (I) | ile (i) |
| leucine | Leu (L) | leu (l) |
| lysine | Lys (K) | lys (k) |
| methionine | Met (M) | met (m) |
| napthylalanine | Nal (Φ) | nal (ϕ) |
| norleucine | Nle (Ω) | nle |
| phenylalanine | Phe (F) | phe (F) |
| phenylglycine | Phg (Ψ) | phg |
| 4-(phosphonodifluoromethyl)phenylalanine | F₂Pmp (A) | f₂pmp |
| proline | Pro (P) | pro (p) |
| sarcosine | Sar (Ξ) | sar |
| selenocysteine | Sec (U) | sec (u) |
| serine | Ser (S) | ser (s) |
| threonine | Thr (T) | thr (y) |
| tyrosine | Tyr (Y) | tyr (y) |
| tryptophan | Trp (W) | trp (w) |
| valine | Val (V) | val (v) |
| Tert-butyl-alanine | Tle | tle |
| Penicillamine | Pen | pen |
| Homoarginine | HomoArg | homoarg |
| Nicotinyl-lysine | Lys(NIC) | lys(NIC) |
| Triflouroacetyl-lysine | Lys(TFA) | lys(TFA) |
| Methyl-leucine | MeLeu | meLeu |
| 3-(3-benzothienyl)-alanine | Bta | bta |

| | | |
|---|---|---|
| * single letter abbreviations: when shown in capital letters herein it indicates the L-amino acid form, when shown in lower case herein it indicates the D-amino acid form. | | |

Non-limiting examples of linear CPPs include Polyarginine (e.g., R₉ or R₁₁), Antennapedia sequences, HIV-TAT, Penetratin, Antp-3A (Antp mutant), Buforin II. Transportan, MAP (model amphipathic peptide), K-FGF, Ku70, Prion, pVEC, Pep-1, SynB1, Pep-7, HN-1, BGSC (Bis-Guanidinium-Spermidine-Cholesterol, and BGTC (Bis-Guanidinium-Tren-Cholesterol).

In various embodiments, the cell-penetrating peptides of the present disclosure are cyclic cell-penetrating peptides (cCPPs). In some embodiment, CPPs are cyclized to form cCPP by forming a peptide bond between the N- and C-termini of two amino acids in a peptide sequence. In some embodiments, the cCPPs may include any combination of at least two arginines and at least two hydrophobic amino acids. In some embodiments, the cCPPs may include any combination of two to three arginines and at least two hydrophobic amino acids.

In some embodiments, the cCPP used in compounds described herein has a structure comprising Formula III: 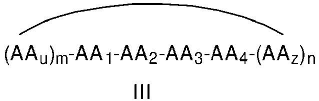 wherein:
each of AA₁, AA₂, AA₃, and AA₄, are independently selected from a D or L amino acid,
each of AAᵤ and AA_{z}, at each instance and when present, are independently selected from a D or L amino acid, and
m and n are independently selected from a number from 0 to 6; and
wherein:
at least two of AAᵤ, when present, AA₁, AA₂, AA₃, AA₄, and AA_{z}, when present, are independently arginine, and
at least two of AAᵤ, when present, AA₁, AA₂, AA₃, AA₄, and AA_{z}, when present, are independently a hydrophobic amino acid.

In some embodiments, each hydrophobic amino acid is independently selected from is independently selected from glycine, alanine, valine, leucine, isoleucine, methionine, phenylalanine, tryptophan, proline, naphthylalanine, phenylglycine, homophenylalanine, tyrosine, cyclohexylalanine, piperidine-2-carboxylic acid, cyclohexylalanine, norleucine, 3-(3-benzothienyl)-alanine, 3-(2-quinolyl)-alanine, O-benzylserine, 3-(4-(benzyloxy)phenyl)-alanine, S-(4-methylbenzyl)cysteine, *N*-(naphthalen-2-yl)glutamine, 3-(1,1'-biphenyl-4-yl)-alanine, tert-leucine, or nicotinoyl lysine, each of which is optionally substituted with one or more substituents. The structures of certain of these non-natural aromatic hydrophobic amino acids (prior to incorporation into the peptides disclosed herein) are provided below. In particular embodiments, each hydrophobic amino acid is independently a hydrophobic aromatic amino acid. In some embodiments, the aromatic hydrophobic amino acid is naphthylalanine, 3-(3-benzothienyl)-alanine, phenylglycine, homophenylalanine, phenylalanine, tryptophan, or tyrosine, each of which is optionally substituted with one or more substituents.

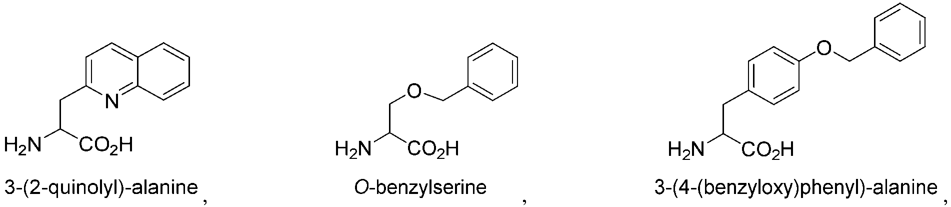

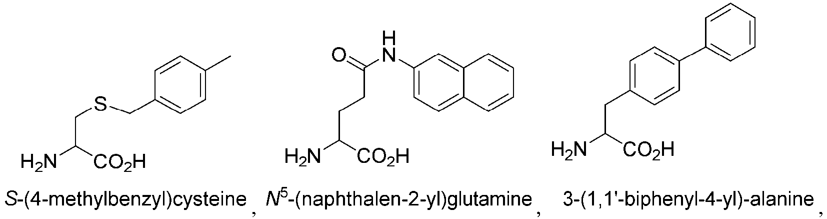

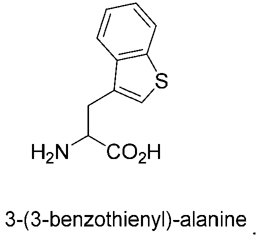

The optional substituent can be any atom or group which does not significantly reduce (e.g., by more than 50%) the cytosolic delivery efficiency of the cCPP, e.g., compared to an otherwise identical sequence which does not have the substituent. In some embodiments, the optional substituent can be a hydrophobic substituent or a hydrophilic substituent. In some embodiments, the optional substituent is a hydrophobic substituent. In some embodiments, the substituent increases the solvent-accessible surface area (as defined herein) of the hydrophobic amino acid. In some embodiments, the substituent can be a halogen, alkyl, alkenyl, alkynyl, cycloalkyl, cycloalkenyl, cycloalkynyl, heterocyclyl, aryl, heteroaryl, alkoxy, aryloxy, acyl, alkylcarbamoyl, alkylcarboxamidyl, alkoxycarbonyl, alkylthio, or arylthio. In some embodiments, the substituent is a halogen.

Amino acids having higher hydrophobicity values can be selected to improve cytosolic delivery efficiency of a cCPP relative to amino acids having a lower hydrophobicity value. In some embodiments, each hydrophobic amino acid independently has a hydrophobicity value which is greater than that of glycine. In other embodiments, each hydrophobic amino acid independently is a hydrophobic amino acid having a hydrophobicity value which is greater than that of alanine. In still other embodiments, each hydrophobic amino acid independently has a hydrophobicity value which is greater or equal to phenylalanine. Hydrophobicity may be measured using hydrophobicity scales known in the art. Table 2 below lists hydrophobicity values for various amino acids as reported by Eisenberg and Weiss (Proc. Natl. Acad. Sci. U. S. A. 1984;81(1):140-144), Engleman, et al. (Ann. Rev. of Biophys. Biophys. Chem.. 1986;1986(15):321-53), Kyte and Doolittle (J. Mol. Biol. 1982;157(1):105-132), Hoop and Woods (Proc. Natl. Acad. Sci. U. S. A. 1981;78(6):3824-3828), and Janin (Nature. 1979;277(5696):491-492), the entirety of each of which is herein incorporated by reference in its entirety. In particular embodiments, hydrophobicity is measured using the hydrophobicity scale reported in Engleman, et al.

**Table 2. Amino Acid Hydrophobicity**

| **Amino Acid** | **Group** | **Eisenberg and Weiss** | **Engleman et al.** | **Kyrie and Doolittle** | **Hoop and Woods** | **Janin** |
|---|---|---|---|---|---|---|
| Ile | Nonpolar | 0.73 | 3.1 | 4.5 | -1.8 | 0.7 |
| Phe | Nonpolar | 0.61 | 3.7 | 2.8 | -2.5 | 0.5 |
| Val | Nonpolar | 0.54 | 2.6 | 4.2 | -1.5 | 0.6 |
| Leu | Nonpolar | 0.53 | 2.8 | 3.8 | -1.8 | 0.5 |
| Trp | Nonpolar | 0.37 | 1.9 | -0.9 | -3.4 | 0.3 |
| Met | Nonpolar | 0.26 | 3.4 | 1.9 | -1.3 | 0.4 |
| Ala | Nonpolar | 0.25 | 1.6 | 1.8 | -0.5 | 0.3 |
| Gly | Nonpolar | 0.16 | 1.0 | -0.4 | 0.0 | 0.3 |
| Cys | Unch/Polar | 0.04 | 2.0 | 2.5 | -1.0 | 0.9 |
| Tyr | Unch/Polar | 0.02 | -0.7 | -1.3 | -2.3 | -0.4 |
| Pro | Nonpolar | -0.07 | -0.2 | -1.6 | 0.0 | -0.3 |
| Thr | Unch/Polar | -0.18 | 1.2 | -0.7 | -0.4 | -0.2 |
| Ser | Unch/Polar | -0.26 | 0.6 | -0.8 | 0.3 | -0.1 |
| His | Charged | -0.40 | -3.0 | -3.2 | -0.5 | -0.1 |
| Glu | Charged | -0.62 | -8.2 | -3.5 | 3.0 | -0.7 |
| Asn | Unch/Polar | -0.64 | -4.8 | -3.5 | 0.2 | -0.5 |
| Gln | Unch/Polar | -0.69 | -4.1 | -3.5 | 0.2 | -0.7 |
| Asp | Charged | -0.72 | -9.2 | -3.5 | 3.0 | -0.6 |
| Lys | Charged | -1.10 | -8.8 | -3.9 | 3.0 | -1.8 |
| Arg | Charged | -1.80 | -12.3 | -4.5 | 3.0 | -1.4 |

The chirality of the amino acids can be selected to improve cytosolic uptake efficiency. In some embodiments, at least two of the amino acids have the opposite chirality. In some embodiments, the at least two amino acids having the opposite chirality can be adjacent to each other. In some embodiments, at least three amino acids have alternating stereochemistry relative to each other. In some embodiments, the at least three amino acids having the alternating chirality relative to each other can be adjacent to each other. In some embodiments, at least two of the amino acids have the same chirality. In some embodiments, the at least two amino acids having the same chirality can be adjacent to each other. In some embodiments, at least two amino acids have the same chirality and at least two amino acids have the opposite chirality. In some embodiments, the at least two amino acids having the opposite chirality can be adjacent to the at least two amino acids having the same chirality. Accordingly, in some embodiments, adjacent amino acids in the cCPP can have any of the following sequences: D-L; L-D; D-L-L-D; L-D-D-L; L-D-L-L-D; D-L-D-D-L; D-L-L-D-L; or L-D-D-L-D.

In some embodiments, an arginine is adjacent to a hydrophobic amino acid. In some embodiments, the arginine has the same chirality as the hydrophobic amino acid. In some embodiments, at least two arginines are adjacent to each other. In still other embodiments, three arginines are adjacent to each other. In some embodiments, at least two hydrophobic amino acids are adjacent to each other. In other embodiments, at least three hydrophobic amino acids are adjacent to each other. In other embodiments, the cCPPs described herein comprise at least two consecutive hydrophobic amino acids and at least two consecutive arginines. In further embodiments, one hydrophobic amino acid is adjacent to one of the arginines. In still other embodiments, the cCPPs described herein comprise at least three consecutive hydrophobic amino acids and there consecutive arginines. In further embodiments, one hydrophobic amino acid is adjacent to one of the arginines. These various combinations of amino acids can have any arrangement of D and L amino acids, e.g., the sequences described above.

In some embodiments, any four adjacent amino acids in the cCPPs described herein (e.g., the cCPPs according to Formula 2) can have one of the following sequences: AA_{H2}-AA_{H1}-R-r, AA_{H2}-AA_{H1}-r-R, R-r-AA_{H1}-AA_{H2}, or r-R-AA_{H1}-AA_{H2}, wherein each of AA_{H1} and AA_{H2} are independently a hydrophobic amino acid. Accordingly, in some embodiments, the cCPPs used in the compounds described herein comprise a structure according any of Formula IV-A-D: 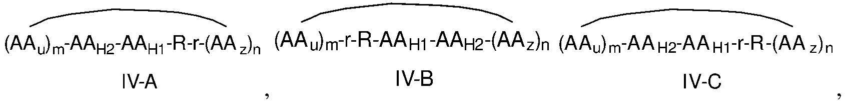 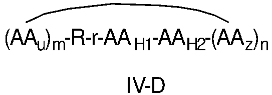 wherein:
each of AA_{H1} and AA_{H2} are independently a hydrophobic amino acid;
at each instance and when present, each of AA_{U} and AA_{Z} are independently any amino acid; and
m and n are independently selected from a number from 0 to 6.

In some embodiments, the total number of amino acids (including r, R, AA_{H1}, AA_{H2}), in the CPPs of Formula 4-A to 4-D are in the range of 6 to 10. In some embodiments, the total number of amino acids is 6. In some embodiments, the total number of amino acids is 7. In some embodiments, the total number of amino acids is 8. In some embodiments, the total number of amino acids is 9. In some embodiments, the total number of amino acids is 10.

In some embodiments, the sum of m and n is from 2 to 6. In some embodiments, the sum of m and n is 2. In some embodiments, the sum of m and n is 3. In some embodiments, the sum of m and n is 4. In some embodiments, the sum of m and n is 5. In some embodiments, the sum of m and n is 6. In some embodiments, m is 0. In some embodiments, m is 1. In some embodiments, m is 2. In some embodiments, m is 3. In some embodiments, m is 4. In some embodiments, m is 5. In some embodiments, m is 6. In some embodiments, n is 0. In some embodiments, n is 1. In some embodiments, n is 2. In some embodiments, n is 3. In some embodiments, n is 4. In some embodiments, n is 5. In some embodiments, n is 6.

In some embodiments, each hydrophobic amino acid is independently selected from glycine, alanine, valine, leucine, isoleucine, methionine, phenylalanine, tryptophan, proline, naphthylalanine, phenylglycine, homophenylalanine, tyrosine, cyclohexylalanine, piperidine-2-carboxylic acid, or norleucine, each of which is optionally substituted with one or more substituents. In particular embodiments, each hydrophobic amino acid is independently a hydrophobic aromatic amino acid. In some embodiments, the aromatic hydrophobic amino acid is piperidine-2-carboxylic acid, naphthylalanine, phenylglycine, homophenylalanine, phenylalanine, tryptophan, or tyrosine, each of which is optionally substituted with one or more substituents. In particular embodiments, the hydrophobic amino acid is piperidine-2-carboxylic acid, naphthylalanine, tryptophan, or phenylalanine, each of which is optionally substituted with one or more substituents.

In some embodiments, each of AA_{H1} and AA_{H2} are independently a hydrophobic amino acid having a hydrophobicity value which is greater than that of glycine. In other embodiments, each of AA_{H1} and AA_{H2} are independently a hydrophobic amino acid having a hydrophobicity value which is greater than that of alanine. In still other embodiments, each of AA_{H1} and AA_{H2} are independently an hydrophobic amino acid having a hydrophobicity value which is greater than that of phenylalanine, e.g., as measured using the hydrophobicity scales described above, including Eisenberg and Weiss (Proc. Natl. Acad. Sci. U. S. A. 1984;81(1):140-144), Engleman, et al. (Ann. Rev. of Biophys. Biophys. Chem. 1986; 1986(15):321-53), Kyte and Doolittle (J. Mol. Biol. 1982;157(1):105-132), Hoop and Woods (Proc. Natl. Acad. Sci. U. S. A. 1981;78(6):3824-3828), and Janin (Nature. 1979;277(5696):491-492), (see Table 1 above). In particular embodiments, hydrophobicity is measured using the hydrophobicity scale reported in Engleman, et al.

The presence of a hydrophobic amino acid on the N- or C-terminal of a D-Arg or L-Arg, or a combination thereof, has also found to improve the cytosolic uptake of the cCPP (and the attached cargo). For example, in some embodiments, the cCPPs disclosed herein may include AA_{H1}-D-Arg or D-Arg-AA_{H1}. In other embodiments, the cCPPs disclosed herein may include AA_{H1}-L-Arg or L-Arg-AA_{H1}.

The size of the hydrophobic amino acid on the N- or C-terminal of the D-Arg or an L-Arg, or a combination thereof (i.e., AA_{H1}), may be selected to improve cytosolic delivery efficiency of the CPP. For example, a larger hydrophobic amino acid on the N- or C-terminal of a D-Arg or L-Arg, or a combination thereof, improves cytosolic delivery efficiency compared to an otherwise identical sequence having a smaller hydrophobic amino acid. The size of the hydrophobic amino acid can be measured in terms of molecular weight of the hydrophobic amino acid, the steric effects of the hydrophobic amino acid, the solvent-accessible surface area (SASA) of the side chain, or combinations thereof. In some embodiments, the size of the hydrophobic amino acid is measured in terms of the molecular weight of the hydrophobic amino acid, and the larger hydrophobic amino acid has a side chain with a molecular weight of at least about 90 g/mol, or at least about 130 g/mol, or at least about 141 g/mol. In other embodiments, the size of the amino acid is measured in terms of the SASA of the hydrophobic side chain, and the larger hydrophobic amino acid has a side chain with a SASA greater than alanine, or greater than glycine. In other embodiments, AA_{H1} has a hydrophobic side chain with a SASA greater than or equal to about piperidine-2-carboxylic acid, greater than or equal to about tryptophan, greater than or equal to about phenylalanine, or equal to or greater than about naphthylalanine. In some embodiments, AA_{H1} has a side chain side with a SASA of at least about 200 Å², at least about 210 Å2, at least about 220 Å², at least about 240 Å², at least about 250 Å², at least about 260 Å², at least about 270 Å², at least about 280 Å², at least about 290 Å², at least about 300 Å², at least about 310 Å², at least about 320 Å²,or at least about 330 Å². In some embodiments, AAH₂ has a side chain side with a SASA of at least about 200 Å², at least about 210 Å2, at least about 220 Å², at least about 240 Å², at least about 250 Å², at least about 260 Å², at least about 270 Å², at least about 280 Å², at least about 290 Å², at least about 300 Å², at least about 310 Å², at least about 320 Å²,or at least about 330 Å². In some embodiments, the side chains of AAH₁ and AAH₂ have a combined SASA of at least about 350 Å², at least about 360 Å², at least about 370 Å², at least about 380 Å₂, at least about 390 Å², at least about 400 Å², at least about 410 Å², at least about 420 Å², at least about 430 Å², at least about 440 Å², at least about 450 Å², at least about 460 Å², at least about 470 Å², at least about 480 Å², at least about 490 Å², greater than about 500 Å², at least about 510 Å², at least about 520 Å², at least about 530 Å², at least about 540 Å², at least about 550 Å², at least about 560 Å², at least about 570 Å², at least about 580 Å², at least about 590 Å², at least about 600 Å², at least about 610 Å², at least about 620 Å², at least about 630 Å², at least about 640 Å², greater than about 650 Å², at least about 660 Å², at least about 670 Å², at least about 680 Å², at least about 690 Å², or at least about 700 Å². In some embodiments, AA_{H2} is a hydrophobic amino acid with a side chain having a SASA that is less than or equal to the SASA of the hydrophobic side chain of AA_{H1}. By way of example, and not by limitation, a cCPP having a Nal-Arg motif exhibits improved cytosolic delivery efficiency compared to an otherwise identical CPP having a Phe-Arg motif; a cCPP having a Phe-Nal-Arg motif exhibits improved cytosolic delivery efficiency compared to an otherwise identical cCPP having a Nal-Phe-Arg motif; and a phe-Nal-Arg motif exhibits improved cytosolic delivery efficiency compared to an otherwise identical cCPP having a nal-Phe-Arg motif.

As used herein, "hydrophobic surface area" or "SASA" refers to the surface area (reported as square Ångstroms; Å²) of an amino acid side chain that is accessible to a solvent. In particular embodiments, SASA is calculated using the 'rolling ball' algorithm developed by Shrake & Rupley (J Mol Biol. 79 (2): 351-71), which is herein incorporated by reference in its entirety for all purposes. This algorithm uses a "sphere" of solvent of a particular radius to probe the surface of the molecule. A typical value of the sphere is 1.4 Å, which approximates to the radius of a water molecule.

SASA values for certain side chains are shown below in Table 3. In some embodiments, the SASA values described herein are based on the theoretical values listed in Table 3 below, as reported by Tien, et al. (PLOS ONE 8(11): e80635. https://doi.org/10.1371/journal.pone.0080635, which is herein incorporated by reference in its entirety for all purposes.

**Table 3. Amino Acid SASA Values**

| **Residue** | **Theoretical** | **Empirical** | **Miller *et al.* (1987)** | **Rose *et al.* (1985)** |
|---|---|---|---|---|
| Alanine | 129.0 | 121.0 | 113.0 | 118.1 |
| Arginine | 274.0 | 265.0 | 241.0 | 256.0 |
| Asparagine | 195.0 | 187.0 | 158.0 | 165.5 |
| Aspartate | 193.0 | 187.0 | 151.0 | 158.7 |
| Cysteine | 167.0 | 148.0 | 140.0 | 146.1 |
| Glutamate | 223.0 | 214.0 | 183.0 | 186.2 |
| Glutamine | 225.0 | 214.0 | 189.0 | 193.2 |
| Glycine | 104.0 | 97.0 | 85.0 | 88.1 |
| Histidine | 224.0 | 216.0 | 194.0 | 202.5 |
| Isoleucine | 197.0 | 195.0 | 182.0 | 181.0 |
| Leucine | 201.0 | 191.0 | 180.0 | 193.1 |
| Lysine | 236.0 | 230.0 | 211.0 | 225.8 |
| Methionine | 224.0 | 203.0 | 204.0 | 203.4 |
| Phenylalanine | 240.0 | 228.0 | 218.0 | 222.8 |
| Proline | 159.0 | 154.0 | 143.0 | 146.8 |
| Serine | 155.0 | 143.0 | 122.0 | 129.8 |
| Threonine | 172.0 | 163.0 | 146.0 | 152.5 |
| Tryptophan | 285.0 | 264.0 | 259.0 | 266.3 |
| Tyrosine | 263.0 | 255.0 | 229.0 | 236.8 |
| Valine | 174.0 | 165.0 | 160.0 | 164.5 |

In some embodiments, the cCPP does not include a hydrophobic amino acid on the N- and/or C-terminal of AA_{H2}-AA_{H1}-R-r, AA_{H2}-AA_{H1}-r-R, R-r-AA_{H1}-AA_{H2}, or r-R-AA_{H1}-AA_{H2}. In alternative embodiments, the cCPP does not include a hydrophobic amino acid having a side chain which is larger (as described herein) than at least one of AA_{H1} or AA_{H2}. In further embodiments, the cCPP does not include a hydrophobic amino acid with a side chain having a surface area greater than AA_{H1}. For example, in embodiments in which at least one of AA_{H1} or AA_{H2} is phenylalanine, the cCPP does not further include a naphthylalanine (although the cCPP may include at least one hydrophobic amino acid which is smaller than AA_{H1} and AA_{H2}, e.g., leucine). In still other embodiments, the cCPP does not include a naphthylalanine in addition to the hydrophobic amino acids in AA_{H2}-AA_{H1}-R-r, AA_{H2}-AA_{H1}-r-R, R-r-AA_{H1}-AA_{H2}, or r-R-AA_{H1}-AA_{H2}.

The chirality of the amino acids (i.e., D or L amino acids) can be selected to improve cytosolic delivery efficiency of the cCPP (and the attached cargo as described below). In some embodiments, the hydrophobic amino acid on the N- or C-terminal of an arginine (e.g., AA_{H1}) has the same or opposite chirality as the adjacent arginine. In some embodiments, AA_{H1} has the opposite chirality as the adjacent arginine. For example, when the arginine is D-arg (i.e. "r"), AA_{H1} is a D-AA_{H1}, and when the arginine is L-Arg (i.e., "R"), AA_{H1} is a L-AA_{H1}. Accordingly, in some embodiments, the cCPPs disclosed herein may include at least one of the following motifs: D-AA_{H1}-D-arg, D-arg-D-AA_{H1}, L-AA_{H1}-L-Arg, or L-Arg-LAA_{H1}. In particular embodiments, when arginine is D-arg, AA_{H1} can be D-nal, D-trp, or D-phe. In another non-limiting example, when arginine is L-Arg, AA_{H1} can be L-Nal, L-Trp, or L-Phe.

In some embodiments, the cCPPs described herein include at least three arginines. Accordingly, in some embodiments, the cCPPs described herein include one of the following sequences: AA_{HZ}-AA_{H1}-R-r-R, AA_{HZ}-AA_{H1}-R-r-r, AA_{HZ}-AA_{H1}-r-R-R, AA_{HZ}-AA_{H1}-r-R-r, R-R-r-AA_{H1}-AA_{H2}, r-R-r-AA_{H1}-AA_{H2}, r-r-R-AA_{H1}-AA_{H2}, or, R-r-R-AA_{H1}-AA_{H2}. In particular embodiments, the cCPPs have one of the following sequences AA_{H2}-AA_{H1}-R-r-R, AA_{H2}-AA_{H1}-r-R-r, r-R-r-AA_{H1}-AA_{H2}, or R-r-R-AA_{H1}-AA_{H2}. In some embodiments, the chirality of AAH₁ and AAH₂ can be selected to improve cytosolic uptake efficiency, e.g., as described above, where AAH₁ has the same chirality as the adjacent arginine, and AAH₁ and AAH₂ have the opposite chirality.

In some embodiments, the cCPPs described herein include three hydrophobic amino acids. Accordingly, in some embodiments, the cCPPs described herein include one of the following sequences: AA_{H3}-AA_{H2}-AA_{H1}-R-r, AA_{H3}-AA_{H2}-AA_{H1}-R-r, AA_{H3}-AA_{H2}-AA_{H1}-r-R, AA_{H3}-AA_{H2}-AA_{H1}-r-R, R-r-AA_{H1}-AA_{H2}-AA_{H3}, R-r-AA_{H1}-AA_{H2}-AA_{H3}, r-R-AA_{H1}-AA_{H2}-AA_{H3}, or, r-R-AA_{H1}-AA_{H2}-AA_{H3}, wherein AA_{H3} is any hydrophobic amino acid described above, e.g., piperidine-2-carboxylic acid, naphthylalanine, tryptophan, or phenylalanine. In some embodiments, the chirality of AA_{H1}, AA_{H2}, and AA_{H3} can be selected to improve cytosolic uptake efficiency, e.g., as described above, where AAH₁ has the same chirality as the adjacent arginine, and AA_{H1} and AA_{H2} have the opposite chirality. In other embodiments, the size of AA_{H1}, AA_{H2}, and AA_{H3} can be selected to improve cytosolic uptake efficiency, e.g., as described above, where AA_{H3} has a SAS of less than or equal to AA_{H1} and/or AA_{H2}.

In some embodiments, AA_{H1} and AA_{H2} have the same or opposite chirality. In some embodiments, AA_{H1} and AA_{H2} have the opposite chirality. Accordingly, in some embodiments, the cCPPs disclosed herein include at least one of the following sequences: D-AA_{H2}-L-AA_{H1}-R-r; L-AA_{H2}-D-AA_{H1}-r-R; R-r-D-AA_{H1}-L-AA_{H2}; or r-R- L-AA_{H1}-D-AA_{H1}, wherein each of D-AA_{H1} and D-AA_{H2} is a hydrophobic amino acid having a D configuration, and each of L-AA_{H1} and L-AA_{H2} is a hydrophobic amino acid having an L configuration. In some embodiments, each of D-AA_{H1} and D-AA_{H2} is independently selected from the group consisting of D-pip, D-nal, D-trp, and D-phe. In particular embodiments, D-AA_{H1} or D-AA_{H2} is D-nal. In other particular embodiments, D-AA_{H1} is D-nal. In some embodiments, each of L-AA_{H1} and L-AA_{H2} is independently selected from the group consisting of L-Pip, L-Nal, L-Trp, and L-Phe. In particular embodiments, each of L-AA_{H1} and L-AA_{H2} is L-Nal. In other particular embodiments, L-AA_{H1} is L-Nal.

As discussed above, the disclosure provides for various modifications to a cCPP which may improve cytosolic delivery (also called uptake) efficiency. In some embodiments, improved cytosolic uptake efficiency can be measured by comparing the cytosolic delivery efficiency of the cCPP having the modified sequence to a proper control sequence. In some embodiments, the control sequence does not include a particular modification (e.g., matching chirality of R and AA_{H1}) but is otherwise identical to the modified sequence. In other embodiments, the control has the following sequence: cyclic(FΦRRRRQ) (SEQ ID NO: 146). In some embodiments, improved cytosolic uptake efficiency can be measured by comparing the cytosolic delivery efficiency of a compound described herein having a cCPP to a control compound that does not have a cCPP.

As used herein cytosolic delivery efficiency refers to the ability of a cCPP to traverse a cell membrane and enter the cytosol. In embodiments, cytosolic delivery efficiency of the cCPP is not dependent on a receptor or a cell type. Cytosolic delivery efficiency can refer to absolute cytosolic delivery efficiency or relative cytosolic delivery efficiency.

Absolute cytosolic delivery efficiency is the ratio of cytosolic concentration of a cCPP (or a cCPP-AC conjugate) over the concentration of the CPP (or the CPP-AC conjugate) in the growth medium. Relative cytosolic delivery efficiency refers to the concentration of a cCPP in the cytosol compared to the concentration of a control cCPP in the cytosol. Quantification can be achieved by fluorescently labeling the cCPP (e.g., with a FITC dye) and measuring the fluorescence intensity using techniques well-known in the art.

In particular embodiments, relative cytosolic delivery efficiency is determined by comparing (i) the amount of a compound containing a cCPP and oligonucleotide sequence internalized by a cell type (e.g., HeLa cells) to (ii) the amount of the control cCPP internalized by the same cell type. To measure relative cytosolic delivery efficiency, the cell type may be incubated in the presence of a cCPP for a specified period of time (e.g., 30 minutes, 1 hour, 2 hours, etc.) after which the amount of the cCPP internalized by the cell is quantified using methods known in the art, e.g., fluorescence microscopy. Separately, the same concentration of the control cCPP is incubated in the presence of the cell type over the same period of time, and the amount of the control cCPP internalized by the cell is quantified.

In other embodiments, relative cytosolic delivery efficiency can be determined by measuring the IC₅₀ of a cCPP having a modified sequence for an intracellular target, and comparing the IC₅₀ of the cCPP having the modified sequence to a proper control sequence (as described herein).

In some embodiments, the relative cytosolic delivery efficiency of the cCPP-AC conjugates described herein in the range of from about 1% to about 1000% compared to cCPP, e.g., about 5%, about 10%, about 20%, about 30%, about 40%, about 50%, about 60%, about 70%, about 80%, about 90%, about 100%, about 110%, about 120%, about 130%, about 140%, about 150%, about 160%, about 170%, about 180%, about 190%, about 200%, about 210%, about 220%, about 230%, about 240%, about 250%, about 260%, about 270%, about 280%, about 290%, about 300%, about 310%, about 320%, about 330%, about 340%, about 350%, about 360%, about 370%, about 380%, about 390%, about 400%, about 410%, about 420%, about 430%, about 440%, about 450%, about 460%, about 470%, about 480%, about 490%, about 500%, about 510%, about 520%, about 530%, about 540%, about 550%, about 560%, about 570%, about 580%, about 590%, about 600%, about 610%, about 620%, about 630%, about 640%, about 650%, about 660%, about 670%, about 680%, about 690%, about 700%, about 710%, about 720%, about 730%, about 740%, about 750%, about 760%, about 770%, about 780%, about 790%, about 800%, about 810%, about 820%, about 830%, about 840%, about 850%, about 860%, about 870%, about 880%, about 890%, about 900%, about 910%, about 920%, about 930%, about 940%, about 950%, about 960%, about 970%, about 980%, about 990%, about 1000%, inclusive of all values and subranges therebetween

In other embodiments, the absolute cytosolic delivery efficacy of from about 40% to about 100%, e.g., about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, about 99%, inclusive of all values and subranges therebetween.

In some embodiments, the cCPP may be or include any of the sequences listed in Table 4. That is, the cCPPs used in the compounds disclosed herein may comprise any one of the sequences listed in Table 4, along with additional amino acids to form a cyclic sequence, or the sequences in the Table 4 may be cyclized (via a peptide bond) to form a cCPP. In some embodiments, the amino acids listed in Table 4 further include a glutamine residue or other amino acid that has a side chain that allows for conjugation of the AC.

**Table 4. cCPP sequences**

| **ID** | **Sequence** | **SEQ ID NO** |
|---|---|---|
| **PCT 1** | F**Φ**RRR | 1 |
| **PCT 2** | F**Φ**RRRC | 2 |
| **PCT 3** | F**Φ**RRRU | 3 |
| **PCT 4** | RRR**Φ**F | 4 |
| **PCT 5** | RRRRΦF | 5 |
| **PCT 6** | F**Φ**RRRR | 6 |
| **PCT 7** | FϕrRrR | 7 |
| **PCT 8** | FϕrRrR | 8 |
| **PCT 9** | F**Φ**RRRR | 9 |
| **PCT 10** | f**Φ**RrRr | 10 |
| **PCT 11** | RRFR**Φ**R | 11 |
| **PCT 12** | FRRRR**Φ** | 12 |
| **PCT 13** | rRFR**Φ**R | 13 |
| **PCT 14** | RR**Φ**FRR | 14 |
| **PCT 15** | CRRRRFW | 15 |
| **PCT 16** | Ff**Φ**RrRr | 16 |
| **PCT 17** | FF**Φ**RRRR | 17 |
| **PCT 18** | RFRFR**Φ**R | 18 |
| **PCT 19** | URRRRFW | 19 |
| **PCT 20** | CRRRRFW | 20 |
| **PCT 21** | F**Φ**RRRRQK | 21 |
| **PCT 22** | F**Φ**RRRRQC | 22 |
| **PCT 23** | f**Φ**RrRrRQ | 23 |
| **PCT 24** | F**Φ**RRRRRQ | 24 |
| **PCT 25** | RRRR**Φ**FDΩC | 25 |
| **PCT 26** | F**Φ**RRR | 26 |
| **PCT 27** | FWRRR | 27 |
| **PCT 28** | RRR**Φ**F | 28 |
| **PCT 29** | RRRWF | 29 |
| **SAR 1** | FΦRRRR | 30 |
| **SAR 19** | FFRRR | 31 |
| **SAR 20** | FFrRr | 32 |
| **SAR 21** | FFRrR | 33 |
| **SAR 22** | FRFRR | 34 |
| **SAR 23** | FRRFR | 35 |
| **SAR 24** | FRRRF | 36 |
| **SAR 25** | GΦRRR | 37 |
| **SAR 26** | FFFRA | 38 |
| **SAR 27** | FFFRR | 39 |
| **SAR 28** | FFRRRR | 40 |
| **SAR 29** | FRRFRR | 41 |
| **SAR 30** | FRRRFR | 42 |
| **SAR 31** | RFFRRR | 43 |
| **SAR 32** | RFRRFR | 44 |
| **SAR 33** | FRFRRR | 45 |
| **SAR 34** | FFFRRR | 46 |
| **SAR 35** | FFRRRF | 47 |
| **SAR 36** | FRFFRR | 48 |
| **SAR 37** | RRFFFR | 49 |
| **SAR 38** | FFRFRR | 50 |
| **SAR 39** | FFRRFR | 51 |
| **SAR 40** | FRRFFR | 52 |
| **SAR 41** | FRRFRF | 53 |
| **SAR 42** | FRFRFR | 54 |
| **SAR 43** | RFFRFR | 55 |
| **SAR 44** | GΦRRRR | 56 |
| **SAR 45** | FFFRRRR | 57 |
| **SAR 46** | RFFRRRR | 58 |
| **SAR 47** | RRFFRRR | 59 |
| **SAR 48** | RFFFRRR | 60 |
| **SAR 49** | RRFFFRR | 61 |
| **SAR 50** | FFRRFRR | 62 |
| **SAR 51** | FFRRRRF | 63 |
| **SAR 52** | FRRFFRR | 64 |
| **SAR 53** | FFFRRRRR | 65 |
| **SAR 54** | FFFRRRRRR | 66 |
| **SAR 55** | FΦRrRr | 67 |
| **SAR 56** | XXRRRR | 68 |
| **SAR 57** | FfFRrR | 69 |
| **SAR 58** | fFfrRr | 70 |
| **SAR 59** | fFfRrR | 71 |
| **SAR 60** | FfFrRr | 72 |
| **SAR 61** | fFϕrRr | 73 |
| **SAR 62** | fΦfrRr | 74 |
| **SAR 63** | ϕFfrRr | 75 |
| **SAR 64** | FΦrRr | 76 |
| **SAR 65** | fΦrRr | 77 |
| **SAR 66** | Ac-(Lys-fFRrRrD) | 78 |
| **SAR 67** | Ac-(Dap-fFRrRrD) | 79 |
| **SAR 68** | CWWRRRRC S—S | 80 |
| **SAR 69** | CWWVRRRRC S S | 81 |
| **SAR 70** | 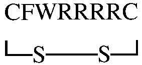 CFWRRRRC S S | 82 |
| **SAR 71** | 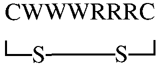 CWWWRRRC S S | 83 |
| **Pin1 15** | Pip-Nal-Arg-Glu-arg-arg-glu | 84 |
| **Pin1 16** | Pip-Nal-Arg-Arg-arg-arg-glu | 85 |
| **Pin1 17** | Pip-Nal-Nal-Arg-arg-arg-glu | 86 |
| **Pin1 18** | Pip-Nal-Nal-Arg-arg-arg-Glu | 87 |
| **Pin1 19** | Pip-Nal-Phe-Arg-arg-arg-glu | 88 |
| **Pin1 20** | Pip-Nal-Phe-Arg-arg-arg- Glu | 89 |
| **Pin1 21** | Pip-Nal-phe-Arg-arg-arg- glu | 90 |
| **Pin1 22** | Pip-Nal-phe-Arg-arg-arg- Glu | 91 |
| **Pin1 23** | Pip-Nal-nal-Arg-arg-arg- Glu | 92 |
| **Pin1 24** | Pip-Nal-nal-Arg-arg-arg- glu | 93 |
| **Rev-13** | [Pim-RQRR-Nlys]GRRR*^{b}* | 94 |
| **hLF** | 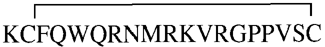 KCFQWQRNMRKVRGPPVSC | 95 |
| **cTat** | [KrRrGrKkRrE]*^{c}* | 96 |
| **cR10** | [KrRrRrRrRrRE]*^{c}* | 97 |
| **L-50** | [RVRTRGKRRIRRpP] | 98 |
| **L-51** | [RTRTRGKRRIRVpP] | 99 |
| **[WR]₄** | [WRWRWRWR] | 100 |
| **MCoTI-II** | 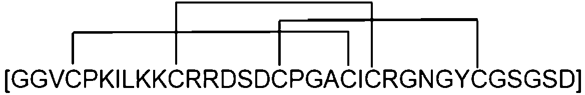 [GGVCPKILKKCRRDSDCPGACICRGNGYCGSGSD] | 101 |
| Rotstein et al. Chem. Eur. J. 2011 | [P-Cha-r-Cha-r-Cha-r-Cha-r-G]*^{d}* | 102 |
| Lian et al. J. Am. Chem. Soc. 2014 | Tm(SvP-F₂Pmp-H)-Dap-(FΦRRRR-Dap)]*^{f}* | 103 |
| Lian et al. J. Am. Chem. Soc. 2014 | [Tm(a-Sar-D-pThr-Pip-ΦRAa)-Dap-(FΦRRRR-Dap)]*^{f}* | 104 |
| **IA8b** | [CRRSRRGCGRRSRRCG]*^{g}* | 105 |
| **Dod-[R₅]** | [K(Dod)RRRR] | 106 |
| **LK-3** | | 107 |
| | RRRR-[KRRRE]*^{c}* | 108 |
| | RRR-[KRRRRE]*^{c}* | 109 |
| | RR-[KRRRRRE)*^{c}* | 110 |
| | R-[KRRRRRRE]*^{c}* | 111 |
| **[CR]₄** | [CRCRCRCR] | 112 |
| **cyc3** | [Pra-LRKRLRKFRN-AzK]*^{h}* | 113 |
| **PMB** | T-Dap-[Dap-Dap-f-L-Dap-Dap-T] | 114 |
| **GPMB** | T-Agp-[Dap-Agp-f-L-Agp-Agp-T] | 115 |
| **cCPP1** | F**Φ**RRRR | 116 |
| **cCPP12** | Ff**Φ**RrRr | 117 |
| **cCPP9** | f**Φ**RrRr | 118 |
| **cCPP11** | f**Φ**RrRrR | 119 |
| **cCPP18** | FϕrRrR | 120 |
| **cCPP13** | FϕrRrR | 121 |
| **cCPP6** | F**Φ**RRRRR | 122 |
| **cCPP3** | RRFR**Φ**RQ | 123 |
| **cCPP7** | FF**Φ**RRRR | 124 |
| **cCPP8** | RFRFR**Φ**R | 125 |
| **cCPP5** | F**Φ**RRR | 126 |
| **cCPP4** | FRRRR**Φ** | 127 |
| **cCPP10** | rRFR**Φ**R | 128 |
| **cCPP2** | RR**Φ**FRR | 129 |
| **cCPP62** | f**Φ**frRr | 130 |

| | | |
|---|---|---|
| Φ, L-2-naphthylalanine; Pim, pimelic acid; Nlys, lysine peptoid residue; D-pThr, D-phosphothreonine; Pip, L-piperidine-2-carboxylic acid; Cha, L-3-cyclohexyl-alanine; Tm, trimesic acid; Dap, L-2,3-diaminopropionic acid; Sar, sarcosine; F₂Pmp, L-difluorophosphonomethyl phenylalanine; Dod, dodecanoyl; Pra, L-propargylglycine; AzK, L-6-Azido-2-amino-hexanoic; Agp, L-2-amino-3-guanidinylpropionic acid; *^{b}*Cyclization between Pim and Nlys; *^{c}*Cyclization between Lys and Glu; *^{d}*Macrocyclization by multicomponent reaction with aziridine aldehyde and isocyanide; *^{e}*Cyclization between the main-chain of Gln residue; *^{f}*N-terminal amine and side chains of two Dap residues bicyclized with Tm; *^{g}*Three Cys side chains bicyclized with tris(bromomethyl)benzene; *^{h}*Cyclization by the click reaction between Pra and Azk. | | |

Additionally, the cCPP used in the compounds and methods described herein can include any sequence disclosed in: U.S. App. No. 15/312,878; U.S. App. No. 15/360,719; International PCT Application Publication No. WO/2018/089648 (including the corresponding US publication), and International PCT Application Publication No. WO 2018/098231, each of which is incorporated by reference in its entirety for all purposes.

In some embodiments, provided herein are ACs conjugated to cCPP12. Non-limiting examples of the structures of ACs conjugated to cCPP12 are provided below. Underlining represents the antisense oligonucleotide (SEQ ID NO: 217). The antisense oligonucleotide sequences shown below are for illustrative purposes only, and can be substituted for another antisense oligonucleotide sequence depending on the target of interest. 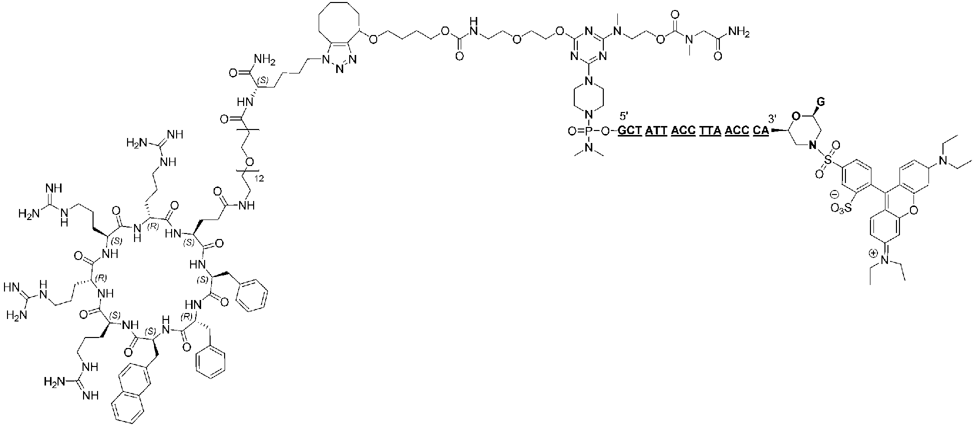 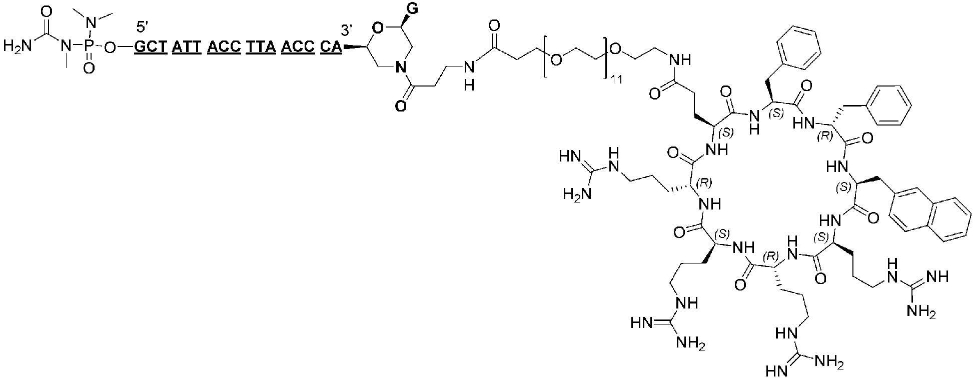 In some embodiments, provided herein are ACs that are conjugated to two CPPs. Non-limiting examples of the structures of ACCs that are conjugated to two CPPs are provided below. Underlining represents the antisense oligonucleotide (SEQ ID NO: 217). The antisense oligonucleotide sequences shown below are for illustrative purposes only, and can be substituted for another antisense oligonucleotide sequence depending on the target of interest. 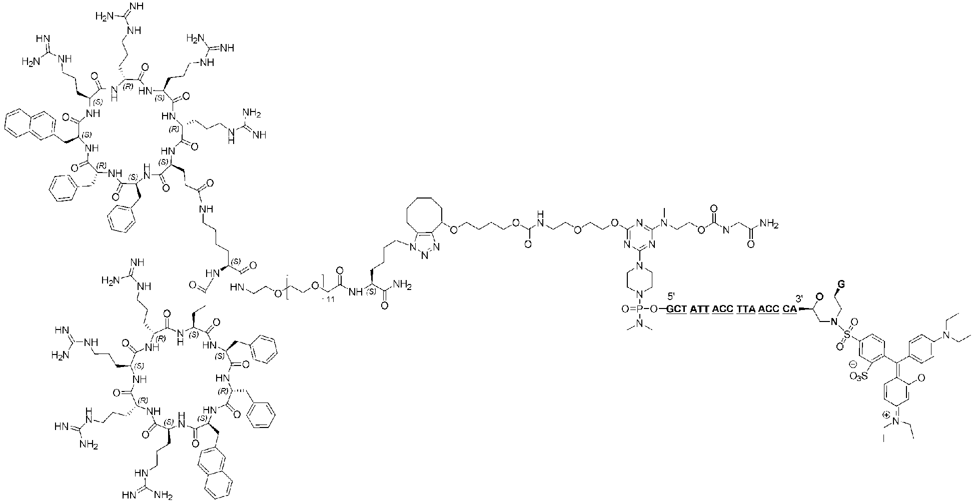 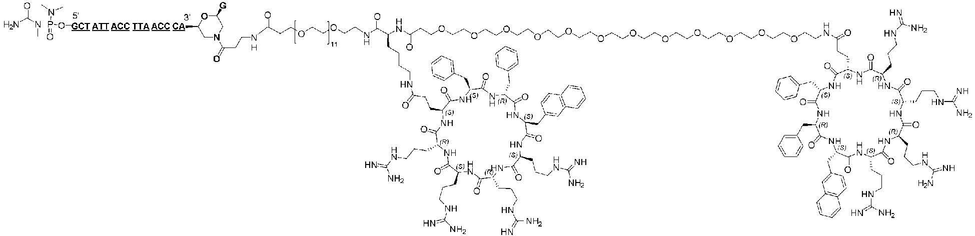 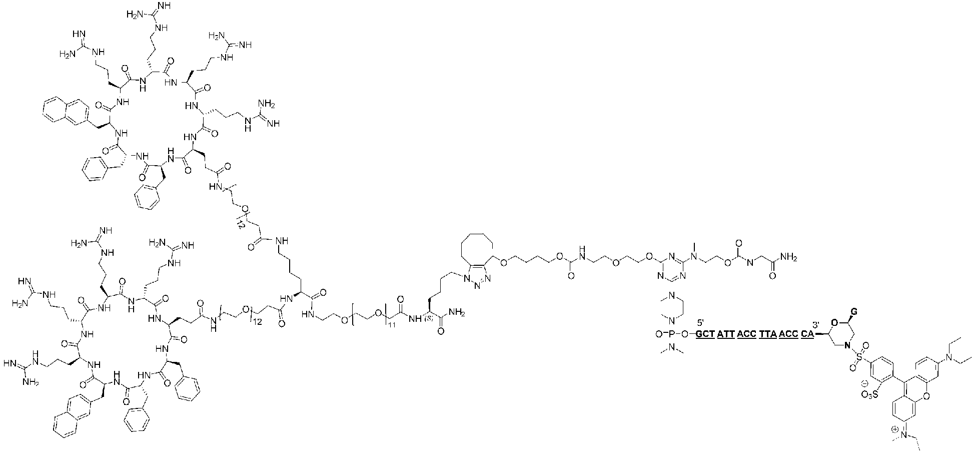 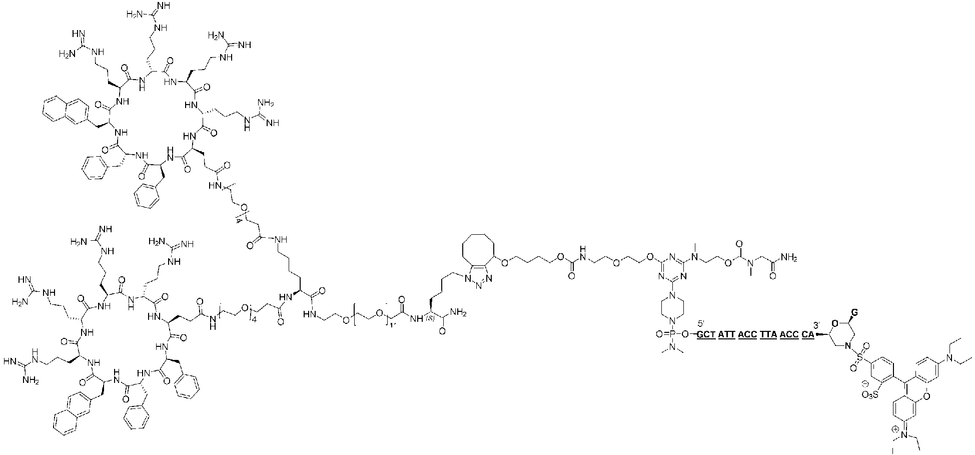 In some embodiments, provided herein are ACs that are conjugated to three CPPs. Non-limiting examples of the structures of ACCs that are conjugated to three CPPs are provided below. Underlining represents the antisense oligonucleotide (SEQ ID NO: 217). The antisense oligonucleotide sequences shown below are for illustrative purposes only, and can be substituted for another antisense oligonucleotide sequence depending on the target of interest. 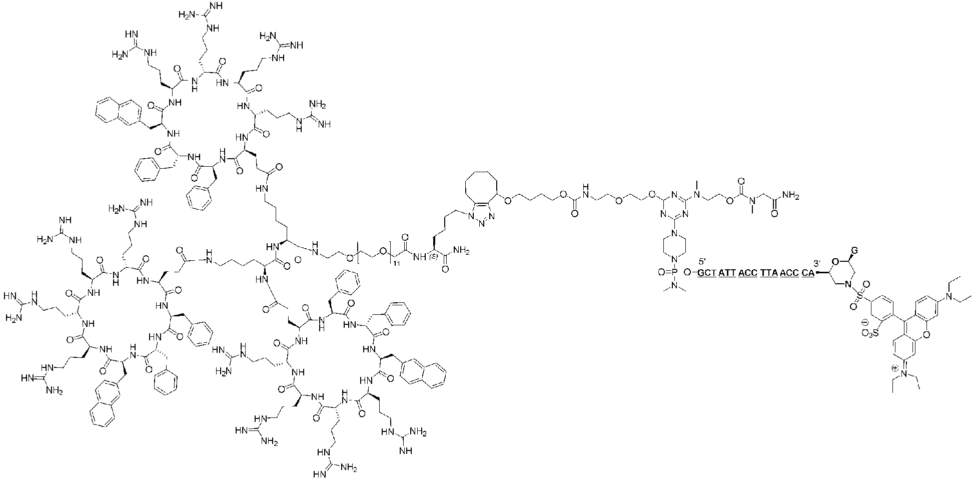 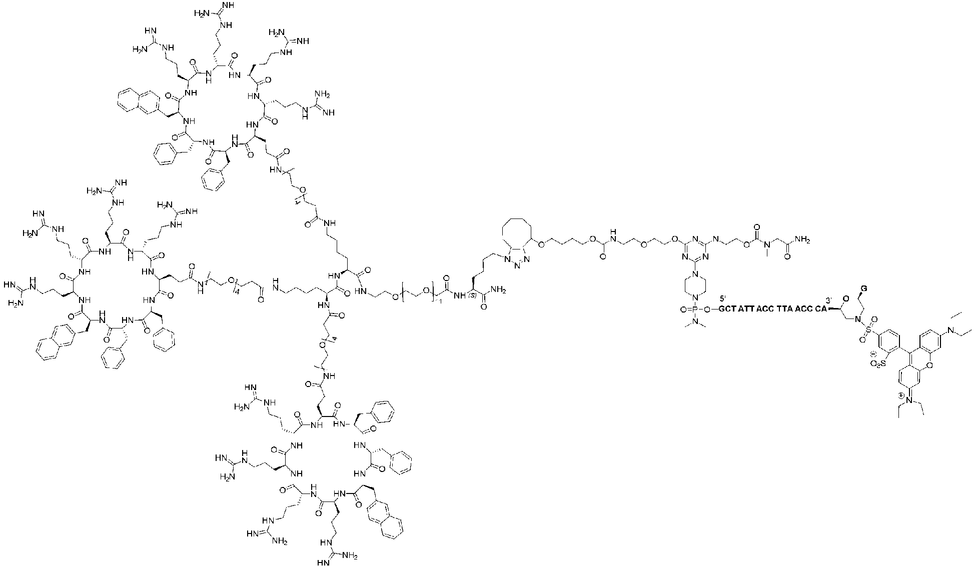 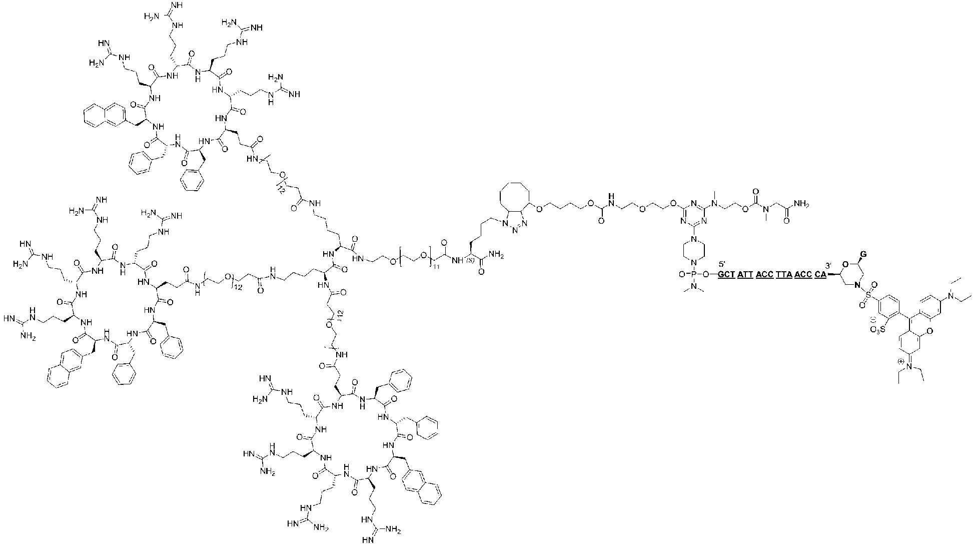 In some embodiments, the AC is independently selected from one of the following structures. Underlining represents the antisense oligonucleotide (SEQ ID NO: 217). The antisense oligonucleotide sequences shown below are for illustrative purposes only, and can be substituted for another antisense oligonucleotide sequence depending on the target of interest.

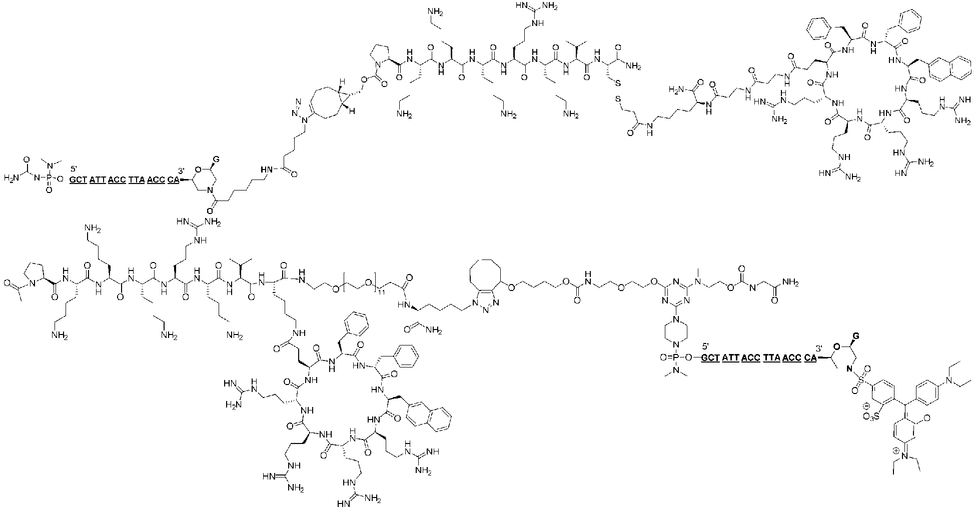

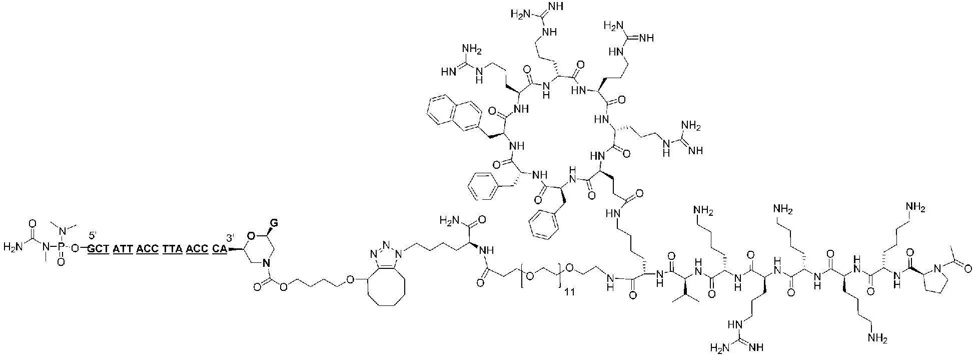

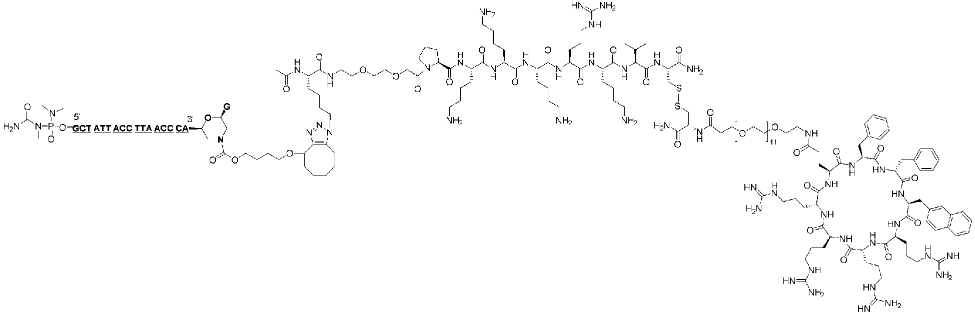

In some embodiments, the compounds of the disclosure have the following structure:

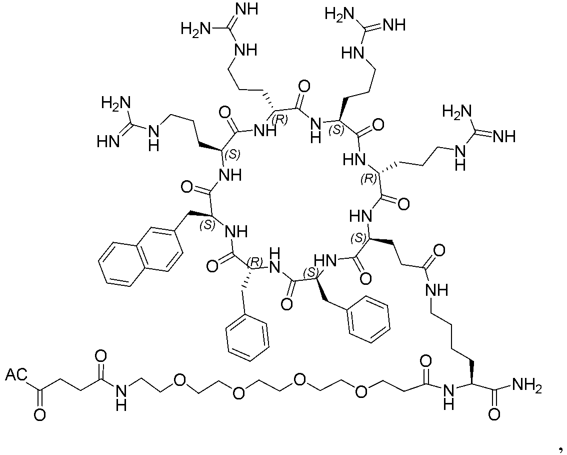

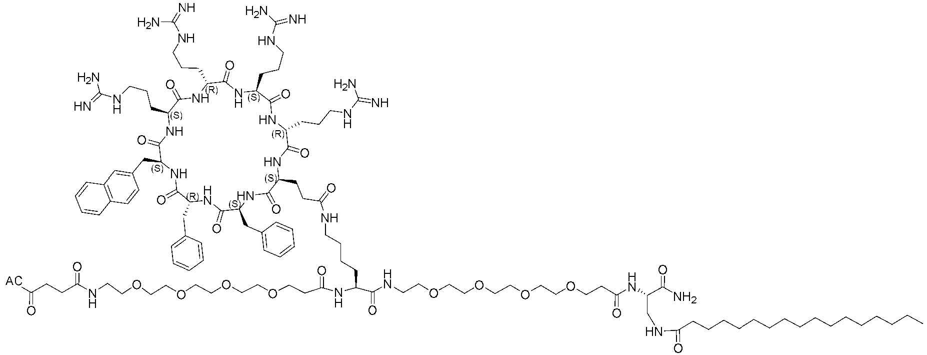

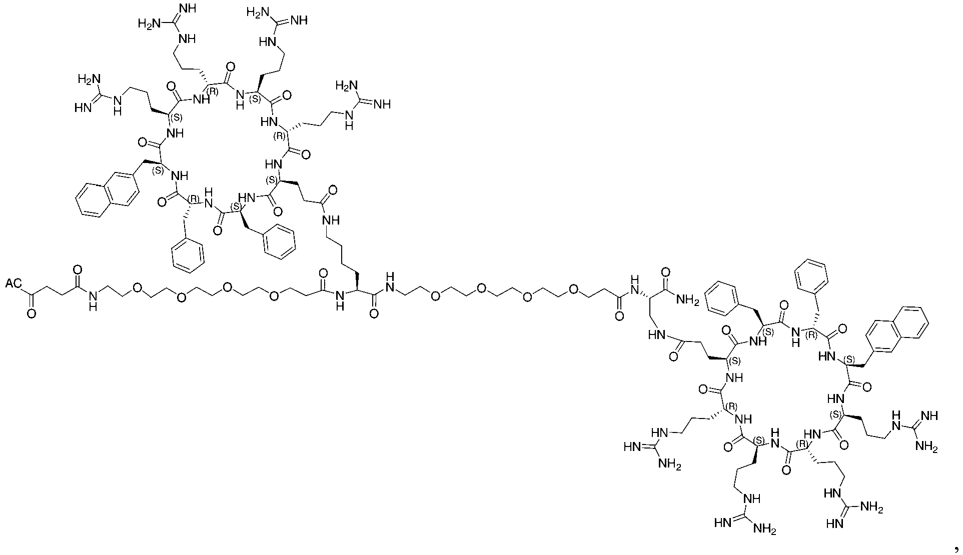

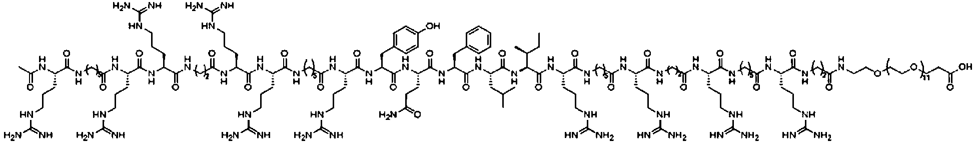

### Oligonucleotides

In various embodiments, the compounds disclosed herein comprise a cell penetrating peptide conjugated to an antisense compound (AC). In some embodiments, the AC comprises an antisense oligonucleotide, siRNA, microRNA, antagomir, aptamer, ribozyme, immunostimulatory oligonucleotide, decoy oligonucleotide, supermir, miRNA mimic, miRNA inhibitor, U1 adaptor, or combinations thereof.

### Antisense Oligonucleotides

In various embodiments, the oligonucleotide moiety of the present invention is an antisense oligonucleotide directed to a target polynucleotide. The term "antisense oligonucleotide" or simply "antisense" is meant to include oligonucleotides that are complementary to a targeted polynucleotide sequence. Antisense oligonucleotides are single strands of DNA or RNA that are complementary to a chosen sequence, e.g. a target gene mRNA.

The antisense oligonucleotides may modulate one or more aspects of protein transcription, translation, and expression. The antisense oligonucleotides described herein modulate aspects of transcription, translation, and expression through various mechanisms as shown in **FIGS. 31** and **32****.**

In some embodiments, antisense oligonucleotides block expansions of nucleotide repeats (e.g., trinucleotide repeat expansions, pentanucleotide repeat expansions, or hexanucleotide repeat expansions). **FIG. 32** shows an exemplary mechanism through which an AC blocks trinucleotide repeats. In some embodiments, the antisense oligonucleotide blocks transcription of the trinucleotide repeat. The following review article describes additional applications for steric blocking antisense oligonucleotides and is incorporated by reference herein in its entirety: Roberts et al. Nature Reviews Drug Discovery (2020) 19: 673-694.

Several diseases are associated with expanded nucleotide repeats, for example, Fragile X mental retardation 1, Friedreich's ataxia (FRDA), Huntington's Disease, myotonic dystrophy type 1 (DM1), myotonic dystrophy type 2 (DM2), spinal and bulbar muscular atrophy, spinal cerebellar ataxia type 1, spinal cerebellar ataxia type 2, and spinal cerebellar ataxia type 3. Table 5 provides examples of nucleotide repeat disorders, and characteristics of genes with expanded nucleotide repeats. The following document describes exemplary oligonucleotides for treating tandem repeat diseases and is incorporated by reference herein in its entirety: Zain et al. Neurotherapeutics. 2019; 16(2): 248-262.

**Table 5. Tandem Repeat Diseases**

| **Disease (abbreviation)** | **Gene** | **Normal repeat length** | **Expanded repeat length** | **Gene product** | **Repeat sequence** | **Location of Repeat** |
|---|---|---|---|---|---|---|
| Fragile X mental retardation 1 (Fragile X) | *FMR1* | 5-55 | > 200 | Fragile X mental retardation protein | CGG•CCG | 5' UTR |
| Friedreich's ataxia (FRDA) | *FXN* | 5-34 | 66-1700 | Frataxin | GAA•CTT | Intron |
| Huntington's Disease (HD) | *HTT* | 6-35 | 36-250 | Huntingtin | CAG•CTG | Exon |
| Myotonic dystrophy type 1 (DM1) | *DMPK* | 5-34 | > 50 | Dystrophia myotonica protein kinase | CTG•CAG | 3' UTR |
| Myotonic dystrophy type 2 (DM2) | *CNBP* | 11-26 | 75-11,000 | Cellular nucleic acid-binding protein | CCTG•CAGG | Intron |
| Spinal and bulbar muscular atrophy (SBMA) | *AR* | 9-34 | 38-68 | Androgen receptor | CAG•CTG | Exon |
| Spinal cerebellar ataxia type 1 (SCA1) | *ATXN1* | 6-44 | 39-82 | Ataxin 1 | CAG•CTG | Exon |
| Spinal cerebellar ataxia type 2 (SCA2) | *ATXN2* | 12-44 | 55-87 | Ataxin 2 | CAG•CTG | Exon |
| Spinal cerebellar ataxia type 3 (SCA3) | *ATXN3* | 12-44 | 55-87 | Ataxin 3 | CAG•CTG | Exon |

In some embodiments, the antisense oligonucleotide is complementary to trinucleotide repeats, such as CAG repeats, CGG repeats, GCC repeats, GAA repeats, or CUG repeats. In some embodiments, the trinucleotide repeat is a CAG repeat. In some embodiments, the target RNA sequence comprises at least 10 trinucleotide repeats (e.g., CAG, CGG, GCC, GAA, or CUG repeats), e.g., at least 5, at least 10, at least 15, at least 20, at least 25, at least 30, at least 35, at least 40, at least 45, at least 50, at least 60, at least 70, at least 80, at least 90, at least 100, at least 150, at least 200, at least 300, at least 400, at least 500, at least 600, at least 700, at least 800, at least 900, at least 1000 at least 2000 trinucleotide repeats. In some embodiments, the AC is complementary sequence comprises to at least 5, at least 10, at least 15, at least 20, at least 25, at least 30, at least 35, at least 40, at least 45, at least 50, at least 60, at least 70, at least 80, at least 90, at least 100, at least 150, at least 200, at least 300, at least 400, at least 500, at least 600, at least 700, at least 800, at least 900, at least 1000 at least 2000 of the trinucleotide repeats in the target mRNA.

In some embodiments, the compounds disclosed herein block at least 1, at least 5, at least 10, at least 15, at least 20, at least 25, at least 30, at least 35, at least 40, at least 45, at least 50, at least 60, at least 70, at least 80, at least 90, at least 100, at least 150, at least 200, at least 300, at least 400, at least 500, at least 600, at least 700, at least 800, at least 900, at least 1000 at least 2000 nucleotide repeats. In some embodiments, the compound disclosed herein prevent translation of at least 1, at least 5, at least 10, at least 15, at least 20, at least 25, at least 30, at least 35, at least 40, at least 45, at least 50, at least 60, at least 70, at least 80, at least 90, at least 100, at least 150, at least 200, at least 300, at least 400, at least 500, at least 600, at least 700, at least 800, at least 900, at least 1000 at least 2000 of the nucleotide repeats in the target mRNA.

In some embodiments, the compounds of the disclosure result in decreased translation of the nucleotide repeats about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the expanded repeat length in the disease state.

Examples of AC sequences for FRDA and DM1 are provided in Table 8. When cells were transfected with these sequences, translation of at least a portion of the expanded repeat was blocked.

In some embodiments, the antisense oligonucleotide degrades trinucleotide repeats. In some embodiments, after binding of an antisense oligonucleotide to a target mRNA, the target mRNA is degraded by RNase H.

In some embodiments, a pair of antisense oligonucleotides are utilized to stabilize target mRNA. In some embodiments, a pair of antisense oligonucleotides are utilized to stabilize the coding region of a target mRNA also referred to herein as a "CDS". In some embodiments, a first antisense oligonucleotide binds 5' of the mRNA CDS, and a second antisense oligonucleotide binds 3' of the mRNA CDS.

In some embodiments, an antisense oligonucleotide increases the half-life of an mRNA. In some embodiments, an antisense oligonucleotide increases the half-life of a target mRNA. In some embodiments, an antisense oligonucleotide increases expression of the protein product of an mRNA (**FIG. 32**).

In some embodiments, the antisense oligonucleotides to a target sequence within a target pre-mRNA modulates one or more aspects of pre-mRNA splicing. As used herein, modulation of splicing refers to altering the processing of a pre-mRNA transcript such that the spliced mRNA molecule contains either a different combination of exons as a result of exon skipping or exon inclusion, a deletion in one or more exons, or the deletion or addition of a sequence not normally found in the spliced mRNA (e.g., an intron sequence). In some embodiments, antisense oligonucleotides hybridization to a target sequence comprised by a pre-mRNA molecule restores native splicing to a mutated pre-mRNA sequence. In some embodiments, antisense oligonucleotides hybridization results in alternative splicing of the target pre-mRNA. In some embodiments, antisense oligonucleotides hybridization results in exon inclusion or exon skipping of one or more exons. In some embodiments, the skipped exon sequence comprises a frameshift mutation, a nonsense mutation, or a missense mutation. In some embodiments, the skipped exon sequence comprises a nucleic acid deletion, substitution, or insertion. In some embodiments, the skipped exon itself does not comprise a sequence mutation, but a neighboring intron comprises a mutation leading to a frameshift mutation or a nonsense mutation. In some embodiments, antisense oligonucleotides hybridization to a target sequence within a target pre-mRNA prevents inclusion of an intron sequence in the mature mRNA molecule. In some embodiments, antisense oligonucleotides hybridization to a target sequence within a target pre-mRNA results in preferential expression of a wild type target protein isomer. In some embodiments, antisense oligonucleotides hybridization to a target sequence within a target pre-mRNA results in expression of a re-spliced target protein comprising an active fragment of a wild type target protein.

The antisense mechanism functions via hybridization of an antisense oligonucleotide compound with a target nucleic acid. In some embodiments, the antisense oligonucleotide hybridizing to its target sequence suppresses expression of the target protein. In some embodiments, the antisense oligonucleotide hybridizing to its target sequence suppresses expression of one or more wild type target protein isomers. In some embodiments, the antisense oligonucleotide hybridizing to its target sequence upregulates expression of the target protein. In some embodiments, the antisense oligonucleotide hybridizing to its target sequence increases expression of one or more wild type target protein isomers.

In other embodiments, the antisense compound of the present invention can inhibit gene expression by binding to a complementary mRNA. Binding to the target mRNA can lead to inhibition of gene expression either by preventing translation of complementary mRNA strands by binding to it or by leading to degradation of the target mRNA. Antisense DNA can be used to target a specific, complementary (coding or non-coding) RNA. If binding takes places this DNA/RNA hybrid can be degraded by the enzyme RNase H. In particular embodiment, antisense oligonucleotides contain from about 10 to about 50 nucleotides, or about 15 to about 30 nucleotides. The term also encompasses antisense oligonucleotides that may not be fully complementary to the desired target gene. Thus, the invention can be utilized in instances where non-target specific-activities are found with antisense, or where an antisense sequence containing one or more mismatches with the target sequence is the most preferred for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABAA receptor and human EGF (Jaskulski et ai, Science. 1988 Jun 10;240(4858): 1544-6; Vasanthakumar and Ahmed, Cancer Commun. 1989;1(4):225-32; Peris et ai, Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, e.g. cancer (U. S. Patent 5,747,470; U. S. Patent 5,591,317 and U. S. Patent 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, Tm, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et ai, Nucleic Acids Res. 1997, 25(17):3389-402).

### RNA Interference Nucleic Acids

In some embodiments, the oligonucleotide moiety of the present invention is a RNA interference (RNAi) molecule or a small interfering RNA molecule. RNA interference methods using RNAi or siRNA molecules may be used to disrupt the expression of a gene or polynucleotide of interest.

Small interfering RNAs (siRNAs) are RNA duplexes normally 16-30 nucleotides long that can associate with a cytoplasmic multi-protein complex known as RNAi-induced silencing complex (RISC). RISC loaded with siRNA mediates the degradation of homologous mRNA transcripts, therefore siRNA can be designed to knock down protein expression with high specificity. Unlike other antisense technologies, siRNA function through a natural mechanism evolved to control gene expression through non-coding RNA. A variety of RNAi reagents, including siRNAs targeting clinically relevant targets, are currently under pharmaceutical development, as described, e.g., in de Fougerolles, A. et al , Nature Reviews 6:443-453 (2007).

While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology 24:111-119). Thus, the invention includes the use of RNAi molecules comprising any of these different types of double-stranded molecules. In addition, it is understood that RNAi molecules may be used and introduced to cells in a variety of forms. Accordingly, as used herein, RNAi molecules encompasses any and all molecules capable of inducing an RNAi response in cells, including, but not limited to, double- stranded oligonucleotides comprising two separate strands, i.e. a sense strand and an antisense strand, e.g., small interfering RNA (siRNA); double-stranded oligonucleotide comprising two separate strands that are linked together by non-nucleotidyl linker; oligonucleotides comprising a hairpin loop of complementary sequences, which forms a double-stranded region, e.g., shRNAi molecules, and expression vectors that express one or more polynucleotides capable of forming a double- stranded polynucleotide alone or in combination with another polynucleotide.

A "single strand siRNA compound" as used herein, is an siRNA compound which is made up of a single molecule. It may include a duplexed region, formed by intra-strand pairing, e.g., it may be, or include, a hairpin or pan-handle structure. Single strand siRNA compounds may be antisense with regard to the target molecule.

A single strand siRNA compound may be sufficiently long that it can enter the RISC and participate in RISC mediated cleavage of a target mRNA. A single strand siRNA compound is at least 14, and in other embodiments at least 15, 20, 25, 29, 35, 40, or 50 nucleotides in length. In certain embodiments, it is less than 200, 100, or 60 nucleotides in length.

Hairpin siRNA compounds may have a duplex region equal to or at least 17, 18, 19, 29, 21, 22, 23, 24, or 25 nucleotide pairs. The duplex region may be equal to or less than 200, 100, or 50, in length. In certain embodiments, ranges for the duplex region are 15-30, 17 to 23, 19 to 23, and 19 to 21 nucleotides pairs in length. The hairpin may have a single strand overhang or terminal unpaired region. In certain embodiments, the overhangs are 2-3 nucleotides in length. In some embodiments, the overhang is at the same side of the hairpin and in some embodiments on the antisense side of the hairpin.

A "double stranded siRNA compound" as used herein, is an siRNA compound which includes more than one, and in some cases two, strands in which interchain hybridization can form a region of duplex structure.

The antisense strand of a double stranded siRNA compound may be equal to or at least, 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to21 nucleotides in length. As used herein, term "antisense strand" means the strand of an siRNA compound that is sufficiently complementary to a target molecule, e.g. a target RNA.

The sense strand of a double stranded siRNA compound may be equal to or at least 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to 21 nucleotides in length.

The double strand portion of a double stranded siRNA compound may be equal to or at least, 14, 15, 16 17, 18, 19, 20, 21, 22, 23, 24, 25, 29, 40, or 60 nucleotide pairs in length, It may be equal to or less than 200, 100, or 50, nucleotides pairs in length, Ranges may be 15-30, 17 to 23, 19 to 23, and 19 to 21 nucleotides pairs in length.

In many embodiments, the siRNA compound is sufficiently large that it can be cleaved by an endogenous molecule, e.g., by Dicer, to produce smaller siRNA compounds, e.g., siRNAs agents

The sense and antisense strands may be chosen such that the double-stranded siRNA compound includes a single strand or unpaired region at one or both ends of the molecule. Thus, a double- stranded siRNA compound may contain sense and antisense strands, paired to contain an overhang, e.g., one or two 5' or 3' overhangs, or a 3' overhang of 1 - 3 nucleotides. The overhangs can be the result of one strand being longer than the other, or the result of two strands of the same length being staggered. Some embodiments will have at least one 3' overhang. In one embodiment, both ends of an siRNA molecule will have a 3' overhang. In some embodiments, the overhang is 2 nucleotides.

In certain embodiments, the length for the duplexed region is between 15 and 30, or 18, 19, 20, 21, 22, and 23 nucleotides in length, e.g., in the ssiRNA (siRNA with sticky overhangs) compound range discussed above. ssiRNA compounds can resemble in length and structure the natural Dicer processed products from long dsiRNAs. Embodiments in which the two strands of the ssiRNA compound are linked, e.g., covalently linked are also included. Hairpin, or other single strand structures which provide the required double stranded region, and a 3' overhang are also within the invention.

The siRNA compounds described herein, including double-stranded siRNA compounds and single- stranded siRNA compounds can mediate silencing of a target RNA, e.g., mRNA, e.g., a transcript of a gene that encodes a protein. For convenience, such mRNA is also referred to herein as mRNA to be silenced. Such a gene is also referred to as a target gene. In general, the RNA to be silenced is an endogenous gene or a pathogen gene. In addition, RNAs other than mRNA, e.g., tRNAs, and viral RNAs, can also be targeted.

As used herein, the phrase "mediates RNAi" refers to the ability to silence, in a sequence specific manner, a target RNA. While not wishing to be bound by theory, it is believed that silencing uses the RNAi machinery or process and a guide RNA, e.g., an ssiRNA compound of 21 to 23 nucleotides.

In one embodiment, an siRNA compound is "sufficiently complementary" to a target RNA, e.g., a target mRNA, such that the siRNA compound silences production of protein encoded by the target mRNA. In another embodiment, the siRNA compound is "exactly complementary" to a target RNA, e.g., the target RNA and the siRNA compound anneal, for example to form a hybrid made exclusively of Watson-Crick base pairs in the region of exact complementarity. A "sufficiently complementary" target RNA can include an internal region (e.g., of at least 10 nucleotides) that is exactly complementary to a target RNA. Moreover, in certain embodiments, the siRNA compound specifically discriminates a single-nucleotide difference. In this case, the siRNA compound only mediates RNAi if exact complementary is found in the region (e.g., within 7 nucleotides of) the single-nucleotide difference.

### MicroRNAs

In some embodiments, the oligonucleotide moiety of the present invention is a microRNA molecule. MicroRNAs (miRNAs) are a highly conserved class of small RNA molecules that are transcribed from DNA in the genomes of plants and animals, but are not translated into protein. Processed miRNAs are single stranded -17-25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3 '-untranslated region of specific mRNAs. RISC mediates down-regulation of gene expression through translational inhibition, transcript cleavage, or both. RISC is also implicated in transcriptional silencing in the nucleus of a wide range of eukaryotes.

The number of miRNA sequences identified to date is large and growing, illustrative examples of which can be found, for example, in: "miRBase: microRNA sequences, targets and gene nomenclature" Griffiths- Jones S, Grocock RJ, van Dongen S, Bateman A, Enright AJ. NAR, 2006, 34, Database Issue, D140-D144; "The microRNA Registry" Griffiths -Jones S. NAR, 2004, 32, Database Issue, D109-D111; and also at http://www.mirbase.org/.

### Antagomirs

In some embodiments, the oligonucleotide moiety of the present invention is an antagomir. Antagomirs are RNA-like oligonucleotides that harbor various modifications for RNAse protection and pharmacologic properties, such as enhanced tissue and cellular uptake. They differ from normal RNA by, for example, complete 2'-0-methylation of sugar, phosphorothioate backbone and, for example, a cholesterol-moiety at 3'-end. Antagomirs may be used to efficiently silence endogenous miRNAs by forming duplexes comprising the antagomir and endogenous miRNA, thereby preventing miRNA-induced gene silencing. An example of antagomir-mediated miRNA silencing is the silencing of miR-122, described in Krutzfeldt et al, Nature, 2005, 438: 685-689, which is expressly incorporated by reference herein in its entirety. Antagomir RNAs may be synthesized using standard solid phase oligonucleotide synthesis protocols. See U.S. Patent Application Ser. Nos. 11/502,158 and 11/657,341 (the disclosure of each of which are incorporated herein by reference).

An antagomir can include ligand-conjugated monomer subunits and monomers for oligonucleotide synthesis. Exemplary monomers are described in U.S. Application No. 10/916,185, filed on August 10, 2004. An antagomir can have a ZXY structure, such as is described in PCT Application No. PCT/US2004/07070 filed on March 8, 2004. An antagomir can be complexed with an amphipathic moiety. Exemplary amphipathic moieties for use with oligonucleotide agents are described in PCT Application No. PCT/US2004/07070, filed on March 8, 2004.

### Aptamers

In some embodiments, the oligonucleotide moiety of the present invention is an aptamer. Aptamers are nucleic acid or peptide molecules that bind to a particular molecule of interest with high affinity and specificity (Tuerk and Gold, Science 249:505 (1990); Ellington and Szostak, Nature 346:818 (1990)). DNA or RNA aptamers have been successfully produced which bind many different entities from large proteins to small organic molecules. See Eaton, Curr. Opin. Chem. Biol. 1: 10-16 (1997), Famulok, Curr. Opin. Struct. Biol. 9:324-9(1999), and Hermann and Patel, Science 287:820-5 (2000). Aptamers may be RNA or DNA based, and may include a riboswitch. A riboswitch is a part of an mRNA molecule that can directly bind a small target molecule, and whose binding of the target affects the gene's activity. Thus, an mRNA that contains a riboswitch is directly involved in regulating its own activity, depending on the presence or absence of its target molecule. Generally, aptamers are engineered through repeated rounds of in vitro selection or equivalently, SELEX (systematic evolution of ligands by exponential enrichment) to bind to various molecular targets such as small molecules, proteins, nucleic acids, and even cells, tissues and organisms. The aptamer may be prepared by any known method, including synthetic, recombinant, and purification methods, and may be used alone or in combination with other aptamers specific for the same target. Further, the term "aptamer" also includes "secondary aptamers" containing a consensus sequence derived from comparing two or more known aptamers to a given target. In some embodiments, the aptamer is an "intracellular aptamer", or "intramer", which specifically recognize intracellular targets. See Famulok et al., Chem Biol. 2001, Oct, 8(10):931-939; Yoon and Rossi, Adv Drug Deliv Rev. 2018, Sep, 134:22-35, each incorporated by reference herein.

### Ribozymes

In some embodiments, the oligonucleotide moiety of the present invention is a ribozyme. Ribozymes are RNA molecules complexes having specific catalytic domains that possess endonuclease activity (Kim and Cech, Proc Natl Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al, Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374): 173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds in trans (and thus can cleave other RNA molecules) under physiological conditions, In general, enzymatic nucleic acids act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis δ virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel et al. (Eur. Pat. Appl. Publ. No. EP 0360257), Hampel and Tritz, Biochemistry 1989 Jun 13;28(12):4929- 33; Hampel et al, Nucleic Acids Res. 1990 Jan 25;18(2):299-304 and U. S. Patent 5,631,359. An example of the hepatitis virus motif is described by Perrotta and Been, Biochemistry. 1992 Dec 1 ;31(47): 11843-52; an example of the RNaseP motif is described by Guerrier-Takada et al , Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in U. S. Patent 4,987,071. Important characteristics of enzymatic nucleic acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to any polynucleotide sequence are known in the art. Ribozymes may be designed as described in Int. Pat. Appl. Publ. No. WO 93/23569 and Int. Pat. Appl. Publ. No. WO 94/02595, each specifically incorporated herein by reference, and synthesized to be tested in vitro and in vivo, as described therein.

Ribozyme activity can be optimized by altering the length of the ribozyme binding arms or chemically synthesizing ribozymes with modifications that prevent their degradation by serum ribonucleases (see e.g. , Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711 ; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

### Immunostimulatory Oligonucleotides

In some embodiments, the oligonucleotide moiety of the present invention is an immunostimulatory oligonucleotide. Immunostimulatory oligonucleotides (ISS; single-or double-stranded) are capable of inducing an immune response when administered to a subject, which may be a mammal or other patient. ISS include, e.g., certain palindromes leading to hairpin secondary structures (see Yamamoto S., et al. (1992) J. Immunol. 148: 4072-4076), or CpG motifs, as well as other known ISS features (such as multi-G domains, see WO 96/11266).

The immune response may be an innate or an adaptive immune response. The immune system is divided into a more innate immune system, and acquired adaptive immune system of vertebrates, the latter of which is further divided into humoral cellular components. In particular embodiments, the immune response may be mucosal.

Immunostimulatory nucleic acids are considered to be non-sequence specific when it is not required that they specifically bind to and reduce the expression of a target polynucleotide in order to provoke an immune response. Thus, certain immunostimulatory nucleic acids may comprise a sequence corresponding to a region of a naturally occurring gene or mRNA, but they may still be considered non-sequence specific immunostimulatory nucleic acids.

In one embodiment, the immunostimulatory nucleic acid or oligonucleotide comprises at least one CpG dinucleotide. The oligonucleotide or CpG dinucleotide may be unmethylated or methylated. In another embodiment, the immunostimulatory nucleic acid comprises at least one CpG dinucleotide having a methylated cytosine. In one embodiment, the nucleic acid comprises a single CpG dinucleotide, wherein the cytosine in said CpG dinucleotide is methylated. In a specific embodiment, the nucleic acid comprises the sequence 5' TAACGTTGAGGGGCAT 3' (SEQ ID NO: 147). In an alternative embodiment, the nucleic acid comprises at least two CpG dinucleotides, wherein at least one cytosine in the CpG dinucleotides is methylated. In a further embodiment, each cytosine in the CpG dinucleotides present in the sequence is methylated. In another embodiment, the nucleic acid comprises a plurality of CpG dinucleotides, wherein at least one of said CpG dinucleotides comprises a methylated cytosine.

Additional specific nucleic acid sequences of oligonucleotides (ODNs) suitable for use in the compositions and methods of the invention are described in Raney et al, Journal of Pharmacology and Experimental Therapeutics, 298:1185-1192 (2001). In certain embodiments, ODNs used in the compositions and methods of the present invention have a phosphodiester ("PO") backbone or a phosphorothioate ("PS") backbone, and/or at least one methylated cytosine residue in a CpG motif.

### Decoy Oligonucleotides

In some embodiments, the oligonucleotide moiety of the present invention is a decoy oligonucleotide. Because transcription factors recognize their relatively short binding sequences, even in the absence of surrounding genomic DNA, short oligonucleotides bearing the consensus binding sequence of a specific transcription factor can be used as tools for manipulating gene expression in living cells. This strategy involves the intracellular delivery of such "decoy oligonucleotides", which are then recognized and bound by the target factor. Occupation of the transcription factor's DNA-binding site by the decoy renders the transcription factor incapable of subsequently binding to the promoter regions of target genes. Decoys can be used as therapeutic agents, either to inhibit the expression of genes that are activated by a transcription factor, or to upregulate genes that are suppressed by the binding of a transcription factor. Examples of the utilization of decoy oligonucleotides may be found in Mann et al., J. Clin. Invest, 2000, 106: 1071-1075, which is expressly incorporated by reference herein, in its entirety.

### Supermir

In some embodiments, the oligonucleotide moiety of the present invention is a supermir. A supermir refers to a single stranded, double stranded or partially double stranded oligomer or polymer of ribonucleic acid (RNA) or deoxyribonucleic acid (DNA) or both or modifications thereof, which has a nucleotide sequence that is substantially identical to an miRNA and that is antisense with respect to its target, This term includes oligonucleotides composed of naturally-occurring nucleobases, sugars and covalent internucleoside (backbone) linkages and which contain at least one non-naturally- occurring portion which functions similarly. Such modified or substituted oligonucleotides are preferred over native forms because of desirable properties such as, for example, enhanced cellular uptake, enhanced affinity for nucleic acid target and increased stability in the presence of nucleases. In a preferred embodiment, the supermir does not include a sense strand, and in another preferred embodiment, the supermir does not self-hybridize to a significant extent. A supermir featured in the invention can have secondary structure, but it is substantially single-stranded under physiological conditions. A supermir that is substantially single-stranded is single-stranded to the extent that less than about 50% {e.g., less than about 40%, 30%, 20%, 10%, or 5%) of the supermir is duplexed with itself. The supermir can include a hairpin segment, e.g., sequence, preferably at the 3' end can self hybridize and form a duplex region, e.g., a duplex region of at least 1, 2, 3, or 4 and preferably less than 8, 7, 6, or n nucleotides, e.g., 5 nuclotides. The duplexed region can be connected by a linker, e.g., a nucleotide linker, e.g., 3, 4, 5, or 6 dTs, e.g., modified dTs. In another embodiment the supermir is duplexed with a shorter oligo, e.g., of 5, 6, 7, 8, 9, or 10 nucleotides in length, e.g., at one or both of the 3' and 5' end or at one end and in the non-terminal or middle of the supermir.

### miRNA mimics

In some embodiments, the oligonucleotide moiety of the present invention is an miRNA mimic. miRNA mimics represent a class of molecules that can be used to imitate the gene silencing ability of one or more miRNAs. Thus, the term "microRNA mimic" refers to synthetic non-coding RNAs (i.e. the miRNA is not obtained by purification from a source of the endogenous miRNA) that are capable of entering the RNAi pathway and regulating gene expression. miRNA mimics can be designed as mature molecules (e.g. single stranded) or mimic precursors (e.g., pri- or pre-miRNAs). miRNA mimics can be comprised of nucleic acid (modified or modified nucleic acids) including oligonucleotides comprising, without limitation, RNA, modified RNA, DNA, modified DNA, locked nucleic acids, or 2'-0,4'-C-ethylene-bridged nucleic acids (ENA), or any combination of the above (including DNA-RNA hybrids). In addition, miRNA mimics can comprise conjugates that can affect delivery, intracellular compartmentalization, stability, specificity, functionality, strand usage, and/or potency. In one design, miRNA mimics are double stranded molecules (e.g., with a duplex region of between about 16 and about 31 nucleotides in length) and contain one or more sequences that have identity with the mature strand of a given miRNA. Modifications can comprise 2' modifications (including 2'-0 methyl modifications and 2' F modifications) on one or both strands of the molecule and internucleotide modifications (e.g. phorphorthioate modifications) that enhance nucleic acid stability and/or specificity. In addition, miRNA mimics can include overhangs. The overhangs can consist of 1-6 nucleotides on either the 3' or 5' end of either strand and can be modified to enhance stability or functionality. In one embodiment, a miRNA mimic comprises a duplex region of between 16 and 31 nucleotides and one or more of the following chemical modification patterns: the sense strand contains 2'-0-methyl modifications of nucleotides 1 and 2 (counting from the 5' end of the sense oligonucleotide), and all of the Cs and Us; the antisense strand modifications can comprise 2' F modification of all of the Cs and Us, phosphorylation of the 5' end of the oligonucleotide, and stabilized internucleotide linkages associated with a 2 nucleotide 3 ' overhang.

### miRNA inhibitor

In some embodiments, the oligonucleotide moiety of the present invention is an miRNA inhibitor. The terms "antimir" "microRNA inhibitor", "miR inhibitor", or "miRNA inhibitor" are synonymous and refer to oligonucleotides or modified oligonucleotides that interfere with the ability of specific miRNAs. In general, the inhibitors are nucleic acid or modified nucleic acids in nature including oligonucleotides comprising RNA, modified RNA, DNA, modified DNA, locked nucleic acids (LNAs), or any combination of the above.

Modifications include 2' modifications (including 2'-0 alkyl modifications and 2' F modifications) and internucleotide modifications (e.g. phosphorothioate modifications) that can affect delivery, stability, specificity, intracellular compartmentalization, or potency. In addition, miRNA inhibitors can comprise conjugates that can affect delivery, intracellular compartmentalization, stability, and/or potency. Inhibitors can adopt a variety of configurations including single stranded, double stranded (RNA/RNA or RNA/DNA duplexes), and hairpin designs, in general, microRNA inhibitors comprise contain one or more sequences or portions of sequences that are complementary or partially complementary with the mature strand (or strands) of the miRNA to be targeted, in addition, the miRNA inhibitor may also comprise additional sequences located 5' and 3' to the sequence that is the reverse complement of the mature miRNA. The additional sequences may be the reverse complements of the sequences that are adjacent to the mature miRNA in the pri-miRNA from which the mature miRNA is derived, or the additional sequences may be arbitrary sequences (having a mixture of A, G, C, or U). In some embodiments, one or both of the additional sequences are arbitrary sequences capable of forming hairpins. Thus, in some embodiments, the sequence that is the reverse complement of the miRNA is flanked on the 5' side and on the 3' side by hairpin structures. Micro-RNA inhibitors, when double stranded, may include mismatches between nucleotides on opposite strands. Furthermore, micro-RNA inhibitors may be linked to conjugate moieties in order to facilitate uptake of the inhibitor into a cell. For example, a micro-RNA inhibitor may be linked to cholesteryl 5-(bis(4- methoxyphenyl)(phenyl)methoxy)-3 hydroxypentylcarbamate) which allows passive uptake of a micro-RNA inhibitor into a cell. Micro-RNA inhibitors, including hairpin miRNA inhibitors, are described in detail in Vermeulen et al., "Double-Stranded Regions Are Essential Design Components Of Potent Inhibitors of RISC Function," RNA 13: 723-730 (2007) and in WO2007/095387 and WO 2008/036825 each of which is incorporated herein by reference in its entirety. A person of ordinary skill in the art can select a sequence from the database for a desired miRNA and design an inhibitor useful for the methods disclosed herein.

### U1 adaptor

In some embodiments, the oligonucleotide moiety of the present invention is a U1 adaptor. U1 adaptors inhibit polyA sites and are bifunctional oligonucleotides with a target domain complementary to a site in the target gene's terminal exon and a 'U1 domain' that binds to the U1 smaller nuclear RNA component of the U1 snRNP (Goraczniak, et al., 2008, Nature Biotechnology, 27(3), 257-263, which is expressly incorporated by reference herein, in its entirety). U1 snRNP is a ribonucleoprotein complex that functions primarily to direct early steps in spliceosome formation by binding to the pre-mRNA exon- intron boundary (Brown and Simpson, 1998, Annu Rev Plant Physiol Plant Mol Biol 49:77-95). Nucleotides 2-11 of the 5'end of U1 snRNA base pair bind with the 5'ss of the pre mRNA. In one embodiment, oligonucleotides of the invention are U1 adaptors. In one embodiment, the U1 adaptor can be administered in combination with at least one other iRNA agent.

### Antisense compounds (ACs)

According to the present disclosure, an antisense compound (AC) is employed in order to alter one or more aspects of the splicing, translation, or expression of a target gene, e.g., by altering the splicing of a eukaryotic target pre-mRNA. The AC according to the disclosure comprises a nucleic acid sequence that is complementary to a sequence found within a target pre-mRNA sequence. The use of these ACs provides a direct genetic approach that has the ability to modulate splicing of specific disease-causing genes. The principle behind antisense technology is that an antisense compound, which hybridizes to a target nucleic acid, modulates gene expression activities such as splicing or translation through one of a number of antisense mechanisms. The sequence-specificity of the AC makes this technique extremely attractive as a therapeutic to selectively modulate the splicing of pre-mRNA involved in the pathogenesis of any one of a variety of diseases. Antisense technology is an effective means for changing the expression of one or more specific gene products and can therefore prove to be useful in a number of therapeutic, diagnostic, and research applications.

The compounds described herein may contain one or more asymmetric centers and thus give rise to enantiomers, diastereomers, and other stereoisomeric configurations that may be defined, in terms of absolute stereochemistry, as (R) or (S), α or β, or as (D) or (L). Included in the antisense compounds provided herein are all such possible isomers, as well as their racemic and optically pure forms.

### Antisense compound hybridization site

Antisense mechanisms rely on hybridization of the antisense compound to the target nucleic acid. Accordingly, the present disclosure provides antisense compounds that are complementary to a target nucleic acid. In some embodiments, the target nucleic acid sequence is present in a pre-mRNA molecule.

Pre-mRNA molecules are made in the nucleus and are processed before or during transport to the cytoplasm for translation. Processing of the pre-mRNAs includes addition of a 5' methylated cap and an approximately 200-250 base poly(A) tail to the 3' end of the transcript. The next step in mRNA processing is splicing of the pre-mRNA, which occurs in the maturation of 90-95% of mammalian mRNAs. Introns (or intervening sequences) are regions of a primary transcript (or the DNA encoding it) that are not included in the coding sequence of the mature mRNA. Exons are regions of a primary transcript that remain in the mature mRNA when it reaches the cytoplasm. The exons are spliced together to form the mature mRNA sequence. Splice junctions are also referred to as splice sites with the 5' side of the junction often called the "5' splice site," or "splice donor site" and the 3' side called the "3' splice site" or "splice acceptor site." In splicing, the 3' end of an upstream exon is joined to the 5' end of the downstream exon. Thus the unspliced RNA (or pre-mRNA) has an exon/intron junction at the 5' end of an intron and an intron/exon junction at the 3' end of an intron. After the intron is removed, the exons are contiguous at what is sometimes referred to as the exon/exon junction or boundary in the mature mRNA. Cryptic splice sites are those which are less often used but may be used when the usual splice site is blocked or unavailable. Alternative splicing, defined as the splicing together of different combinations of exons, often results in multiple mRNA transcripts from a single gene.

In some embodiments, the AC hybridizes with a sequence in a splice site. In some embodiments, the AC hybridizes with a sequence comprising part of a splice site. In some embodiments, the AC hybridizes with a sequence comprising all of a splice site. In some embodiments, the AC hybridizes with a sequence comprising part or all of a splice donor site. In some embodiments, the AC hybridizes with a sequence comprising part or all of a splice acceptor site. In some embodiments, the AC hybridizes with a sequence comprising part or all of a cryptic splice site. In some embodiments, the AC hybridizes with a sequence comprising an exon/intron junction.

Pre-mRNA splicing involves two sequential biochemical reactions. Both reactions involve the spliceosomal transesterification between RNA nucleotides. In a first reaction, the 2'-OH of a specific branch-point nucleotide within an intron, which is defined during spliceosome assembly, performs a nucleophilic attack on the first nucleotide of the intron at the 5' splice site forming a lariat intermediate. In a second reaction, the 3'-OH of the released 5' exon performs a nucleophilic attack at the last nucleotide of the intron at the 3' splice site thus joining the exons and releasing the intron lariat. Pre-mRNA splicing is regulated by intronic silencer sequence (ISS) and terminal stem loop (TSL) sequences. As used herein, the terms "intronic silencer sequences (ISS)" and "terminal stem loop (TSL)" refer to sequence elements within introns and exons, respectively, that control alternative splicing by the binding of trans-acting protein factors within a pre-mRNA thereby resulting in differential use of splice sites. Typically, intronic silencer sequences are between 8 and 16 nucleotides and are less conserved than the splice sites at exon-intron junctions. Terminal stem loop sequences are typically between 12 and 24 nucleotides and form a secondary loop structure due to the complementarity, and hence binding, within the 12-24 nucleotide sequence.

In some embodiments, the AC hybridizes with a sequence comprising part or all of an intronic silencer sequence. In some embodiments, the AC hybridizes with a sequence comprising part or all of a terminal stem loop.

Up to 50% of human genetic diseases resulting from a point mutation are caused by aberrant splicing. Such point mutations can either disrupt a current splice site or create a new splice site, resulting in mRNA transcripts comprised of a different combination of exons or with deletions in exons. Point mutations also can result in activation of a cryptic splice site or disrupt regulatory cis elements (i.e. splicing enhancers or silencers).

In some embodiments, the AC hybridizes with a sequence comprising part or all of an aberrant splice site resulting from a mutation in the target gene. In some embodiments, the AC hybridizes with a sequence comprising part or all of a regulatory element. Also provided are antisense compounds targeted to cis regulatory elements. In some embodiments, the regulatory element is in an exon. In some embodiments, the regulatory element is in an intron.

In some embodiments, the AC may be specifically hybridizable with a translation initiation codon region, a 5' cap region, an intron/exon junction, a coding sequence, a translation termination codon region or sequences in the 5'- or 3'-untranslated region. In some embodiments, the AC may hybridize with part or all of a pre-mRNA splice site, an exon-exon junction, or an intron-exon junction. In some embodiments, the AC may hybridize with an aberrant fusion junction due to a rearrangement or a deletion. In some embodiments, the AC may hybridize with particular exons in alternatively spliced mRNAs.

In some embodiments, the AC hybridizes with a sequence between 5 and 50 nucleic acids in length, which can also be referred to as the length of the AC. In some embodiments, the AC is between 5 and 10, 10 and 15, 15 and 20, 20 and 25, 25 and 30, 30 and 35, 35 and 40, 40 and 45, or 45 and 50 nucleic acids in length. In some embodiments, the AC is approximately 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleic acids in length. In some embodiments, the AC is approximately 10 nucleic acids in length. In some embodiments, the AC is approximately 15 nucleic acids in length. In some embodiments, the AC is approximately 20 nucleic acids in length. In some embodiments, the AC is approximately 25 nucleic acids in length. In some embodiments, the AC is approximately 30 nucleic acids in length.

In some embodiments, the AC may be less than 100 percent complementary to a target nucleic acid sequence. As used herein, the term "percent complementary" refers to the number of nucleobases of an AC that have nucleobase complementarity with a corresponding nucleobase of an oligomeric compound or nucleic acid divided by the total length (number of nucleobases) of the AC. One skilled in the art recognizes that the inclusion of mismatches is possible without eliminating the activity of the antisense compound. Therefore, in some embodiments, an AC may contain up to about 20% nucleotides that disrupt base pairing of the AC to the target nucleic acid. In some embodiments, the ACs contain no more than about 15%, no more than about 10%, no more than 5%, or no mismatches. In some embodiments, the ACs are at least 80%, at least 85%, at least 90%, at least 95%, at least 96%, at least 97%, at least 98%, at least 99% or 100% complementary to a target nucleic acid. Percent complementarity of an oligonucleotide is calculated by dividing the number of complementary nucleobases by the total number of nucleobases of the oligonucleotide. Percent complementarity of a region of an oligonucleotide is calculated by dividing the number of complementary nucleobases in the region by the total number of nucleobases region.

In some embodiments, incorporation of nucleotide affinity modifications allows for a greater number of mismatches compared to an unmodified compound. Similarly, certain oligonucleotide sequences may be more tolerant to mismatches than other oligonucleotide sequences. One of ordinary skill in the art is capable of determining an appropriate number of mismatches between oligonucleotides, or between an oligonucleotide and a target nucleic acid, such as by determining melting temperature (Tm). Tm or ΔTm can be calculated by techniques that are familiar to one of ordinary skill in the art. For example, techniques described in Freier et al. (Nucleic Acids Research, 1997, 25, 22: 4429-4443) allow one of ordinary skill in the art to evaluate nucleotide modifications for their ability to increase the melting temperature of an RNA:DNA duplex.

### Antisense mechanisms

The ACs according to the present disclosure may modulate one or more aspects of protein transcription, translation, and expression. In some embodiments, the AC hybridizing to a target sequence within a target pre-mRNA modulates one or more aspects of pre-mRNA splicing. As used herein, modulation of splicing refers to altering the processing of a pre-mRNA transcript such that the spliced mRNA molecule contains either a different combination of exons as a result of exon skipping or exon inclusion, a deletion in one or more exons, or the deletion or addition of a sequence not normally found in the spliced mRNA (e.g., an intron sequence). In some embodiments, AC hybridization to a target sequence comprised by a pre-mRNA molecule restores native splicing to a mutated pre-mRNA sequence. In some embodiments, AC hybridization results in alternative splicing of the target pre-mRNA. In some embodiments, AC hybridization results in exon inclusion or exon skipping of one or more exons. In some embodiments, the skipped exon sequence comprises a frameshift mutation, a nonsense mutation, or a missense mutation. In some embodiments, the skipped exon sequence comprises a nucleic acid deletion, substitution, or insertion. In some embodiments, the skipped exon itself does not comprise a sequence mutation, but a neighboring intron comprises a mutation leading to a frameshift mutation or a nonsense mutation. In some embodiments, AC hybridization to a target sequence within a target pre-mRNA prevents inclusion of an intron sequence in the mature mRNA molecule. In some embodiments, AC hybridization to a target sequence within a target pre-mRNA results in preferential expression of a wild type target protein isomer. In some embodiments, AC hybridization to a target sequence within a target pre-mRNA results in expression of a re-spliced target protein comprising an active fragment of a wild type target protein.

In some embodiments, the AC regulates transcription, translation, or protein expression through steric blocking. The following review article describes the mechanisms of steric blocking and applications thereof and is incorporated by reference herein in its entirety: Roberts et al. Nature Reviews Drug Discovery (2020) 19: 673-694.

The antisense mechanism functions via hybridization of an antisense compound with a target nucleic acid. In some embodiments, the AC hybridizing to its target sequence suppresses expression of the target protein. In some embodiments, the AC hybridizing to its target sequence suppresses expression of one or more wild type target protein isomers. In some embodiments, the AC hybridizing to its target sequence upregulates expression of the target protein. In some embodiments, the AC hybridizing to its target sequence increases expression of one or more wild type target protein isomers.

The efficacy of the ACs of the present disclosure may be assessed by evaluating the antisense activity effected by their administration. As used herein, the term "antisense activity" refers to any detectable and/or measurable activity attributable to the hybridization of an antisense compound to its target nucleic acid. Such detection and or measuring may be direct or indirect. In some embodiments, antisense activity is assessed by detecting and or measuring the amount of target protein. In some embodiments, antisense activity is assessed by detecting and or measuring the amount of re-spliced target protein. In some embodiments, antisense activity is assessed by detecting and/or measuring the amount of target nucleic acids and/or cleaved target nucleic acids and/or alternatively spliced target nucleic acids

### Antisense compound design

Design of ACs according to the present disclosure will depend upon the sequence being targeted. Targeting an AC to a particular target nucleic acid molecule can be a multistep process. The process usually begins with the identification of a target nucleic acid whose expression is to be modulated. As used herein, the terms "target nucleic acid" and "nucleic acid encoding a target gene" encompass DNA encoding a selected target gene, RNA (including pre-mRNA and mRNA) transcribed from such DNA, and also cDNA derived from such RNA. For example, the target nucleic acid can be a cellular gene (or mRNA transcribed from the gene) whose expression is associated with a particular disorder or disease state, or a nucleic acid molecule from an infectious agent.

One of skill in the art will be able to design, synthesize, and screen antisense compounds of different nucleobase sequences to identify a sequence that results in antisense activity. For example, one may design an antisense compound that alters splicing of a target pre-mRNA or inhibits expression of a target protein. Methods for designing, synthesizing and screening antisense compounds for antisense activity against a preselected target nucleic acid can be found, for example in "Antisense Drug Technology, Principles, Strategies, and Applications" Edited by Stanley T. Crooke, CRC Press, Boca Raton, Florida, which is incorporated by reference in its entirety for any purpose.

In some embodiments, the present invention provides antisense compounds comprising oligonucleotides. Certain oligonucleotides comprise 8 to 30 linked nucleosides. In some embodiments, the antisense compounds comprise modified nucleosides, modified internucleoside linkages and/or conjugate groups.

In some embodiments, the antisense compound is a "tricyclo-DNA (tc-DNA)", which refers to a class of constrained DNA analogs in which each nucleotide is modified by the introduction of a cyclopropane ring to restrict conformational flexibility of the backbone and to optimize the backbone geometry of the torsion angle γ. Homobasic adenine- and thymine-containing tc-DNAs form extraordinarily stable A-T base pairs with complementary RNAs.

### Exemplary Nucleosides

In some embodiments, the invention provides antisense compounds comprising linked nucleosides. In some embodiments, some or all of the nucleosides are modified nucleosides. In some embodiments, one or more nucleoside comprises a modified nucleobase. In some embodiments, one or more nucleosides comprises a modified sugar. Chemically modified nucleosides are routinely used for incorporation into antisense compounds to enhance one or more properties, such as nuclease resistance, pharmacokinetics or affinity for a target RNA. Non-limiting examples of nucleosides are provided in **FIG. 69** and in Khvorova et al. Nature Biotechnology (2017) 35: 238-248, which is incorporated by reference herein in its entirety.

In general, a nucleobase is any group that contains one or more atom or groups of atoms capable of hydrogen bonding to a base of another nucleic acid. In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable with the compounds described herein. The terms modified nucleobase and nucleobase mimetic can overlap but generally a modified nucleobase refers to a nucleobase that is fairly similar in structure to the parent nucleobase, such as for example a 7-deaza purine, a 5-methyl cytosine, or a G-clamp, whereas a nucleobase mimetic would include more complicated structures, such as for example a tricyclic phenoxazine nucleobase mimetic. Methods for preparation of the above noted modified nucleobases are well known to those skilled in the art.

In some embodiments, ACs provided herein comprise one or more nucleosides having a modified sugar moiety. In some embodiments, the furanosyl sugar ring of a natural nucleoside can be modified in a number of ways including, but not limited to, addition of a substituent group, bridging of two non-geminal ring atoms to form a bicyclic nucleic acid (BNA) and substitution of an atom or group such as -S-, -N(R)- or -C(R1)(R2) for the ring oxygen at the 4'-position. Modified sugar moieties are well known and can be used to alter, typically increase, the affinity of the antisense compound for its target and/or increase nuclease resistance. A representative list of modified sugars includes but is not limited to non-bicyclic substituted sugars, especially non-bicyclic 2'-substituted sugars having a 2'-F, 2'-OCH3 or a 2'-O(CH2)2-OCH3 substituent group; and 4'-thio modified sugars. Sugars can also be replaced with sugar mimetic groups among others. Methods for the preparations of modified sugars are well known to those skilled in the art. Some representative patents and publications that teach the preparation of such modified sugars include, but are not limited to, U.S. Patents: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; 5,700,920; and 6,600,032; and WO 2005/121371 .

In some embodiments, nucleosides comprise bicyclic modified sugars (BNA's), including LNA (4'-(CH2)-O-2' bridge), 2'-thio-LNA (4'-(CH2)-S-2' bridge),, 2'-amino-LNA (4'-(CH2)-NR-2' bridge),, ENA (4'-(CH2)2-O-2' bridge), 4'-(CH2)3-2' bridged BNA, 4'-(CH2CH(CH3))-2' bridged BNA" cEt (4'-(CH(CH3)-O-2' bridge), and cMOE BNAs (4'-(CH(CH2OCH3)-O-2' bridge). Certain such BNA's have been prepared and disclosed in the patent literature as well as in scientific literature (See, e.g., Srivastava, et al. J. Am. Chem. Soc. 2007, ACS Advanced online publication, 10.1021/ja071106y, Albaek et al. J. Org. Chem., 2006, 71, 7731 -7740, Fluiter, et al. Chembiochem 2005, 6, 1104-1109, Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; WO 94/14226; WO 2005/021570; Singh et al., J. Org. Chem., 1998, 63, 10035-10039, WO 2007/090071; Examples of issued US patents and published applications that disclose BNAs include, for example, U.S. Patent Nos. 7,053,207; 6,268,490; 6,770,748; 6,794,499; 7,034,133; and 6,525,191; and U.S. Pre-Grant Publication Nos. 2004-0171570; 2004-0219565; 2004-0014959; 2003-0207841; 2004-0143114; and 20030082807 .

Also provided herein are "Locked Nucleic Acids" (LNAs) in which the 2'-hydroxyl group of the ribosyl sugar ring is linked to the 4' carbon atom of the sugar ring thereby forming a 2'-C,4'-C-oxymethylene linkage to form the bicyclic sugar moiety (reviewed in Elayadi et al., Curr. Opinion Invens. Drugs, 2001, 2, 558-561; Braasch et al., Chem. Biol., 2001, 8 1-7; and Orum et al., Curr. Opinion Mol. Ther., 2001, 3, 239-243; see also U.S. Patents: 6,268,490 and 6,670,461 ). The linkage can be a methylene (-CH2-) group bridging the 2' oxygen atom and the 4' carbon atom, for which the term LNA is used for the bicyclic moiety; in the case of an ethylene group in this position, the term ENA^{™} is used (Singh et al., Chem. Commun., 1998, 4, 455-456; ENA^{™}: Morita et al., Bioorganic Medicinal Chemistry, 2003, 11, 2211-2226). LNA and other bicyclic sugar analogs display very high duplex thermal stabilities with complementary DNA and RNA (Tm = +3 to +10° C), stability towards 3'-exonucleolytic degradation and good solubility properties. Potent and nontoxic antisense oligonucleotides containing LNAs have been described (Wahlestedt et al., Proc. Natl. Acad. Sci. U.S.A., 2000, 97, 5633-5638).

An isomer of LNA that has also been studied is alpha-L-LNA which has been shown to have superior stability against a 3'-exonuclease. The alpha-L-LNA's were incorporated into antisense gapmers and chimeras that showed potent antisense activity (Frieden et al., Nucleic Acids Research, 2003, 21, 6365-6372).

The synthesis and preparation of the LNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). LNAs and preparation thereof are also described in WO 98/39352 and WO 99/14226.

Analogs of LNA, phosphorothioate-LNA and 2'-thio-LNAs, have also been prepared (Kumar et al., Bioorg. Med. Chem. Lett., 1998, 8, 2219-2222). Preparation of locked nucleoside analogs containing oligodeoxyribonucleotide duplexes as substrates for nucleic acid polymerases has also been described ( Wengel et al., WO 99/14226 ). Furthermore, synthesis of 2'-amino-LNA, a novel conformationally restricted high-affinity oligonucleotide analog has been described in the art (Singh et al., J. Org. Chem., 1998, 63, 10035-10039). In addition, 2'-Amino- and 2'-methylamino-LNA's have been prepared and the thermal stability of their duplexes with complementary RNA and DNA strands has been previously reported.

### Exemplary Internucleoside Linkages

Described herein are internucleoside linking groups that link the nucleosides or otherwise modified monomer units together thereby forming an antisense compound. The two main classes of internucleoside linking groups are defined by the presence or absence of a phosphorus atom. Representative phosphorus containing internucleoside linkages include, but are not limited to, phosphodiesters, phosphotriesters, methylphosphonates, phosphoramidate, and phosphorothioates. Representative non-phosphorus containing internucleoside linking groups include, but are not limited to, methylenemethylimino (-CH2-N(CH3)-O-CH2-), thiodiester (-O-C(O)-S-), thionocarbamate (-O-C(O)(NH)-S-); siloxane (-O-Si(H)2-O-); and N,N'-dimethylhydrazine (-CH2-N(CH3)-N(CH3)-). Antisense compounds having non-phosphorus internucleoside linking groups are referred to as oligonucleosides. Modified internucleoside linkages, compared to natural phosphodiester linkages, can be used to alter, typically increase, nuclease resistance of the antisense compound. Internucleoside linkages having a chiral atom can be prepared racemic, chiral, or as a mixture. Representative chiral internucleoside linkages include, but are not limited to, alkylphosphonates and phosphorothioates. Methods of preparation of phosphorous-containing and non-phosphorous-containing linkages are well known to those skilled in the art.

In some embodiments, a phosphate group can be linked to the 2', 3' or 5' hydroxyl moiety of the sugar. In forming oligonucleotides, the phosphate groups covalently link adjacent nucleosides to one another to form a linear polymeric compound. Within oligonucleotides, the phosphate groups are commonly referred to as forming the internucleoside backbone of the oligonucleotide. The normal linkage or backbone of RNA and DNA is a 3' to 5' phosphodiester linkage.

### Conjugate Groups

In some embodiments, ACs are modified by covalent attachment of one or more conjugate groups. In general, conjugate groups modify one or more properties of the attached AC including but not limited to pharmacodynamic, pharmacokinetic, binding, absorption, cellular distribution, cellular uptake, charge and clearance. Conjugate groups are routinely used in the chemical arts and are linked directly or via an optional linking moiety or linking group to a parent compound such as an AC. A preferred list of conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. In some embodiments, the conjugate group is a polyethylene glycol (PEG), and the PEG is conjugated to either the AC or the CPP.

Certain conjugate groups amenable to the present invention include lipid moieties such as a cholesterol moiety (Letsinger et al., Proc. Natl. Acad. Sci. USA, 1989, 86, 6553); cholic acid (Manoharan et al., Bioorg. Med. Chem. Lett., 1994, 4, 1053); a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765); a thiocholesterol (Oberhauser et al., Nucl. Acids Res., 1992, 20, 533); an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49); a phospholipid, e.g., di-hexadecyl-rac-glycerol or triethylammonium-1,2-di-O-hexadecyl-rac-glycero-3-H-phosphonate (Manoharan et al., Tetrahedron Lett., 1995, 36, 3651; Shea et al., Nucl. Acids Res., 1990, 18, 3777); a polyamine or a polyethylene glycol chain (Manoharan et al., Nucleosides & Nucleotides, 1995, 14, 969); adamantane acetic acid (Manoharan et al., Tetrahedron Lett., 1995, 36, 3651); a palmityl moiety (Mishra et al., Biochim. Biophys. Acta, 1995, 1264, 229); or an octadecylamine or hexylamino-carbonyl-oxycholesterol moiety (Crooke et al., J. Pharmacol. Exp. Ther., 1996,277,923).

Linking groups or bifunctional linking moieties such as those known in the art are amenable to the compounds provided herein. Linking groups are useful for attachment of chemical functional groups, conjugate groups, reporter groups and other groups to selective sites in a parent compound such as for example an AC. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. Any of the linkers described here may be used. In some embodiments, the linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C1-C10 alkyl, substituted or unsubstituted C2-C10 alkenyl or substituted or unsubstituted C2-C10 alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

In some embodiments, the AC may be linked to a 10 arginine-serine dipeptide repeat. ACs linked to 10 arginine-serine dipeptide repeats for the artificial recruitment of splicing enhancer factors have been applied in vitro to induce inclusion of mutated BRCA1 and SMN2 exons that otherwise would be skipped. See Cartegni and Krainer 2003, incorporated by reference herein.

In some embodiments, the AC may be between 5 and 50 nucleotides in length. In some embodiments, the AC may be 5-10 nucleotides in length. In some embodiments, the AC may be 10-15 nucleotides in length. In some embodiments, the AC may be 15-20 nucleotides in length. In some embodiments, the AC may be 20-25 nucleotides in length. In some embodiments, the AC may be 25-30 nucleotides in length. In some embodiments, the AC may be 30-35 nucleotides in length. In some embodiments, the AC may be 35-40 nucleotides in length. In some embodiments, the AC may be 40-45 nucleotides in length. In some embodiments, the AC may be 45-50 nucleotides in length.

### Clustered Regularly Interspaced Short Palindromic Repeats (CRISPR) Gene-Editing Machinery

In some embodiments, the compounds disclosed herein comprise one or more CPP (or cCPP) conjugated to CRISPR gene-editing machinery. As used herein, "CRISPR gene-editing machinery" refers to protein, nucleic acids, or combinations thereof, which may be used to edit a genome. Non-limiting examples of gene-editing machinery include gRNAs, nucleases, nuclease inhibitors, and combinations and complexes thereof. The following patent documents describe CRISPR gene-editing machinery: U.S. Pat. No. 8,697,359, U.S. Pat. No. 8,771,945, U.S. Pat. No. 8,795,965, U.S. Pat. No. 8,865,406, U.S. Pat. No. 8,871,445, U.S. Pat. No. 8,889,356, U.S. Pat. No. 8,895,308, U.S. Pat. No. 8,906,616, U.S. Pat. No. 8,932,814, U.S. Pat. No. 8,945,839, U.S. Pat. No. 8,993,233, U.S. Pat. No. 8,999,641, U.S. Pat. App. No. 14/704,551, and U.S. Pat. App. No. 13/842,859. Each of the aforementioned patent documents is incorporated by reference herein in its entirety.

In some embodiments, a linker conjugates the CPP (or cCPP) to the CRISPR gene-editing machinery. Any linker described in this disclosure or that is known to a person of skill in the art may be utilized.

### gRNA

In some embodiments, the compounds comprise the CPP (or cCPP) is conjugated to a gRNA. A gRNA targets a genomic loci in a prokaryotic or eukaryotic cell.

In some embodiments, the gRNA is a single-molecule guide RNA (sgRNA). A sgRNA comprises a spacer sequence and a scaffold sequence. A spacer sequence is a short nucleic acid sequence used to target a nuclease (e.g., a Cas9 nuclease) to a specific nucleotide region of interest (e.g., a genomic DNA sequence to be cleaved). In some embodiments, the spacer may be about 17-24 base pairs in length, such as about 20 base pairs in length. In some embodiments, the spacer may be about 15, about 16, about 17, about 18, about 19, about 20, about 21, about 22, about 23, about 24, about 25, about 26, about 27, about 28, about 29, or about 30 base pairs in length. In some embodiments, the spacer may be at least 15, at least 16, at least 17, at least 18, at least 19, at least 20, at least 21, at least 22, at least 23, at least 24, at least 25, at least 26, at least 27, at least 28, at least 29, or at least 30 base pairs in length. In some embodiments, the spacer may be 15, 16, 17, 18, 19, 20, 21, 22, 23, 24, 25, 26, 27, 28, 29, or 30 base pairs in length. In some embodiments, the spacer sequence has between about 40% to about 80% GC content.

In some embodiments, the spacer targets a site that immediately precedes a 5' protospacer adjacent motif (PAM). The PAM sequence may be selected based on the desired nuclease. For example, the PAM sequence may be any one of the PAM sequences shown in Table 5 below, wherein N refers to any nucleic acid, R refers to A or G, Y refers to C or T, W refers to A or T, and V refers to A or C or G.

**Table 6. Exemplary Nucleases and PAM sequences**

| **PAM sequence (5' to 3')** | **Nuclease** | **Isolated from** |
|---|---|---|
| NGG | SpCas9 | *Streptococcus pyogenes* |
| NGRRT or NGRRN | SaCas9 | *Staphylococcus aureus* |
| NNNNGATT | NmeCas9 | *Neisseria meningitidis* |
| NNNNRYAC | CjCas9 | *Campylobacter jejuni* |
| NNAGAAW | StCas9 | *Streptococcus thermophiles* |
| TTTV | LbCpf1 | *Lachnospiraceae bacterium* |
| TTTV | AsCpf1 | *Acidaminococcus sp.* |

In some embodiments, a spacer may target a sequence of a mammalian gene, such as a human gene. In some embodiments, the spacer may target a mutant gene. In some embodiments, the spacer may target a coding sequence. In some embodiments, the spacer may target an exonic sequence.

The scaffold sequence is the sequence within the sgRNA that is responsible for nuclease (e.g., Cas9) binding. The scaffold sequence does not include the spacer/targeting sequence. In some embodiments, the scaffold may be about 1 to about 10, about 10 to about 20, about 20 to about 30, about 30 to about 40, about 40 to about 50, about 50 to about 60, about 60 to about 70, about 70 to about 80, about 80 to about 90, about 90 to about 100, about 100 to about 110, about 110 to about 120, or about 120 to about 130 nucleotides in length. In some embodiments, the scaffold may be about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60,about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, about 99, about 100, about 101, about 102, about 103, about 104, about 105, about 106, about 107, about 108, about 109, about 110, about 111, about 112, about 113, about 114, about 115, about 116, about 117, about 118, about 119, about 120, about 121, about 122, about 123, about 124, or about 125 nucleotides in length. In some embodiments, the scaffold may be at least 10, at least 20, at least 30, at least 40, at least 50, at least 60, at least 70, at least 80, at least 90, at least 100, at least 110, at least 120, or at least 125 nucleotides in length.

In some embodiments, the gRNA is a dual-molecule guide RNA, e.g, crRNA and tracrRNA. In some embodiments, the gRNA may further comprise a polyA tail.

In some embodiments, a compound comprising a CPP is conjugated to a nucleic acid comprising a gRNA. In some embodiments, the nucleic acid comprises about 1, 2, 3, 4, 5, 6, 7, 8, 9, 10, 11, 12, 13, 14, 15, 16, 17, 18, 19, or 20 gRNAs. In some embodiments, the gRNAs recognize the same target. In some embodiments, the gRNAs recognize different targets. In some embodiments, the nucleic acid comprising a gRNA comprises a sequence encoding a promoter, wherein the promoter drives expression of the gRNA.

### Nuclease

In some embodiments, the compounds comprise a cell penetrating peptide conjugated to a nuclease. In some embodiments, the nuclease is a Type II, Type V-A, Type V-B, Type VC, Type V-U, Type VI-B nuclease. In some embodiments, the nuclease is a transcription, activator-like effector nuclease (TALEN), a meganuclease, or a zinc-finger nuclease. In some embodiments, the nuclease is a Cas9, Cas12a (Cpfl), Cas12b, Cas12c, Tnp-B like, Cas13a (C2c2), Cas13b, or Cas14 nuclease. For example, in some embodiments, the nuclease is a Cas9 nuclease or a Cpfl nuclease.

In some embodiments, the nuclease is a modified form or variant of a Cas9, Cas12a (Cpf1), Cas12b, Cas12c, Tnp-B like, Cas13a (C2c2), Cas13b, or Cas14 nuclease. In some embodiments, the nuclease is a modified form or variant of a TAL nuclease, a meganuclease, or a zinc-finger nuclease. A "modified" or "variant" nuclease is one that is, for example, truncated, fused to another protein (such as another nuclease), catalytically inactivated, etc. In some embodiments, the nuclease may have at least 80%, at least 85%, at least 90%, at least 95%, at least 98%, at least 99%, or 100% sequence identity to a naturally occurring Cas9, Cas12a (Cpfl), Cas12b, Cas12c, Tnp-B like, Cas13a (C2c2), Cas13b, Cas14 nuclease, or a TALEN, meganuclease, or zinc-finger nuclease. In embodiments, the nuclease is a Cas9 nuclease derived from *S. pyogenes* (SpCas9). In some embodiments, a nuclease has at least 90%, at least 95%, at least 96%, at least 97%, at least 98%, or at least 99% sequence identity to a Cas9 nuclease derived from *S. pyogenes* (SpCas9). In embodiments, the nuclease is a Cas9 derived from *S. aureus* (SaCas9). In some embodiments, the nuclease has at least 90%, at least 95%, at least 96%, at least 97%, at least 98%, or at least 99% sequence identity to a Cas9 derived from *S*. *aureus* (SaCas9). In embodiments, the Cpfl is a Cpf1 enzyme from *Acidaminococcus* (species BV3L6, UniProt Accession No. U2UMQ6). In some embodiments, the nuclease has at least 90%, at least 95%, at least 96%, at least 97%, at least 98%, or at least 99% sequence identity to a Cpfl enzyme from *Acidaminococcus* (species BV3L6, UniProt Accession No. U2UMQ6).

In some embodiments, the Cpfl is a Cpfl enzyme from *Lachnospiraceae* (species ND2006, UniProt Accession No. A0A182DWE3). In some embodiments, the nuclease has at least 90%, at least 95%, at least 96%, at least 97%, at least 98%, or at least 99% sequence identity to a Cpfl enzyme from *Lachnospiraceae.* In some embodiments, a sequence encoding the nuclease is codon optimized for expression in mammalian cells. In some embodiments, the sequence encoding the nuclease is codon optimized for expression in human cells or mouse cells.

In some embodiments, a compound comprising a CPP is conjugated to a nuclease. In some embodiments, the nuclease is a soluble protein.

In some embodiments, a compound comprising a CPP is conjugated to a nucleic acid encoding a nuclease. In some embodiments, the nucleic acid encoding a nuclease comprises a sequence encoding a promoter, wherein the promoter drives expression of the nuclease.

### gRNA and Nuclease Combinations

In some embodiments, the compounds comprise one or more CPP (or cCPP) conjugated to a gRNA and a nuclease. In some embodiments, the one or more CPP (or cCPP) are conjugated to a nucleic acid encoding a gRNA and/or a nuclease. In some embodiments, the nucleic acid encoding a nuclease and a gRNA comprises a sequence encoding a promoter, wherein the promoter drives expression of the nuclease and the gRNA. In some embodiments, the nucleic acid encoding a nuclease and a gRNA comprises two promoters, wherein a first promoter controls expression of the nuclease and a second promoter controls expression of the gRNA. In some embodiments, the nucleic acid encoding a gRNA and a nuclease encodes 1, 2, 3, 4, 5, 6, 7, 8, 9, 10, 11, 12, 13, 14, 15, 16, 17, 18, 19, or 20 gRNAs. In some embodiments, the gRNAs recognize different targets. In some embodiments, the gRNAs recognize the same target.

In some embodiments, the compounds comprise a cell penetrating peptide (or cCPP) conjugated to a ribonucleoprotein (RNP) comprising a gRNA and a nuclease.

In some embodiments, a composition comprising: (a) a CPP conjugated to a gRNA and (b) a nuclease is delivered to a cell. In some embodiments, a composition comprising: (a) a CPP conjugated to a nuclease and (b) an gRNA is delivered to a cell.

In some embodiments, a composition comprising: (a) a first CPP conjugated to a gRNA and (b) a second CPP conjugated to a nuclease is delivered to a cell. In some embodiments, the first CPP and second CPP are the same. In some embodiments, the first CPP and second CPP are different.

### Genetic Element of Interest

In some embodiments, the compounds disclosed herein comprise a cell penetrating peptide conjugated to a genetic element of interest. In some embodiments, a genetic element of interest replaces a genomic DNA sequence cleaved by a nuclease. Non-limiting examples of genetic elements of interest include genes, a single nucleotide polymorphism, promoter, or terminators.

### Nuclease Inhibitors

In some embodiments, the compounds disclosed herein comprise a cell penetrating peptide conjugated to an inhibitor of a nuclease (e.g. Cas9). A limitation of gene editing is potential off-target editing. The delivery of a nuclease inhibitor will limit off-target editing. In some embodiments, the nuclease inhibitor is a polypeptide, polynucleotide, or small molecule. Exemplary nuclease inhibitors are described in U.S. Publication No. 2020/087354, International Publication No. 2018/085288, U.S. Publication No. 2018/0382741, International Publication No. 2019/089761, International Publication No. 2020/068304, International Publication No. 2020/041384, and International Publication No. 2019/076651, each of which is incorporated by reference herein in its entirety.

### Mechanism of Modulation and Target Molecules

Many types of oligonucleotides are capable of modulating gene transcription, translation and/or protein function in cells. Non-limiting examples of such oligonucleotides include, e.g. , small interfering RNA (siRNA), microRNA (miRNA), antisense oligonucleotides, ribozymes, plasmids, immune stimulating nucleic acids, antisense, antagomir, antimir, microRNA mimic, supermir, U1 adaptor, and aptamer. Additional examples include DNA-targeting, triplex-forming oligonucleotide, strand-invading oligonucleotide, and synthetic guide strand for CRISPR/Cas, These nucleic acids act via a variety of mechanisms. See Smith and Zain, Annu Rev Pharmacol Toxicol. 2019, 59:605-630, incorporated by reference herein.

Splice-switching antisense oligonucleotides are short, synthetic, antisense, modified nucleic acids that base-pair with a pre-mRNA and disrupt the normal splicing repertoire of the transcript by blocking the RNA-RNA base-pairing or protein-RNA binding interactions that occur between components of the splicing machinery and the pre-mRNA. Splicing of pre-mRNA is required for the proper expression of the vast majority of protein-coding genes, and thus, targeting the process offers a means to manipulate protein production from a gene. Splicing modulation is particularly valuable in cases of disease caused by mutations that lead to disruption of normal splicing or when interfering with the normal splicing process of a gene transcript may be therapeutic. Such antisense oligonucleotides offer an effective and specific way to target and alter splicing in a therapeutic manner. See Havens and Hastings, Nucleic Acids Res. 2016 Aug 19;44(14):6549-6563, incorporated by reference herein.

In the case of siRNA or miRNA, these nucleic acids can down-regulate intracellular levels of specific proteins through a process termed RNA interference (RNAi). Following introduction of siRNA or miRNA into the cell cytoplasm, these double-stranded RNA constructs can bind to a protein termed RISC. The sense strand of the siRNA or miRNA is displaced from the RISC complex providing a template within RISC that can recognize and bind mRNA with a complementary sequence to that of the bound siRNA or miRNA. Having bound the complementary mRNA the RISC complex cleaves the mRNA and releases the cleaved strands. RNAi can provide down-regulation of specific proteins by targeting specific destruction of the corresponding mRNA that encodes for protein synthesis.

The therapeutic applications of RNAi are extremely broad, since siRNA and miRNA constructs can be synthesized with any nucleotide sequence directed against a target protein. To date, siRNA constructs have shown the ability to specifically down- regulate target proteins in both in vitro and in vivo models, as well as in clinical studies.

Antisense oligonucleotides and ribozymes can also inhibit mRNA translation into protein. In the case of antisense constructs, these single stranded deoxynucleic acids have a complementary sequence to that of the target protein mRNA and can bind to the mRNA by Watson-Crick base pairing. This binding either prevents translation of the target mRNA and/or triggers RNase H degradation of the mRNA transcripts, Consequently, antisense oligonucleotides have tremendous potential for specificity of action (i.e., down-regulation of a specific disease-related protein). To date, these compounds have shown promise in several in vitro and in vivo models, including models of inflammatory disease, cancer, and HIV (reviewed in Agrawal, Trends in Biotech. 14:376-387 (1996)). Antisense can also affect cellular activity by hybridizing specifically with chromosomal DNA.

Immune-stimulating nucleic acids include deoxyribonucleic acids and ribonucleic acids. In the case of deoxyribonucleic acids, certain sequences or motifs have been shown to illicit immune stimulation in mammals. These sequences or motifs include the CpG motif, pyrimidine-rich sequences and palindromic sequences. It is believed that the CpG motif in deoxyribonucleic acids is specifically recognized by an endosomal receptor, tolllike receptor 9 (TLR-9), which then triggers both the innate and acquired immune stimulation pathway. Certain immune stimulating ribonucleic acid sequences have also been reported. It is believed that these RNA sequences trigger immune activation by binding to toll-like receptors 6 and 7 (TLR-6 and TLR-7). In addition, double-stranded RNA is also reported to be immune stimulating and is believe to activate via binding to TLR-3.

Non-limiting examples of mechanism and targets of antisense oligonucleotides (ASOs) to modulate gene transcription, translation and/or protein function are illustrated in Table 7A and 7B.

**Table 7A. Mechanism of ASO Modulation and Target Molecules**

| **Types** | **Location of target** | **Subcellular Location** | **Mechanism** |
|---|---|---|---|
| **mRNA** | **Intracellular** | **cytoplasm** | **inhibition of translation** |
| **Pre-mRNA** | **Intracellular** | **nucleus** | **alternative splicing** |
| **micro-RNA** | **Intracellular** | **cytoplasm and nucleus** | **miRNA inhibition or activation** |
| **long non-coding RNA** | **Intracellular** | **cytoplasm and nucleus** | **inhibition of IncRNA function** |
| **Telomerase RNA component** | **Intracellular** | **cytoplasm and nucleus** | **inhibition of telomerase** |
| **Protein** | **Extra- and intracellular** | **cytoplasm and nucleus** | **inhibition of protein target** |

**Table 7B. Mechanism of ASO Modulation and Target Molecules**

| Mechanism | Target | Description | Examples of drugs |
|---|---|---|---|
| Regulation of pre-mRNA splicing | pre-mRNA | ASOs bind to pre-mRNA and alter the splicing by steric blocking, which result in disruption of the recognition by splicing factors | Nusinersen, Eteplirsen |
| Regulation of RNA translation by recruiting RNase H | pre-mRNA and mRNA | ASOs containing DNA bases bind to target RNA and induce the cleavage of RNA by RNase H | Mipomersen, Inotersen |
| Regulation of RNA translation by steric blocking | mRNA | ASOs and duplex RNA can both sterically block the translation machinery to inhibit RNA translation or enhance RNA translation by blocking aberrant sites that reduce RNA translation | |
| Regulation of RNA translation by RNAi | mRNA | siRNA and miRNA inhibit translation by RNA interference and induce the cleavage of target RNA | Patisiran, Inclisiran, Fitusiran, Givosiran |
| Regulation of protein activity by binding with target proteins | protein | Aptamers bind with target proteins as antagonists | Pegaptanib |

Clustered regularly interspaced short palindromic repeats (CRISPR) and associated Cas proteins constitute the CRISPR-Cas system. CRISPR-Cas is a mechanism for gene-editing. The RNA-guided (e.g., gRNA) Cas9 endonuclease specifically targets and cleaves DNA in a sequence-dependent manner. The Cas9 endonuclease can be substituted with any nuclease of the disclosure. The gRNA targets a nuclease (e.g., a Cas9 nuclease) to a specific nucleotide region of interest (e.g., a genomic DNA sequence to be cleaved) and cleaves genomic DNA. Genomic DNA can then be replaced with a genetic element of interest.

### Diseases and Target Genes

The human genome comprises more than 40,000 genes, approximately half of which correspond to protein-coding genes. However, the number of human protein species is predicted to be orders of magnitude higher due to single amino acid polymorphisms, post translational modifications, and, importantly, alternative splicing. RNA splicing, generally taking place in the nucleus, is the process by which precursor messenger RNA (pre-mRNA) is transformed into mature messenger RNA (mRNA) by removing non-coding regions (introns) and joining together the remaining coding regions (exons). The resulting mRNA can then be exported from the nucleus and translated into protein. Alternative splicing, or differential splicing, is a regulated process during gene expression that results in a single gene coding for multiple proteins. In this process, particular exons of a gene may be included within or excluded from the final, processed mRNA produced from that gene. While alternative splicing is a normal phenomenon in eukaryotic organisms, and contributes to the biodiversity of proteins encoded by a genome, abnormal variations in splicing are heavily implicated in disease. A large proportion of human genetic disorders result from splicing variants; abnormal splicing variants contribute to the development of cancer; and splicing factor genes are frequently mutated in different types of cancer.

About 10% of ~80,000 mutations reported in the human gene mutation database (HGMD) affect splice sites. In the HGMD, there are 3390 disease-causing mutations that occur at the +1 donor splice site. These mutations affect 2754 exons in 901 genes. The prevalence is even higher for neuromuscular disorders (NMDs) due to the unusually large size and multiexonic structure of genes encoding muscle structural proteins, further highlighting the importance of these mutations in NMDs.

Previously, the correction of point mutations, e.g. splice site mutations, has been attempted via the homology-directed repair (HDR) pathway, which is extremely inefficient in post-mitotic tissues such as skeletal muscles, hampering its therapeutic utility in NMD. In addition, standard gene therapy approaches to reintroduce corrected coding regions into the genome are impeded by the large size of genes encoding, e.g., muscular structural proteins. Furthermore, many existing therapies rely on inefficient introduction of the therapeutic compound into the disease cells, such that in vivo treatment is impractical and higher toxicities are experienced.

The target gene of the present disclosure may be any eukaryotic gene comprising one or more introns and one or more exons. In some embodiments, the target gene is a mammalian gene. In some embodiments, the mammal is a human, mouse, bovine, rat, pig, horse, chicken, sheep, or the like. In some embodiments, the target gene is a human gene.

In some embodiments, the target gene is a gene comprising mutations leading to aberrant splicing. In some embodiments, the target gene is a gene that comprises one or more mutations. In some embodiments, the target gene is a gene that comprises one or more mutations, such that transcription and translation of the target gene does not lead to a functional protein. In some embodiments, the target gene is a gene that comprises one or more mutations, such that transcription and translation of the target gene leads to a target protein that is less active or less functional than a wild type target protein.

In some embodiments, the target gene is a gene underlying a genetic disorder. In some embodiments, the target gene has abnormal gene expression in the central nervous system. In some embodiments, the target gene is a gene involved in the pathogenesis of a neuromuscular disorder (NMD). In some embodiments, the target gene is a gene involved in the pathogenesis of a musculoskeletal disorder (NMD). In some embodiments, the neuromuscular disease is Pompe disease, and the target gene is GYS1.

Antisense compounds may be used to target genes comprising mutations that lead to aberrant splicing underlying a genetic disease in order to redirect splicing to give a desired splice product (Kole, Acta Biochimica Polonica, 1997, 44, 231-238).

CRISPR gene-editing machinery may be used to target aberrant genes for removal or to regulate gene transcription and translation.

In some embodiments, the disease is β-thalassemia (Dominski and Kole, Proc. Natl. Acad. Sci. USA, 1993, 90, 8673-8677; Sierakowska et al., Nucleosides & Nucleotides, 1997, 16,1173-1182; Sierakowska et al., Proc. Natl. Acad. Sci. USA, 1996, 93, 12840-44; Lacerra et al., Proc. Natl. Acad. Sci. USA, 2000, 97, 9591-9596).

In some embodiments, the disease is dystrophin Kobe (Takeshima et al., J. Clin. Invest., 1995, 95, 515-520).

In some embodiments, the disease is Duchenne muscular dystrophy (Dunckley et al. Nucleosides & Nucleotides, 1997, 16, 1665-1668; Dunckley et al. Human Mol. Genetics, 1998, 5, 1083-90). In some embodiments, the target gene is the DMD gene, which codes for dystrophin. The protein consists of an N-terminal domain that binds to actin filaments, a central rod domain, and a C-terminal cysteine-rich domain that binds to the dystrophin-glycoprotein complex (Hoffman et al. 1987; Koenig et al. 1988; Yoshida and Ozawa 1990). Mutations in the DMD gene that interrupt the reading frame result in a complete loss of dystrophin function, which causes severe Duchenne muscular dystrophy (DMD) [MIM 310200]). The milder Becker muscular dystrophy (BMD [MIM 300376]), on the other hand, is the result of mutations in the same gene that are not frameshifting and result in an internally deleted but partially functional dystrophin that has retained its N- and C-terminal ends (Koenig et al. 1989; Di Blasi et al. 1996). Over two-thirds of patients with DMD and BMD have a deletion of >1 exon (den Dun-nen et al. 1989). Remarkably, patients have been described who exhibit very mild BMD and who lack up to 67% of the central rod domain (England et al. 1990; Winnard et al. 1993; Mirabella et al. 1998). This suggests that, despite large deletions, a partially functional dystrophin can be generated, provided that the deletions render the transcript in frame. These observations have led to the idea of using ACs to alter splicing so that the open reading frame is restored and the severe DMD phenotype is converted into a milder BMD phenotype. Several studies have shown therapeutic AC-induced single-exon skipping in cells derived from the mdx mouse model (Dunckley et al. 1998; Wilton et al. 1999; Mann et al. 2001, 2002; Lu et al. 2003) and various DMD patients (Takeshima et al. 2001; van Deutekom et al. 2001; Aartsma-Rus et al. 2002, 2003; De Angelis et al. 2002). In some embodiments, the AC of the present disclosure is used to skip one or more exons selected from exons 2, 8, 11, 17, 19, 23, 29, 40, 41, 42, 43, 44, 45, 46, 48, 49, 50, 51, 52, 53, 55, and 59 of DMD. See Aartsma-Rus et al. 2002, incorporated by reference herein. In some embodiments, the AC of the present disclosure is used to skip one or more exons selected from exons 8, 11, 43, 44, 45, 50, 51, 53, and 55 of DMD. Of all patients with DMD, ~75% would benefit from the skipping of these exons. The skipping of exons flanking out-of-frame deletions or an in-frame exon containing a nonsense mutation can restore the reading frame and induce the synthesis of BMD-like dystrophins in treated cells. (van Deutekom et al. 2001; Aartsma-Rus et al. 2003). In some embodiments, the AC hybridizing to its target sequence within a target DMD pre-mRNA induces the skipping of one or more exons. In some embodiments, the AC induces expression of a re-spliced target protein comprising an active fragment of dystrophin.Non-limiting examples of AC for exon 52 are described in US Pub. No. 2019/0365918, which is incorporated by reference in its entirety for all purposes.

In some embodiments, provided herein are compounds comprising an AC and CPP that target the DMD gene. Non-limiting examples of the aforementioned compounds are shown below. The antisense oligonucleotide is underlined (SEQ ID NO: 218).

### ENTR-0088:

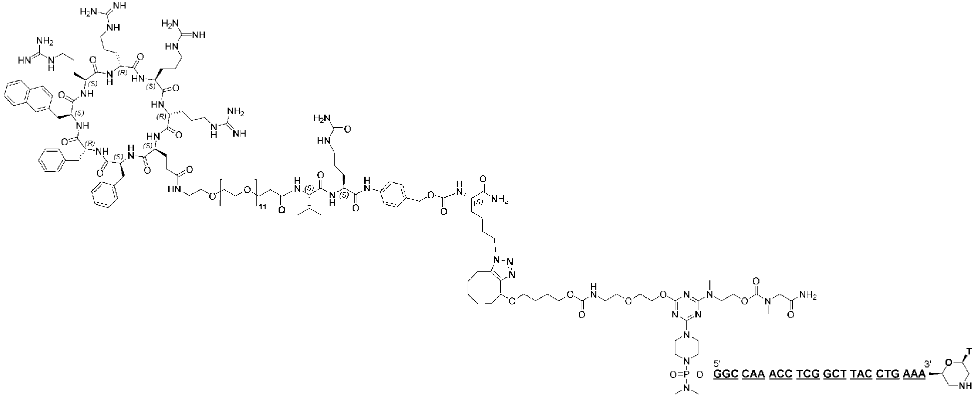

### ENTR-0093:

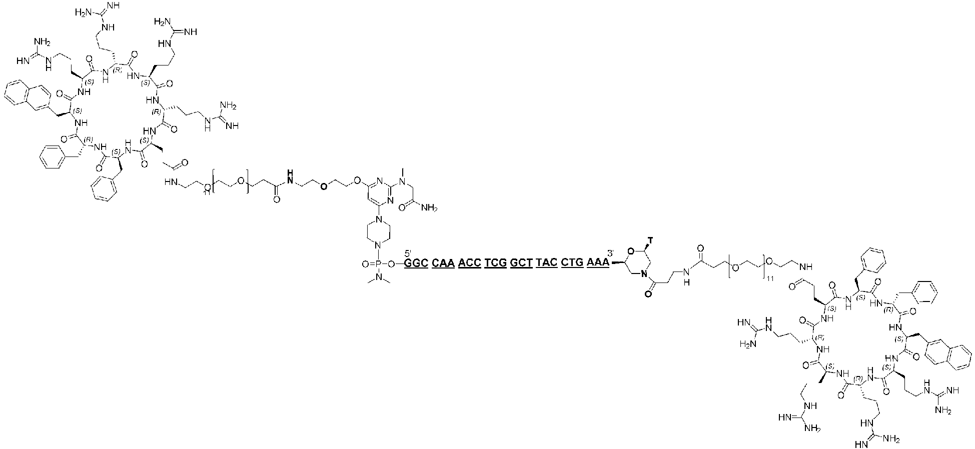

### ENTR-0098:

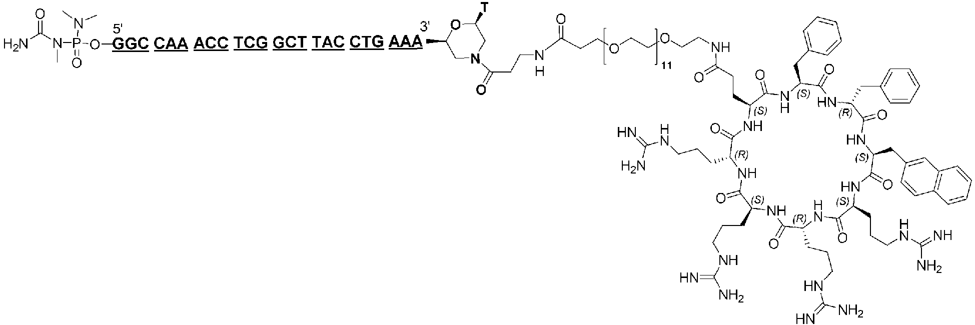

### ENTR-0099:

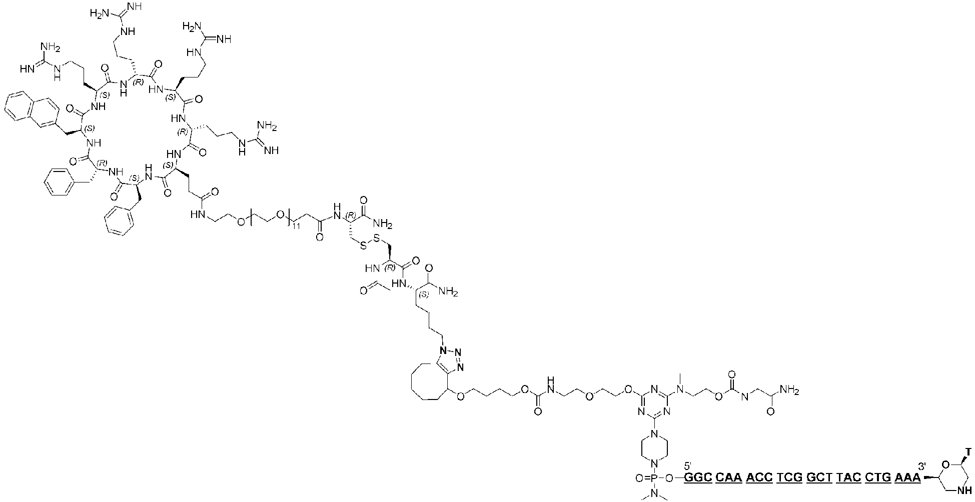

### ENTR-0014:

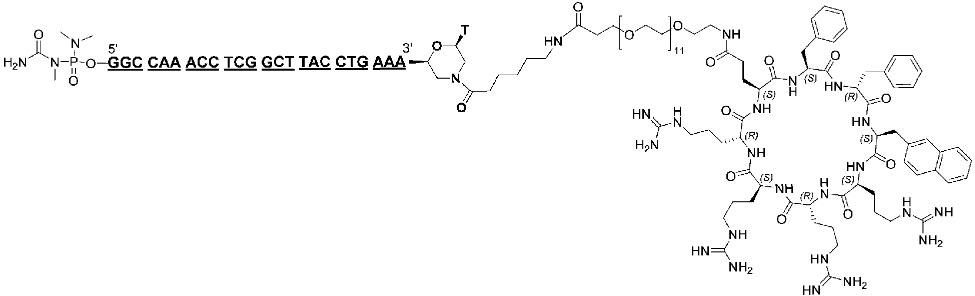

### ENTR-0100:

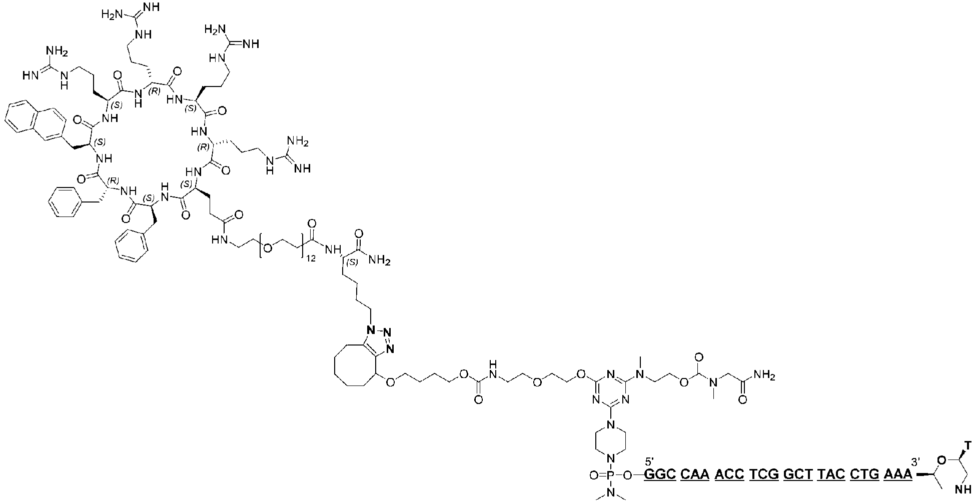

### ENTR-0115:

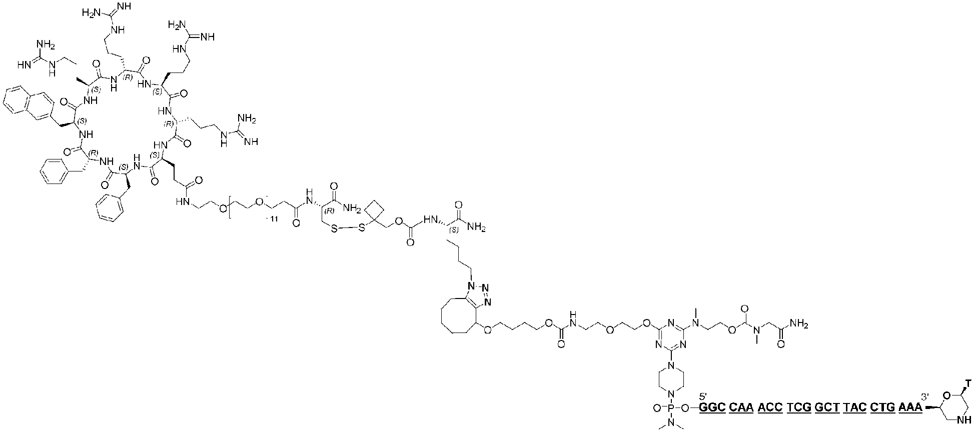

### ENTR-0120:

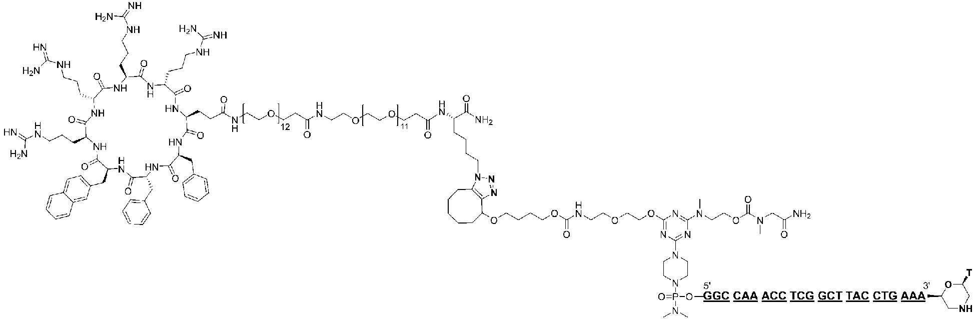

### ENTR-0161:

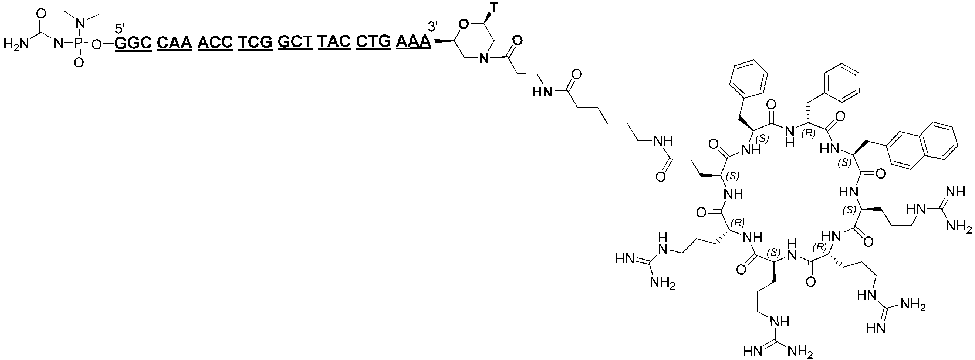

### ENTR-0089:

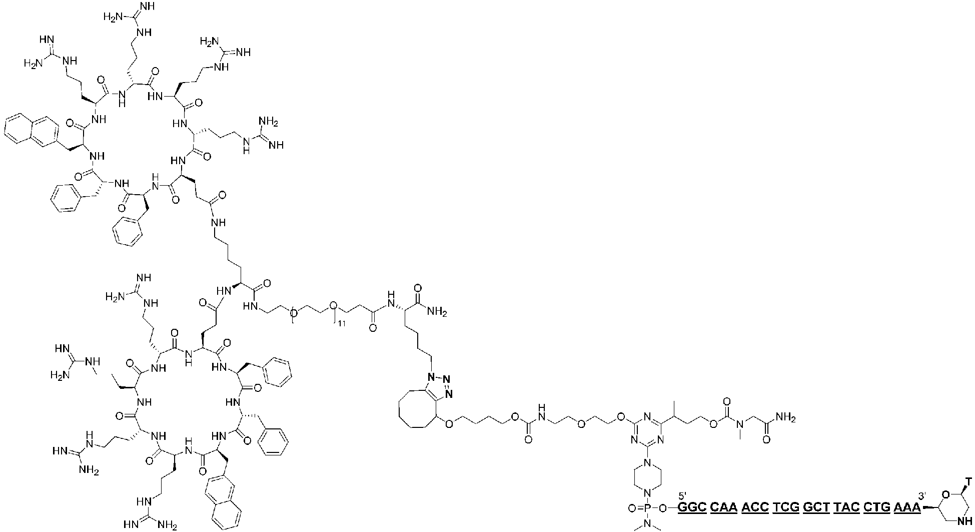

### ENTR-0119:

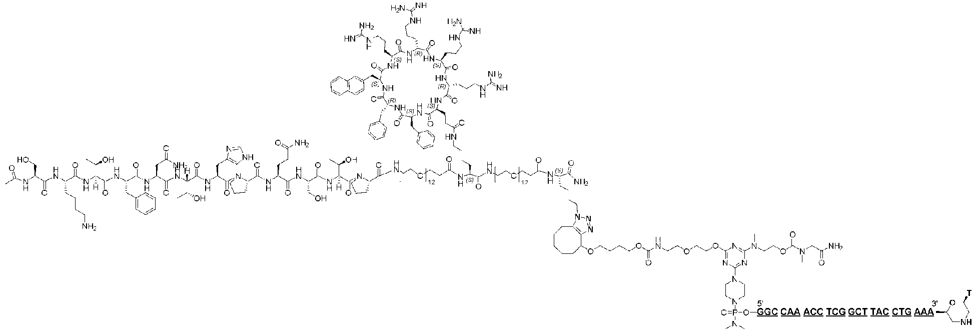

### ENTR-0092:

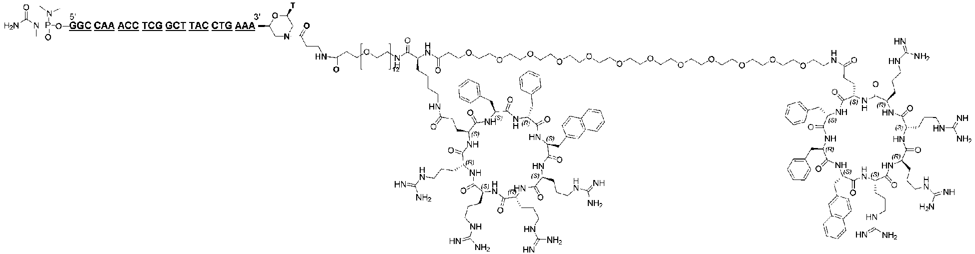

### ENTR-0163:

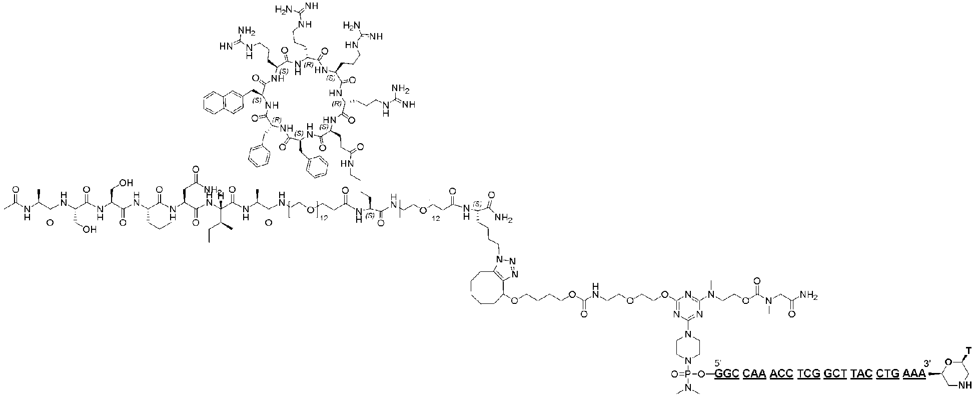

In some embodiments, the disease is an ocular disease. In some embodiments, the ocular disease is refractive errors, macular degeneration, cataracts, diabetic retinopathy, glaucoma, amblyopia, or strabismus. In some embodiments, the target gene is VEGF, ABCA4, CEP290, RHO, USH2A, OPA1, CNGB3, PRPF31, RPGR.

In some embodiments, the disease is associated with insulin resistance. In some embodiments, the disease is diabetes. In some embodiments, the target gene is PTP.

In some embodiments, the disease is a CNS disorder. In some embodiments, the disease is Alzheimer's Disease (AD) (Zhao et al. Gerontology 2019;65:323-331). In some embodiments, the target gene is the CD33 gene. The CD33 gene maps on chromosome 19q13.33 in humans encoding a 67-kDa transmembrane glycoprotein. Human CD33 binds preferentially to alpha-2,6-linked sialic acid. CD33 is expressed exclusively on immune cells. CD33 is an inhibitory receptor that recruits inhibitory proteins such as SHP phosphatases via its immunoreceptor tyrosine-based inhibition motif (ITIM). CD33 is also involved in adhesion processes in immune or malignant cells, inhibition of cytokine release by monocytes, immune cell growth and survival through the inhibition of proliferation, and induction of apoptosis. Polymorphisms of CD33 have been implicated in modulating AD susceptibility. rs3865444C is an allele associated with an increased risk of AD in European, Chinese, and North American populations due to increased expression of CD33. The skipping of exon 2 of CD33 leads to a decreased expression of CD33 and an increased expression of D2-CD33, which is a CD33 isoform that lacks a ligand binding domain. Expression of D2-CD33 is associated with a decreased risk of developing AD. In some embodiments, the AC of the present disclosure is used to skip an exon of CD33 selected from the group consisting of exon 1, exon 2, exon 3, exon 4, exon 5, exon 6, exon 7a, and exon 7b. In some embodiments, the exon is exon 2. In some embodiments, the AC hybridizing to its target sequence within a target CD33 pre-mRNA induces the skipping of one or more exons. In some embodiments, the AC induces expression of a re-spliced target protein comprising an inactive fragment of CD33.

In some embodiments, the disease is cancer (Laszlo et al. Oncotarget. 2016 Jul 12; 7(28): 43281-43294.). In some embodiments, the cancer is acute myeloid leukemia (AML). In some embodiments, the cancer is glioma, thyroid cancer, lung cancer, colorectal cancer, head and neck cancer, stomach cancer, liver cancer, pancreatic cancer, renal cancer, urothelial cancer, prostate cancer, testis cancer, breast cancer, cervical cancer, endometrial cancer, ovarian cancer, or melanoma. In some embodiments, the target gene is the CD33 gene. Each of the aforementioned cancers express CD33. In some embodiments, the AC of the present disclosure is used to skip an exon of CD33. In some embodiments, the exon is selected from exon 1, exon 2, exon 3, exon 4, exon 5, exon 6, exon 7a, and exon 7b. In some embodiments, the target gene is Myc, STAT3, MDM4, ERRB4, BCL2L1, GLDC, PKM2, MCL1, MDM2, BRCA2, IL5R, FGFR1, MSTR1, USP5, or CD33.

In some embodiments, provided herein are compounds comprising an AC and CPP that target the CD33 gene. Non-limiting examples of the aforementioned compounds are shown below. The antisense oligonucleotide is underlined (SEQ ID NOs: 219 and 220).

### ENTR-0036:

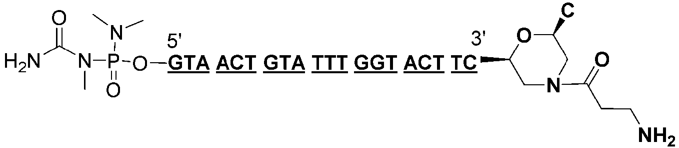

### ENTR-0081:

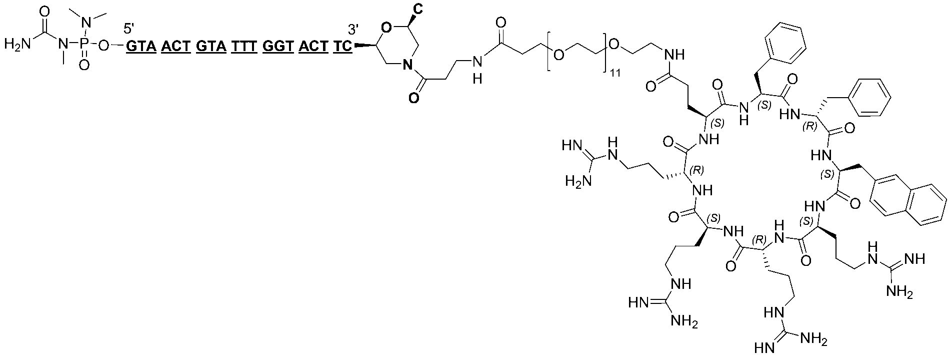

### ENTR-0087:

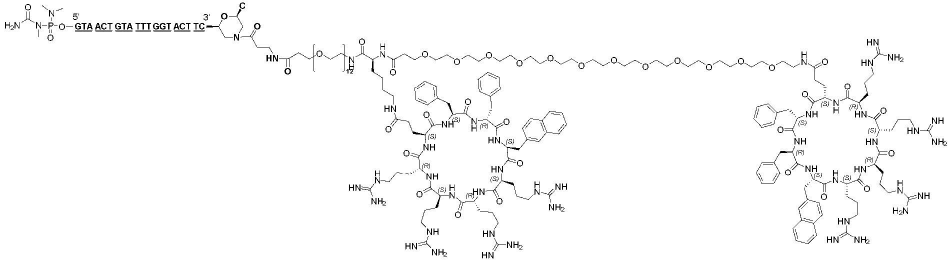

### ENTR-0085:

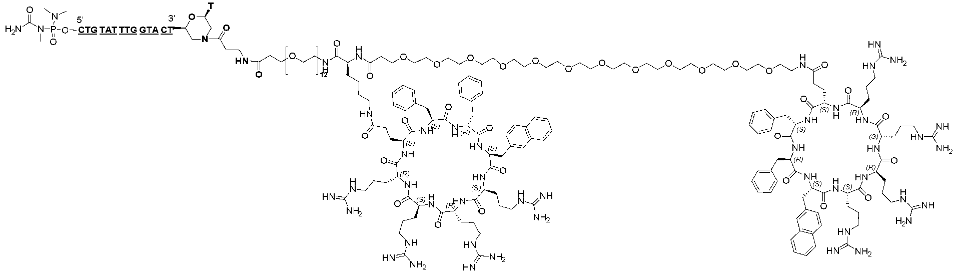

### ENTR-0179:

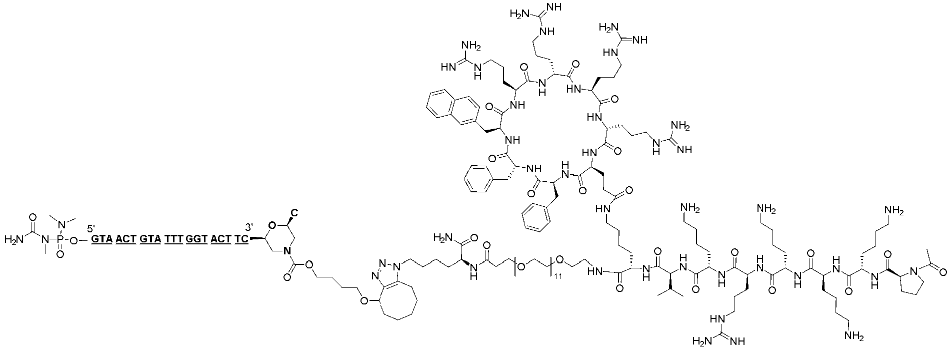

In some embodiments, the disease is an inflammatory or autoimmune disease. In some embodiments, the target gne is NLRP3 or CD6.

In some embodiments, the disease is osteogenesis imperfecta (Wang and Marini, J. Clin Invest., 1996, 97, 448-454).

In some embodiments, the disease is cystic fibrosis (Friedman et al., J. Biol. Chem., 1999, 274, 36193-36199).

In some embodiments, the disease is Merosin-deficient congenital muscular dystrophy type 1A (MDC1A). MDC1A is an autosomal recessive neuromuscular disease characterized by neonatal onset of muscle weakness, hypotonia, dysmyelinating neuropathy, and minor brain abnormalities. Splice site mutations are estimated to affect ~40% of the MDC1A patient population. Causative mutations are located in the LAMA2 gene, which encodes the a2 chain (Lama2) of laminin-211 (or merosin) heterotrimeric protein complex expressed in the basement membrane of muscle and Schwann cells. In MDC1A, laminin-211 loses its proper interactions with receptors such as integrin α7β1 and dystroglycan, resulting in muscle and Schwann cells apoptosis and degeneration, which leads to fibrosis and loss of muscle function. In some embodiments, the AC hybridizes with a LAMA2 target pre-mRNA. So far, development of therapeutic strategies for MDC1A have been mainly focused on preventing fibrosis and apoptosis. The degree of LAMA2 deficiency highly correlates with the clinical severity in patients and mouse models. The lack of a functional Lama2 leads to the development of severe muscle atrophy and hind limb paralysis in mice. Therefore, restoration of LAMA2 expression holds a tremendous potential for the treatment of MDC1A. It has previously been demonstrated that muscle-specific overexpression of Laminin-211 in merosin-deficient mice improved muscle pathology, but not the associated paralysis, indicating that correction of the peripheral neuropathy requires restoration of Lama2 beyond skeletal muscles. In some embodiments, the AC restores proper splicing to the gene.

In some embodiments, antisense compounds may be used to alter the ratio of the long and short forms of bcl-x pre-mRNA. See U.S. Pat. Nos. 6,172,216; 6,214,986; Taylor et al., Nat. Biotechnol. 1999, 17, 1097-1100, each incorporated herein by reference. An increasing number of genes and gene products have been implicated in apoptosis. One of these is bcl-2, which is an intracellular membrane protein shown to block or delay apoptosis. Overexpression of bcl-2 has been shown to be related to hyperplasia, autoimmunity and resistance to apoptosis, including that induced by chemotherapy (Fang et al., J. Immunol. 1994, 153, 4388-4398). A family of bcl-2-related genes has been described. All bcl-2 family members share two highly conserved domains, BH1 and BH2. These family members include, but are not limited to, A-1, mcl-1, bax and bcl-x. Bcl-x was isolated using a bcl-2 cDNA probe at low stringency due to its sequence homology with bcl-2. Bcl-x was found to function as a bcl-2-independent regulator of apoptosis (Boise et al., Cell, 1993, 74, 597-608). Two isoforms of bcl-x were reported in humans. Bcl-xl (long) contains the highly conserved BH1 and BH2 domains. When transfected into an IL-3 dependent cell line, bcl-xl inhibited apoptosis during growth factor withdrawal in a manner similar to bcl-2. In contrast, the bcl-x short isoform, bcl-xs, which is produced by alternative splicing and lacks a 63-amino acid region of exon 1 containing the BH1 and BH2 domains, antagonizes the anti-apoptotic effect of either bcl-2 or bcl-xl. As numbered in Boise et al., Cell, 1993 74:, 597-608, the bcl-x transcript can be categorized into regions described by those of skill in the art as follows: nucleotides 1-134, 5' untranslated region (5'-UTR); nucleotides 135-137, translation initiation codon (AUG); nucleotides 135-836, coding region, of which 135-509 are the shorter exon 1 of the bcl-xs transcript and 135-698 are the longer exon 1 of the bcl-xl transcript; nucleotides 699-836, exon 2; nucleotides 834-836, stop codon; and nucleotides 837-926, 3' untranslated region (3'-UTR). Between exons 1 and 2 (between nucleotide 698 and 699) an intron is spliced out of the pre-mRNA when the mature bcl-xl (long) mRNA transcript is produced. An alternative splice from position 509 to position 699 produces the bcl-xs (short) mRNA transcript which is 189 nucleotides shorter than the long transcript, encoding a protein product (bcl-xs) which is 63 amino acids shorter than bcl-xl. Thus nucleotide position 698 is sometimes referred to in the art as the "5' splice site" and position 509 as the "cryptic 5' splice site," with nucleotide 699 sometimes referred to as the "3' splice site." In some embodiments, the AC hybridizes with a sequence comprising the cryptic 5' splice site of the bcl-x pre-mRNA, thereby inhibiting production of the short isoform and increasing the ratio of bcl-xl to bcl-xs isoforms.

In some embodiments, the AC promotes skipping of specific exons containing premature termination codons. See Wilton et al., Neuromuscul. Disord., 1999, 9, 330-338, incorporated by reference herein.

In some embodiments, the AC counteracts or corrects aberrant splicing in a target pre-mRNA. See U.S. Pat. No. 5,627,274 and WO 94/26887, each of which is incorporated by reference herein, and which disclose compositions and methods for combating aberrant splicing in a pre-mRNA molecule containing a mutation using antisense oligonucleotides which do not activate RNAse H.

In some embodiments, the disease is proximal spinal muscular atrophy (SMA). SMA is a genetic, neurodegenerative disorder characterized by the loss of spinal motor neurons. SMA is an autosomal recessive disease of early onset and is currently the leading cause of death among infants. SMA is caused by the loss of both copies of survival of motor neuron 1 (SMN1), a protein that is part of a multi-protein complex thought to be involved in snRNP biogenesis and recycling. A nearly identical gene, SMN2, exists in a duplicated region on chromosome 5q13. Although SMN1 and SMN2 have the potential to code for the same protein, SMN2 contains a translationally silent mutation at position +6 of exon 7, which results in inefficient inclusion of exon 7 in SMN2 transcripts. Thus, the predominant form of SMN2 is a truncated version, lacking exon 7, which is unstable and inactive (Cartegni and Drainer, Nat. Genet., 2002, 30, 377-384). In some embodiments, the AC is targeted to intron 6, exon 7 or intron 7 of SMN2. In some embodiments, the AC modulates splicing of SMN2 pre-mRNA. In some embodiments, modulation of splicing results in an increase in exon 7 inclusion.

In some embodiments, the target gene is the beta globin gene. See Sierakowska et al. 1996, incorporated by reference herein. In some embodiments, the target gene is the cystic fibrosis transmembrane conductance regulator gene. See Friedman et al. 1999, incorporated by reference herein. In some embodiments, the target gene is the BRCA1 gene. In some embodiments, the target gene is the eIF4E gene. In some embodiments, the target gene is a gene involved in the pathogenesis of Duchenne muscular dystrophy, spinal muscular atrophy, or Steinert myotonic dystrophy. In some embodiments, the target gene is a DMD gene. In some embodiments, the target gene is BRCA1. In some embodiments, the target gene is a gene encoding a muscular structural protein. In some embodiments, the target gene is a gene implicated in a neuromuscular disorder (NMD). In some embodiments, the target gene is a gene implicated in cancer.

In some embodiments, the target gene is a gene that is subject to alternative splicing. In some embodiments, the present compounds and methods may be used to preferentially increase the ratio of a protein isoform by preferentially increasing the splicing of the target pre-mRNA to produce the mRNA encoding that isoform.

In some embodiments, the disease is a disease that is caused by repeat expansions of nucleotide repeat (e.g., trinucleotide repeat expansions, tetranucleotide repeat expansions, pentanucleotide repeat expansions, or hexanucleotide repeat expansions). In some embodiments, the disease is Huntington's disease, Huntington disease-like 2 (HDL2), myotonic dystrophy, spinocerebellar ataxia, spinal and bulbar muscular atrophy (SBMA), dentatorubral-pallidoluysian atrophy (DRPLA), amyotrophic lateral sclerosis, frontotemporal dementia, Fragile X syndrome, fragile X mental retardation 1 (FMR1), fragile X mental retardation 2 (FMR2), Fragile XE mental retardation (FRAXE), Friedreich's ataxia (FRDA), fragile X-associated tremor/ataxia syndrome (FXTAS), myoclonic epilepsy, oculopharyngeal muscular dystrophy (OPMD), or syndromic or non-syndromic X-linked mental retardation. In some embodiments, the disease is Huntington's disease. In some embodiments, the disease is amyotrophic lateral sclerosis. In some embodiments, the disease is a form of spinocerebellar ataxia (e.g., SCA1, SCA2, SAC3/MJD, SCA6, SCA7, SCA8, SCA10, SCA12, or SCA17).

In some embodiments, the disease is Friedreich's ataxia. In some embodiments, the target gene is *FXN*, which encodes for frataxin. In some embodiments, the compounds provided herein comprise an antisense oligonucleotide that targets *FXN.* Exemplary oligonucleotides that target *FXN* are provided in Table 8.

**Table 8. Exemplary Oligonucleotides targeting FXN**

| **Oligo chemistry** | **Name (oligo chemistry)** | **Design** | **Target** |
|---|---|---|---|
| MOE blocker | M-4 | 5'- TT^{m}C TT^{m}C TT^{m}C TT^{m}C TT^{m}C TT^{m}C-3' (all 2'-O-MOE, all PS bonds, m=5-methyl C) (SEQ ID NO: 167) | FXN (GAA)n |
| MOE gapmer | Gap-0039 | 5'- **^{m}CTT ^{m}CTT** ^{m}CTT ^{m}CTT ^{m}CTT **^{m}CTT ^{m}CT**-3' (all PS bonds, not bold=DNA, bold=2-MOE, m=5-methyl C) (SEQ ID NO: 168) | FXN (GAA)n |
| MOE gapmer | Gap-0040 | 5'- **T^{m}CT T^{m}CT** T^{m}CT T^{m}CT T^{m}CT **T^{m}CT T^{m}C**-3' (all PS bonds, not-bold=DNA, bold=2-MOE, m=5-methyl C) (SEQ ID NO: 169) | FXN (GAA)n |
| 2'-F, siRNA | ENTR-siRNA-0027 | ss 5'-GSASAS GAA GAA GAA GAA GASASG-3' (SEQ ID NO: 170) as 5'-CSUSUC UUC UUC UUC UUC SUSUSC-3' (SEQ ID NO: 171) (all 2'-F, S =PS bond) | FXN (GAA)n |
| 2'-F, siRNA EEV | ENTR-siRNA-0027A | ss CPP₁₂-NH-5'-GSASAS GAA GAA GAA GAA GASASG-3' (SEQ ID NO: 172) as 5'-CSUSUC UUC UUC UUC UUC SUSUSC-3' (SEQ ID NO: 173) (all 2'-F, S =PS bond) | FXN (GAA)n |
| 2'-F, siRNA EEV | ENTR-siRNA-0027B | ss 5'-GSASAS GAA GAA GAA GAA GASASG-3'-NH-CPP₁₂ (SEQ ID NO: 174) as 5'-CSUSUC UUC UUC UUC UUC SUSUSC-3' (SEQ ID NO: 175) (all 2'-F, S =PS bond) | FXN (GAA)n |
| siRNA | siGAA | ss 5'-GAAGAAGAAGAAGAAGAAGT_{d}T_{d}-3' (SEQ ID NO: 176) as 5'-CUUCUUCUUCUUCUUCUUCT_{d}T_{d}-3' (d=DNA) (SEQ ID NO: 177) | FXN (GAA)n |
| siRNA | Control (Ctrl) | as 5'-GCUAUACCAGCGUCGUCAUT_{d}T_{d}-3' (SEQ ID NO: 178) ss 5'-ATGACGACGCTGGTATAGCT_{d}T_{d}-3' (d=DNA) (SEQ ID NO: 179) | Mismatched negative control |
| siRNA | siExon2 | ss 5'-GAGUGUCUAUUUGAUGAAUT_{d}T_{d}-3' (SEQ ID NO: 180) as 5'-AUUCAUCAAAUAGACACUCT_{d}T_{d}-3' (d=DNA) (SEQ ID NO: 181) | FXN exon2, positive control for transfection |
| MOE blocker | ET4 | 5'-^{m}C^{m}CT ^{m}CAA AAG ^{m}CAG GAA UA-3' (all 2'-O-MOE, all PS bonds, m=5-methyl C) (SEQ ID NO: 182) | FXN 3'-UTR |
| MOE blocker | ET14 | 5-^{m}C^{m}CG GGT ^{m}CTG ^{m}C^{m}CG ^{m}C^{m}C^{m}C-3' (all 2'-O-MOE, all PS bonds, m=5-methyl C) (SEQ ID NO: 183) | FXN 5'-UTR |
| PMO | ENTR-Oligo-0180 | 5'-CCT CAA AAG CAG GAA TA-3' (all PMO monomers) (SEQ ID NO: 184) | FXN 3'-UTR |
| PMO | ENTR-Oligo-0181 | 5'-CCG GGT CTG CCG CCC-3' (all PMO monomers) (SEQ ID NO: 185) | FXN 5'-UTR |
| PMO | ENTR-Oligo-0182 | 5'-CCA ACT GTC CTC AAA AGC AGG AAT A-3' (all PMO monomers) (SEQ ID NO: 186) | FXN 3'-UTR |
| PMO | ENTR-Oligo-0183 | 5'-CCG GGT CTG CCG CCC GCT CCG CCC T-3' (all PMO monomers) (SEQ ID NO: 187) | FXN 5'-UTR |
| PMO | ENTR-Oligo-0000 | 5'-CTT CTT CTT CTT CTT CTT CTT CTT C-3' (all PMO monomers) (SEQ ID NO: 188) | FXN (GAA)n |
| O-MOE | ENTR-Oligo-0002 | 5'-CTT CTT CTT CTT CTT CTT-3' (all 2'-O-MOE RNA monomers, all PS bonds) (SEQ ID NO: 189) | FXN (GAA)n |
| LNA | ENTR-Oligo-0004 | 5'-LnT-dC-LnT-LnT-dC-LnT-LnT-dC-LnT-LnT-dC-LnT-LnT-dC-LnT-LnT-dC-LnT-LnT-3' (LnT=LNA T; dC=DNA C; all PS bonds) (SEQ ID NO: 190) | FXN (GAA)n |
| Gapmer (all PS bonds) | ENTR-Oligo-0039 | 5'- **CTTCT**TCTTCTTCTT**CTTCT**-3' (all PS bonds, non-bold=DNA, bold=2-MOE) (SEQ ID NO: 191) | FXN (GAA)n |
| Gapmer (all PS bonds) | ENTR-Oligo-0040 | 5'- **TCTTC**TTCTTCTTCT**TCTTC**-3' (all PS bonds, non-bold=DNA, bold=2-MOE) (SEQ ID NO: 192) | FXN (GAA)n |
| MOE (all PS bonds) | ENTR-Oligo-0041 | 5'-CTT CTT CTT CTT CTT CTT-3' (all 2'-O-MOE RNA monomers, all PS bonds) (SEQ ID NO: 193) | FXN (GAA)n |

In some embodiments, the disease is a form of myotonic dystrophy (e.g., myotonic dystrophy type 1 or myotonic dystrophy type 2). In some embodiments, the target gene is the *DMPK* gene, which encodes myotonic-protein kinase. In some embodiments, the compounds provided herein comprise an antisense oligonucleotide that targets *DMPK.* Exemplary oligonucleotides that target *DMPK* are provided in Table 9.

**Table 9. Exemplary Oligonucleotides targeting DMPK**

| **Oligo ID** | **Oligo name** | **Target** | **Sequence (5'-3')** |
|---|---|---|---|
| ENTR-Oligo-0022 | DMPK-2MOE-M+N+H (quote as DM+N+H) | DMPK | alkyne-5'-**ACAGA**CAATAAATAC**CGAGG**-3'-primary amine (all PS bonds, black=DNA, black=2-MOE) (SEQ ID NO: 194) |
| ENTR-Oligo-0023 | DMPK-2MOE-M+N+H-dual modification-1 | DMPK | cycloctyne-5'-**ACAGA**CAATAAATAC**CGAGG**-3'-primary amine (all PS bonds, black=DNA, black=2-MOE) (SEQ ID NO: 195) |
| ENTR-Oligo-0023A | DMPK-2MOE-M+N+H-NH2-1 | DMPK | 5'-**ACAGA**CAATAAATAC**CGAGG**-3'-primary amine (all PS bonds, black=DNA, black=2-MOE) (SEQ ID NO: 196) |
| ENTR-Oligo-0028 | DMPK-cEt-M+N+H | DMPK | cyclooctyne-5'-**ACA**ATAAATACCG**AGG-**3'-primary amine (all PS bonds, black=DNA, bold=(s)-cEt) (SEQ ID NO: 197) |
| ENTR-Oligo-0029 | DMPK-cEt-M+N+H-CP12 | DMPK | cyclooctyne-5'-**ACA**ATAAATACCG**AGG-**3'-CP12 (all PS bonds, black=DNA, bold=(s)-cEt) (SEQ ID NO: 198) |
| ENTR-Oligo-0031 | DMPK-2MOE-H-2 (quote as DH-1) | DMPK | 5'- **CGGAG**CGGTTGTGAA**CTGGC** -3'-primary amine (all PS bonds, non-bold=DNA, bold=2-MOE) (SEQ ID NO: 199) |
| ENTR-Oligo-0032 | DMPK-2MOE-N-2 (wrongly labeled as DM-1 in quote) | DMPK | alkyne-5'-**CGGAG**CGGTGTGAAC**TGGCA** -3'-primary amine (20 bases, all PS bonds, non-bold=DNA, bold=2-MOE) (SEQ ID NO: 200) |
| ENTR-Oligo-0053 | DMPK-2MOE-M+N+H | DMPK | 5'-**ACAGA**CAATAAATAC**CGAGG**-3' (all PS bonds, non-bold=DNA, bold=2-MOE) (SEQ ID NO: 201) |
| ENTR-oligo-0077 | ENTR-oligo-0022-PEG12-CPP12-Amide | DMPK | alkyne-5'-**ACAGA**CAATAAATAC**CGAGG**-3'-PEG12-CPP12 (all PS bonds, non-bold=DNA, bold=2-MOE) (SEQ ID NO: 202) |
| ENTR-oligo-0078 | CPP12-PEG12-click-ENTR-oligo-0022 | DMPK | CPP12-PEG12-Lys-click-5'-**ACAGA**CAATAAATAC**CGAGG**-3' (all PS bonds, non-bold=DNA, bold=2-MOE) (SEQ ID NO: 203) |
| ENTR-oligo-0189 | ETRDWUXI-617+ ENTR-oligo-0023 (click) | DMPK | CPP12-PEG12-click-5'-**ACAGA**CAATAAATAC**CGAGG**-3'-primary amine (all PS bonds, non-bold=DNA, bold=2-MOE) (SEQ ID NO: 204) |
| ENTR-oligo-0190 | ETRDWUXI-642+ ENTR-oligo-0023 (click) | DMPK | CPP12-K(CPP12) PEG12-K-click-5'-**ACAGA**CAATAAATAC**CGAGG**-3'-primary amine (all PS bonds, non- |
| | | | bold=DNA, bold=2-MOE) (SEQ ID NO: 205) |
| ENTR-oligo-0191 | ETRDWUXI-684+ ENTR-oligo-0023 (click) | DMPK | Ac-NLS-Lys(CPP12)-PEG12-K-click-5'-**ACAGA**CAATAAATAC**CGAGG-**3'-primary amine (all PS bonds, non-bold=DNA, bold=2-MOE) (SEQ ID NO: 206) |
| ENTR-oligo-0192 | ENTR-oligo-0023a+SMCC | DMPK | 5'-**ACAGA**CAATAAATAC**CGAGG**-3'-primary amine+SMCC (all PS bonds, non-bold=DNA, bold=2-MOE) (SEQ ID NO: 207) |
| ENTR-Oligo-0071 | PMO^{CAG} | DMPK | 5'-CAG CAG CAG CAG CAG CAG CAG-3'-NH2 (all PMO monomers) (SEQ ID NO: 208) |
| ENTR-Oligo-0034 | PMO^{CAG}-CP12 | DMPK | 5'-CAG CAG CAG CAG CAG CAG CAG-3' -CP12 (all PMO monomers) (SEQ ID NO: 209) |
| ENTR-Oligo-0022 | DMPK-2MOE-M+N+H (quote as DM+N+H) | DMPK | alkyne-5'-**ACAGA**CAATAAATAC**CGAGG**-3'-primary amine (all PS bonds, non-bold=DNA, bold=2-MOE) (SEQ ID NO: 210) |
| ENTR-Oligo-0031 | DMPK-2MOE-H-2 (quote as DH-1) | DMPK | 5'- **CGGA**GCGGTTGTGAA**CTGGC**-3'-primary amine (all PS bonds, non-bold=DNA, bold=2-MOE) (SEQ ID NO: 211) |

In some embodiments, the disease is Dravet syndrome. Dravet syndrome is a severe and progressive genetic epilepsy. Dravet syndrome is an autosomal dominant condition caused by more than 1250 *de novo* mutations in *SCN1A,* resulting in 50 % NaV1.1 protein expression. Dravet syndrome is caused by pathogenic mutation or deletion of the *SCN1A* gene in 85 % of patients. Existing antiepileptic drug sonly address the occurrence of seizures, and more than 90 % of Dravet syndrome patients still report suffering from incomplete seizure control. In some embodiments, the antisense oligonucleotide targets *SCN1A.* In some embodiments, the antisense oligonucleotide targeting *SCN1A* has a sequence of 5'-CCATAATAAAGGGCTCAG-3' (SEQ ID NO: 212). In some embodiments, the efficacy of antisense compounds targeting *SCN1A* is evaluated in a mouse model. Non-limiting examples of mouse models include mouse models with a targeted deletion of *SCN1A* exon 1 (Scn1a^{tm1Kea}) and exon 26 (Scn1a^{tm1Wac}), mouse models with specific point mutation knock-ins, such as Scnla R1407X, Scnla R1648H, and Scnla E1099X, and a transgenic mouse model expressing a bacterial artificial chromosome (BAC) with a human SCN1A R1648H mutation. In some embodiments, the efficacy of antisense compounds targeting *SCN1A* is evaluated in an in vitro model, for example, in wild-type fibroblasts.

In some embodiments, the disease is Fragile X Syndrome (FXS). FXS is the most common form of inherited intellectual and developmental disease. FXS is caused by silenced expression of fragile X mental retardation protein (FMRP) due to the presence of > 200 CGG trinucleotide repeats in *FMR1* which encodes for FMRP. FMRP is encoded by *FMR1.* In some embodiments, an antisense compound of the disclosure targets *FMR1.* In some embodiments, the efficacy of antisense compounds targeting *FMR1* is evaluated in a mouse model (e.g., those described in Dahlhaus et al.), which is incorporated by reference herein in its entirety: Dahlhaus, R. (2018). Of men and mice: modeling the fragile X syndrome. Frontiers in molecular neuroscience, 11, 41.

In some embodiments, the disease is Fragile X tremor ataxia syndrome (FXTAS). FXTAS is a late-onset, progressive neurodegenerative disorder characterized by cerebellar ataxia and intention tremor. FXTAS is caused by an *FMR1* premutation, which is defined as having 55 to 200 CGG repeats in the 5' untranslated region of *FMR1.* In some embodiments, an antisense compound of the disclosure targets *FMR1.*

In some embodiments, the disease is Huntington's Disease (HD). HD is an autosomal dominant disease, characterized by cognitive decline, psychiatric illness, and chorea. HD is often fatal. HD is caused by an expanded CAG triplet repeat in the *HTT* gene, which results in the production of mutant huntingtin protein (mHTT). Accumulation of mHTT causes progressive loss of neurons in the brain. In some embodiments, the target gene is *HTT.* In some embodiments, an antisense compound of the disclosure targets *HTT.* In some embodiments, the efficacy of antisense compounds and/or oligonucleotides are evaluated in in vivo models. Exemplary models are described in Pouladi et al. which is incorporated by reference herein in its entirety: Pouladi, Mahmoud A., et al. "Choosing an animal model for the study of Huntington's disease." Nature Reviews Neuroscience 14.10 (2013): 708-721. In some embodiments, the antisense oligonucleotide is non-allele selective. In some embodiments, the non-allele selective antisense oligonucleotide is an HTTRx gapmer (Ionis) or a divalent siRNA (UMass). In some embodiments, the antisense oligonucleotide is allele selective. In some embodiments, the allele selective antisense oligonucleotide is a stereopure gapmer targeting a single nucleotide polymorphism in *HTT.* In some embodiments, the antisense oligonucleotide targets exon 1, exon 30, exon 36, exon 50, or exon 67 of *HTT.* Exemplary antisense oligonucleotides and their pre-mRNA targets for HD are illustrated in **FIG. 33**. The following references describe exemplary antisense oligonucleotides and are incorporated herein by reference in their entirety: Yu, Dongbo, et al. Cell 150.5 (2012): 895-908; Alterman, Julia F., et al. Nature biotechnology 37.8 (2019): 884-894. Tabrizi, Sarah J., et al. New England Journal of Medicine 380.24 (2019): 2307-2316.; Kordasiewicz, Holly B., et al. Neuron 74.6 (2012): 1031-1044.

In some embodiments, the disease is Wilson's Disease (WD). WD is a recessive fatal copper homeostasis disorder, typically diagnosed in patients between the ages of 5 and 35, leading to hepatic and neurologic symptoms due to free copper accumulation. WD is caused by loss-of-function mutations in the *ATP7B* gene. *ATP7B* encodes copper-transporting ATPase 2, which is a transmembrane copper transporter and responsible in the transport of copper from the liver to other parts of the body. In some embodiments, provided herein is an antisense oligonucleotide or compound thereof that targets *ATP7B.* In some embodiments, the antisense oligonucleotide or compound thereof targets a T1934G (or Met-645-Arg) mutation in *ATP7B.* The aforementioned ATP7B variant is described in Merico et al. which is incorporated by reference herein in its entirety: Merico, Daniele, et al. NPJ Genomic Medicine 5.1 (2020): 1-7. In some embodiments, the antisense oligonucleotide has a sequence of 5'-CAGCTGGAGTTTATCTTTTG-3' (SEQ ID NO: 213).

In some embodiments of this aspect, the sequence of the corresponding gene underlying such diseases is prone to forming clusters of RNA comprises tandem nucleotide repeats (e.g., multiple nucleotide repeats comprising at least 10, 15, 20, 25, 30, 40, 50, 60, 70 or more adjacent repeated nucleotide sequences). In some embodiments, the tandem nucleotide repeats are trinucleotide repeats. The trinucleotide repeat sequences may be CAG repeats, CGG repeats, GCC repeats, GAA repeats, or CUG repeats. In some embodiments, the trinucleotide repeat is a CAG repeat. In some embodiments, the RNA sequence comprises at least 10 trinucleotide repeats (e.g., CAG, CGG, GCC, GAA, or CUG repeats), e.g., at least 10, at least 15, at least 20, at least 25, at least 30, at least 35, at least 40, at least 45, at least 50, at least 60, or at least 70 trinucleotide repeats. In some embodiments, the target gene is selected from the group consisting of FMR1, AFF2, FXN, DMPK, SCA8, PPP2R2B, ATN1, DRPLA, HTT, AR, ATXN1, ATXN2, ATXN3, CACNA1A, ATXN7, TBP. See U.S. Pat. Appl. Publ. No. 2016/0355796 and U.S. Pat. Appl. Publ. No. 2018/0344817, each of which is incorporated by reference herein, and which discloses diseases and corresponding genes prone to forming and/or expanding tandem nucleotide repeats.

In some embodiments, an AC of the disclosure is administered to treat any disease described by the disclosure, for example, Huntington's disease, Huntington disease-like 2 (HDL2), myotonic dystrophy, spinocerebellar ataxia, spinal and bulbar muscular atrophy (SBMA), dentatorubral-pallidoluysian atrophy (DRPLA), amyotrophic lateral sclerosis, frontotemporal dementia, Fragile X syndrome, fragile X mental retardation 1 (FMR1), fragile X mental retardation 2 (FMR2), Fragile XE mental retardation (FRAXE), Friedreich's ataxia (FRDA), fragile X-associated tremor/ataxia syndrome (FXTAS), myoclonic epilepsy, oculopharyngeal muscular dystrophy (OPMD), syndromic or non-syndromic X-linked mental retardation, Cystic fibrosis, proximal spinal muscular atrophy, of Duchenne muscular dystrophy, spinal muscular atrophy, Steinert myotonic dystrophy, Merosin-deficient congenital muscular dystrophy type 1A, osteogenesis imperfect, cancer, glioma, thyroid cancer, lung cancer, colorectal cancer, head and neck cancer, stomach canker, liver cancer, pancreatic cancer, renal cancer, urothelial cancer, prostate cancer, testis cancer, breast cancer, cervical cancer, endometrial cancer, ovarian cancer, melanoma, or Alzheimer's Disease. In some embodiments, an AC of the disclosure is administered to a patient diagnosed with a disease of the disclosure at a dose of between about 0.1 mg/kg and about 1000 mg/kg, for example, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, about 30 mg/kg, about 31 mg/kg, about 32 mg/kg, about 33 mg/kg, about 34 mg/kg, about 35 mg/kg, about 36 mg/kg, about 37 mg/kg, about 38 mg/kg, about 39 mg/kg, about 40 mg/kg, about 41 mg/kg, about 42 mg/kg, about 43 mg/kg, about 44 mg/kg, about 45 mg/kg, about 46 mg/kg, about 47 mg/kg, about 48 mg/kg, about 49 mg/kg, about 50 mg/kg, about 51 mg/kg, about 52 mg/kg, about 53 mg/kg, about 54 mg/kg, about 55 mg/kg, about 56 mg/kg, about 57 mg/kg, about 58 mg/kg, about 59 mg/kg, about 60 mg/kg, about 61 mg/kg, about 62 mg/kg, about 63 mg/kg, about 64 mg/kg, about 65 mg/kg, about 66 mg/kg, about 67 mg/kg, about 68 mg/kg, about 69 mg/kg, about 70 mg/kg, about 71 mg/kg, about 72 mg/kg, about 73 mg/kg, about 74 mg/kg, about 75 mg/kg, about 76 mg/kg, about 77 mg/kg, about 78 mg/kg, about 79 mg/kg, about 80 mg/kg, about 81 mg/kg, about 82 mg/kg, about 83 mg/kg, about 84 mg/kg, about 85 mg/kg, about 86 mg/kg, about 87 mg/kg, about 88 mg/kg, about 89 mg/kg, about 90 mg/kg, about 91 mg/kg, about 92 mg/kg, about 93 mg/kg, about 94 mg/kg, about 95 mg/kg, about 96 mg/kg, about 97 mg/kg, about 98 mg/kg, about 99 mg/kg, about 100 mg/kg, about 110 mg/kg, about 120 mg/kg, about 130 mg/kg, about 140 mg/kg, about 150 mg/kg, about 160 mg/kg, about 170 mg/kg, about 180 mg/kg, about 190 mg/kg, about 200 mg/kg, about 210 mg/kg, about 220 mg/kg, about 230 mg/kg, about 240 mg/kg, about 250 mg/kg, about 260 mg/kg, about 270 mg/kg, about 280 mg/kg, about 290 mg/kg, about 300 mg/kg, about 310 mg/kg, about 320 mg/kg, about 330 mg/kg, about 340 mg/kg, about 350 mg/kg, about 360 mg/kg, about 370 mg/kg, about 380 mg/kg, about 390 mg/kg, about 400 mg/kg, about 410 mg/kg, about 420 mg/kg, about 430 mg/kg, about 440 mg/kg, about 450 mg/kg, about 460 mg/kg, about 470 mg/kg, about 480 mg/kg, about 490 mg/kg, about 500 mg/kg, about 510 mg/kg, about 520 mg/kg, about 530 mg/kg, about 540 mg/kg, about 550 mg/kg, about 560 mg/kg, about 570 mg/kg, about 580 mg/kg, about 590 mg/kg, about 600 mg/kg, about 610 mg/kg, about 620 mg/kg, about 630 mg/kg, about 640 mg/kg, about 650 mg/kg, about 660 mg/kg, about 670 mg/kg, about 680 mg/kg, about 690 mg/kg, about 700 mg/kg, about 710 mg/kg, about 720 mg/kg, about 730 mg/kg, about 740 mg/kg, about 750 mg/kg, about 760 mg/kg, about 770 mg/kg, about 780 mg/kg, about 790 mg/kg, about 800 mg/kg, about 810 mg/kg, about 820 mg/kg, about 830 mg/kg, about 840 mg/kg, about 850 mg/kg, about 860 mg/kg, about 870 mg/kg, about 880 mg/kg, about 890 mg/kg, about 900 mg/kg, about 910 mg/kg, about 920 mg/kg, about 930 mg/kg, about 940 mg/kg, about 950 mg/kg, about 960 mg/kg, about 970 mg/kg, about 980 mg/kg, about 990 mg/kg, or about 1000 mg/kg, including all values and ranges therein and in between.

In some embodiments, an AC of the disclosure is a gapmer oligonucleotide as disclose in U.S. Patent No. 9,550,988, the disclosure of which is incorporated by reference herein.

In some embodiments, an AC of the disclosure comprises the sequence and/or structure of any one of the ACs targeting SMN2 disclosed in U.S. Patent No. 8,361,977, the disclosure of which is incorporated by reference herein.

In some embodiments, an AC of the disclosure comprises the sequence and/or structure of any one of the ACs targeting DMD, SMN2, or DMPK disclosed in U.S. Patent Publication No. 2017/0260524, the disclosure of which is incorporated by reference herein.

In some embodiments, an AC of the disclosure comprises the sequence and/or structure of any one of the ACs or oligonucleotides disclosed in U.S. Patent Publications US20030235845A1, US20060099616A1, US 2013/0072671 A1, US 2014/0275212 A1, US 2009/0312532 A1, US20100125099A1, US 2010/0125099 A1, US 2009/0269755 A1, US 2011/0294753 A1, US 2012/0022134 A1, US 2011/0263682 A1, US 2014/0128592 A1, US 2015/0073037 A1, and US20120059042A1, the contents of each of which are incorporated herein in their entirety for all purposes.

### Re-spliced target proteins

The "target protein" is the amino acid sequence resulting from transcription and translation of the target gene. The "re-spliced target protein" as used herein refers to the protein encoded as a result of binding of the AC to the target pre-mRNA transcribed from the target gene. The "wild type target protein" refers to a naturally occurring, correctly translated protein isomer resulting from proper splicing of the target pre-mRNA encoded by a wild type target gene. The present compounds and methods may result in a re-spliced target protein containing one or more amino acid substitutions, deletions, and/or insertions as compared to a wild type target protein, while retaining some wild type target protein activity. In some embodiments, the re-spliced target protein produced by administration of the present compounds is homologous to a wild type target protein. In some embodiments, the re-spliced target protein has an amino acid sequence that is at least about 50%, at least about 55%, at least about 60%, at least about 65%, at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98%, at least about 99% or more identical to a wild type target protein. In some embodiments, the re-spliced target protein is substantially identical to a wild type target protein. In some embodiments, the amino acid sequence of the re-spliced target protein is at least 50% identical to the amino acid sequence of a wild type target protein. In some embodiments, the amino acid sequence of the re-spliced target protein is at least 75% identical to the amino acid sequence of a wild type target protein. In some embodiments, the amino acid sequence of the re-spliced target protein is at least 90% identical to the amino acid sequence of a wild type target protein. In some embodiments, the re-spliced target protein is a truncated version of a wild type target protein.

In some embodiments, the re-spliced target protein can rescue one or more phenotypes or symptoms of a disease associated with the transcription and translation of the target gene. In some embodiments, the re-spliced target protein can rescue one or more phenotypes or symptoms of a disease associated with the expression of the target protein. In some embodiments, the re-spliced target protein is an active fragment of a wild type target protein. In some embodiments, the re-spliced target protein functions in a substantially similar manner to the wild type target protein. In some embodiments, the re-spliced target protein allows the cell to function substantially similar to a similar cell which expresses a wild type target protein. In some embodiments, the re-spliced target protein does not cure the disease associated with the target gene or with the target protein, but ameliorates one or more symptoms of the disease. In some embodiments, the re-spliced target protein results in an improvement of target protein function of about 1%, about 2%, about 3%, about 4%, about 5%, about 6%, about 7%, about 8%, about 9%, about 10%, about 15%, about 205, about 25%, about 30%, about 35%, about 40%, about 45%, about 50%, about 55%, about 60%, about 65%, about 70%, about 75%, about 80%, about 85%, about 90%, about 95%, or about 100%.

In some embodiments, the re-spliced target protein may have an amino acid sequence that is reduced from the size of a wild type target protein by about 1 or more amino acids, e.g., about 5, about 10, about 15, about 20, about 25, about 30, about 35, about 40, about 45, about 50, about 55, about 60, about 65, about 70, about 75, about 80, about 90, about 95, about 100, about 105, about 110, about 115, about 120, about 125, about 130, about 135, about 140, about 145, about 150, about 155, about 160, about 165, about 170, about 175, or about 180 or more amino acids.

In some embodiments, the re-spliced target protein may have one or more properties that are improved relative to the target protein. In some embodiments, the re-spliced target protein may have one or more properties that are improved relative to a wild type target protein. In some embodiments, the enzymatic activity or stability may be enhanced by promoting different splicing of the target pre-mRNA. In some embodiments, the re-spliced target protein may have a sequence identical or substantially similar to a wild type target protein isomer having improved properties compared to another wild type target protein isomer.

In some embodiments, one or more properties of the target protein are either not present (eliminated) or are reduced in the re-spliced target protein. In some embodiments, one or more properties of the wild type target protein are either not present (eliminated) or are reduced in the re-spliced target protein. Non-limiting examples of properties that may be reduced or eliminated include immunogenic, angiogenic, thrombogenic, aggregation, and ligand-binding activity.

In some embodiments, the re-spliced target protein contains one or more amino acid substitutions compared to a wild type target protein. In some embodiments, the substitutions may be conservative substitutions or non-conservative substitutions. Examples of conservative amino acid substitutions include substitution of one amino acid for another amino acid within one from one of the following groups: basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). In some embodiments, structurally similar amino acids are substituted to reverse the charge of a residue (e.g., glutamine for glutamic acid or vice-versa, aspartic acid for asparagine or vice-versa). In some embodiments, tyrosine is substituted for phenylalanine or vice-versa. Other non-limiting examples of amino acid substitutions are described, for example, by H. Neurath and R. L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In some embodiments, the re-spliced target protein may comprise a substitution, deletion, and/or insertion at one or more (e.g., several) positions compared to a wild type target protein. In some embodiments, the number of amino acid substitutions, deletions and/or insertions comprised by the re-spliced target protein amino acid sequence is not more than 200, not more than 150, not more than 100, not more than 50, not more than 40, not more than 30, not more than 20, or not more than 10, e.g., 1, 2, 3, 4, 5, 6, 7, 8, 9, or 10.

In some embodiments, cCPP may be conjugated, via the linker, to the 5' or 3' end of the AC. In some embodiments, the linker further comprises an amino acid (e.g., lysine) to facilitate chemical conjugation of the AC to a side chain of an amino acid on the cCCP

In some embodiments, a water-soluble polymer can be conjugated to the AC.

### Methods of Treatment

The present disclosure provides a method of treating disease in a subject in need thereof, comprising administering a compound disclosed herein. In some embodiments, the disease is any of the diseases provided in the present disclosure. In some embodiments, the target gene is any of the target genes provided in the present disclosure.

In various embodiments, treatment refers to partial or complete alleviation, amelioration, relief, inhibition, delaying onset, reducing severity and/or incidence of one or more symptoms in a subject.

In some embodiments, a method is provided for altering the expression of a target gene in a subject in need thereof, comprising administering a compound disclosed herein. In some embodiments, the treatment results in the lowered expression of a target protein. In some embodiments, the treatment results in the expression of a re-spliced target protein. In some embodiments, the treatment results in the preferential expression of a wild type target protein isomer.

In some embodiments, treatment according to the present disclosure results in decreased expression of a target protein in a subject by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject before the treatment or of one or more control individuals with similar disease without treatment.

In some embodiments, treatment according to the present disclosure results in increased expression of a re-spliced target protein in a subject by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject before the treatment or of one or more control individuals with similar disease without treatment.

In some embodiments, treatment according to the present disclosure results in increased or decreased expression of a wild type target protein isomer in a subject by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject before the treatment or of one or more control individuals with similar disease without treatment.

In some embodiments, treatment according to the present disclosure results in decreased expression of a target protein in a subject's muscle tissue, diaphragm tissue, quadriceps, or heart by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject's muscle tissue, diaphragm tissue, quadriceps, or heart before the treatment,compared to one or more control individuals with similar disease without treatment, or compared to treatment with an AC not conjugated to a cyclic CPP disclosed herein.

In some embodiments, treatment according to the present disclosure results in increased expression of a re-spliced target protein in a subject's muscle tissue, diaphragm tissue, quadriceps, or heart by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 250%, about 300%, about 350%, about 400%, about 450%, about 500%, about 550%, about 600%, about 650%, about 700%, about 750%, about 800, about 850%, about 900%, about 950%, or about 1000% or more, as compared to the average level of the target protein in the subject's muscle tissue, diaphragm tissue, quadriceps, or heart before the treatment, compared toone or more control individuals with similar disease without treatment, or compared to treatment with an AC not conjugated to a cyclic CPP disclosed herein.

In some embodiments, treatment according to the present disclosure results in increased or decreased expression of a wild type target protein isomer in a subject's muscle tissue, diaphragm tissue, quadriceps, or heart by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject's muscle tissue, diaphragm tissue, quadriceps, or heart before the treatment, compared to one or more control individuals with similar disease without treatment, or compared to treatment with an AC not conjugated to a cyclic CPP disclosed herein.

In some embodiments, treatment according to the present disclosure results in decreased expression of a target protein in a subject's heart by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject's heart before the treatment, compared to one or more control individuals with similar disease without treatment, or compared to treatment with an AC not conjugated to a cyclic CPP disclosed herein.

In some embodiments, treatment according to the present disclosure results in increased expression of a re-spliced target protein in a subject's heart by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 250%, about 300%, about 350%, about 400%, about 450%, about 500%, about 550%, about 600%, about 650%, about 700%, about 750%, about 800, about 850%, about 900%, about 950%, or about 1000% or more, as compared to the average level of the target protein in the subject's heart before the treatment, compared to one or more control individuals with similar disease without treatment, or compared to treatment with an AC not conjugated to a cyclic CPP disclosed herein.

In some embodiments, treatment according to the present disclosure results in increased or decreased expression of a wild type target protein isomer in a subject by more than about 5%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%, as compared to the average level of the target protein in the subject's heart before the treatment, compared to one or more control individuals with similar disease without treatment, or compared to treatment with an AC not conjugated to a cyclic CPP disclosed herein.

The terms, "improve," "increase," "reduce," "decrease," and the like, as used herein, indicate values that are relative to a control. In some embodiments, a suitable control is a baseline measurement, such as a measurement in the same individual prior to initiation of the treatment described herein, or a measurement in a control individual (or multiple control individuals) in the absence of the treatment described herein. A "control individual" is an individual afflicted with the same disease, who is about the same age and/or gender as the individual being treated (to ensure that the stages of the disease in the treated individual and the control individual(s) are comparable).

The individual (also referred to as "patient" or "subject") being treated is an individual (fetus, infant, child, adolescent, or adult human) having a disease or having the potential to develop a disease. The individual may have a disease mediated by aberrant gene expression or aberrant gene splicing. In various embodiments, the individual having the disease may have wild type target protein expression or activity levels that are less than about 1-99% of normal protein expression or activity levels in an individual not afflicted with the disease. In some embodiments, the range includes, but is not limited to less than about 80-99%, less than about 65-80%, less than about 50-65%, less than about 30-50%, less than about 25-30%, less than about 20-25%, less than about 15-20%, less than about 10-15%, less than about 5-10%, less than about 1-5% of normal thymidine phosphorylase expression or activity levels. In some embodiments, the individual may have target protein expression or activity levels that are 1-500% higher than normal wild type target protein expression or activity levels. In some embodiments, the range includes, but is not limited to, greater than about 1-10%, about 10-50%, about 50-100%, about 100-200%, about 200-300%, about 300-400%, about 400-500%, or about 500-1000%.

In some embodiments, the individual is an individual who has been recently diagnosed with the disease. Typically, early treatment (treatment commencing as soon as possible after diagnosis) is important to minimize the effects of the disease and to maximize the benefits of treatment.

### Methods of Making

The compounds described herein can be prepared in a variety of ways known to one skilled in the art of organic synthesis or variations thereon as appreciated by those skilled in the art. The compounds described herein can be prepared from readily available starting materials. Optimum reaction conditions can vary with the particular reactants or solvents used, but such conditions can be determined by one skilled in the art.

Variations on the compounds described herein include the addition, subtraction, or movement of the various constituents as described for each compound. Similarly, when one or more chiral centers are present in a molecule, the chirality of the molecule can be changed. Additionally, compound synthesis can involve the protection and deprotection of various chemical groups. The use of protection and deprotection, and the selection of appropriate protecting groups can be determined by one skilled in the art. The chemistry of protecting groups can be found, for example, in Wuts and Greene, Protective Groups in Organic Synthesis, 4th Ed., Wiley & Sons, 2006, which is incorporated herein by reference in its entirety.

The starting materials and reagents used in preparing the disclosed compounds and compositions are either available from commercial suppliers such as Aldrich Chemical Co., (Milwaukee, WI), Acros Organics (Morris Plains, NJ), Fisher Scientific (Pittsburgh, PA), Sigma (St. Louis, MO), Pfizer (New York, NY), GlaxoSmithKline (Raleigh, NC), Merck (Whitehouse Station, NJ), Johnson & Johnson (New Brunswick, NJ), Aventis (Bridgewater, NJ), AstraZeneca (Wilmington, DE), Novartis (Basel, Switzerland), Wyeth (Madison, NJ), Bristol-Myers-Squibb (New York, NY), Roche (Basel, Switzerland), Lilly (Indianapolis, IN), Abbott (Abbott Park, IL), Schering Plough (Kenilworth, NJ), or Boehringer Ingelheim (Ingelheim, Germany), or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989). Other materials, such as the pharmaceutical carriers disclosed herein can be obtained from commercial sources.

Reactions to produce the compounds described herein can be carried out in solvents, which can be selected by one of skill in the art of organic synthesis. Solvents can be substantially nonreactive with the starting materials (reactants), the intermediates, or products under the conditions at which the reactions are carried out, *i.e.,* temperature and pressure. Reactions can be carried out in one solvent or a mixture of more than one solvent. Product or intermediate formation can be monitored according to any suitable method known in the art. For example, product formation can be monitored by spectroscopic means, such as nuclear magnetic resonance spectroscopy (*e.g.,* ¹H or ¹³C) infrared spectroscopy, spectrophotometry (*e.g.,* UV-visible), or mass spectrometry, or by chromatography such as high performance liquid chromatography (HPLC) or thin layer chromatography.

The disclosed compounds can be prepared by solid phase peptide synthesis wherein the amino acid α-N-terminal is protected by an acid or base protecting group. Such protecting groups should have the properties of being stable to the conditions of peptide linkage formation while being readily removable without destruction of the growing peptide chain or racemization of any of the chiral centers contained therein. Suitable protecting groups are 9-fluorenylmethyloxycarbonyl (Fmoc), t-butyloxycarbonyl (Boc), benzyloxycarbonyl (Cbz), biphenylisopropyloxycarbonyl, t-amyloxycarbonyl, isobornyloxycarbonyl, α,α-dimethyl-3,5-dimethoxybenzyloxycarbonyl, o-nitrophenylsulfenyl, 2-cyano-t-butyloxycarbonyl, and the like. The 9-fluorenylmethyloxycarbonyl (Fmoc) protecting group is particularly preferred for the synthesis of the disclosed compounds. Other preferred side chain protecting groups are, for side chain amino groups like lysine and arginine, 2,2,5,7,8-pentamethylchroman-6-sulfonyl (pmc), nitro, p-toluenesulfonyl, 4-methoxybenzene- sulfonyl, Cbz, Boc, and adamantyloxycarbonyl; for tyrosine, benzyl, o-bromobenzyloxy-carbonyl, 2,6-dichlorobenzyl, isopropyl, t-butyl (t-Bu), cyclohexyl, cyclopentyl and acetyl (Ac); for serine, t-butyl, benzyl and tetrahydropyranyl; for histidine, trityl, benzyl, Cbz, p-toluenesulfonyl and 2,4-dinitrophenyl; for tryptophan, formyl; for asparticacid and glutamic acid, benzyl and t-butyl and for cysteine, triphenylmethyl (trityl). In the solid phase peptide synthesis method, the α-C-terminal amino acid is attached to a suitable solid support or resin. Suitable solid supports useful for the above synthesis are those materials which are inert to the reagents and reaction conditions of the stepwise condensation-deprotection reactions, as well as being insoluble in the media used. Solid supports for synthesis of α-C-terminal carboxy peptides is 4-hydroxymethylphenoxymethyl-copoly(styrene-1% divinylbenzene) or 4-(2',4'-dimethoxyphenyl-Fmoc-aminomethyl)phenoxyacetamidoethyl resin available from Applied Biosystems (Foster City, Calif.). The α-C-terminal amino acid is coupled to the resin by means of N,N'-dicyclohexylcarbodiimide (DCC), N,N'-diisopropylcarbodiimide (DIC) or O-benzotriazol-1-yl-N,N,N',N'-tetramethyluroniumhexafluorophosphate (HBTU), with or without 4-dimethylaminopyridine (DMAP), 1-hydroxybenzotriazole (HOBT), benzotriazol-1-yloxy-tris(dimethylamino)phosphoniumhexafluorophosphate (BOP) or bis(2-oxo-3-oxazolidinyl)phosphine chloride (BOPCl), mediated coupling for from about 1 to about 24 hours at a temperature of between 10°C and 50°C in a solvent such as dichloromethane or DMF. When the solid support is 4-(2',4'-dimethoxyphenyl-Fmoc-aminomethyl)phenoxy-acetamidoethyl resin, the Fmoc group is cleaved with a secondary amine, preferably piperidine, prior to coupling with the α-C-terminal amino acid as described above. One method for coupling to the deprotected 4 (2',4'-dimethoxyphenyl-Fmoc-aminomethyl)phenoxy-acetamidoethyl resin is O-benzotriazol-1-yl-N,N,N',N'-tetramethyluroniumhexafluorophosphate (HBTU, 1 equiv.) and 1-hydroxybenzotriazole (HOBT, 1 equiv.) in DMF. The coupling of successive protected amino acids can be carried out in an automatic polypeptide synthesizer. In one example, the α-N-terminal in the amino acids of the growing peptide chain are protected with Fmoc. The removal of the Fmoc protecting group from the α-N-terminal side of the growing peptide is accomplished by treatment with a secondary amine, preferably piperidine. Each protected amino acid is then introduced in about 3-fold molar excess, and the coupling is preferably carried out in DMF. The coupling agent can be O-benzotriazol-1-yl-N,N,N',N'-tetramethyluroniumhexafluorophosphate (HBTU, 1 equiv.) and 1-hydroxybenzotriazole (HOBT, 1 equiv.). At the end of the solid phase synthesis, the polypeptide is removed from the resin and deprotected, either in successively or in a single operation. Removal of the polypeptide and deprotection can be accomplished in a single operation by treating the resin-bound polypeptide with a cleavage reagent comprising thioanisole, water, ethanedithiol and trifluoroacetic acid. In cases wherein the α-C-terminal of the polypeptide is an alkylamide, the resin is cleaved by aminolysis with an alkylamine. Alternatively, the peptide can be removed by transesterification, e.g. with methanol, followed by aminolysis or by direct transamidation. The protected peptide can be purified at this point or taken to the next step directly. The removal of the side chain protecting groups can be accomplished using the cleavage cocktail described above. The fully deprotected peptide can be purified by a sequence of chromatographic steps employing any or all of the following types: ion exchange on a weakly basic resin (acetate form); hydrophobic adsorption chromatography on underivatized polystyrene-divinylbenzene (for example, Amberlite XAD); silica gel adsorption chromatography; ion exchange chromatography on carboxymethylcellulose; partition chromatography, e.g. on Sephadex G-25, LH-20 or countercurrent distribution; high performance liquid chromatography (HPLC), especially reverse-phase HPLC on octyl- or octadecylsilyl-silica bonded phase column packing.

The above polymers, such as PEG groups, can be attached to the AC under any suitable conditions used to react a protein with an activated polymer molecule. Any means known in the art can be used, including via acylation, reductive alkylation, Michael addition, thiol alkylation or other chemoselective conjugation/ligation methods through a reactive group on the PEG moiety (e.g., an aldehyde, amino, ester, thiol, α-haloacetyl, maleimido or hydrazino group) to a reactive group on the AC (e.g., an aldehyde, amino, ester, thiol, α-haloacetyl, maleimido or hydrazino group). Activating groups which can be used to link the water soluble polymer to one or more proteins include without limitation sulfone, maleimide, sulfhydryl, thiol, triflate, tresylate, azidirine, oxirane, 5-pyridyl, and alpha-halogenated acyl group (e.g., α-iodo acetic acid, α-bromoacetic acid, α-chloroacetic acid). If attached to the AC by reductive alkylation, the polymer selected should have a single reactive aldehyde so that the degree of polymerization is controlled. See, for example, Kinstler et al., Adv. Drug. Delivery Rev. 54: 477-485 (2002); Roberts et al., Adv. Drug Delivery Rev. 54: 459-476 (2002); and Zalipsky et al., Adv. Drug Delivery Rev. 16: 157-182 (1995).

In order to direct covalently link the AC to the CPP, appropriate amino acid residues of the CPP may be reacted with an organic derivatizing agent that is capable of reacting with a selected side chain or the N- or C-termini of an amino acids. Reactive groups on the peptide or conjugate moiety include, e.g., an aldehyde, amino, ester, thiol, α-haloacetyl, maleimido or hydrazino group. Derivatizing agents include, for example, maleimidobenzoyl sulfosuccinimide ester (conjugation through cysteine residues), N-hydroxysuccinimide (through lysine residues), glutaraldehyde, succinic anhydride or other agents known in the art.

Methods of making AC and conjugating AC to linear CPP are generally described in US Pub. No. 2018/0298383, which is herein incorporated by reference for all purposes. The methods may be applied to the cyclic CPPs disclosed herein.

Synthetic schemes are provided in Fig. 7A-7D, Fig. 8, and Fig. 9

Non-limiting examples of CPPs containing reactive groups (e.g., TFP) for conjugation to an AC are shown below. Although each of the below examples contain CPP12, any CPP can be substituted for CPP12.

### TFP-PEG₄-K(CPP12):

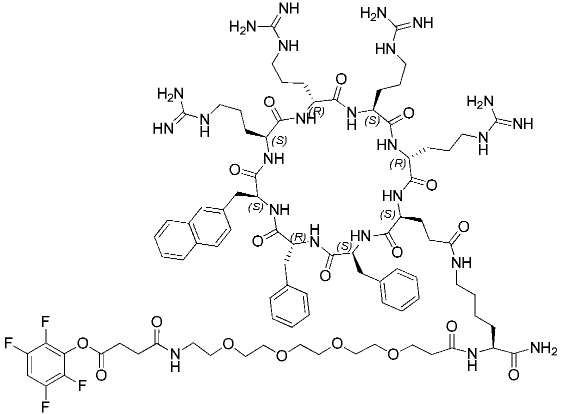

### TFP-PEG₄-K(CPP12)-PEG₄-Dap(palmitoyl):

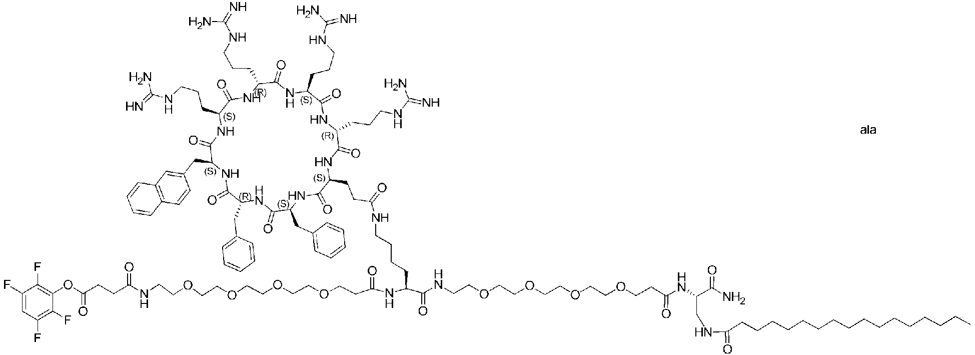

### TFP-PEG₄-K(CPP12)-PEG₄-Dap(CPP12):

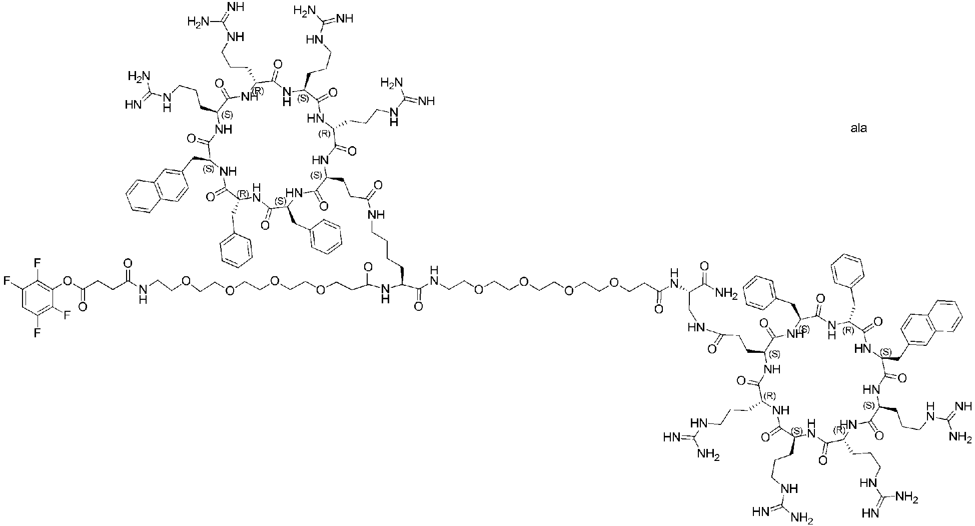

### TFP-Pip6a:

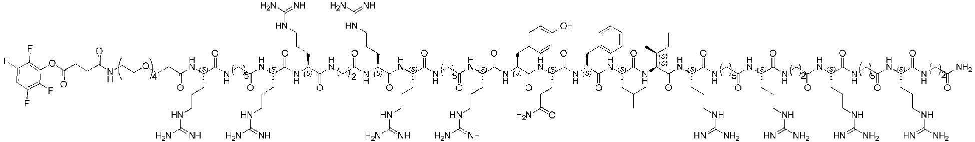

### CPP12-PEG12-TFP:

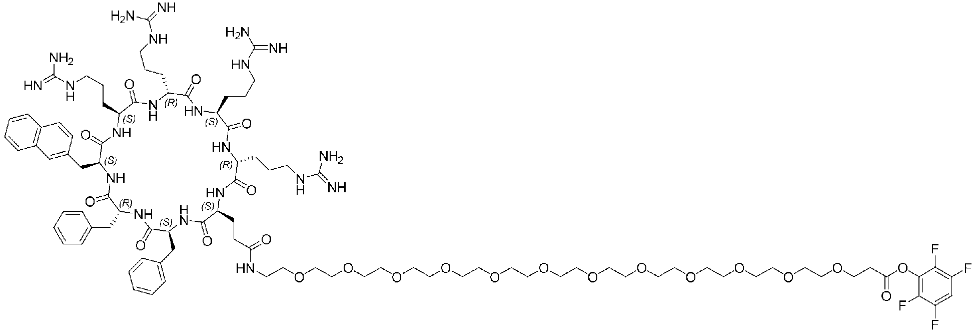

### CPP12-PEG12-K(CPP12)-PEG12-TFP ETRD-peptide-344:

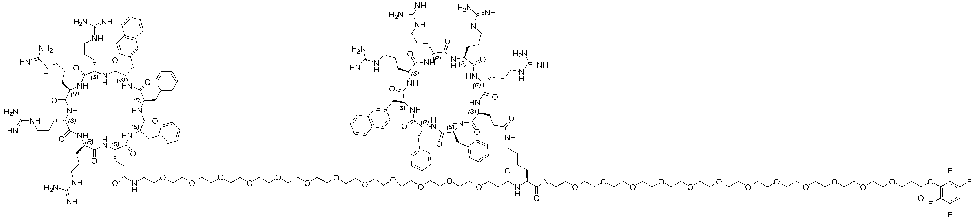

### CPP12-PEG12-Lys(N3):

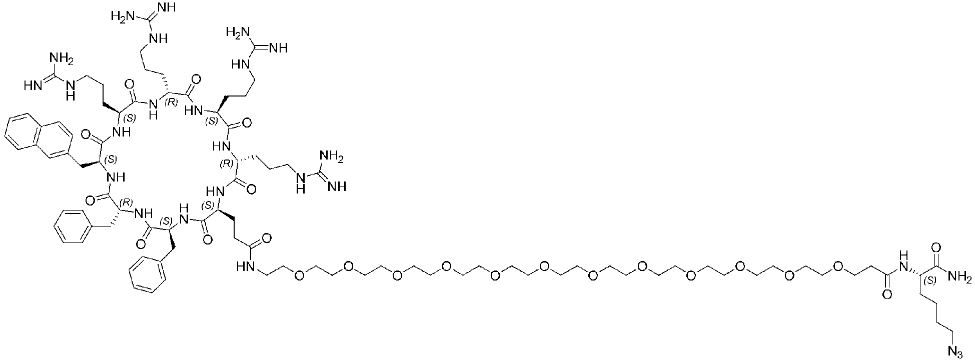

### CPP-12-K(CPP12)-PEG12-K(N3):

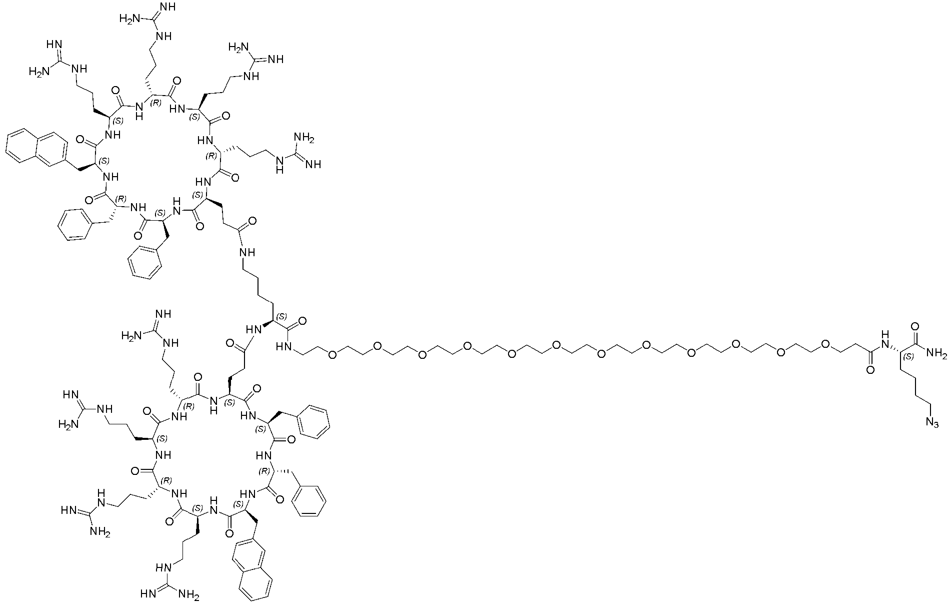

### CPP12-PEG4-K(PEG4-CPP12)-PEG12-K(N3):

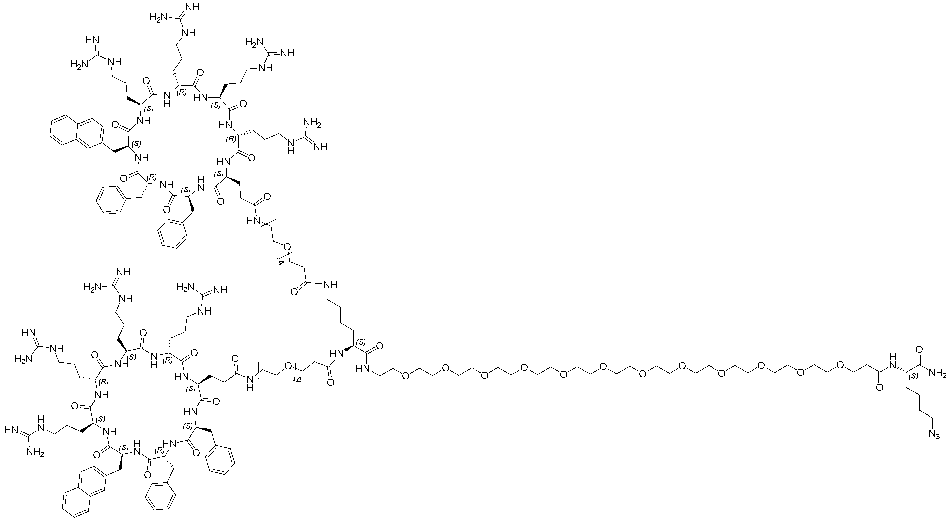

### CPP12-PEG12-K(PEG12-CPP12)-PEG12-K(N3):

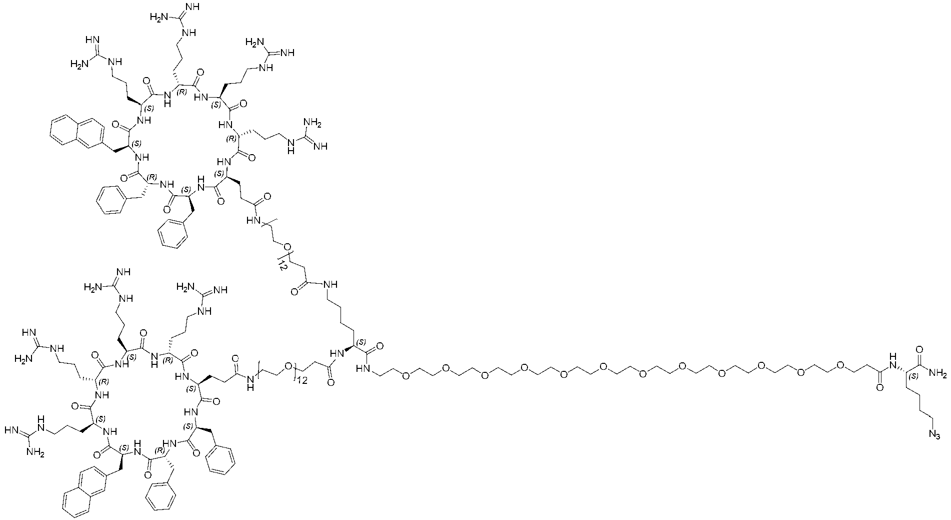

### CPP12-K(CPP12)-K(CPP12)-PEG12-K(N3):

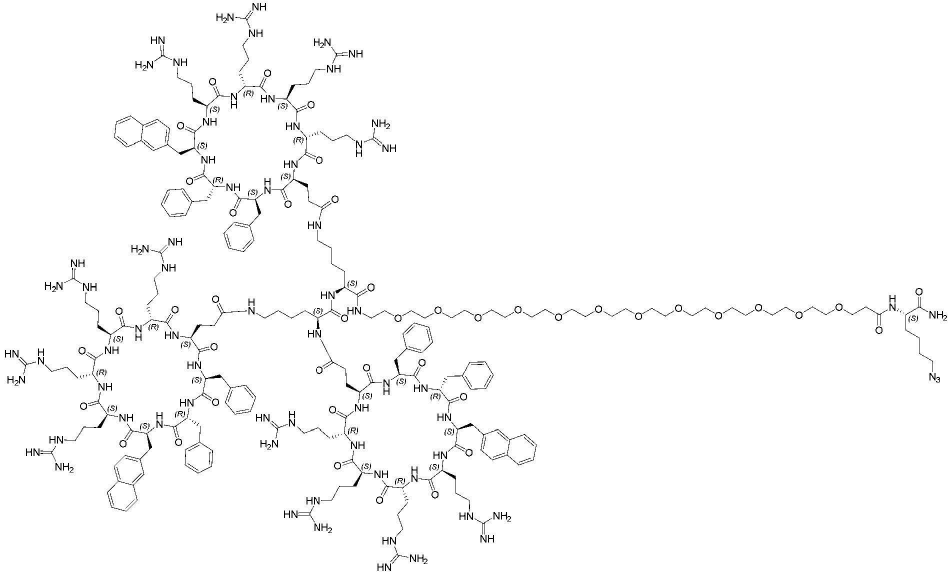

### CPP12-PEG4-K(PEG4-CPP12)-K(PEG4-CPP12)-PEG12-K(N3):

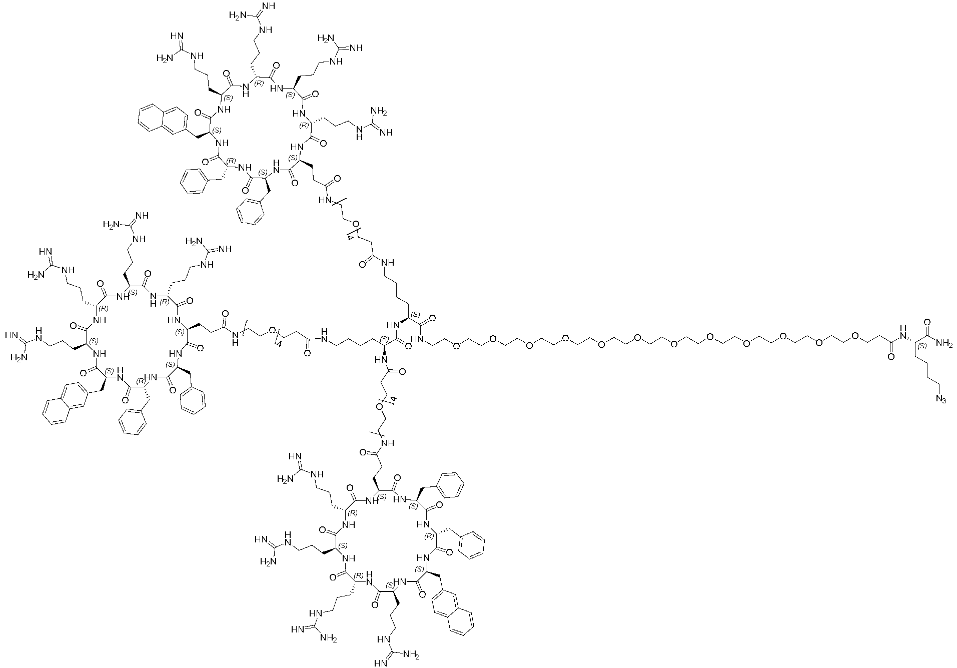

### CPP12-PEG12-K(PEG12-CPP12)-K(PEG12-CPP12)-PEG12-K(N3):

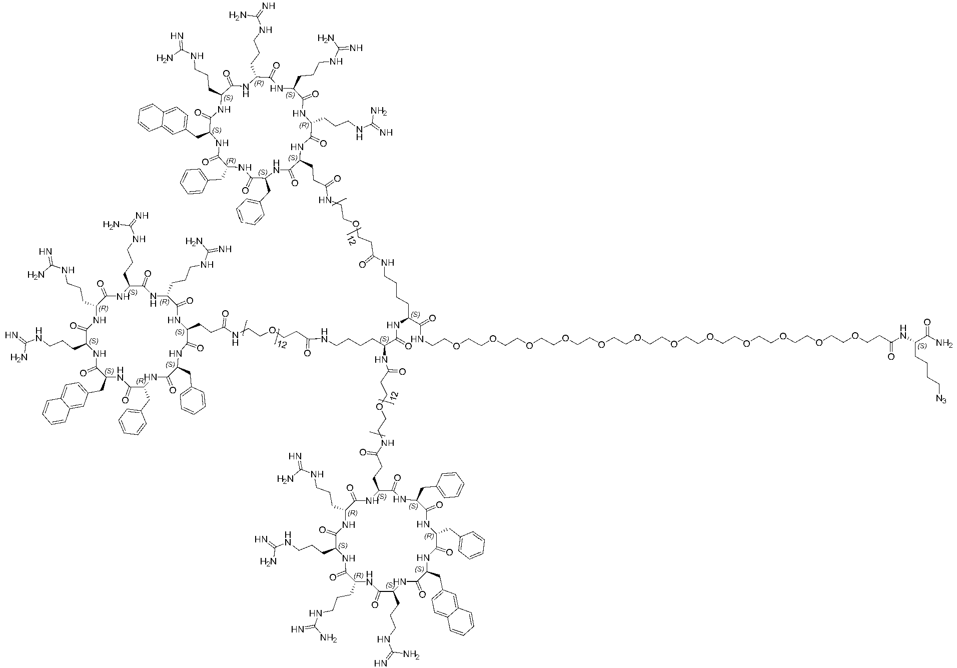

### Ac-NLS-Lys(CPP12)-PEG12-K(N3):

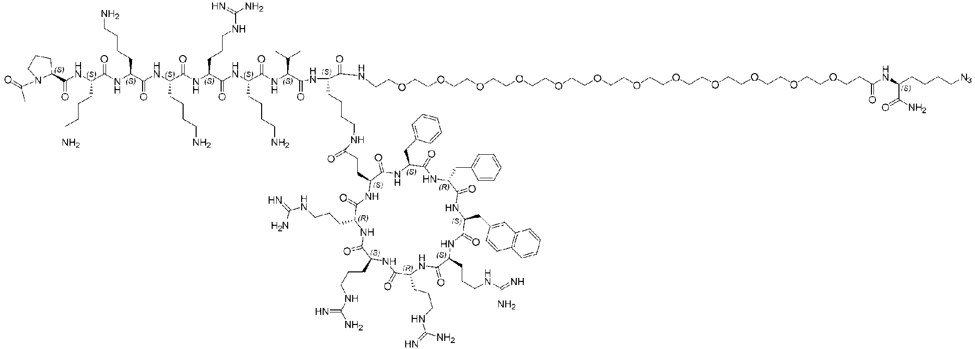

### Lys(N3)-miniPEG-NLS-ss-PEG12-CPP12:

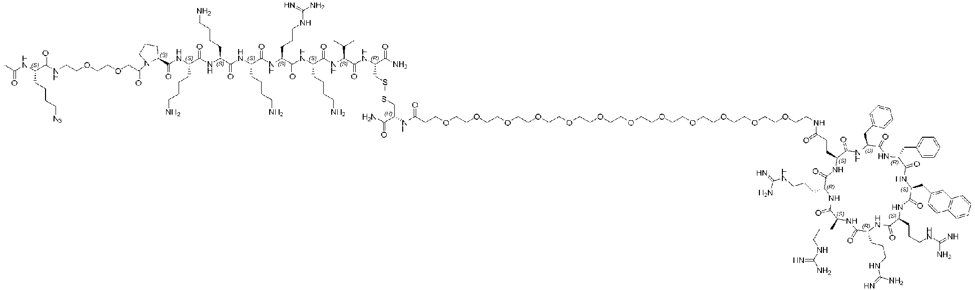

### BCN-NLS-ss-CPP12:

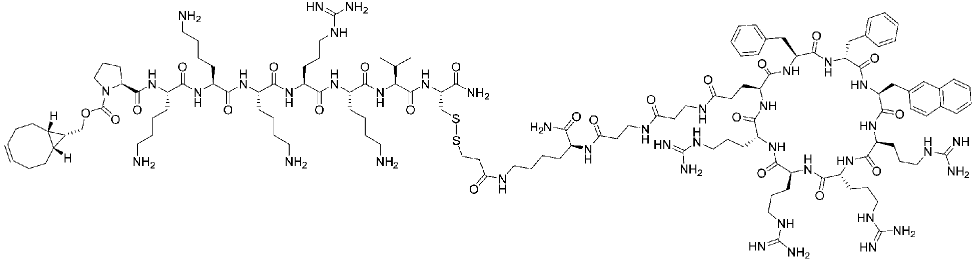

### CPP12-PEG12-Val-Cit-PABC-Lys(N3):

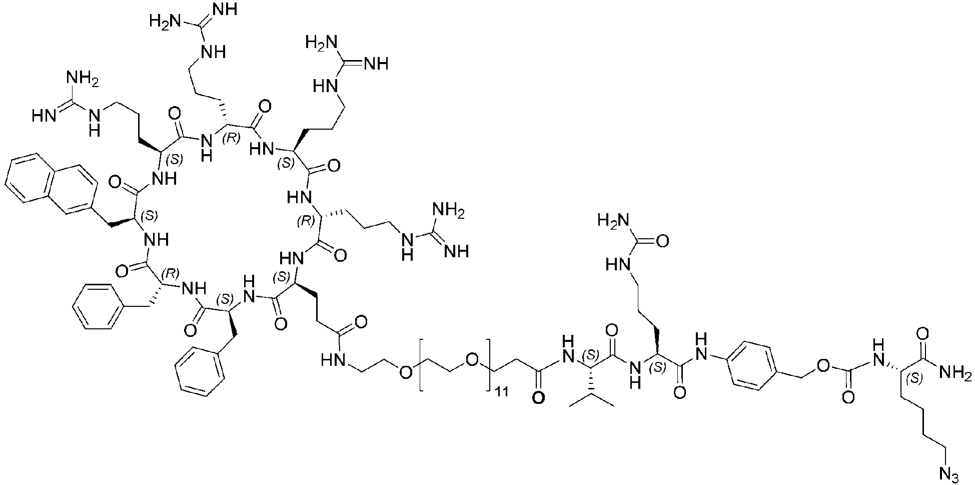

### CPP12-PEG12-Cys-ss-Cys-Lys(N3):

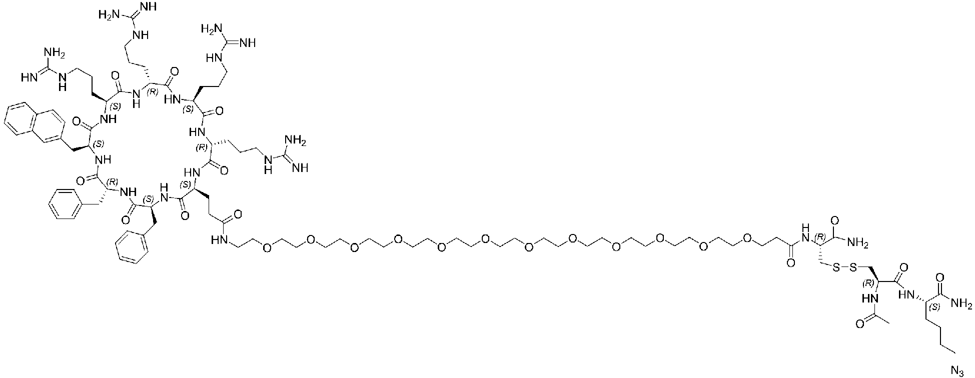

### CPP12-PEG12-Cys-ss-Cys-Lys(N3):

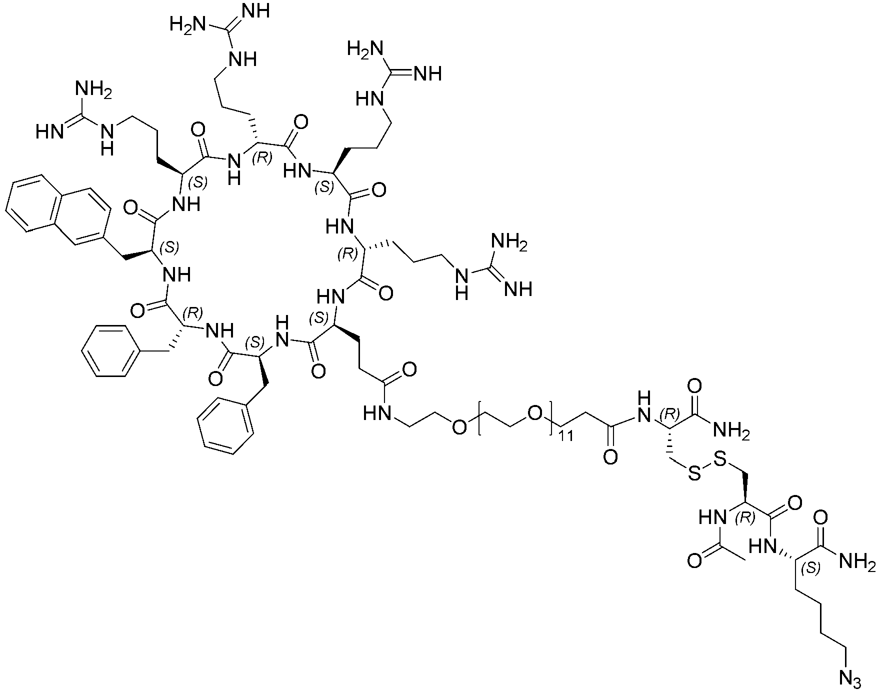

### CPP 12-PEG 12-TFP ETRD802:

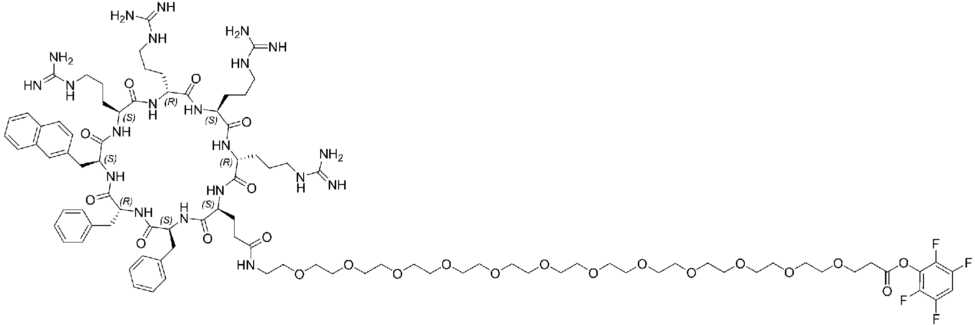

### CPP12-PEG12-Lys(N3):

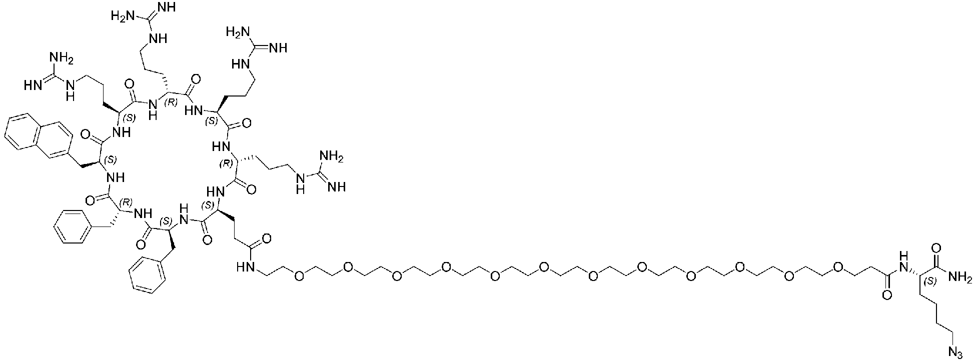

### CPP12-PEG12-Cys-prodisulfide-Lys(N3):

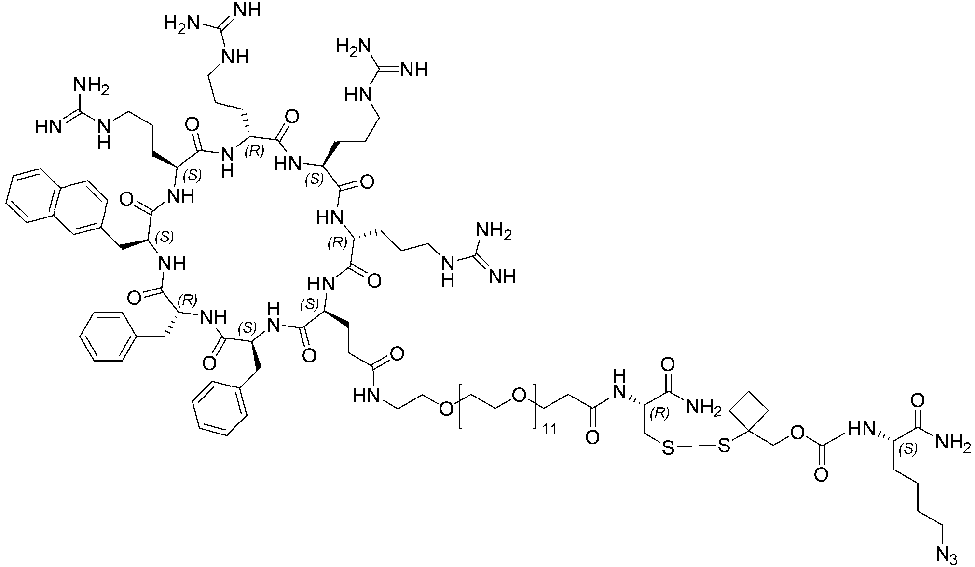

### CPP12-PEG24-Lys(N3):

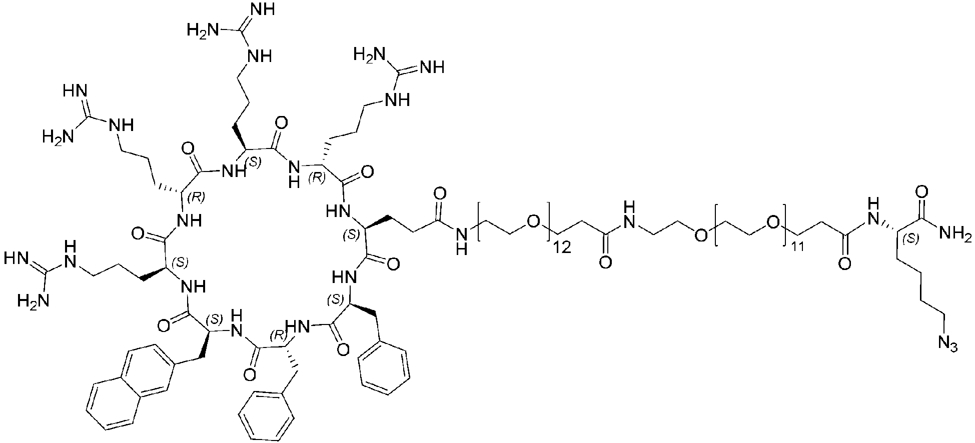

### CPP12-K(CPP12)-PEG12-K(N3):

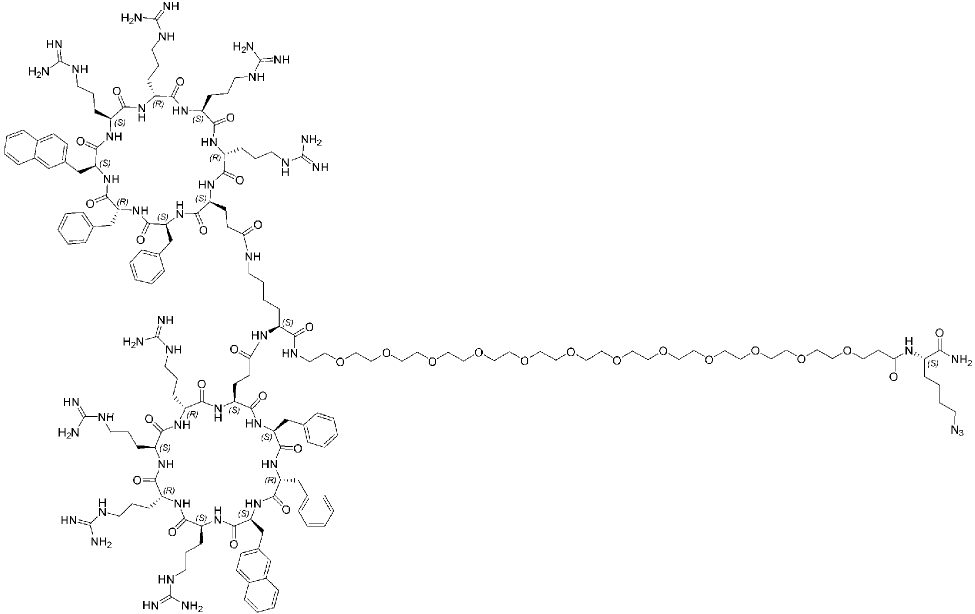

### CPP12-PEG12-K(CPP12)-PEG12-TFP ETRD-344:

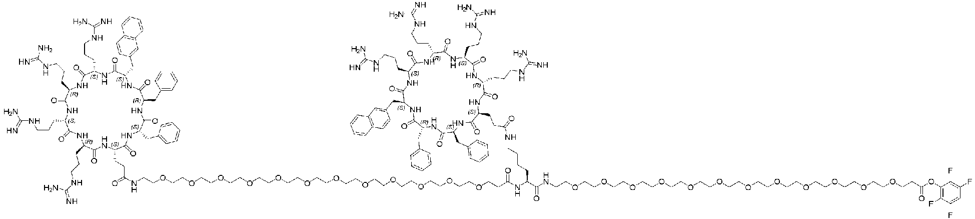

### CPP12-C6-TFP:

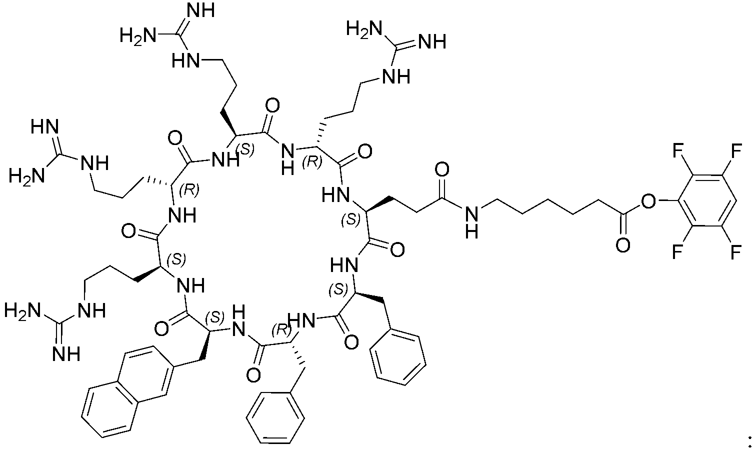

### CPP12-PEG12-K(PEG12-CPP12)PEG12-K(N3):

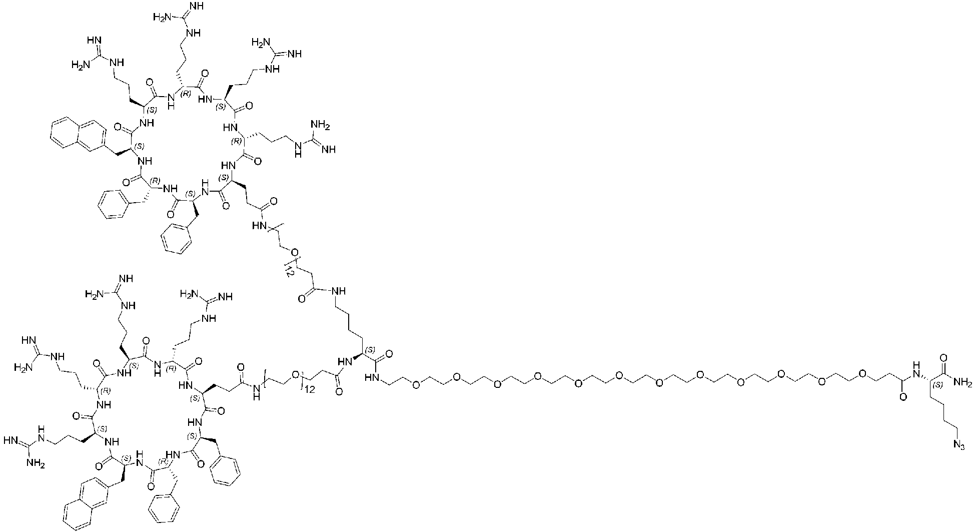

### Ac-T9-PEG12-Lys(CPP12-PEG12)-K(N3):

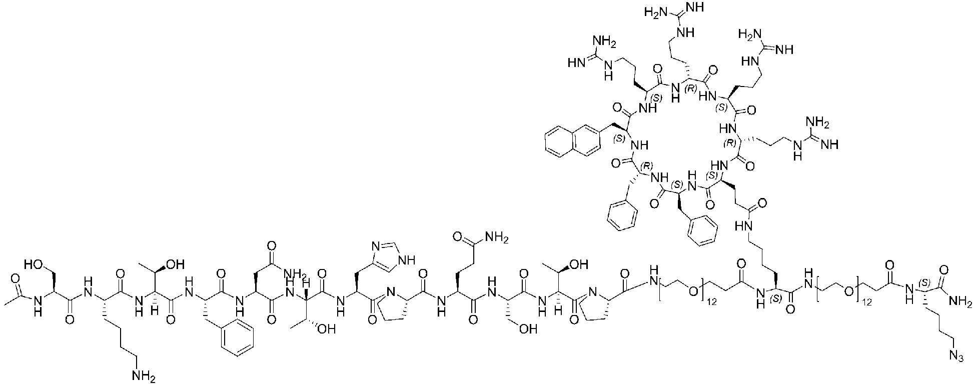

### Ac-MSP-PEG12-Lys(CPP12-PEG12)-K(N3):

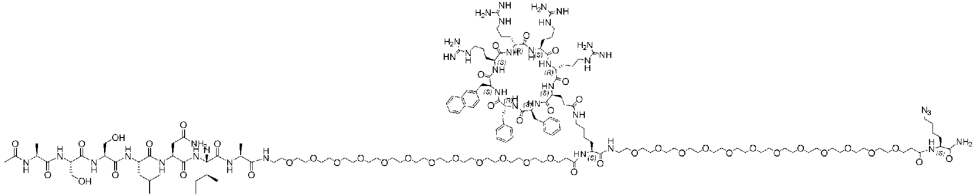

The following CPPs have free carboxylic acid groups that may be utilized for conjugation to an AC.

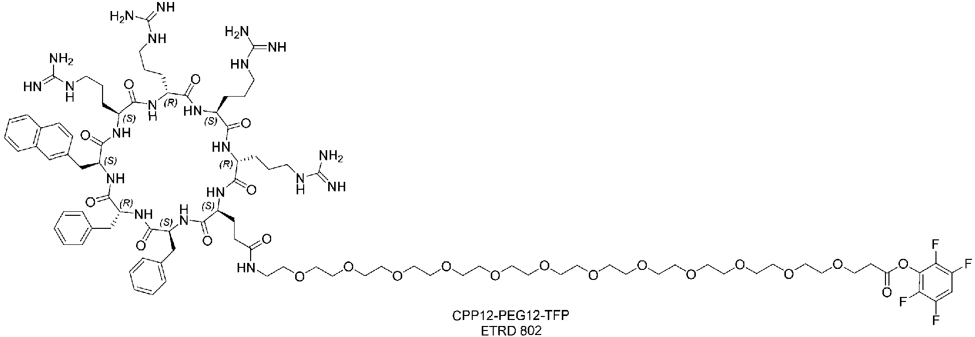

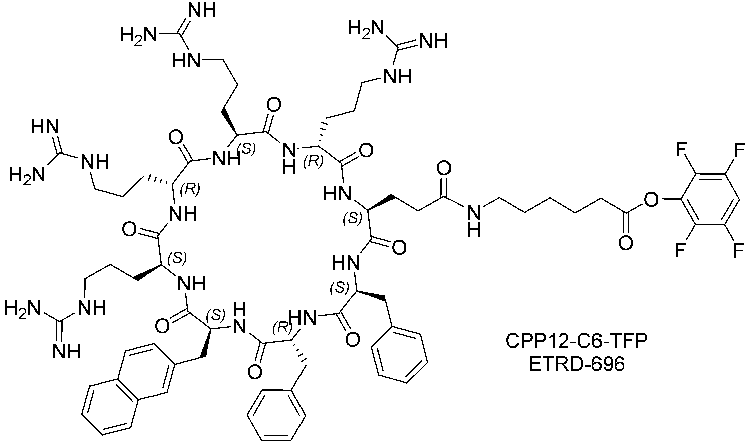

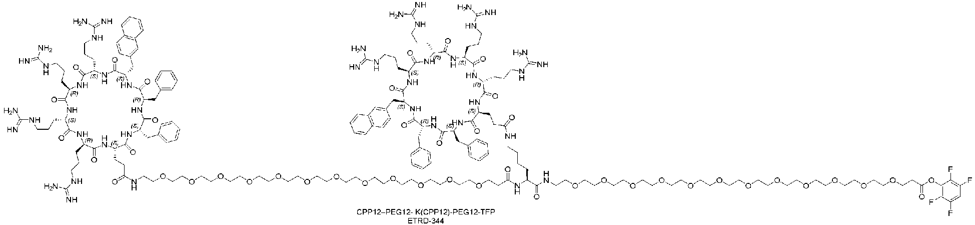

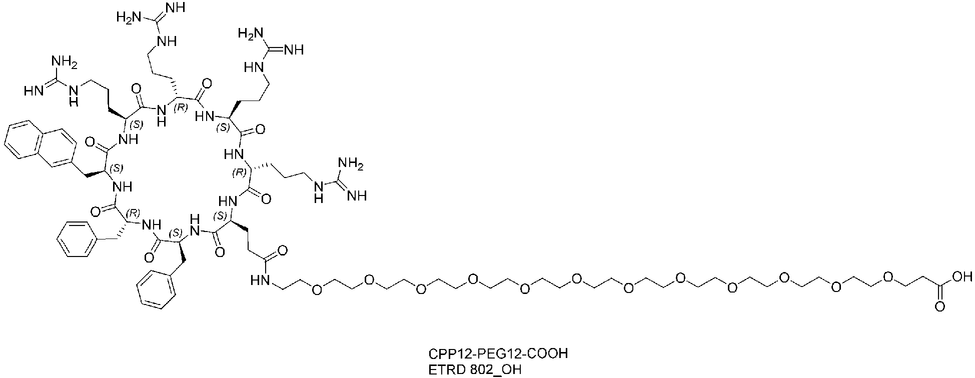

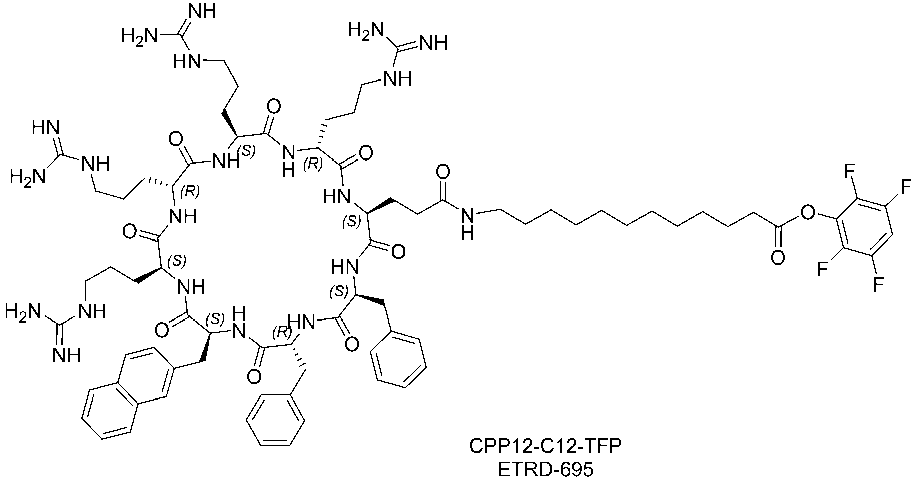

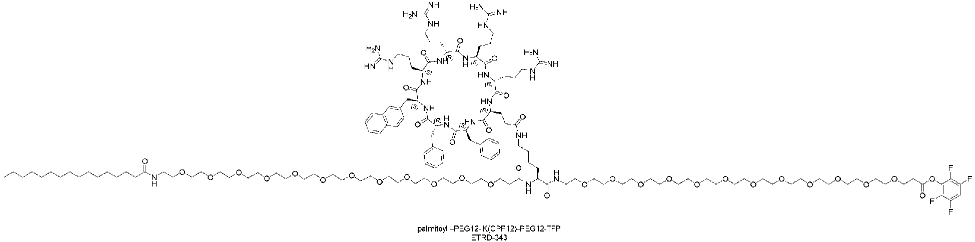

The following CPPs contain azide functional groups on the linker that may be utilized to facilitate addition of an AC. As shown below, in some embodiments, the CPP may also include an NLS conjugated to the side chain of an amino acid in the CPP.

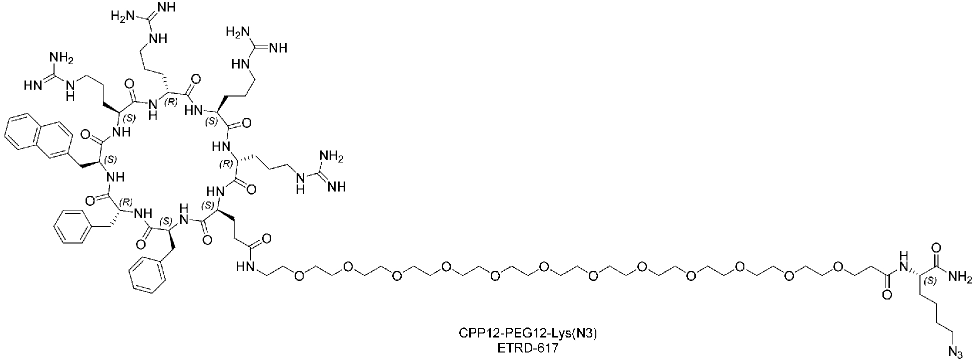

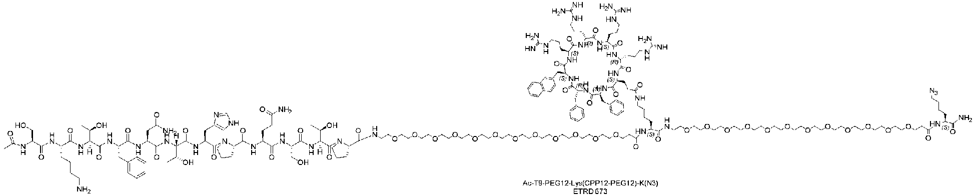

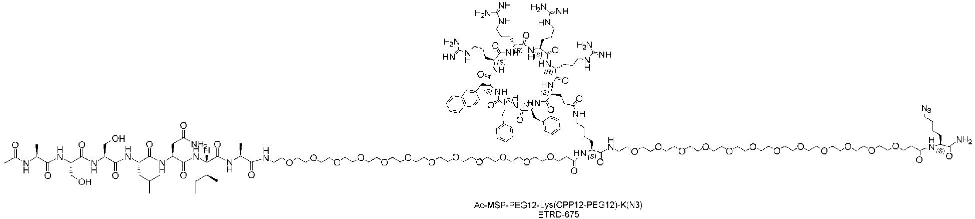

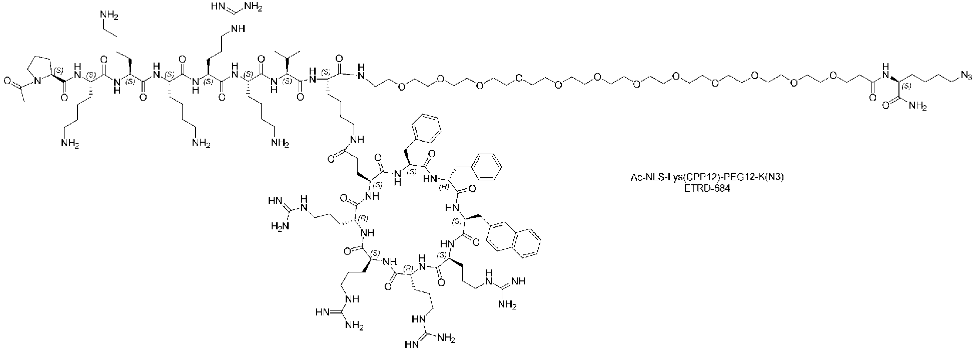

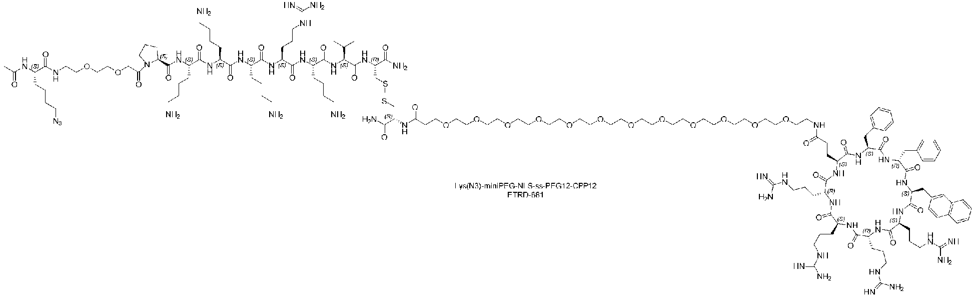

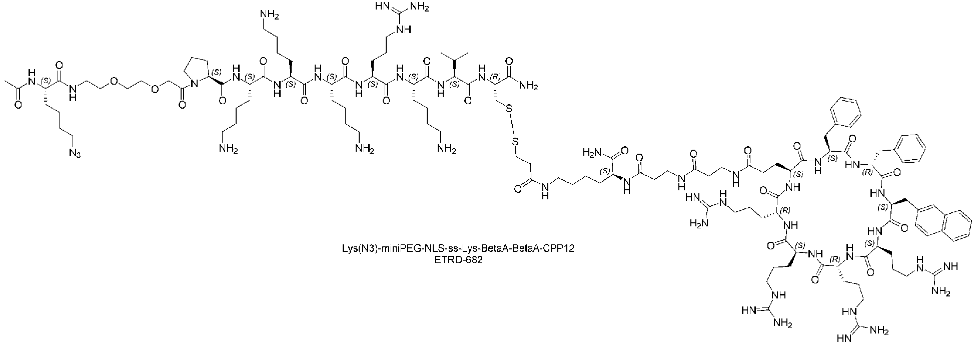

The structure below is a 3' cyclooctyne modified PMO used for a click reaction with CPPs and/or NLS containing an azide:

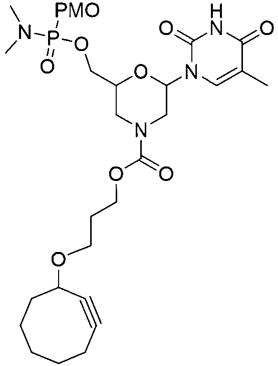

An example scheme of conjugation of a CPP and linker to the 3' end of an AC via an amide bond is shown below.

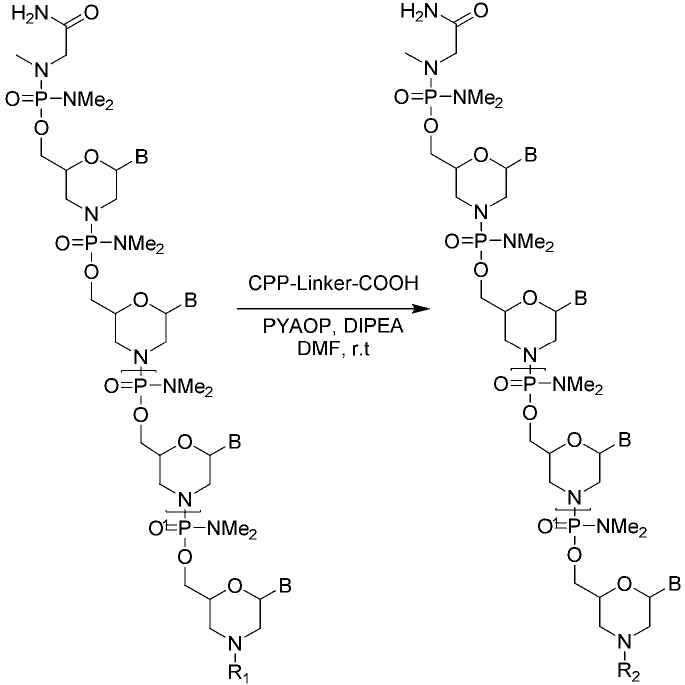

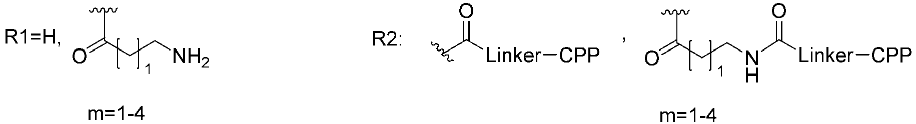

An example scheme of conjugation of a CPP and linker to a 3'-cyclooctyne modified PMO via strain-promoted azide-alkyne cycloaddition is shown below:

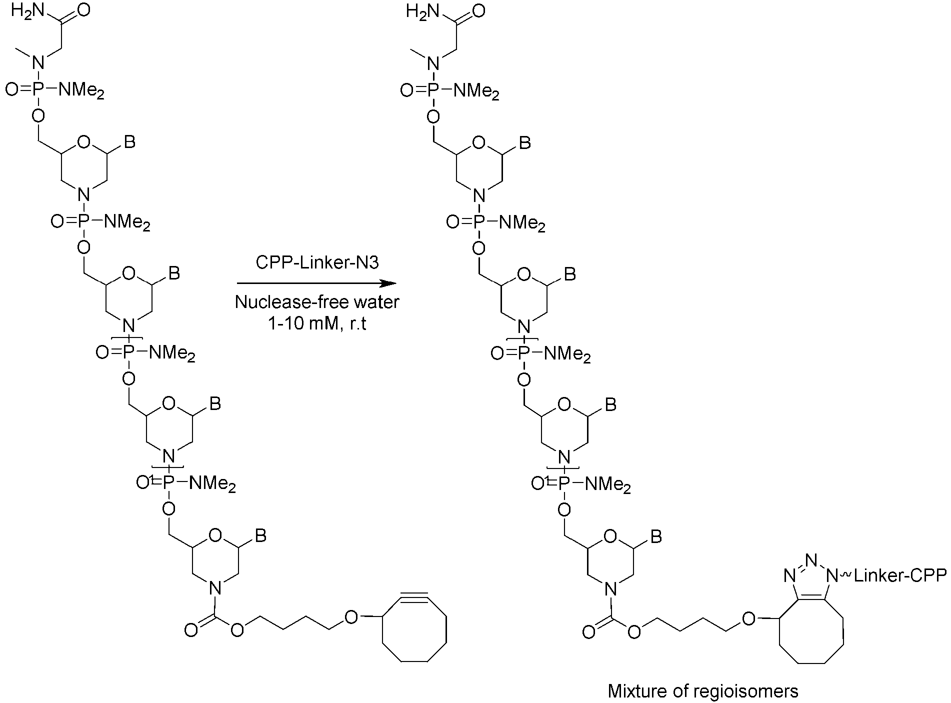

An example of the conjugation chemistry used to connect an AC and CPP with an additional linker containing a polyethylene glycol moiety is shown below:

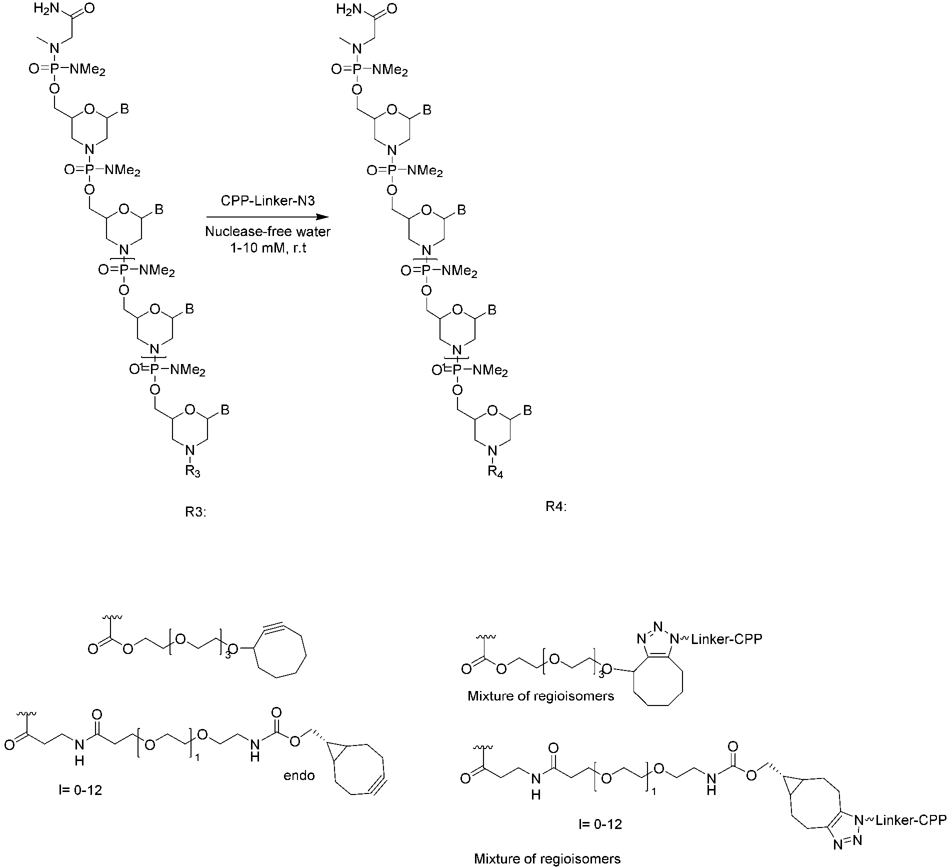

An example of conjugation of a CPP-linker to a 5'-cyclooctyne modified PMO via strain-promoted azide-alkyne cycloaddition (click chemistry) is shown below:

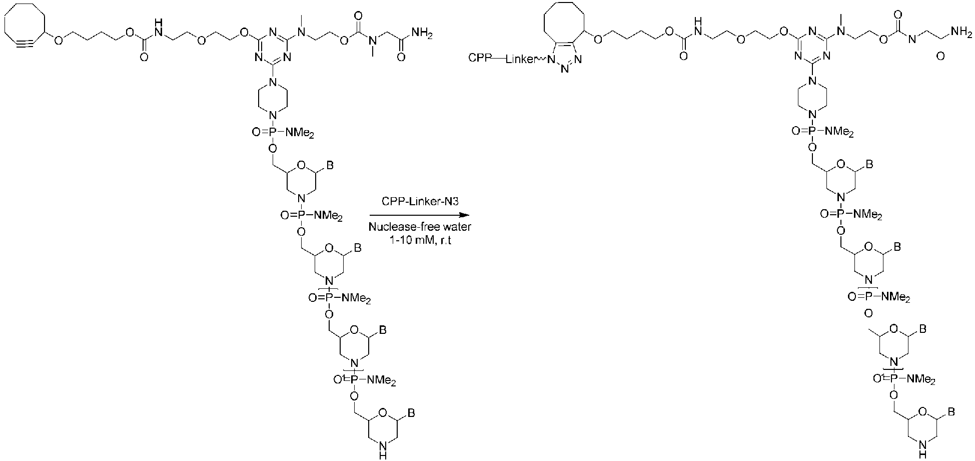

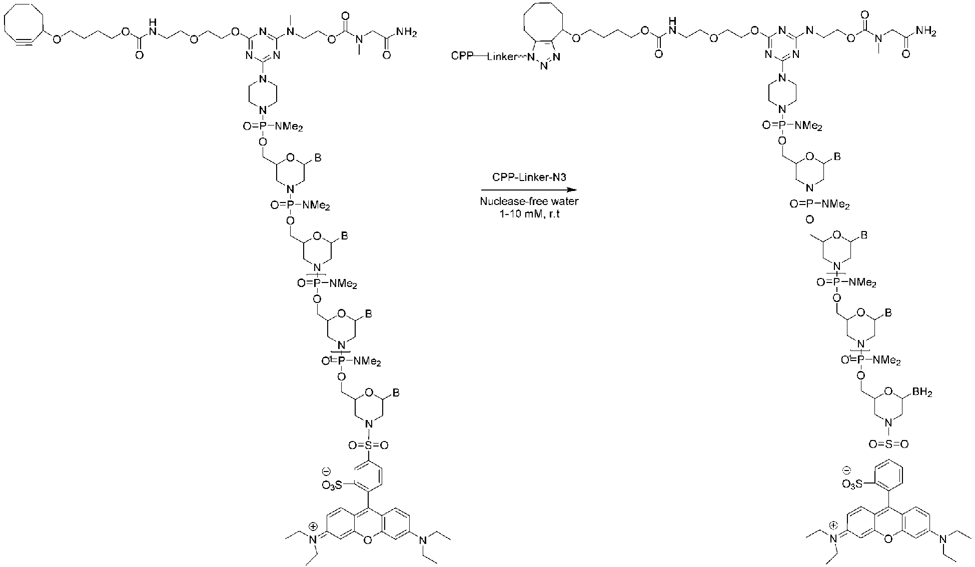

The following compounds contain an AC attached to a linker through click chemistry and a nuclear localization sequence attached to the linker via the C-terminus and the side chain of glutamine on a CPP. Alternative CPPs and/or AC that bind to different targets are envisioned.

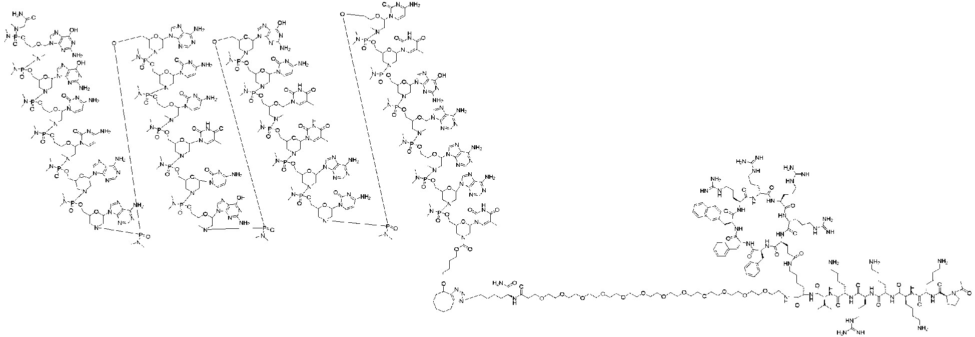

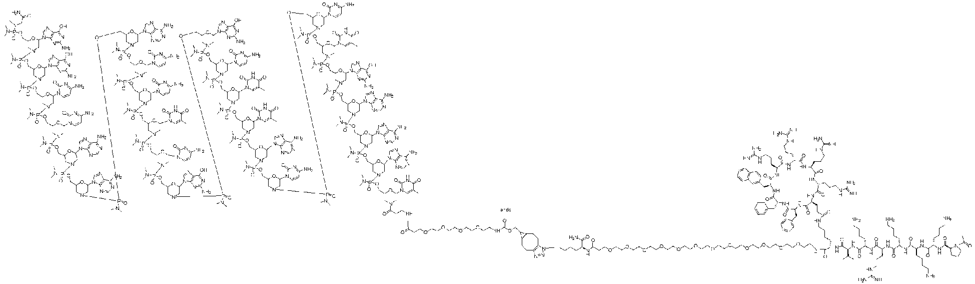

Methods of synthesizing oligomeric antisense compounds are known in the art. The present disclosure is not limited by the method of synthesizing the AC. In some embodiments, provided herein are compounds having reactive phosphorus groups useful for forming internucleoside linkages including for example phosphodiester and phosphorothioate internucleoside linkages. Methods of preparation and/or purification of precursors or antisense compounds are not a limitation of the compositions or methods provided herein. Methods for synthesis and purification of DNA, RNA, and the antisense compounds are well known to those skilled in the art.

Oligomerization of modified and unmodified nucleosides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Antisense compounds provided herein can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

Methods of oligonucleotide purification and analysis are known to those skilled in the art. Analysis methods include capillary electrophoresis (CE) and electrospray-mass spectroscopy. Such synthesis and analysis methods can be performed in multi-well plates. The method of the invention is not limited by the method of oligomer purification.

### Methods of Administration

*In vivo* application of the disclosed compounds, and compositions containing them, can be accomplished by any suitable method and technique presently or prospectively known to those skilled in the art. For example, the disclosed compounds can be formulated in a physiologically- or pharmaceutically-acceptable form and administered by any suitable route known in the art including, for example, oral and parenteral routes of administration. As used herein, the term parenteral includes subcutaneous, intradermal, intravenous, intramuscular, intraperitoneal, intrasternal, and intrathecal administration, such as by injection. Administration of the disclosed compounds or compositions can be a single administration, or at continuous or distinct intervals as can be readily determined by a person skilled in the art.

The compounds disclosed herein, and compositions comprising them, can also be administered utilizing liposome technology, slow release capsules, implantable pumps, and biodegradable containers. These delivery methods can, advantageously, provide a uniform dosage over an extended period of time. The compounds can also be administered in their salt derivative forms or crystalline forms.

The compounds disclosed herein can be formulated according to known methods for preparing pharmaceutically acceptable compositions. Formulations are described in detail in a number of sources which are well known and readily available to those skilled in the art. For example, Remington's Pharmaceutical Science by E.W. Martin (1995) describes formulations that can be used in connection with the disclosed methods. In general, the compounds disclosed herein can be formulated such that an effective amount of the compound is combined with a suitable carrier in order to facilitate effective administration of the compound. The compositions used can also be in a variety of forms. These include, for example, solid, semi-solid, and liquid dosage forms, such as tablets, pills, powders, liquid solutions or suspension, suppositories, injectable and infusible solutions, and sprays. The preferred form depends on the intended mode of administration and therapeutic application. The compositions also preferably include conventional pharmaceutically-acceptable carriers and diluents which are known to those skilled in the art. Examples of carriers or diluents for use with the compounds include ethanol, dimethyl sulfoxide, glycerol, alumina, starch, saline, and equivalent carriers and diluents. To provide for the administration of such dosages for the desired therapeutic treatment, compositions disclosed herein can advantageously comprise between about 0.1% and 100% by weight of the total of one or more of the subject compounds based on the weight of the total composition including carrier or diluent.

Formulations suitable for administration include, for example, aqueous sterile injection solutions, which can contain antioxidants, buffers, bacteriostats, and solutes that render the formulation isotonic with the blood of the intended recipient; and aqueous and nonaqueous sterile suspensions, which can include suspending agents and thickening agents. The formulations can be presented in unit-dose or multi-dose containers, for example sealed ampoules and vials, and can be stored in a freeze dried (lyophilized) condition requiring only the condition of the sterile liquid carrier, for example, water for injections, prior to use. Extemporaneous injection solutions and suspensions can be prepared from sterile powder, granules, tablets, *etc.* It should be understood that in addition to the ingredients particularly mentioned above, the compositions disclosed herein can include other agents conventional in the art having regard to the type of formulation in question.

Compounds disclosed herein, and compositions comprising them, can be delivered to a cell either through direct contact with the cell or via a carrier means. Carrier means for delivering compounds and compositions to cells are known in the art and include, for example, encapsulating the composition in a liposome moiety. Another means for delivery of compounds and compositions disclosed herein to a cell comprises attaching the compounds to a protein or nucleic acid that is targeted for delivery to the target cell. U.S. Patent No. 6,960,648 and U.S. Application Publication Nos. 20030032594 and 20020120100 disclose amino acid sequences that can be coupled to another composition and that allows the composition to be translocated across biological membranes. U.S. Application Publication No. 20020035243 also describes compositions for transporting biological moieties across cell membranes for intracellular delivery. Compounds can also be incorporated into polymers, examples of which include poly (D-L lactide-co-glycolide) polymer for intracranial tumors; poly[bis(p-carboxyphenoxy) propane:sebacic acid] in a 20:80 molar ratio (as used in GLIADEL); chondroitin; chitin; and chitosan.

Compounds and compositions disclosed herein, including pharmaceutically acceptable salts or prodrugs thereof, can be administered intravenously, intramuscularly, or intraperitoneally by infusion or injection. Solutions of the active agent or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations can contain a preservative to prevent the growth of microorganisms.

The pharmaceutical dosage forms suitable for injection or infusion can include sterile aqueous solutions or dispersions or sterile powders comprising the active ingredient, which are adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. Optionally, the prevention of the action of microorganisms can be brought about by various other antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the inclusion of agents that delay absorption, for example, aluminum monostearate and gelatin.

Sterile injectable solutions are prepared by incorporating a compound and/or agent disclosed herein in the required amount in the appropriate solvent with various other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

Useful dosages of the compounds and agents and pharmaceutical compositions disclosed herein can be determined by comparing their *in vitro* activity, and *in vivo* activity in animal models. Methods for the extrapolation of effective dosages in mice, and other animals, to humans are known to the art.

The dosage ranges for the administration of the compositions are those large enough to produce the desired effect in which the symptoms or disorder are affected. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counterindications. Dosage can vary, and can be administered in one or more dose administrations daily, for one or several days.

Also disclosed are pharmaceutical compositions that comprise a compound disclosed herein in combination with a pharmaceutically acceptable carrier. Pharmaceutical compositions adapted for oral, topical or parenteral administration, comprising an amount of a compound constitute a preferred aspect. The dose administered to a patient, particularly a human, should be sufficient to achieve a therapeutic response in the patient over a reasonable time frame, without lethal toxicity, and preferably causing no more than an acceptable level of side effects or morbidity. One skilled in the art will recognize that dosage will depend upon a variety of factors including the condition (health) of the subject, the body weight of the subject, kind of concurrent treatment, if any, frequency of treatment, therapeutic ratio, as well as the severity and stage of the pathological condition.

Also disclosed are kits that comprise a compound disclosed herein in one or more containers. The disclosed kits can optionally include pharmaceutically acceptable carriers and/or diluents. In one embodiment, a kit includes one or more other components, adjuncts, or adjuvants as described herein. In another embodiment, a kit includes one or more anti-cancer agents, such as those agents described herein. In one embodiment, a kit includes instructions or packaging materials that describe how to administer a compound or composition of the kit. Containers of the kit can be of any suitable material, *e.g.*, glass, plastic, metal, *etc.*, and of any suitable size, shape, or configuration. In one embodiment, a compound and/or agent disclosed herein is provided in the kit as a solid, such as a tablet, pill, or powder form. In another embodiment, a compound and/or agent disclosed herein is provided in the kit as a liquid or solution. In one embodiment, the kit comprises an ampoule or syringe containing a compound and/or agent disclosed herein in liquid or solution form.

A number of embodiments of the invention have been described. Nevertheless, it will be understood that various modifications may be made without departing from the spirit and scope of the invention. Accordingly, other embodiments are within the scope of the following claims.

### EXAMPLES

### Example 1A. Use of cell-penetrating peptides conjugated to oligonucleotides for restoring protein expression in eukaryotic cells.

The compounds of Table A are evaluated in Examples 1A and 1B. Exemplary experiments are described below for ENTR-0006. The same techniques are used to evaluate any compound of Table A.

**Table A. Compounds that target EGFP-654**

| **Oligo#** | **Design** | **Sequence** |
|---|---|---|
| ENTR-0006 | PMO-654 | 5'-GCT ATT ACC TTA ACC CAG-3' (SEQ ID NO: 221) |
| ENTR-0070 | PMO654-NH2 | 5'-GCT ATT ACC TTA ACC CAG-3' -C6-NH2 (all PMO monomers) (SEQ ID NO: 222) |
| ENTR-0059 | PMO654-LSR | cyclooctyne-5'-GCT ATT ACC TTA ACC CAG-3'-Lissamine Rho (all PMO monomers) (SEQ ID NO: 223) |
| ENTR-0123 | CPP12-PMO654-LSR | (CPP12-PEG12)-5'-GCT ATT ACC TTA ACC CAG-3'-Lissamine Rho (all PMO monomers) (SEQ ID NO: 224) |
| ENTR-0121 | PMO654-CPP12 | 5'-GCT ATT ACC TTA ACC CAG-3'-NH2 (all PMO monomers)+CPP12-PEG12-TFP-Amide chemistry (SEQ ID NO: 225) |
| ENTR-0106 | PMO654-3'-Asymmetric bidentate | 5'-GCT ATT ACC TTA ACC CAG-3'-NH2 (all PMO monomers)+CPP1212-TFP; Amide chemistry (SEQ ID NO: 226) |
| ENTR-0108 | 5'-Bidentate-PMO654-LSR | (CPP12-(bidentate-no linker)-PEG12)-5'-GCT ATT ACC TTA ACC CAG-3'-Lissamine Rho (all PMO monomers) (SEQ ID NO: 227) |
| ENTR-0109 | 5'-Bidentate-PMO654-LSR | (CPP12-(bidentate-PEG4)-PEG12)-5'-GCT ATT ACC TTA ACC CAG-3'-Lissamine Rho (all PMO monomers) (SEQ ID NO: 228) |
| ENTR-0110 | 5'-Bidentate-PMO654-LSR | (CPP12-(bidentate-PEG12)-PEG12)-5'-GCT ATT ACC TTA ACC CAG-3'-Lissamine Rho (all PMO monomers) (SEQ ID NO: 229) |
| ENTR-0111 | 5'-Tridentate-PMO654-LSR | (CPP12-(tridentate-no linker)-PEG12)-5'-GCT ATT ACC TTA ACC CAG-3'-Lissamine Rho (all PMO monomers) (SEQ ID NO: 230) |
| ENTR-0112 | 5'-Tridentate-PMO654-LSR | (CPP12-(tridentate-PEG4)-PEG12)-5'-GCT ATT ACC TTA ACC CAG-3'-Lissamine Rho (all PMO monomers) (SEQ ID NO: 231) |
| ENTR-0113 | 5'-Tridentate-PMO654-LSR | (CPP12-(tridentate-PEG12)-PEG12)-5'-GCT ATT ACC TTA ACC CAG-3'-Lissamine Rho (all PMO monomers) (SEQ ID NO: 232) |
| ENTR-0047 | PMO654-NLS-ss-CPP12 | 5'-GCT ATT ACC TTA ACC CAG-3-NLS-ss-CPP12 (all PMO monomers) (SEQ ID NO: 233) |
| ENTR-0168 | NLS-CPP12-PMO654-LSR | (Ac-NLS-Lys(CPP12)-PEG12-K(N3))-5'-GCT ATT ACC TTA ACC CAG-3'-Lissamine Rho (all PMO monomers) (SEQ ID NO: 234) |
| ENTR-0203 | PMO654-CPP12-NLS | 5'-GCT ATT ACC TTA ACC CAG-3'-click-K-PEG12-Lys(CPP12)-NLS-Ac (all PMO monomers) (SEQ ID NO: 235) |
| ENTR-0207 | PMO654-NLS-CPP12 | 5'-GCT ATT ACC TTA ACC CAG-3'-click-Lys(N3)-miniPEG-NLS-ss-PEG12-CPP12 (all PMO monomers) (SEQ ID NO: 236) |

**Target gene design.** A missense EGFP gene ("EGFP-654") was designed with a mutated intron 2 of the human β globin gene interrupting the EGFP coding sequence. A mutation was introduced at nucleotide 654 of intron 2, which activated aberrant splice sites and led to retention of the intron fragment in spliced, mature mRNA, thereby preventing proper translation of EGFP. A schematic of the target gene, mutation site, splicing patterns, and resulting protein is provided in **FIG. 1****.**

**Oligonucleotide design.** An antisense compound (AC) was designed to bind to and block the aberrant splice site of the target gene in order to correct pre-mRNA splicing and restore EGFP expression. The AC had the sequence "5'-GCTATTACCTTAACCCAG-3'" (SEQ ID NO:148) and was designed as a phosphorodiamidate morpholino oligomer (PMO) with a C6-thiol 5' modification (ENTR-0006, Table A). A schematic of the binding of the PMO to the target gene is provided in **FIG. 1****.**

**Cell penetrating peptide.** A cell-penetrating peptide comprising cyclo(Phe-D-Phe-SNal-Arg-D-Arg-Arg-D-Arg-γ-Glu)-b-Ala-b-Ala-Lys(Maleimide)-NH2 ("CPP12-Maleimide") (SEQ ID NO: 149) was formulated as a TFA salt. The peptide was synthesized using standard Fmoc chemistry according to the following procedure:
a. 1. DCM added to the vessel containing Rink amide resin (1 mmol, 1 g, 1.0 mmol/g) and Fmoc-Lys(Trt)-OH (1 eq) with N₂ bubbling.
b. 2. DIEA (4.0 eq) was added dropwise and mix for 4 hours.
c. 3. MeOH (0.2 mL) was added to the resin and mixed for 30 min.
d. 4. Solution was drained and washed with DMF for 5 times.
e. 5. 20% piperidine/DMF was added and reacted for 30 min.
f. 6. The solution was drained and washed with DMF for 5 times.
g. 7. Fmoc-amino acid solution was added to resin and mixed for 30 seconds, then activation buffer was added and mixed with N₂ bubbling for about 1 hour.
h. 8. Step 4 to 7 was repeated for the coupling of following amino acids. All couplings completions were confirmed by negative ninhydrin test. After couplings, resin was washed with DMF for 5 times.
i. 9. Allyl protecting group was removed by adding PhSiH3 (10eq) and Pd(PPh3)4 (0.1 eq) in DCM, repeat twice.
j. 10. Peptide was cyclized by HATU (1.0 eq) and DIEA (2 eq). The cyclization reaction was monitored by ninhydrin test. Resin was washed with DMF five times.
k. 11. The Trt protecting group was removed with 20%HIFP/80%DCM and the resulting primary amine was reacted with OAt activated 3-Maleimidopropionic acid using HATU/DIPEA for 2 hours
l. 12. After coupling, the resin was washed with MeOH for 3 times and dried under reduce pressure.

The table below shows the materials used for solid-phase peptide synthesis and coupling reagents:

| **#** | **Materials** | **Coupling reagents** |
|---|---|---|
| 1 | Fmoc-NH-PEG12-CH₂CH₂COOH (1.0 eq) | DIEA (4.0 eq) |
| 2 | Fmoc-Glu-OAll (1.5 eq) | HATU (1.45 eq) and DIEA (3.0 eq) |
| 3 | D-Fmoc-Arg(Pbf)-OH (3 eq) | HBTU (2.85 eq) and DIEA (6.0 eq) |
| 4 | Fmoc-Arg(Pbf)-OH (3 eq) | HBTU (2.85 eq) and DIEA (6.0 eq) |
| 5 | D-Fmoc-Arg(Pbf)-OH (3 eq) | HBTU (2.85 eq) and DIEA (6.0 eq) |
| 6 | Fmoc-Arg(Pbf)-OH (3 eq) | HBTU (2.85 eq) and DIEA (6.0 eq) |
| 7 | Fmoc-3-(2-Nal)-Ala-OH (1.5 eq) | HATU (1.45 eq) and DIEA (3.0 eq) |
| 8 | D-Fmoc-Phe-OH (3 eq) | HBTU (2.85 eq) and DIEA (6.0 eq) |
| 9 | Fmoc-Phe-OH (3 eq) | HBTU (2.85 eq) and DIEA (6.0 eq) |
| 10 | Cyclization | HATU (1.00 eq) and DIEA (2.0 eq) |

The peptide was cleaved from the solid-phase peptide synthesis resin and purified according to the following procedure:
a. Add cleavage buffer (95 % TFA, 2.5 % TIPS, 2.5 % H₂O) to the flask containing the side chain protected peptide at room temperature and stir for 1 hour and repeat one time.
b. Filter and collect the peptide solution.
c. The peptide is concentrated under reduce press to give residue.
d. The resulting solid is dissolved in CH₃CN and H₂O, then lyophilized to give crude peptide (2 g, 70.1% yield) as white solid.

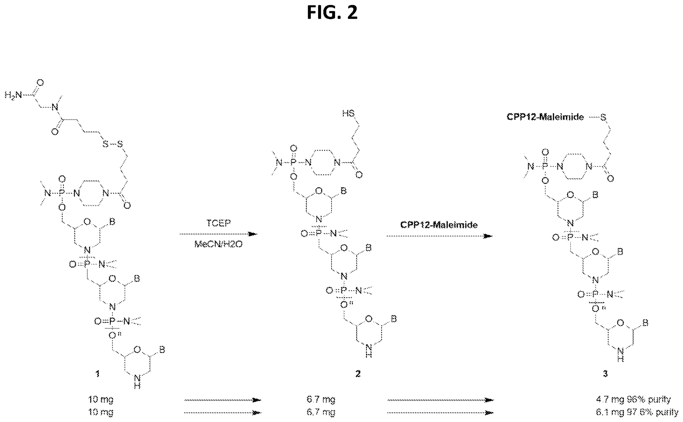

**CPP-AC conjugate formation.** The steps of the conjugation process are shown in **FIG. 2****.** Compound 1 was treated with 1M TCEP and MeCN/H₂O to reduce the 5' end resulting in compound 2..To this solution of compound **2** (~13 mg, from reduction of ~10 mg*2 PMO654-G) in H2O/CH3CN (3:2, 2 mL) was added **CPP12-Maleimide** (4.5 mg, 1.2 eq, previously dissolved in H2O/CH₃CN (3:2), 98.4 uL) in one portion. Then to the mixture was added PB buffer (PH=7, 1mL) and stirred for 1 hour at 25 °C. Another batch (~12 mg) processed in parallel. LCMS shown the compound **1** was consumed completely and the solution was directly injected and purified by C18 reverse phase column. The mixture was first eluted with TEEA condition (2 mM TEAA in water, CH₃CN) and then eluted with TFA condition (0.075% TFA in water, CH3CN) to give **CPP12-Maleimide-PMO-654** (28 mg, 96.3% purity, total yield: 56.8%).

### Purification Conditions:

| Separation condition | |
|---|---|
| Dissolution condition | Dissolve in H₂O |
| Instrument | GX-281D |
| Mobile Phase 1 | A: H₂O (2mM TEEA in H₂O) |
| | B: CH₃CN |
| Gradient 1 | 10-60%B 60min. Separate the impurity that could be elute with TEEA condition. |
| Mobile Phase 2 | A: H₂O (0.075% TFA in H₂O) |
| | B: CH₃CN |
| Gradient 2 | 20-50%B 60min. Retention time: 14.5 min |
| Column | Luna 200*25 mm, C18, 10 um, 100Å+Gemini^{®} 150*30 mm, C18, 5 um, 110Å |
| Flow Rate | 20 mL/Min |
| Wavelength | 220/254 nm |
| Oven Tem. | Room temperature |

**PMO synthesis protocol and linker installation at the 3' end.** The following protocol was used to synthesize the PMO. For every step of the following synthesis protocol, it was ensured that the volume of reagent or solvent used completely covers resin (add more, if needed). Volumes listed below are estimates in number of milliliters per gram of resin used, which will increase during synthesis due to increasing resin size. All synthesis steps in the table were performed at room temperature. Prior to synthesis the resin is swelled in NMP for 1 hour. The resin was washed two times with DCM, followed by washing resin 2 times with 30% TFE/DCM (15 mL/g of resin). The table below describes the PMO synthesis and linker installation protocol:

| Entry | Step | Volume | Time | Mixture |
|---|---|---|---|---|
| 1 | Wash | 7-24 mL/g | 15 seconds | 30%TFE/DCM |
| 2 | Deprotection | 7-24 mL/g | 15 minutes | CYTFA Solution |
| 3 | Deprotection | 7-24 mL/g | 15 minutes | CYTFA Solution |
| 4 | Wash | 7-24 mL/g | 15 seconds | DCM |
| 5 | Wash | 7-24 mL/g | 15 seconds | DCM |
| 6 | Neutralization | 7-24 mL/g | 5 minutes | Neutralization Solution |
| 7 | Neutralization | 7-24 mL/g | 5 minutes | Neutralization Solution |
| 8 | Wash | 7-24 mL/g | 15 seconds | DCM |
| 9 | Wash | 7-24 mL/g | 15 seconds | DCM or anhydrous DMI |
| 10 | Coupling | 4.6-7.3 mL/g | 4.25-5.0 hours | 3-6 eq monomer, 5-8 eq NEM in DMI (0.2-0.4 M monomer & 0.5 M NEM) |
| 11 | Wash | 7-24 mL/g | 15 seconds | DCM |
| 12 | Capping | 7-24 mL/g | 15 minutes | 0.55 M Benzoic anhydride + 0.55 M NEM in NMP |
| 13 | Neutralization | 7-24 mL/g | 5 minutes | Neutralization Solution |
| 14 | Wash | 7-24 mL/g | 15 seconds | DCM |
| 15 | Wash | 7-24 mL/g | 15 seconds | 30% TFE/DCM |
| 16 | Wash | 7-24 mL/g | 15 seconds | 30% TFE/DCM |

| | | | | |
|---|---|---|---|---|
| CYTFA Solution = 100 mM 4-cyanopyridine + 100 mM TFA in 4:1 DCM:TFE + 1% Ethanol. | | | | |

Below are the solutions used for synthesis:
a. Neutralization Solution = 5% DIPEA in 3:1 DCM:iPrOH.
b. Coupling Solution = The number of equivalents and concentration will increase throughout synthesis using the following guide:
c. Residues 1-10 = 3 eq morpholino monomer, 5 eq NEM in DMI (0.2 M morpholino monomer, 0.5 M NEM), rt, 4.25h.
d. Residue 1: couple for 5h at rt.
e. Residues 11-20 = 4 eq morpholino monomer, 6.5 eq NEM in DMI (0.3 M morpholino monomer, 0.5 M NEM), rt, 4.25h.
f. Residues 21-25 = 5 eq morpholino monomer, 8 eq NEM in DMI (0.3 M morpholino monomer, 0.5 M NEM), rt, 4.25h.
g. Guanine morpholino monomers: couple for 4.75h at rt.

Some couplings employed 6 eq morpholino monomer, 8 eq NEM in DMI (0.4 M morpholino monomer, 0.5 M NEM) at 45 °C for 4.75 hours.

The PMO synthesis resin with linker has the following structure:

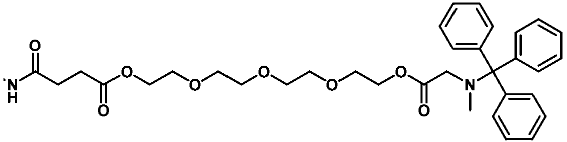

The morpholino monomers used during coupling have the following structures:

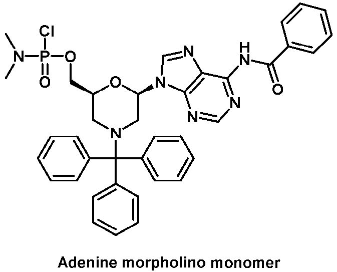

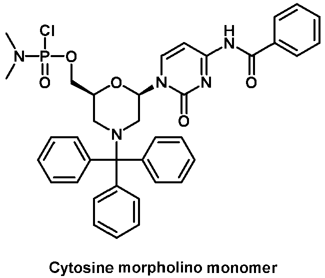

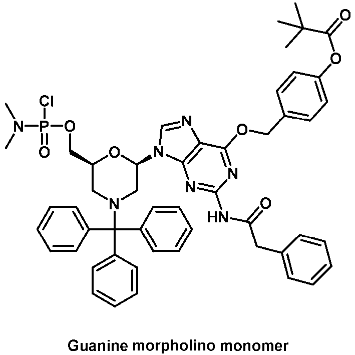

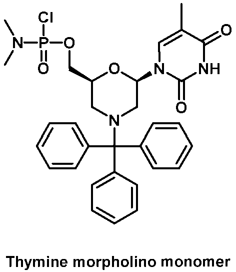

Below is the protocol for PMO synthesis:
Deprotection: Resin was first washed with 30% TFE/DCM solution and was allowed to stand for 15 seconds before draining. CYTFA solution was then added to the drained resin and reacted for 15 minutes. The resin was drained, then fresh CYTFA solution was added and reacted again for 15 minutes. The resin was drained and rinsed twice with DCM for 15 seconds before proceeding to neutralization.

Neutralization: Neutralization solution was added to the resin, stirred, and was allowed to stand for 5 minutes, then drained. A second wash of fresh neutralization solution was delivered to the resin, stirred, and reacted for 5 minutes. The resin was washed once with DCM and once with either DCM or anhydrous DMI before coupling.

Coupling: Using the guide listed above, two coupling solutions were made: 1) PMO monomer dissolved in DMI, and 2) NEM dissolved in DMI. These two solutions were mixed immediately before adding to the resin. The resin was stirred and reacted for 4.25-5 hours. The resin was washed one time with DCM.

Capping: A capping solution consisting of 0.55 M benzoic anhydride and 0.55 M NEM in NMP was added to the resin and reacted for 15 minutes. The resin was drained, and Neutralization solution was added to the resin to react for 5 minutes. The resin was drained again and was washed once with DCM, then twice with 30% TFE/DCM solution.

Post-synthesis: After the final coupling step, the resin-bound PMO can be stored until it is cleaved by washing the resin eight times with iPrOH, then drying the resin under vacuum at room temperature (Note: 3'-Trityl protecting group must still be on PMO for this). For PMO modifications at 3', the Trityl protecting group was removed, resin was neutralized, then appropriate bifunctional linker (TFA-protected amino or cyclooctyne) PFP ester (4 eq) in NMP and DIPEA (8 eq) were added to the resin and reacted for 3 hours. Solution was drained and resin was washed once with DCM, then twice with 30% TFE/DCM solution.

Cleavage:The following options can be utilized to perform PMO cleavage:
a. PMO was cleaved from resin with a 1:1 solution of ammonium hydroxide (25% ammonia, aqueous) and methylamine (8 mL/g) at 65 °C for 15 minutes.
b. PMO was cleaved from resin with ammonium hydroxide (25% ammonia, aqueous) at 65 °C for 16 hours.
c. PMO was cleaved from resin with 7M Ammonia in methanol solution (8 mL/g of resin) at 65 °C for 16 hours.
d. The deprotected PMO solution was then desalted prior to lyophilization and purification.
e. Resulting PMO was purified by Clarity 5 µm , C18 oligo reverse phase (250 mm×30 mm), AXIA packed, with 10-30% gradient over 40 min, flow rate of 30 mL/min, solvent A as water with 0.05% TFA and solvent B as Acetonitrile.

**Design and preparation of CPP-PMO654 conjugates.** Design of monovalent CPP-PMO (ENTR-0121, ENTR-0123), bivalent CPP-PMO (ENTR-0106, ENTR-0108, ENTR-0109 and ENTR-0110), trivalent CPP-PMO (ENTR-0111, ENTR-0112 and ENTR-0113) and mono CPP-NLS-PMO (ENTR-0047, ENTR-0168, ENTR-0203 and ENTR-0207) are shown in Table A. Specific structures of the conjugated AC are shown throughout this disclosure. For 3' covalent conjugation of primary amine modified PMOs, a solution of desired peptide-TFP ester in DMF (4 eq, 5 mM) was added to a solution of PMO-3'-primary amine (1 equivalent, 2 mM) in PBS-10X. Reaction proceed to completion in 4-8 hours at room temperature as confirmed by LCMS (Q-TOF), using BEH C18 column (130Å, 1.7 µm, 2.1mm×150 mm), buffer A: water, 0.1% FA), buffer B: acetonitrile, 0.1% FA), flow rate (0.3 mL/min), starting with 2% buffer B and ramping up to 70% for 11 min for a total of 20 min run. For 3' or 5' conjugation via click reaction, a solution of peptide-azide in nuclease-free water (1 mM) was added to the PMO-3'-cyclooctyne or cyclooctyne-5'-PMO solids. The mixture was vortexed to dissolve the peptide-PMO conjugate, centrifuged to settle the solution, and incubated at room temperature for 8-12 hours for completion as confirmed by LCMS (Q-TOF). For purifications, crude mixtures were diluted with DMSO, loaded onto a C18 reverse-phase column (150mm* 21.2 mm), flow rate of 20 mL/min and purified by an appropriate gradient over 20 min using water with 0.05% TFA and acetonitrile as solvents. Desired fractions were pooled, pH of the solution was adjusted to 5-6 by 1M NaOH and the solution underwent the lyophilization process, affording white lyophilized powder. For *in vitro* and *in vivo* formulations, the conjugates were reconstituted in appropriate amount of PBS or Saline for the desired concentration (2-10 mg/mL). Concentration of the non LSR labeled conjugates were measured by preparing 10, 20, and 50-fold dilutions in formulated buffer and reading the absorbance at 260 nm or 280 nm using a nanodrop. Once the linear range of dilution achieved, the absorbance was measured in triplicates and concentration was calculated using the average absorbance and ε₂₆₀ or ε₂₈₀. ε₂₈₀ for conjugates were calculated by the following formula: ε₂₈₀= 100356+ (n*3550); n= number of CPP. For LSR modified PMOs, the concentrations were measured at 566 nm with ε₅₆₆= 100000 LMol⁻¹Cm⁻¹ The diluted samples were analyzed by LCMS (Q-TOF) for the conjugate identity confirmation. Table below summarizes the calculated MW and experimental MWs. All experimental MWs reasonably matched the calculated average MW with expected ± 6 Da assay variation.

| **Name** | **Calculated MW** | **Experimental Average MW** | **Purity** |
|---|---|---|---|
| ENTR 0006 | 6038.19 | | |
| ENTR 0070 | 6151.27 | 6148.22 | >99 |
| ENTR 0059 | 7411.06 | 7409.78 | 99 |
| ENTR 0123 | 9409.43 | 9407.89 | 91 |
| ENTR 0121 | 7938.3 | 7933.15 | 97 |
| ENTR 0106 | 9893.56 | 9887.37 | 96 |
| ENTR 0108 | 10765.09 | 10763.74 | 90 |
| ENTR 0109 | 11259.8 | 11256.91 | 95 |
| ENTR 0110 | 11964.52 | 11961.21 | 99 |
| ENTR 0111 | 12120.7 | 12117.40 | 97 |
| ENTR 0112 | 12862.6 | 12858.85 | >99 |
| ENTR 0113 | 13919.86 | 13916.36 | 97 |
| ENTR 0047 | 9039.81 | 9034.81 | 99 |
| ENTR 0168 | 10444.78 | 10447.3 | 95 |
| ENTR 0203 | 9293.79 | 9295.2 | 92 |
| ENTR 0207 | 9532.05 | 9532.8 | >99 |

**Target cells.** HeLa cells were engineered to stably express EGFP-654 ("HeLa-654"). Untreated cells produced the improperly spliced mRNA retaining a portion of the intron sequence, resulting in non-functional missense EGFP protein expression.

**Fluorescence assays.** HeLa-654 cells were mock-treated (control), treated with the PMO alone at different concentrations (5 or 20 µM), or treated with the CPP12-PMO conjugate at different concentrations (0.6, 1.2, 1.8, 2.5, 5, or 10 µM) and incubated for 24 hours. The cells were then imaged with fluorescence microscopy and the fluorescence of the cells was quantified via FACS.

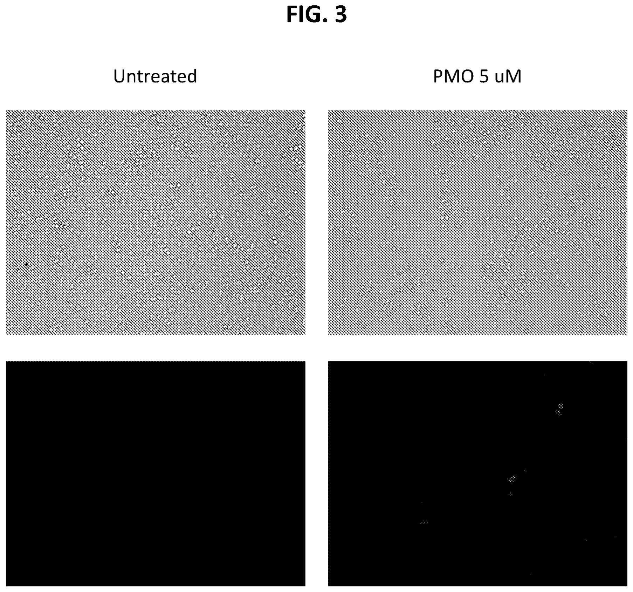

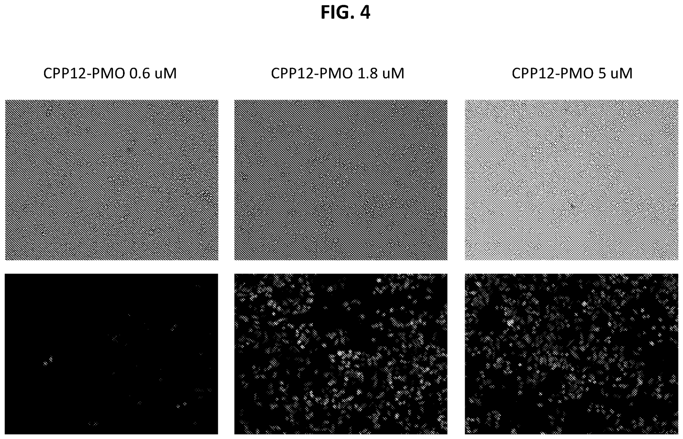

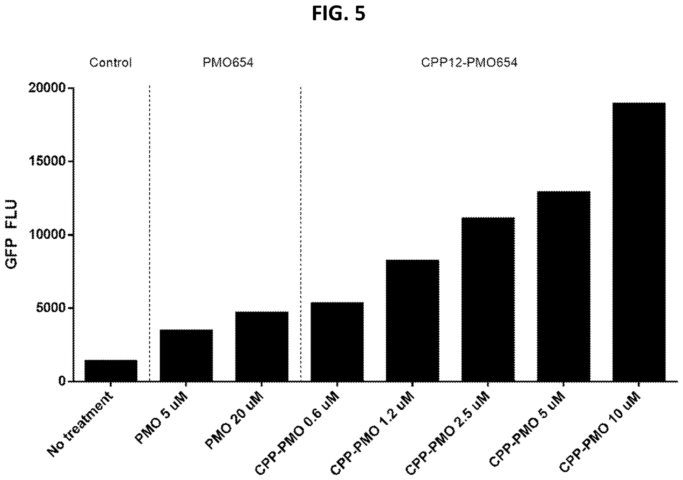

**Results.** **FIG. 3** and **FIG. 4** show the results of the fluorescence microscopy imaging of the mock-treated and treated cells. **FIG. 3** demonstrates that mock-treated cells did not produce functional fluorescent EGFP protein and that treatment with 5 µM PMO barely increased the functional fluorescent protein expression. **FIG. 4** shows that treatment with 0.6 µM CPP-PMO produced a small increase in fluorescence, and that treatment with 1.8 µM or 5 µM CPP-PMO produced a significant increase in fluorescence, indicating the proper splicing of the pre-mRNA sequence. **FIG. 5** shows the results of the FACS quantification of fluorescence intensity for the control and treated cells, demonstrating that PMO treatment and CPP-PMO treatment produced dose-dependent increases in functional fluorescent protein production. The results shown in **FIG. 5** also show the remarkable increase in efficacy of treatment with the PMO conjugated to CPP versus the PMO alone: treatment with 10 µM CPP-PMO produced nearly 4x the fluorescence reading at half the concentration, compared to 20 µM PMO treatment. These results demonstrate that CPP conjugated ACs are effective in binding to a target pre-mRNA sequence and restoring functional protein production.

Structure activity relationship of CPP for PMO654 delivery. **FIG. 34** shows the cellular uptake results of 0.7 µM or 2 µM rhodamine (LSR) labeled PMO and CPP-PMO conjugates quantified by FACS using HeLa-654 cells treated for 48 hours. Untreated and PMO (ENTR-0059) treated cells did not show lissamine-rhodamine (LSR) red fluorescent signal. Conjugation of mono-valent CPP12 (ENTR-0123) showed limited increase of the LSR fluorescence, while conjugation of bivalent (ENTR-0108, ENTR-0109) and trivalent (ENTR-0112, ENTR-0113) CPP12 modified PMOs significantly increase the cellular uptake in a dose dependent manner as indicated by the LSR fluorescence. **FIG. 35** shows the cellular activity as quantified by GFP correction by FACS analysis treated with 0.7 µM or 2 µM PMO and CPP-PMO conjugates using HeLa-654 cells for 48 hours. Untreated and PMO (ENTR-0059 and ENTR-0070) treated cells show minimal GFP correction as expected. Monovalent CPP-PMO treated cells showed xx fold increase of GFP signal increase over PMO itself (comparing ENTR-0123 vs ENTR-0059). Additionally, the bivalent (ENTR-0108, ENTR-0109) and trivalent (ENTR-0112, ENTR-0113) conjugates significantly increase the GFP correction. Surprisingly, conjugation of PMO with monovalent CPP12 but with additional Nuclear Localization Sequence (PKKKRKV (SEQ ID NO: 131)) NLS sequence outperformed all the bivalent and trivalent CPP12 conjugation, by at least 3-fold (ENTR-0047). **FIG. 36** shows the bright field microscopy and fluorescence microscopy images of HeLa-654 cells treated with vehicle PBS control (untreated), bidentate CPP-PMO (ENTR-0108), tridentate CPP-PMO (ENTR-0111), and CPP-NLS-PMO construct (ENTR-0047), which is consistent with FACS analysis shown in **FIG. 35****.** To further explore different conjugation chemistry for the CPP-NLS constructs for PMO conjugation, EGFP654 cells were also treated with CPP-NLS-PMO conjugates (ENTR-0168) before analyzed by FACS and microscopic analysis. **FIG. 37** shows that CPP-NLS-PMO-LSR (ENTR-0168) increased the GFP fluorescence by 10-fold and LSR fluorescence by 15-fold relative to monovalent CPP-PMO-LSR (ENTR-0123).

**Comparison of cellular uptake with transfection reagent.** Endo-Porter (GENETOOLS, LLC) is a transfection reagent designed to deliver neutrally charged PMO oligos into cells and have been commonly used in the literature. The cellular uptake efficiency and cellular activity of CPP-NLS-mediated delivery was also compared to Endo-Porter mediated uptake as analyzed by FACS **FIG. 38****. and** **FIG. 39****.** ENTR-0168 exhibit higher GFP and LSR fluorescence than the commercially transfection reagent Endo-Porter transfected PMO (ENTR-0059 + endo), indicating that CPP-NLS conjugation has higher functional cellular uptake capability than Endo-Porter. **FIG. 39** shows the bright field microscopic and fluorescence microscopic images of the treated HeLa-654 cells, which confirms the findings from FACS analysis shown in **FIG. 38****.** To ensure that LSR fluorescent dye label did not modulate the cellular uptake, two additional CPP-NLS-PMO for EGFP654 were also prepared: ENTR-0203 and ENTR-0207. ENTR-0207 contained a cleavable linker, whereas ENTR-0203 contained a non-cleavable linker.

The structure of ENTR-0203 is below:

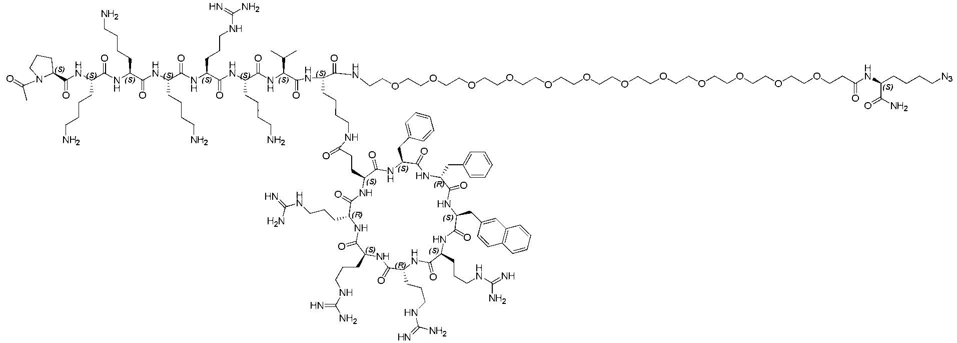

The structure of ENTR-0207 is below:

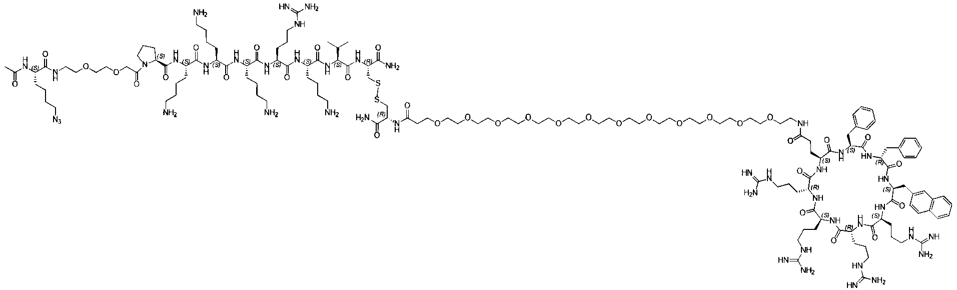

The table below provides additional information about the constructs evaluated:

| **Identifier** | **Description** | **Sequence** |
|---|---|---|
| ENTR-0047 | PMO654-NLS-ss-CPP12 | 5'-GCT ATT ACC TTA ACC CAG-3-NLS-ss-CPP12 (all PMO monomers) (SEQ ID NO: 233) |
| ENTR-0168 | NLS-CPP12-PMO654-LSR | (Ac-NLS-Lys(CPP12)-PEG12-K(N3))-5'-GCT ATT ACC TTA ACC CAG-3'-Lissamine Rho (all PMO monomers) (SEQ ID NO: 234) |
| ENTR-0203 | PMO654-CPP12-NLS | 5'-GCT ATT ACC TTA ACC CAG-3'-click-K-PEG12-Lys(CPP12)-NLS-Ac (all PMO monomers) (SEQ ID NO: 235) |
| ENTR-0207 | PMO654-NLS-CPP12 | 5'-GCT ATT ACC TTA ACC CAG-3'-click-Lys(N3)-miniPEG-NLS-ss-PEG12-CPP12 (all PMO monomers) (SEQ ID NO: 236) |

Both constructs showed dose dependent cellular activity (0 - 10 µM) as analyzed by the corrected GFP fluorescence (**FIG. 24, FIG. 25****,** **FIG. 26****,** **FIG. 27**).

### Example 2. Use of cell-penetrating peptides conjugated to oligonucleotides for splicing correction in mice.

**Mice.** Mice used in this study were genetically engineered to express the missense EGFP-654 gene.

**Study design.** Mice expression EGFP-654 were injected with PBS, CPP-PMO (also called "CPP12-PMO"), or PMO without CPP, through IV/IP/IM administrations at different doses as indicated (IV=10 mpk, IP=30 mpk, IM=1/3/10 mpk). (IV: intravenous; IP: intraperitoneal; IM: intramuscular; mpk: mg of compound/kg of body weight) Each mouse received one injection per day for 4 days. Mice were sacrificed and tissue samples were collected either 24 or 96 hours after last injection. Total RNA were extracted from tissue samples and analyzed by RT-PCR to visualize the efficiency of splicing correction.

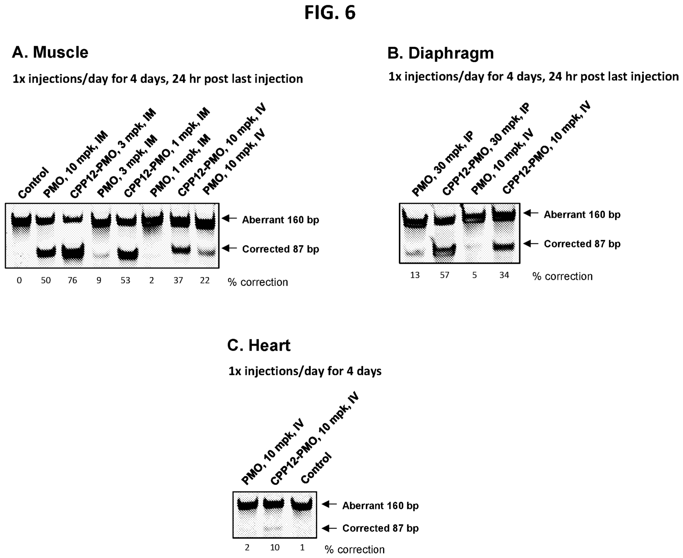

**Detection of splicing correction by RT-PCR.** Untreated cells in control mice produced improperly spliced EGFP-654 mRNA retaining a portion of the intron sequence, resulting in non-functional missense EGFP mRNA expression. As shown in **FIG. 6****,** RT-PCR using forward primer 5'-CGTAAACGGCCACAAGTTCAGCG-3' (SEQ ID NO: 150) and reverse primer 5'-GTGGTGCAGATGAACTTCAGGGTC3' (SEQ ID NO: 151) of the tissue samples without splicing correction resulted predominantly in an "aberrant", 160 bp gene fragment. On the other hand, splicing corrected EGFP-654 mRNAs displayed a "corrected", 87 bp fragment by RT-PCR.

**Analysis of splicing correction.** The degree (percentage) of splicing correction detected by RT-PCR was calculated using the following equation:
% correction = (intensity of 87 bp fragment band) / (intensity of 160 bp fragment band + intensity of 87 bp fragment band). As shown in **FIGS. 6A-C**, PMO treatment increased the percentage of splicing corrected EGFP-654 in the tissue sample. Remarkably, as shown in Table B, tissue samples from CPP-PMO treated mice displayed a higher percentage of splicing correction as compared to samples from PMO treated mice, demonstrating that CPP conjugated ACs are more efficient at cell penetration and mediating splicing correction in mice.

**Table B. In vivo splice modulation potency**

| **Tissue** | **IM Muscle** | **IV diaphragm** | **IV heart** |
|---|---|---|---|
| *PMO* | 2% | 5% | 1% |
| *EEV12-PMO* | 53% | 34% | 10% |
| *Fold Difference* | 26.5x | 6.8x | 10x |

### Example 3A. Use of cell-penetrating peptides conjugated to oligonucleotides for splicing correction of dystrophin in wild type mouse model

The compounds of Tables B1, B2, B3, and B4 are evaluated in Examples 3A and 3B. Exemplary experiments are described below. The same techniques are used to evaluate any compounds of Table B1, Table B2, Table B3, or Table B4.

**Table B1. Compounds that target DMD**

| **Oligo#** | **Design** | **Target** |
|---|---|---|
| ENTR-0013 | 5'-GGC CAA ACC TCG GCT TAC CTG AAA T-3' (all PMO monomers) (SEQ ID NO: 237) | Murine DMD exon 23 |
| ENTR-0017 | 5'-GGC CAA ACC TCG GCT TAC CTG AAA T-3'-C3-NH2 (all PMO monomers) (SEQ ID NO: 238) | Murine DMD exon 23 |
| ENTR-0066 | Cyclooctyne-5'- GGC CAA ACC TCG GCT TAC CTG AAA T -3' (all PMO monomers) (SEQ ID NO: 239) | Murine DMD exon 23 |
| ENTR-0068 | NH2-5'-C3- GGCCAAACCTCGGCTTACCTGAAAT -3'-C3-NH2 (all PMO monomers) (SEQ ID NO: 240) | Murine DMD exon 23 |
| ENTR-0149 | 5'- GGC CAA ACC TCG GCT TAC CTG AAA T -3'-C4-cyclooctyne (all PMO monomers) (SEQ ID NO: 241) | Murine DMD exon 23 |

**Table B2. Compounds that target DMD**

| **Oligo#** | **Design** | **Target** |
|---|---|---|
| ENTR-0088 | (CPP12-PEG12-ValCitPABC)-5'- GGC CAA ACC TCG GCT TAC CTG AAA T -3' (all PMO monomers) (SEQ ID NO: 242) | Murine DMD exon 23 |
| ENTR-0093 | CPP12-PEG12-NH-5'-GGCCAAACCTCGGCTTACCTGAAAT -3'-NH-PEG12-CPP12 (all PMO monomers) (SEQ ID NO: 243) | Murine DMD exon 23 |
| ENTR-0098 | 5'-GGC CAA ACC TCG GCT TAC CTG AAA T-3'-C3-NH-PEG12-CPP12 (all PMO monomers) (SEQ ID NO: 244) | Murine DMD exon 23 |
| ENTR-0099 | 5'-CPP12-PEG12-Cys-ss-Cys-Lys(N3)-cyclooctyne-5'- GGC CAA ACC TCG GCT TAC CTG AAA T-3' (all PMO monomers) (SEQ ID NO: 245) | Murine DMD exon 23 |
| ENTR-0100 | CPP12-PEG12-Lys(N3)-cyclooctyne-5'- GGC CAA ACC TCG GCT TAC CTG AAA T -3' (all PMO monomers) (SEQ ID NO: 246) | Murine DMD exon 23 |
| ENTR-0115 | (CPP12-PEG12-Prodisulfide)-5'- GGC CAA ACC TCG GCT TAC CTG AAA T -3' (all PMO monomers) (SEQ ID NO: 247) | Murine DMD exon 23 |
| ENTR-0120 | (CPP12-PEG24)-5'- GGC CAA ACC TCG GCT TAC CTG AAA T -3' (all PMO monomers) (SEQ ID NO: 248) | Murine DMD exon 23 |
| ENTR-0161 | 5'- GGC CAA ACC TCG GCT TAC CTG AAA T - 3'-C3NH-C6-CPP12 (all PMO monomers) (SEQ ID NO: 249) | Murine DMD exon 23 |
| ENTR-0089 | (CPP12-(bidentate-no linker)-PEG12)-5'- GGC CAA ACC TCG GCT TAC CTG AAA T -3' (all PMO monomers) (SEQ ID NO: 250) | Murine DMD exon 23 |
| ENTR-0090 | (CPP12-(bidentate-PEG12)-PEG12)-5'- GGC CAA ACC TCG GCT TAC CTG AAA T -3' (all PMO monomers) (SEQ ID NO: 251) | Murine DMD exon 23 |
| ENTR-0092 | 5'-GGC CAA ACC TCG GCT TAC CTG AAA T-3'-C3-NH-CPP1212; Amide chemistry (all PMO monomers) (SEQ ID NO: 252) | Murine DMD exon 23 |

**Table B3. Compounds that target DMD**

| **Oligo#** | **Design** | **Peptide Fusion** |
|---|---|---|
| ENTR-0119 | (T9-PEG12-CPP12-PEG12)-5'- GGC CAA ACC TCG GCT TAC CTG AAA T -3' (all PMO monomers) (SEQ ID NO: 253) | T9: SKTFNTHPQSTP (SEQ ID NO: 258) |
| ENTR-0163 | Ac-MSP-PEG12-Lys(CPP12-PEG12)-K(N3)+5'- GGC CAA ACC TCG GCT TAC CTG AAA T -3' (SEQ ID NO: 254) | MSP: ASSLNIA (SEQ ID NO: 259) |
| ENTR-0164 | 5'- GGC CAA ACC TCG GCT TAC CTG AAA T -3'-C3NH-PEG4-BCN+Lys(N3)-miniPEG-NLS-ss-bA-bA-CPP12 (all PMO monomers) (SEQ ID NO: 255) | NLS: PKKKRKV (SEQ ID NO: 131) |
| ENTR-0165 | 5'- GGC CAA ACC TCG GCT TAC CTG AAA T -3'-C3NH-PEG4-BCN+Ac-NLS-Lys(CPP12)-PEG12-K(N3) (all PMO monomers) (SEQ ID NO: 256) | NLS: PKKKRKV (SEQ ID NO: 131) |
| ENTR-0201 | 5'- GGC CAA ACC TCG GCT TAC CTG AAA T -3'-click-K-PEG12-Lys(CPP12)-NLS-Ac (all PMO monomers) (SEQ ID NO: 257) | NLS: PKKKRKV (SEQ ID NO: 131) |

The compounds of Tables B1-B3 are evaluated in Examples 3A, 3B, 3C, and 3D. Exemplary experiments are described below.

**Preparation and design of CPP-PMO targeting murine DMD exon 23.** Design of monovalent CPP-PMO (ENTR-0092, ENTR-0098, ENTR-0100, ENTR-0120 and ENTR-0161), monovalent with cathepsin B cleavable linker (ENTR-0088), monovalent with cytosolic reducible linker by glutathione or GILT (ENTR-0099, ENTR-0115) bivalent CPP-PMO (ENTR-0089, ENTR-0090, ENTR-0093 and ENTR-0110), mono CPP with Muscle targeting moieties (ENTR-0119 and ENTR-0163) and mono CPP-NLS-PMO (ENTR-0164, ENTR-0165, ENTR-0201) are shown in Table B2 and B3. The following compounds were synthesized via 3', 5', or both covalent conjugation of primary amine modified PMOs: ENTR-0093, ENTR-0098, 0161, ENTR-0092. Briefly, a solution of desired peptide-TFP ester in DMF (6 eq, 5 mM) was added to a solution of PMO-3'-primary amine (1 equivalent, 2 mM) in PBS-10X. Reaction proceed to completion in 4-8 hours at room temperature as confirmed by LCMS (Q-TOF). For 3' or 5' conjugation via click reaction (ENTR-0088, ENTR-0099, ENTR-0100, ENTR-0120, ENTR-0089, ENTR-0090, ENTR-0119, ENTR-0163, ENTR-0164, ENTR-0165, ENTR-0201), a solution of peptide-azide in nuclease-free water (1 mM) was added to the PMO-3'-cyclooctyne or cyclooctyne-5'-PMO solids. The mixture was vortexed to dissolve the peptide-PMO conjugate, centrifuged to settle the solution, and incubated at room temperature for 8-12 hours for completion as confirmed by LCMS (Q-TOF). For purifications, crude mixtures were diluted with DMSO, loaded onto a C18 reverse-phase column (150mm* 21.2 mm), flow rate of 20 mL/min and purified by an appropriate gradient using water with 0.05% TFA and acetonitrile as solvents. Desired fractions were pooled, pH of the solution was adjusted to 5-6 by 1M NaOH and the solution underwent the lyophilization process, affording white lyophilized powder. For *in vitro* and *in vivo* formulations, the conjugates were reconstituted in appropriate amount of PBS or Saline for the desired concentration (2-10 mg/mL). Concentration of the non LSR labeled conjugates were measured by preparing 10, 20, and 50-fold dilutions in formulated buffer and reading the absorbance at 260 nm or 280 nm using a nanodrop. Once the linear range of dilution achieved, the absorbance was measured in triplicates and concentration was calculated using the average absorbance and ε₂₆₀ or ε₂₈₀. ε₂₈₀ for conjugates were calculated by the following formula: ε₂₈₀= 138993+ (n*3550); n= number of CPP. The diluted samples were analyzed by LCMS (Q-TOF) for the conjugate identity confirmation. Table below summarizes the calculated MW and experimental MWs. All experimental MWs reasonably matched the calculated average MW with expected ± 6 Da assay variation.

| **Name** | **Calculated MW** | **Experimental Average MW** | **Purity** |
|---|---|---|---|
| ENTR-0013 | 8413.1 | 8410.02 | 99 |
| ENTR-0017 | 8487 | 8484 | 95 |
| ENTR-0066 | 9001.7 | 9003.8 | 95 |
| ENTR-0068 | 8751.07 | 8748.02 | 85 |
| ENTR-0149 | 8635.07 | 8635.8 | 82 |
| ENTR-0088 | 11404.89 | 11403.62 | 93 |
| ENTR-0093 | 12404.9 | 12400.2 | >99 |
| ENTR-0098 | 10311.46 | 10308.04 | >99 |
| ENTR-0099 | 11263.4 | 11260.43 | 90 |
| ENTR-0100 | 11000.1 | 10996.43 | 92 |
| ENTR-0115 | 11261.77 | 11259.53 | 98 |
| ENTR-0120 | 11599.17 | 11596.89 | 99 |
| ENTR-0161 | 9827 | 9825.8 | 98 |
| ENTR-0089 | 12355.7 | 12351.11 | 96 |
| ENTR-0090 | 13555.1 | 13550.96 | 97 |
| ENTR-0092 | 12266.79 | 12262.14 | 94 |
| ENTR-0119 | 13095.87 | 13092.55 | 96 |
| ENTR-0163 | 12426.1 | 12428.8 | 98 |
| ENTR-0164 | 11838.19 | 11836.3 | 97 |
| ENTR-0165 | 11944.31 | 11942.3 | 99 |
| ENTR-0201 | 11669.5 | 11669.5 | >99 |

**Mice.** This study used wild type mice with normal level of dystrophin expression and without DMD mutations or symptoms.

**Study design.** The composition and method described in Example 1 and 2 (ENTR-0013, TABLE B1 and ENTR-0098, Table B2) were applied to wild mice to evaluate the ability of the claimed compositions to skip exon 23 and thus treat DMD. The AC used in this study had the following sequence 5'-GGCCAAACCTCGGCTTACCTGAAAT-3' (SEQ ID NO: 260), which targeted exon 23. This sequence is referred to as PMO in this example. PBS, PMO conjugated to a CPP ("CPP-PMO," also called "CPP12-PMO^{DMD}" or "PMO^{DMD}-CPP12"), or PMO without CPP (also called PMO^{DMD}) was administered via intramuscular (IM) or intravenous (IV) to MDX mice at the following doses: 1 mpk, 3 mpk, 10 mpk, 30 mpk; (mpk: mg of compound / kg of body weight). The CPP used was cCPP12, which has an amino acid sequence of Ff**Φ**RrRr. A schematic of preparation of PMO^{DMD} and PMO^{DMD}-CPP12 is shown in **FIG. 10A.** Total RNA were extracted from tissue samples and analyzed by RT-PCR to visualize the efficiency of splicing correction.

**Detection of splicing correction by RT-PCR.** WT mice express normal level of full-length dystrophin mRNA in muscles. The delivery of PMO can alter the splicing and result in a truncated dystrophin mRNA after exon 23 skipping. The detection of splicing correction process is measured by RT-PCR where extracted RNAs from tissues are first reverse-transcribed into cDNA and are further analyzed by nested PCR using two primer sets: forward primer 5'-CAGAATTCTGCCAATTGCTGAG-3' (SEQ ID NO: 261) and reverse primer 5'-TTCTTCAGCTTGTGTCATCC-3' (SEQ ID NO: 262) for the first round PCR (outer primer set) and forward primer 5'- CCCAGTCTACCACCCTATCAGAGC-3' (SEQ ID NO: 263) and reverse primer 5'- CCTGCCTTTAAGGCTTCCTT-3' (SEQ ID NO: 264) (inner primer set) for the second round PCR. The RT-PCR readout of tissues without splicing correction result in a 901 bp gene fragment and a new 689 bp gene fragment show up after splicing correction. The degree (percentage) of splicing correction detected by RT-PCR was calculated using the following equation: % correction = (intensity of 689 bp fragment band) / (intensity of 901 bp fragment band + intensity of 689 bp fragment band).

**In vivo activity in wild type mice.** As shown in **FIG. 10B,** 24 hr after twice daily IM administration, WT mice (CD1) treated with CPP12-PMO^{DMD} (ENTR-0098) produced significant amount of dystrophin mRNA lacking exon 23, whereas mice treated with PMO (ENTR-0013) alone produced only dystrophin mRNA containing exon 23 (similar to PBS treated control). WT mice (CD1) were also administrated intravenously with equal mole of material (8 mpk PMO, ENTR-0013, or 10 mpk CPP12-PMO, ENTR-0098). Five days post injection, the dystrophin mRNA products in quadriceps, transverse abdominis, diaphragm, and heart are analyzed by RT-PCR and shown in **FIG. 11****.** In contrast to the low exon 23 skipping efficiency in PMO^{DMD} treated mice tissues, the administration of CPP12-PMO^{DMD} resulted in significantly increased amount of dystrophin mRNA with skipped exon 23 in quadriceps, transverse abdominis, diaphragm, and heart. To further confirm the findings, additional wild type mice (C57BL/10, n = 5 per group) were administered intravenously with (equal mole) of 24 mpk PMO^{DMD} (ENTR-0013) and 30 mpk CPP12-PMO^{DMD} (ENTR-0098). One-week post injection, the dystrophin mRNA products in quadriceps, transverse abdominis, diaphragm, and heart were analyzed by RT-PCR and the percentage of exon 23 skipping are shown in **FIG. 11****.** In contrast to the low exon 23 skipping efficiency in PMO^{DMD} treated mice tissues, the administration of CPP12-PMO^{DMD} resulted in significantly increased amount of dystrophin mRNA with skipped exon 23 in quadriceps, transverse abdominis, diaphragm, and heart, illustrating the effective delivery of oligonucleotide cargos by cyclic CPPs.

### Example 3B. Use of cell-penetrating peptides conjugated to oligonucleotides for splicing correction of dystrophin in mouse model of DMD.

Mice. This study used C57BL/10ScSn-Dmdmdx/J (MDX) mice, which contain a C to T mutation resulting in a termination codon at position 2983 within exon 23 of the dystrophin muscular dystrophy gene (*Dmd*) on the X chromosome. Mice expressing this mutant allele produce a truncated dystrophin protein, and are thus a model of Duchenne's muscular dystrophy ("DMD").

**Study design.** The composition and method described in Example 3A and listed in Tables B2 and B3 were applied to MDX mice to evaluate the ability of the claimed compositions to skip exon 23 and thus treat DMD. The AC used in this study had the following sequence 5'-GGCCAAACCTCGGCTTACCTGAAAT-3' (SEQ ID NO: 260), which targeted exon 23. This sequence is referred to as PMO in this example. PBS, PMO conjugated to a CPP ("CPP-PMO," also called "CPP12-PMO^{DMD}" or "PMO^{DMD}-CPP12"), PMO without CPP (also called PMO^{DMD}), PMO with various CPP12 through different linkers or multivalence (TABLE 3), PMO with CPP12 as well as various peptide fusions (TABLE 4) was administered via intravenous (IV) to MDX mice at the following doses: 10 mpk, 20 mpk, 30 mpk; (mpk: mg of compound / kg of body weight). The CPP used was cCPP12, which has an amino acid sequence of Ff**Φ**RrRr. A schematic of preparation of PMO^{DMD} and PMO^{DMD}-CPP12 is shown in **FIG. 10A.** The structures of the compounds are provided throughout this disclosure. Total RNA was extracted from tissue samples and analyzed by RT-PCR to visualize the efficiency of splicing correction. Dystrophin protein level was analyzed by Western blot to evaluate the expression of dystrophin upon treatment.

**Detection of dystrophin expression by Western Blot.** Lysis buffer (9% SDS, 4% glycerol, 5 mM Tris, and 5% beta-mercaptoethanol, along with HALT protease inhibitors) was added to minced mouse tissue from either mouse heart, transverse abdominis, quadriceps, or diaphragm. Metal beads were used in conjunction with a Qiagen Tissuelyser to mechanically homogenize the tissue. Lysate was cleared by centrifugation, and the supernatant was subjected either to SDS-PAGE using 3-8% Tris Acetate gels followed by transfer to nitrocellulose membranes and western blotting followed by fluorescent imaging using the LICOR system or to the Jess Simple Western system using the 66-440 kDa capillary matrices. Dystrophin was detected using the anti-dystrophin antibodies from Abcam (Ab52777 or Ab154168); alpha-actinin was detected using anti-alpha-actinin antibodies from R&D Systems (MAB8279) or Abcam (ab68167). Traditional western blot bands were quantified using LICOR software. Jess Simple Western peaks were fit and the area under the peaks was calculated using the Simple Western software. Each run included a standard curve using wildtype mouse lysate diluted with different amounts of mdx mouse lysate from the respective tissue. The dystrophin detected in each sample was normalized to alpha-actinin as a loading control, and a linear regression was performed for the standard curve, which was used to determine the amount of dystrophin in each sample as a percentage of wildtype dystrophin levels.

**Dose dependent activity of CPP12-PMO in MDX mice.** MDX mice were administered with 10 mpk or 30 mpk of CPP 12-PMO^{DMD} (ENTR-0098). One week post injection, the dystrophin mRNA products in various muscle groups (the quadriceps, transverse abdominis, diaphragm, and heart) were analyzed by RT-PCR and the quantification of exon 23 correction are shown in **FIGS. 42A-D**. **FIGS. 42A-D** show that compared to vehicle treated MDX mice where there are only minimal dystrophin mRNA product, the administration of CPP12-PMO^{DMD} resulted in dose-dependent increased amount of dystrophin mRNA in the quadriceps (**FIG. 42A**), transverse abdominis (**FIG. 42B**), diaphragm (**FIG. 42C**), and heart (**FIG. 42D**).

**Duration of effects of CPP12-PMO in MDX mice.** To evaluate the duration of effects of ENTR-0998, MDX mice were injected intravenously with 30 mpk CPP12-PMO (ENTR-0098) and various muscle groups were harvested 1 week (n=2), 2 weeks (n=4), or 4 weeks (n=4) post injection. **FIGS. 43A-D** show the effect of exon skipping and dystrophin mRNA correction is significant and consistent up to 4 weeks after a single IV injection in the quadriceps (**FIG. 43A**), transverse abdominis (**FIG. 43B**), diaphragm (**FIG. 43C**), and heart (**FIG. 43D**).

**Correction of dystrophin protein expression with CPP12-PMO in MDX mice.** MDX mice were injected with equal mole amount of PMO (8 mpk, ENTR-0013, n=3) or CPP12-PMO (10 mpk, ENTR-0098, n=2) intravenously, 1 week post injection, various muscle groups (quadriceps, transverse abdominis, diaphragm, and heart) were collected and analyzed by western blotting by blotting dystrophin and alpha-actinin (loading control) as shown in **FIG. 44****.** Notably, mice administered with CPP12-PMO^{DMD} exhibited enhanced dystrophin expression in a CPP-dependent manner. The degree of the dystrophin correction is also semi-quantified by image analysis using actinin as the loading control. As shown in **FIGS. 45A-D**, compared to that of PMO^{DMD}, CPP12-PMO^{DMD} administration increased the dystrophin level in quadriceps (2.5 fold, **FIG. 45A**), transverse abdominis (4.8 fold, F**IG. 45B**), diaphragm (15.7 fold, **FIG. 45C**), and heart (>300 fold, **FIG. 45D**). Also by Western Blot analysis, **FIGS. 46A-B** show the sustainable dystrophin levels correction in heart muscles of MDX mice two weeks (**FIG. 46A**) and four weeks (**FIG. 46B**) after a single IV injection of 30 mpk CPP12-PMO^{DMD} or 24 mpk (equal mole) PMO^{DMD}. The correction of dystrophin is consistent and as expected with the RT-PCR analysis as shown in **FIGS. 42A-D** and **FIGS. 43A-D**.

**Structure activity relationship of CPP for PMO delivery in MDX mice.** Table B4 shows the exon skipping activity one-week or 2-week of various muscle groups (Quadriceps, Heart, and Diaphragm) in MDX mice after a single IV injection of 20 mpk or 30 mpk of various CPP12-connected PMO targeting the exon 23.

**Table B4. Exon Skipping Activity in MDX Mice**

| **Animals** | **Injection** | **Oligo ID** | **Dosage (mpk)** | **Sac.** | **Exon 23 Skipping % Quad** | **Exon 23 Skipping % Heart** | **Exon 23 Skipping % Diaphragm** |
|---|---|---|---|---|---|---|---|
| MDX | IV | ENTR-0098 | 30 | 2 w | 24.4 ± 7.4 | 5.0 ± 3.1 | 17.5 ± 5.9 |
| MDX | IV | ENTR-0100 | 30 | 2 w | 22.9 ± 16.5 | <1% | 11.6 ± 15.5 |
| MDX | IV | ENTR-0120 | 30 | 2 w | 0.4 ± 0.4 | <1% | 0.4 ± 0.2 |
| MDX | IV | ENTR-0088 | 30 | 2 w | 7.2 ± 3.3 | <1% | 0.0 ± 0.0 |
| MDX | IV | ENTR-0099 | 30 | 2 w | 25.6 ± 1.2 | <1% | 20.2 ± 0.0 |
| MDX | IV | ENTR-0115 | 30 | 2 w | 3.2 ± 3.4 | <1% | <1% |
| MDX | IV | ENTR-0161 | 20 | 1 w | 25.7 ± 5.1 | 1.1 ± 2.2 | 16.2 ± 10.7 |
| MDX | IV | ENTR-0098 | 10 | 1 w | 13.4 ± 3.7 | 2.2 ± 0.3 | 4.3 ± 0.8 |
| MDX | IV | ENTR-0089 | 10 | 1 w | <1% | <1% | <1% |
| MDX | IV | ENTR-0090 | 10 | 1 w | <1% | <1% | <1% |
| MDX | IV | ENTR-0092 | 10 | 1 w | <1% | <1% | <10% |
| MDX | IV | ENTR-0093 | 10 | 1 w | <1% | <1% | <1% |
| MDX | IV | ENTR-0119 | 30 | 2 w | 15.6 ± 8.3 | 5.0 ± 7.1 | 3.9 ± 4.7 |
| MDX | IV | ENTR-0163 | 20 | 1 w | 7.9 ± 3.7 | 0.0 ± 0.0 | 9.4 ± 1.8 |
| MDX | IV | ENTR-0164 | 20 | 1 w | 59.5 ± 2.2 | 29.1 ± 0.8 | 61.0 + 13.7 |
| MDX | IV | ENTR-0165 | 20 | 1 w | 38.5± 2.5 | 30.5 ± 17.0 | 30.2 ± 2.8 |
| MDX | IV | ENTR-0201 | 20 | 1 w | 73.5 ± 8.4 | 60.5 ± 17.2 | 79.0 ± 6.8 |

The structures of examples of compounds comprising antisense oligonucleotides (underlined) (SEQ ID NO: 218) that target murine DMD and CPPs are shown below.

### ENTR-0119:

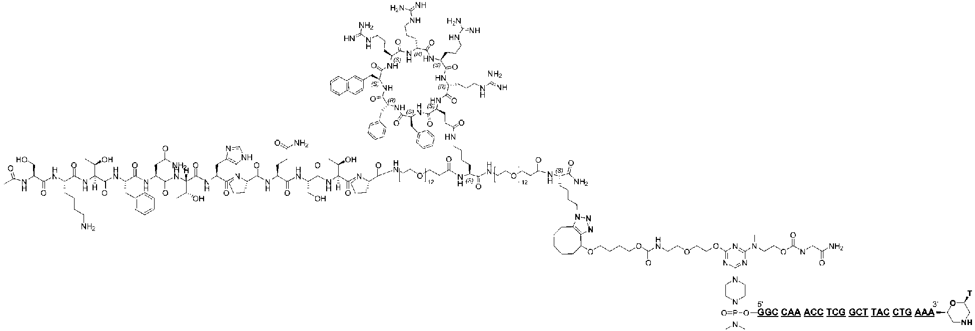

### ENTR-0163:

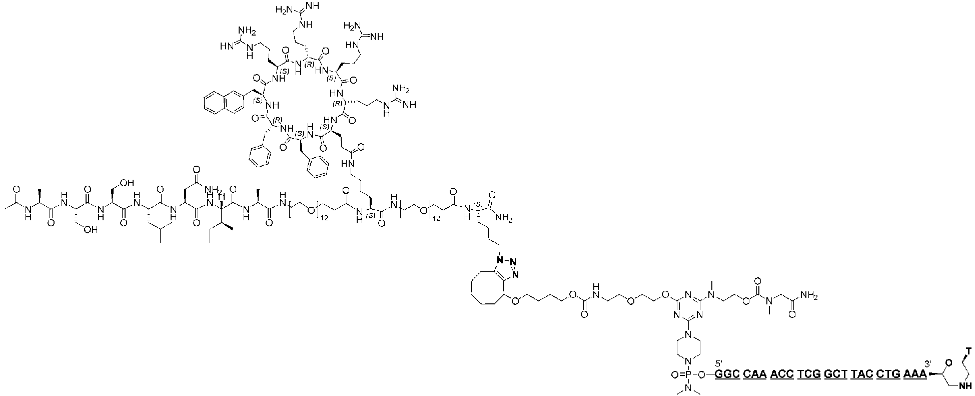

### ENTR-0164:

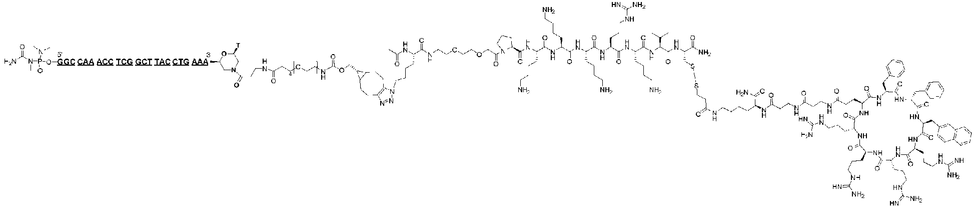

### ENTR-0165:

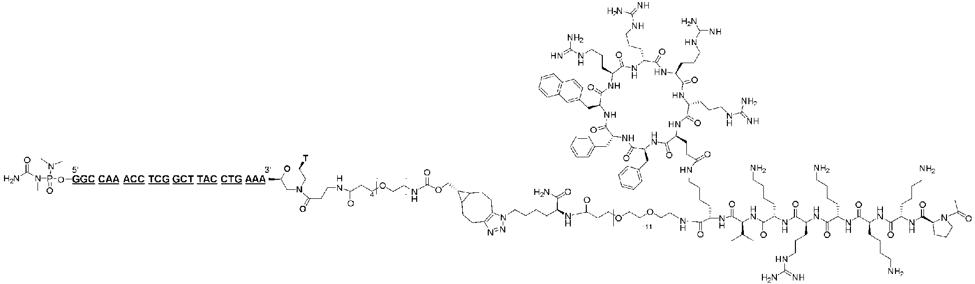

### ENTR-0201:

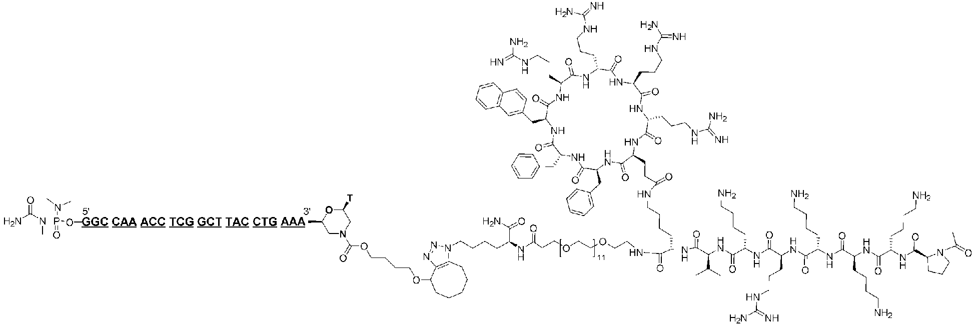

The activity of ENTR-098 is extracted from **FIGS. 43A-D**, and 2 week post single 30 mpk IV injection in MDX mice yielded robust exon skipping in quadricep (Quad) at 24.4 ± 7.4%, in heart at 5.0 ± 3.1%, as well as in diaphragm at 17.5 ± 5.9%. Conjugation of mono-valent CPP12 to the 5' of PMO (e.g. ENTR-0100) vs the 3' of PMO (e.g. ENTR-0098) showed comparable exon skipping activity in Quad (22.9 ± 16.5) and Diaphragm (11.6 ± 15.5) but less in the heart (<1%) compared to that from ENTR-0098. The linker variants of ENTR-0098 also have significantly impact to the activity in MDX mice. For one example, further elongation of the linker between CPP12 and PMO from PEG12 to PEG24 (e.g. ENTR-0120) showed significantly reduced activity. For another example, replacing the amide bond linker as in ENTR-0100 to a cathepsin-B-cleavable linker (Val-CitPABC) as in ENTR-0088 significantly reduced the activity (comparing ENTR-0100 vs ENTR-0088). For another example, replacing the amide bond linker as in ENTR-0100 to a disulfide bond linker as in ENTR-0099 does not have any significant impact of the activity (comparing ENTR-0100 vs ENTR-0099). Interestingly, connecting the CPP and PMO with prodisulfide bond has a reduced activity as shown in ENTR-0115. For another example, replacing the PEG12 linker as in ENTR-0088 to all carbon chain (C6 linker) as in ENTR-161 does not significantly modify the activity. To our surprise, connecting bivalent CPP12 to one PMO cargo molecule yielded significantly reduced activity (e.g. bivalent examples as in ENTR-0089, ENTR-0090, ENTR-0092 and ENTR-0093). Without being bound by theory, this may be due to limited biodistribution of these constructs to the target muscular tissues.

### Example 3C. Use of cell-penetrating peptides conjugated to oligonucleotides for splicing correction of dystrophin in mouse model of DMD.

The study protocols of the previous examples were utilized to evaluate an additional AC that targets exon 23. The additional AC used had the following sequence 5'-GCTATTACCTTAACCCA-3' (PMO modifications) (SEQ ID NO: 152), which targeted exon 23. This sequence is referred to as PMO in this example. PBS, PMO conjugated to a CPP ("CPP-PMO," also called "EEV12-PMO^{DMD}" or "PMO^{DMD}-EEV12"), or PMO without CPP (also called PMO^{DMD}) was administered via intramuscular (IM) or intravenous (IV) to MDX mice at the following doses: 1 mpk, 3 mpk, 10 mpk, 30 mpk; mpk: mg of compound / kg of body weight. The CPP used was cCPP12, which has an amino acid sequence of Ff**Φ**RrRr (SEQ ID NO: 117). A schematic of preparation of PMO^{DMD} and PMO^{DMD}-EEV12 is shown in **FIG. 9****.** Total RNA were extracted from tissue samples and analyzed by RT-PCR to visualize the efficiency of splicing correction.

**Detection of splicing correction by RT-PCR and Western Blot.** As shown in **FIG. 10****,** MDX mice treated with EEV12-PMO^{DMD} produced dystrophin lacking exon 23, whereas MDX mice treated with PMO alone produced only dystrophin containing exon 23 (similar to untreated control). MDX mice were administered 10 mpk I.V. PMO^{DMD} and EEV12-PMO^{DMD}. The dystrophin products in various muscle groups (e.g., the quadriceps, transverse abdominus, diaphragm, and heart) are shown in **FIG. 11. FIG. 11** shows that in contrast to delivery of PMO^{DMD} alone, the delivery of EEV12-PMO^{DMD} to MDX mice resulted in corrected dystrophin splicing (e.g., dystrophin with excised exon 23) in the quadriceps, transverse abdominus, diaphragm, and heart. **FIGS. 12A-D** show the effect of the following IV dosage regimens on exon skipping efficacy: 10 mpk twice per week, 10 mpk once per week, 10 mpk once per two week, and 30 mpk once per week. Notably, 30 mpk EEV12-PMO^{DMD} resulted in the highest percentage of exon skipping. The percentage of exon 23 corrected dystrophin products in quadriceps, transverse abdominus, diaphragm, and heart as determined by RT-PCR is shown in **FIGS. 13A-D**. **FIGS. 20A-D** show the percentage of exon 23 corrected dystrophin products in transverse abdominus (**FIG. 20A**), quadriceps (**FIG. 20B**), diaphragm (**FIG. 20C**), and the heart (**FIG. 20D**) in MDX mice that were administered either 30 mpk of PMO^{DMD} or 30 mpk of EEV12-PMO^{DMD}. Mice administered EEV12-PMO^{DMD} exhibited enhanced splicing correction, compared to mice administered PMO^{DMD} alone. FIGS. **14A-D** show exon skipping efficacy of the above-noted dosing regimens in quadriceps, transverse abdominus, diaphragm, and heart by Western Blot. **FIGS. 19A-B** show the dystrophin levels in MDX mice two weeks (**FIG. 19A**) and four weeks (**FIG. 19B**) after treatment with 30 mpk EEV12-PMO^{DMD} or 30 mpk PMO^{DMD}. **FIGS. 15A-D** show dystrophin protein recovery in muscle groups from MDX mice treated with EEV12-PMO^{DMD} at 10 mpk twice per week, 10 mpk once per week, 10 mpk once per two week, and 30 mpk once per week.

### Example 3D. Use of cell-penetrating peptide coupled to an oligonucleotide and nuclear localization sequence for splicing correction of exon 23 of DMD in an MDX mouse model

**Purpose.** This study employs an MDX mouse model, a model of DMD, to study the effect of compositions comprising an AC, a CPP, and a nuclear localization sequence on dystrophin expression and muscle fiber damage.

**Study design.** Compositions comprising an AC having a sequence of 5'-GGCCAAACCTCGGCTTACCTGAAAT-3' (SEQ ID NO: 260), a cCPP12 (amino acid sequence is Ff**Φ**RrRr (SEQ ID NO: 117)), and a nuclear localization sequence PKKKRKV (SEQ ID NO: 131) (referred to herein as "ENTR-201" are applied to MDX mice to evaluate the ability of the compositions to skip exon 23 and thus treat DMD. A control composition lacks cCPP12 and a nuclear localization sequence. The sequence of the AC of the control composition is 5'-GGC CAA ACC TCG GCT TAC CTG AAA T-3' (SEQ ID NO: 260). The ENTR-201 composition is administered to the mice intravenously (IV) at a dose of 10 mg/kg once per week for four weeks or at a dose of 20 mpk once. The control composition is administered to mice intravenously (IV) at a dose of 20 mpk. Total RNA is extracted from tissue samples and analyzed by RT-PCR and protein is extracted from tissue sample and analyzed by Western Blot to visualize the efficiency of splicing correction and to detect dystrophin products. The percentage of exon 23 corrected products is evaluated. The dystrophin protein level is evaluated with respect to alpha-actinin (loading control) as well as in comparison to dystrophin expression in wild-type mice. Serum levels of creatine kinase, which is increased in DMD patients as a result of muscle fiber damage, were also evaluated by a commercially available kit purchased from Sigma Chemicals.

**Evaluations of peptide fusions to the CPP12-PMO construct.** To further increase the functional delivery of PMO, we also explored various peptides fusions for the CPP-PMO constructs as shown in Table B4. For one example, T9 peptide (SKTFNTHPQSTP (SEQ ID NO: 258)) (Y. Seow et al. / Peptides 31 (2010) 1873-1877) and muscle specific peptide (MSP peptide, ASSLNIA (SEQ ID NO: 259) (Gao et al. Molecular Therapy (2014) 22, 7: 1333-1341) which have been demonstrated to enhanced muscle targeting were also fused to CPP12 construct as in ENTR-0119 and ENTR-0163 respectively. As detailed in Table B4, neither of these peptide fusions improved the activity in MDX mice (comparing ENTR-0119, ENTR-0163 and ENTR-0098). Consistent with what we found the GFP correction in Example 1, CPP12 with a Nuclear Localization Sequence (PKKKRKV (SEQ ID NO: 131)) outperformed CPP12 alone significantly (e.g. ENTR-164, ENTR-0165, and ENTR-0201, Table B4). One week after a single intravenous dose at 20 mpk, ENTR-164 yielded 59.5 ± 2.2%, 29.1 ± 0.8 %, and 61.0 ± 13.7% exon 23 skipping in Quad, heart, and diaphragm respectively. One week after a single intravenous dose at 20 mpk, ENTR-165 yielded 38.5 ± 2.5%, 30.5 ± 17.0 %, and 30.2 ± 2.8% exon 23 skipping in Quad, heart, and diaphragm respectively. One week after a single intravenous dose at 20 mpk, ENTR-201 yielded 73.5 ± 8.4%, 60.5 ± 17.2 %, and 79.0 ± 6.8% exon 23 skipping in Quad, heart, and diaphragm respectively. The preparation of these NLS fusions were described in detail above. The structure of ENTR-164, ENTR-165 are shown above. Consistent with the exon skipping data, by ENTR-0164 and ENTR-0165 also significantly corrected the expression of dystrophin protein levels as analyzed by Western Blot shown in **FIG. 47****.** By comparing to the level in respective tissues from wild type (C57BL/10) and using the actinin as loading control, percentage of dystrophin protein correction is further quantified to that of wild type and shown in **FIGS. 48A-****D.** Instead of using the modified PMO with 3' amide bond formation, we also tested the incorporation of cyclooctyne on solid support by modifying the morpholino amino group with a bifunctional linker comprised of a cyclooctyne moiety for click reaction to a CPP-azide and a PFP easter to form a carbamate with PMO and thus produced the precursor which can be used for synthesis of ENTR-201. Similar to that of ENTR-165, ENTR-201 also demonstrated high exon skipping activity across all the muscle groups as shown in Table B4.

**Activity of ENTR-201 in MDX mice.** The study design in **FIG. 50** and the table below outlines the injection, sample collection and bioanalysis to study the duration of effects of ENTR-201 after single IV injection.

| **Group** | **N** | **Treatment** | **Dosage (mpk)** | **SAC time (p.i.)** | **Endpoints** | |
|---|---|---|---|---|---|---|
| 1 | 3 | PBS | 0 | 1 week | | • Exon skipping (RT-PCR) |
| 2 | 3 | ENTR-201 | 20 | 1 week | | |
| 3 | 3 | ENTR-201 | 20 | 2 weeks | | • Dystrophin expression (Western blot and immunohistochemistry) |
| 4 | 3 | ENTR-201 | 20 | 4 weeks | | |

After a single dose of ENTR-201 at 20 mg/kg on day 1, animals were sacrificed at 1 week, 2 weeks and 4 weeks post injection. Vehicle (PBS) only was used as a negative control. Heart, diaphragm, quadriceps and transverse abdominis were collected for RT-PCR to detect dystrophin exon 23 skipped product, and Western blot analysis to detect dystrophin protein expression (relative to alpha-actinin). Samples from 4 weeks post single IV injection of ENTR-201 at 20 mpk or PBS were also analyzed by immunohistochemistry staining to detect expression and distribution of dystrophin in various muscle tissues.

Treatment of mice with single dose 20 mg/kg ENTR-0201 resulted in splicing correction of dystrophin in the heart (**FIG. 51A**), diaphragm (**FIG. 51B**), quadriceps (**FIG. 51C**) and transverse abdominis (TrA) (**FIG. 51D**). ENTR-0201 delivers significant enhancements in exon skipping efficiency up to four weeks post single IV injection. The corresponding dystrophin protein levels were analyzed by Western Blot and summarized in **FIG. 52A-D**. Restored dystrophin protein sustained up to four weeks after single IV injection at 20 mpk in the heart (**FIG**. **52A**), diaphragm (**FIG. 52B**), quadriceps (**FIG. 52C**) and transverse abdominis (TrA) (**FIG. 52D**). The level of protein correction is consistent with the RNA analysis. The tissue samples from the last injection were also analyzed by immunohistochemistry as shown in **FIG. 53****,** which show that all the skeletal muscle fibers immunostained positive for dystrophin protein as visualized by brown color staining. The intensity of dystrophin expression was significant in the heart muscle tissue reaching near normal levels. Widespread uniform expression of dystrophin protein over multiple tissue sections within each of muscle group analyzed.

**Activity of ENTR-201 in MDX mice after repeated dosage.** The study design schemed in **FIG. 54** and table below outlines the injection, sample collection and bioanalysis to study the activity of ENTR-201 after repeated dosage.

| Group | N | Treatment | Dosage (mpk) | Dosage Frequency | Endpoints | |
|---|---|---|---|---|---|---|
| 1 | 3 | PBS | 0 | QWx4 | | • Exon skipping (RT-PCR) |
| 2 | 3 | ENTR-013 | 20 | QWx4 | | • Creatine Kinase |
| 3 | 3 | ENTR-201 | 10 | QWx4 | | • Dystrophin Expression (Western blot and immunohistochemistry) |

Mice were treated with 10 mg/kg of ENTR-201 once every week for 4 weeks. ENTR-013 at 20 mg/kg (PMO only) and vehicle (PBS) only were used as control groups. All animals were sacrificed at 1 week post the last injection. Heart, diaphragm, quadriceps and transverse abdominis were collected for RT-PCR to detect dystrophin exon skipped products (**FIGS. 55A-D**), Western blot analysis and immunohistochemistry staining to detect dystrophin protein expression (**FIGS. 56A-D** and **FIGS. 57A-D**) to detect expression and distribution of dystrophin in various tissues. Serum creatine kinase level was quantified as a muscle functional biomarker (**FIG. 58**).

Treatment of MDX mice with 10 mg/kg ENTR-201 once per week for four weeks also resulted in significant splicing correction of dystrophin mRNA (**FIGS. 55A-D**) and dystrophin protein levels (**FIGS. 56A-D**) in various muscle tissues (heart, diaphragm, quadricep, and transverse abdominis (TrA)). In comparison to treatment with 20 mpk PMO, treatment with ENTR-201 at 10 mg/kg results in a higher amount of both splicing correction and dystrophin protein in all four muscle tissues (**FIGS. 55A-D** **and** **FIGS. 56A-D**). Notably, the mRNA correction and dystrophin protein expression in the heart are only observed in 10 mpk ENTR-201 treated MDX mice, not in 20 mg/kg PMO treated MDX mice. The findings from IHC study (FIG. 43) was also consistent with RT-PCR and WB analysis. Treatment with 10 mpk ENTR-201 once per week for four weeks also normalized the serum creatine kinase level, which is a muscle damage biomarker, suggesting that Oligo 201 treatment reduces muscle fiber damage in a DMD mouse model (**FIG. 58**). PMO (ENTR-0013) treatment alone in contract did not significantly reduce the elevated serum CK level (**FIG. 58**). Serum samples were collected one week after the last injection from repeated dosing study. Analysis of CK levels was performed using commercially available CK measurement kit (Millipore Sigma chemicals, MAK116) as per instructions from the manufacturer. Quantification of dystrophin protein showed nearly 40 % cells are positive for dystrophin in cardiac tissue compared to 5% or less in vehicle treated or PMO alone treated cardia tissues (**FIG.59**).

Treatment of mice with 20 mg/kg Oligo 201 one time per week resulted in splicing correction of dystrophin in the heart (**FIG. 21A**) and in the diaphragm (**FIG. 21B**). Treatment of mice with 10 mg/kg Oligo 201 or 5 mg/kg Oligo 201 four times per week also resulted in splicing correction of dystrophin in the heart (**FIG. 22A**) and in the diaphragm (**FIG. 22B**). Treatment with Oligo 201 at 10 mg/kg results in a higher amount of splicing correction than treatment with 5 mg/kg in the heart (**FIG. 22C**) and diaphragm (**FIG. 22D**). Treatment with 5 mg/kg or 10 mg/kg Oligo 201 four times per week also resulted in a decrease in creatine kinase expression in comparison to control (**FIG. 23**), suggesting that Oligo 201 treatment reduces muscle fiber damage in patients with DMD.

### Example 4A. Use of cell-penetrating peptides conjugated to oligonucleotides for CD33 knockout in human macrophage cells.

The compounds of Table C are evaluated in Example 4A and 4B. Exemplary experiments are described below.

**Table C. Compounds that target CD33**

| **Oligo#** | **Design** | **Note** |
|---|---|---|
| ENTR-036 | 5'-GTA ACT GTA TTT GGT ACT TCC-3'-primary amine (all PMO monomers) (SEQ ID NO: 265) | PMO^{CD33} Human CD33 exon 2 skipping |
| ENTR-081 | 5'-GTA ACT GTA TTT GGT ACT TCC-3'-PEG12CPP12 (all PMO monomers) (SEQ ID NO: 266) | CPP-PMO^{CD33} Human CD33 exon 2 skipping |
| ENTR-085 | 5'-CTG TAT TTG GTA CTT-3'+ CPP12-PEG12-K(CPP12)-PEG12-TFP (all PMO monomers) (SEQ ID NO: 267) | CPP-CPP-PMO^{CD33} Human CD33 exon 2 skipping |
| ENTR-087 | 5'-GTA ACT GTA TTT GGT ACT TCC-3'+ CPP12-PEG12-K(CPP12)-PEG12-TFP (all PMO monomers) (SEQ ID NO: 268) | CPP-CPP-PMO^{CD33} Human CD33 exon 2 skipping |
| ENTR-179 | 5'-GTA ACT GTA TTT GGT ACT TCC-3' (Ac-NLS-Lys(CPP12)-PEG12-K(N3)- (all PMO monomers) (SEQ ID NO: 269) | CPP-NLS-PMO^{CD33} Human CD33 exon 2 skipping |

**Cells.** Differentiated THP-1 cells (human monocyte cells) and glioblastoma cells (human neuronal cells) were used in this study.

**Study design.** CD33 is implicated in diseases such as cancer and Alzheimer's Disease ("AD"). Targeting CD33 expression represents a treatment strategy for AD and cancer.

Targeting CD33 expression represents a treatment strategy for AD and cancer. Skipping of exon 2 of the gene expressing CD33 produces D2-CD33, a CD33 isoform that lacks a binding domain of sialic acid. (**FIGS. 60A-B**). In the absence of such a ligand binding domain, CD33 cannot inhibit microglial activation and phagocytosis of amyloid beta by microglial cells. Such a result is protective against AD. This example evaluated the efficacy of the platform described in Examples 1-5 for treating AD or cancer. Briefly, THP1 and glioblastoma cells were treated with AC having a nucleic acid sequence of 5'-GTAACTGTATTTGGTACTTCC-3' (SEQ ID NO: 153) ("PMO^{CD13}"),PMO^{CD33} conjugated to a CPP ("CPP-PMO^{CD33}"), or PMO^{CD33} conjugated to both CPP and NLS ("CPP-NLS-PMO^{CD33}"), in the presence of 10 % fetal bovine serum (FBS). The CPP used was cCPP12, which has an amino acid sequence of Ff**Φ**RrRr (SEQ ID NO: 117).

**PMO sequence development and optimization.** The nucleic acid sequence 5'-CTGTATTTGGTACTT-3' (SEQ ID NO: 270) has previously been reported to induce human CD33 exon2 skipping in THP1 cells (Bergeijk P. et al. Molecular and Cellular Biol. 2019). We first modified the oligonucleotide chemistry from 2'-MOE modified RNA to phosphorodiamidate morpholino oligomers (PMO) as in the conjugated construct ENTR-085 but with moderate success (**FIGS. 68A-B**). To improve efficacy, we further developed a 21nt-long PMO, ENTR-036, TABLE 5, 5'-GTAACTGTATTTGGTACTTCC-3' (SEQ ID NO: 265) and its CPP conjugate (i.e. construct ENTR-087) showed superior efficacy (**FIGS. 68A-B**). Thus, PMO sequence ENTR-036 was used for subsequent studies.

**Detection of exon 2 skipping by RT-PCR and flow cytometry.** Reverse Transcription followed by semi-quantitative PCR analysis revealed that treatment of THP1 cells for 48 hours in the presence of 10 % FBS with CPP-CPP-PMO^{CD33} (ENTR-087) resulted in skipping of exon 2 and the production of D2-CD33, a CD33 isoform that lacks a ligand binding domain (**FIG. 61A** and **FIG. 61B**). Treatment of THP1 cells with PMO^{CD33} (ENTR-036) alone resulted in a lower amount of exon skipping in comparison to treatment with CPP-CPP-PMO^{CD33}. Exon 2 skipping was dependent on the dose of CPP-CPP-PMO^{CD33} (ENTR-087) (**FIG. 62A** and **FIG. 62B**). Flow cytometry revealed reduced production of CD33 in cells treated with CPP-CPP-PMO^{CD33} (ENTR_036) in comparison to untreated (NT) cells (**FIGS. 61A-B**).

**Dose-dependent Exon 2 skipping induced by CPP-NLS-PMO^{CD33} in THP1 cells** CD33 mRNA from differentiated THP1 cells (human monocyte cells) with various concentrations of CPP-NLS-PMO^{CD33}(ENTR-179), PMO^{CD33} (ENTR-036) with Endoporter (6µL/mL) transfection reagent, or PMO^{CD33} (ENTR_036) alone for 48 hours in the presence of 10% FBS were analyzed by RT-PCR (**FIGS. 61A-B**). Result shows dose-dependent skipping of exon 2 CD33 by CPP-NLS-PMO^{CD33} treatment, with significant improvement over transfection (over 2-fold) and over 1000-fold improvement compared to unconjugated PMO. CD33 mRNA of glioblastoma cells (human neuronal cell line, U-87 MG) treated with various concentrations of CPP-NLS-PMO^{CD33} (ENTR-179) for 48 hours in the presence of 10% FBS were analyzed by RT-PCR (**FIG. 65**). Result shows dose-dependent skipping of exon 2 CD33 by CPP-NLS-PMO^{CD33} treatment.

**Duration of effects of CPP-PMO^{CD33} in differentiated THP1 cells.** Differentiated THP1 cells (human monocyte cells) treated with CPP-PMO^{CD33} (ENTR-087) for 1 day, and cells were continued to be cultured with full growth medium. 2-8 days post incubation, cells were harvested and the CD33 mRNA were analyzed (**FIGS. 66A-B**). Result shows that uptaken CPP-PMO^{CD33} can induce CD33 exon 2 skipping for a sustained period of time (> 8 days).

**Exon 2 skipping induced by monovalent CPP-PMO^{CD33} and bivalent CPP-PMO^{CD33}** CD33 mRNA of differentiated THP1 cells (human monocyte cells) treated by PMO^{CD33} (ENTR-036), monovalent CPP-PMO^{CD33} (ENTR-081), and bivalent CPP-PMO (ENTR-087) for 48 hours were analyzed by RT-PCR (**FIG. 67**). Result shows effect of bivalent CPP-PMO^{CD33} is more potent than CPP- PMO^{CD33} in inducing CD33 exon 2 skipping.

### Example 4B. Use of cell-penetrating peptides conjugated to oligonucleotides for CD33 knockout in human macrophage cells.

This example used the protocols of Example 4A to evaluate an AC having a nucleic acid sequence of 5'-GTAACTGTATTTGGTACTTCC-3' ("PMO^{CD33}") (SEQ ID NO: 153) or PMO^{CD33} conjugated to a CPP ("EEV-PMO^{CD33}") in the presence of 10 % fetal bovine serum (FBS). The CPP used was cCPP12, which has an amino acid sequence of Ff**Φ**RrRr (SEQ ID NO: 117).

**Detection of exon 2 skipping by RT-PCR and flow cytometry.** RT-PCR analysis revealed that treatment of THP1 cells for 48 hours in the presence of 10 % FBS with EEV-PMO^{CD33} resulted in skipping of exon 2 and the production of D2-CD33, a CD33 isoform that lacks a ligand binding domain (**FIG. 16A** and **FIG. 16B**). Treatment of THP1 cells with PMO^{CD33} alone resulted in a lower amount of exon skipping in comparison to treatment with EEV-PMO^{CD33}. Exon 2 skipping was dependent on the dose of EEV-PMO^{CD33} (**FIG. 17A** and **FIG. 17B**). Flow cytometry revealed reduced production of CD33 in cells treated with EEV-PMO^{CD33} in comparison to untreated (NT) cells (**FIG. 18**).

### Example 4C. Use of cell-penetrating peptides conjugated to oligonucleotides for production of D2-CD33 in nonhuman primates.

The CPP-PMO^{CD33} (ENTR-081 and ENTR-179) described in Examples 4A-C is used animal studies, e.g. in rodents, monkeys, and humans. Animals or humans are administered intravenously or intrathecally via various doses (0.5, 1, 2.5, 5, 10, 20, 40 mpk) of a CPP- PMO^{CD33} or PMO conjugates that targets exon skipping (exon 2 of human CD33 or exon 5 of monkey CD33) of the gene expressing CD33. **FIG. 29** shows the study design. The results show that the oligonucleotide therapeutics induce exon skipping of target CD33 gene, downregulate CD33 level and can treat AD.

**NHP Study design.** To explore the tolerability of CPP-PMO on non-human primate (NHP), two CPP-PMO constructs as well as PMO itself are dosed in staggered fashion at 2 mpk and 5 mpk through intravenous infusion on d0 and d3, respectively. Oligonucleotides include "PMO^{CD33}" (ENTR-036), PMO^{CD33} conjugated to a CPP ("CPP-PMO^{CD33}", ENTR-081), or PMO^{CD33} conjugated to both CPP and NLS ("CPP-NLS-PMO^{CD33}", ENTR-179), and are formulated in saline (0.9% w/v sodium chloride). The CPP used was CPP12, which has a cyclic amino acid sequence of Ff**Φ**RrRr (SEQ ID NO: 117). PBMCs (peripheral blood mononuclear cells) were isolated at 1 day (d4) and 7 days (10) post 5 mpk injection to detect splicing correction. Blood, serum and urine samples were collected at 1 day and or 7 days post each injection for hematology, clinical chemistry, coagulation, urinalysis, cytokine and histamine analysis.

**Detection of exon exclusion by RT-PCR.** Although human and non-human primates share high sequence homology of CD33 gene, the 5'-UTR and splicing pattern is different. The sequence coding for the IgV domain of CD33 is located in exon 2 for human and located in exon 5 for non-human primate CD33 gene. Therefore, the skipping of exon 2 in human CD33 (D2-CD33), resulting in ΔIgV-CD33 protein, corresponds to the skipping of exon 5 of non-human primate CD33. Monkey PBMC was collected at specified time points mentioned above. Total RNA was extracted, and RT-PCR was conducted using forward primer 5'-CTCAGACATGCCGCTGCT-3' (SEQ ID NO: 271) and reverse primer 5'-TTGAGCTGGATGGTTCTCTCCG-3' (SEQ ID NO: 272) resulting in full length CD33 mRNA (FL-CD33) at 700bp and Exon-5 skipped CD33 mRNA (D5-CD33) at 320bp. Reverse Transcription followed by semi-quantitative PCR analysis revealed that treatment of monkey PBMC cells showed that IV administration of CPP-PMO^{CD33} (ENTR-081) and CPP-NLS-PMO^{CD33} (ENTR-179), but not PMO, resulted in skipping of exon 5 of CD33 gene and the production of D2-CD33. And the activity of both ENTR-081 and ENTR-179 last for at least 7 days post treatment (**FIG**. **30**).

### Example 5. Conjugation of oligonucleotide with cell penetrating peptide.

**Conjugation of PMO oligos with cell penetrating peptides.** As shown in **FIG. 7b****,** phosphorodiamidate morpholino oligomer (PMO) was conjugated to cell penetrating peptide (CPP) via either amide bond formation (left) or click chemistry. The linker/CPP can be installed either on the 5' end, or on the 3' end of the oligonucleotide.

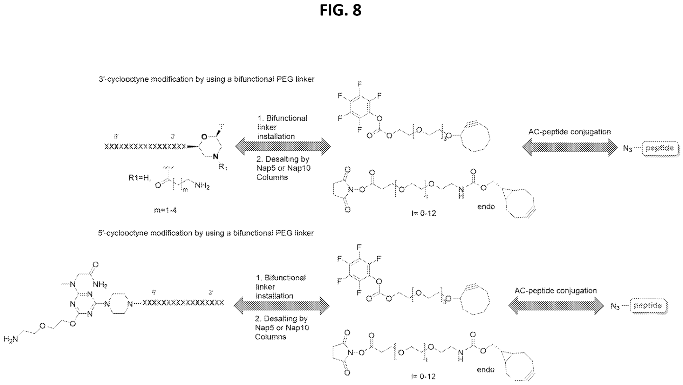

**Synthesis of oligonucleotide-peptide conjugate with PEG spacer.** As shown in **FIG. 8****,** an oligonucleotide-peptide conjugate was synthesized with PEG (polyethylene glycol) linker inserted between oligonucleotide moiety and peptide. Briefly, the 3' or 5' amino group of the AC was conjugated to a bifunctional PEG linker comprising a OSU or TFP or PFP activating carbonate functionality and cyclooctyne. The carbonate is reacted with 3' or 5' amino group of AC to form stable carbamate. The excess bifunctional reagent and other small molecules were removed by desalting column (Nap10 or Nap 5). The resulting solution was concentrated to 1-2 mM modified AC concentration and conjugated with the designated peptide-azide. The click reaction was allowed to proceed for 8-12 hours for completion monitored by LC-MS.

**Synthesis of oligonucleotide-peptide conjugate for various gene targets.** As shown in Table D, **FIG. 7A, FIG. 7B****,** and **FIG. 8****,** for each target selected from the group consisting of FXN, EGFP654, CD33, and DMD, the antisense compounds with specific oligonucleotide sequence were conjugated to corresponding peptides.

**Table D. Compositions Evaluated**

| **Oligo chemistry** | **Name (oligo chemistry)** | **Design** | **Target** |
|---|---|---|---|
| PMO | PMO^{GAA}-CP12 | 5'-CTT CTT CTT CTT CTT CTT CTT CTT C-CP12 (SEQ ID NO: 154) | FXN |
| | | (all PMO monomers; CP12 = peptide) | |
| O-MOE | O-MOE^{GAA} _ CP12 | CP12-5'-CTT CTT CTT CTT CTT CTT-3' (SEQ ID NO: 155) | FXN |
| | | (all 2'-O-MOE RNA monomers; all PS bonds; CP12 = peptide) | |
| LNA | LNA^{GAA}-CP12 | CP12-5'-LnT-dC-LnT-LnT-dC-LnT-LnT-dC-LnT-LnT-dC-LnT-LnT-dC-LnT-LnT-dC-LnT-LnT (SEQ ID NO: 156) (LnT=LNA T; dC=DNA C; all PS bonds; CP12 = peptide) | FXN |
| PMO | PMO654-CP12 | 5'-GCT ATT ACC TTA ACC CAG-3' -CP12 (SEQ ID NO: 157) | EGFP654 |
| | | (all PMO monomers; CP12 = peptide) | |
| PMO | PMO654-CP12-R1 | 5'-GCT ATT ACC TTA ACC CAG-3' -CP12-R1 (SEQ ID NO: 158) | EGFP654 |
| | | (all PMO monomers; CP12 = peptide; R1=lipid group) | |
| PMO | PMO654-CP1212 | 5'-GCT ATT ACC TTA ACC CAG-3' -CP1212 (SEQ ID NO: 159) | EGFP654 |
| | | (all PMO monomers; CP1212 = peptide) | |
| PMO | PMO654-Pip | 5'-GCT ATT ACC TTA ACC CAG-3' -Pip (SEQ ID NO: 160) | EGFP654, control |
| | | (all PMO monomers; Pip = peptide) | |
| PMO | PMO^{FM10}-CP12 | 5'-GGG CAT TTT AAT ATA TCT CTG AAC T-3' - CP12 (SEQ ID NO: 161) (all PMO monomers; CP12 = peptide) | FSHD |
| PMO | PMO^{DMD}-CP12 | 5'-GGC CAA ACC TCG GCT TAC CTG AAA T-3' - CP12 (SEQ ID NO: 162) | DMD |
| PMO | PMO^{DMD}-CP12-R1 | 5'-GGC CAA ACC TCG GCT TAC CTG AAA T-3' - CP12-R1 (SEQ ID NO: 163) (all PMO monomers; CP12 = peptide; R1=lipid group) | DMD |
| PMO | PMO^{CD33}-CP12 | 5'-GTA ACT GTA TTT GGT ACT TCC-3' - CP12 (SEQ ID NO: 164) | CD33 |

**Cell penetrating peptides.** Examples of cell penetrating peptides are presented in **FIG. 9****.**

### Example 6. Intracellular delivery of CRISPR gene-editing machinery

**CPP conjugated to CRISPR gene-editing machinery.** Various compositions comprising a CPP from Table 4 and CRISPR gene-editing machinery (Table E) are prepared. The gRNA is labeled with a fluorescent dye.

**Table E. Compositions Evaluated**

| | Composition |
|---|---|
| 1 | CPP conjugated to gRNA of RNP comprising gRNA and Cas9 |
| 2 | CPP conjugated to Cas9 of RNP comprising gRNA and Cas9 |
| 3 | CPP conjugated to gRNA of RNP comprising gRNA and Cas9 and linker |
| 4 | CPP conjugated to Cas9 of RNP comprising gRNA and Cas9 and linker |
| 5 | CPP conjugated to linker of RNP comprising gRNA and Cas9 and linker |

### Target cells. HeLa cells

**Study Design.** HeLa cells are cultured in six-well plates (5 x 10⁵ cells per well) for 24 hours. After 24 hours, HeLa cells are incubated with the compositions of Table 7. As a negative control, the HeLa cells are incubated with CRISPR gene-editing machinery in the absence of CPP. The uptake efficiency of the compositions comprising a CPP and CRISPR gene-editing machinery is evaluated using fluorescence.

### Example 7. Gene-Editing using cell-penetrating peptides conjugated to CRISPR gene-editing machinery

**Study Design.** Various compositions comprising a CPP from Table 4 and CRISPR gene-editing machinery (Table F) are prepared to evaluate their ability to cleave human target DNA. The protocol described in U.S. Publication No. 2014/ 0068797, which is incorporated by reference herein, in its entirety, is used to evaluate the ability of the compositions to make gene edits. Composition 9 serves as a control to evaluate the effect of the CPP on gene editing. Compositions 10-13 are negative controls which lack either an gRNA or Cas9. Cas9 is labeled with green fluorescent protein (GFP) in the compositions of Table F.

Briefly, human HEK293T cells are transfected with the compositions of Table F. Western blotting is performed to confirm that Cas9 enters the HEK293T cells. Northern blotting is performed to confirm that the gRNA enters the HEK293T cells. Fluorescence microscopy is performed to visualize Cas9. A Surveyor assay is performed to assess site-specific genome cleavage.

**Table F. CPPs conjugated to RNP**

| | Composition |
|---|---|
| 1 | (a) CPP conjugated to Cas9 |
| | (b) CPP conjugated to gRNA |
| 2 | CPP conjugated to gRNA of RNP comprising gRNA and Cas9 |
| 3 | CPP conjugated to Cas9 of RNP comprising gRNA and Cas9 |
| 4 | CPP conjugated to gRNA of RNP comprising gRNA and Cas9 and linker |
| 5 | CPP conjugated to Cas9 of RNP comprising gRNA and Cas9 and linker |
| 6 | CPP conjugated to linker of RNP comprising gRNA and Cas9 and linker |
| 7 | (a) soluble Cas9 |
| | (b) CPP conjugated to gRNA |
| 8 | (a) soluble gRNA |
| | (b) CPP conjugated to Cas9 |
| 9 | (a) RNP comprising Cas9 and gRNA |
| 10 | CPP conjugated to Cas9 |
| 11 | CPP conjugated to gRNA |
| 12 | CPP conjugated to Cas9 through linker |
| 13 | CPP conjugated to gRNA through linker |

### EMBODIMENTS

Embodiment 1. A compound comprising:
(a) a cell penetrating peptide (CPP) sequence and
(b) an antisense compound (AC) that is complementary to a target sequence in a pre-mRNA sequence.

Embodiment 2. The compound of Embodiment 1, wherein the AC comprises at least one modified nucleotide or nucleic acid selected from a phosphorothioate (PS) nucleotide, a phosphorodiamidate morpholino nucleotide, a locked nucleic acid (LNA), a peptide nucleic acid (PNA), a nucleotide comprising a 2'-O-methyl (2'-OMe) modified backbone, a 2'O-methoxyethyl (2'-MOE) nucleotide, a 2',4' constrained ethyl (cEt) nucleotide, and a 2'-deoxy-2'-fluoro-beta-D-arabinonucleic acid (2'F-ANA), and
wherein hybridization of the AC with the target sequence reduces or prevents splicing.

Embodiment 3. The compound of Embodiment 1, wherein the AC comprises small interfering RNA (siRNA), microRNA (miRNA), ribozymes, immune stimulating nucleic acids, antisense, antagomir, antimir, microRNA mimic, supermir, Ul adaptor, aptamer, or a CRISPR gene-editing machinery.

Embodiment 4. The compound of Embodiment 1, wherein the CPP is conjugated, directly or indirectly, to the AC.

Embodiment 5. The compound of Embodiment 4, wherein the CPP is conjugated to the 5' end or the 3' end of the AC.

Embodiment 6. The compound of Embodiment 4, wherein the CPP is conjugated to the backbone of the AC.

Embodiment 7. The compound of any one of Embodiment 4-6, further comprising a linker (L), which conjugates the CPP to the AC.

Embodiment 8. The compound of Embodiment 7, wherein the L is covalently bound to the 5' end of the AC.

Embodiment 9. The compound of Embodiment 7, wherein the L is covalently bound to the 3' end of the AC.

Embodiment 10. The compound of Embodiment 7, wherein the L is covalently bound to the backbone of the AC.

Embodiment 11. The compound of any one of Embodiment 7-10, wherein the L is covalently bound to the side chain of an amino acid on the CPP.

Embodiment 12. The compound of Embodiment 7, having a structure according to Formula I-A or Formula I-B:

CPP-L-AC Formula I-A

or

AC-L-CPP Formula I-B,

wherein L of Formula I-A is covalently bound to the side chain of an amino acid on the CPP and to the 5' end of the AC, and L of Formula I-B is covalently bound to the side chain of an amino acid on the CPP and the 3' end of the AC.

Embodiment 13. The compound of any one of Embodiment 7-12, wherein L comprises one or more D or L amino acids, each of which is optionally substituted; alkylene, alkenylene, alkynylene, carbocyclyl, or heterocyclyl, each of which is optionally substituted; or - (R¹⁻X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is independently selected from H, alkyl, alkenyl, alkynyl, carbocyclyl, and heterocyclyl, each of which is optionally substituted, and z is an integer from 1 to 20; or combinations thereof.

Embodiment 15. The compound of any one of Embodiment 8-14, wherein L has a structure according to Formula II: 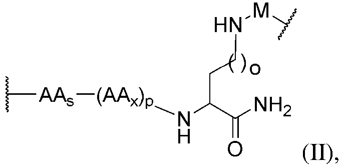 wherein
M is a group that conjugates L to an oligonucleotide;
AAₛ is a side chain or terminus of an amino acid on the CPP;
AAₓ is an amino acid;
o is an integer from 0 to 10; and
p is an integer from 0 to 5.

Embodiment 16. The compound of Embodiment 15, wherein p is 2 and each AAₓ is β alanine

Embodiment 17. The compound of Embodiment 15 or 16, wherein M is present and comprises an alkylene, alkenylene, alkynylene, carbocyclyl, or heterocyclyl, each of which is optionally substituted.

Embodiment 18. The compound of any one of Embodiments 15-17, wherein M is present and selected from the group consisting of: 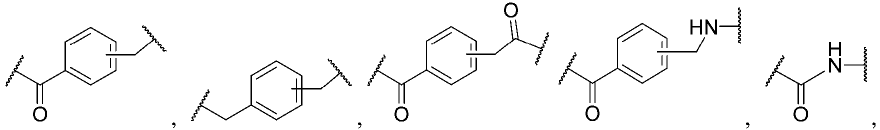 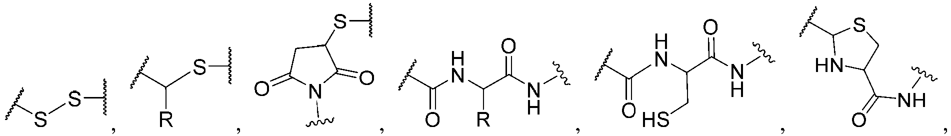 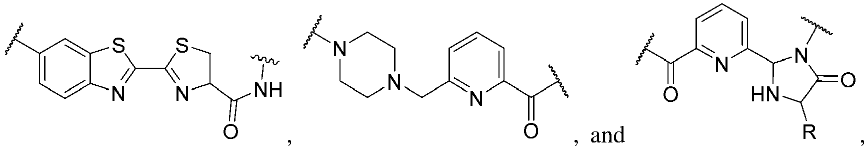 wherein R is alkyl, alkenyl, alkynyl, carbocyclyl, or heterocyclyl; or wherein M is selected from the group consisting of: 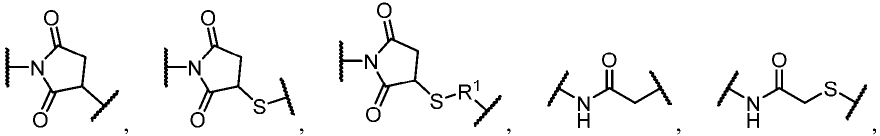 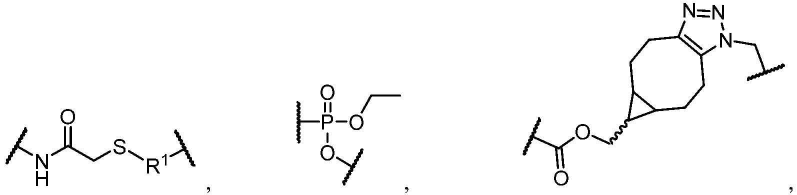 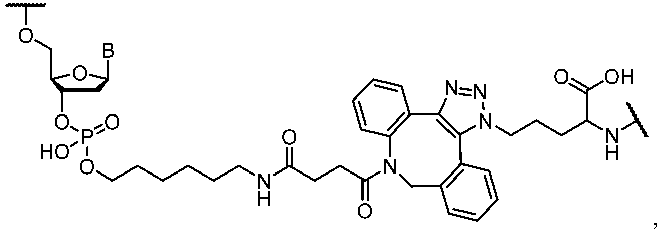 and 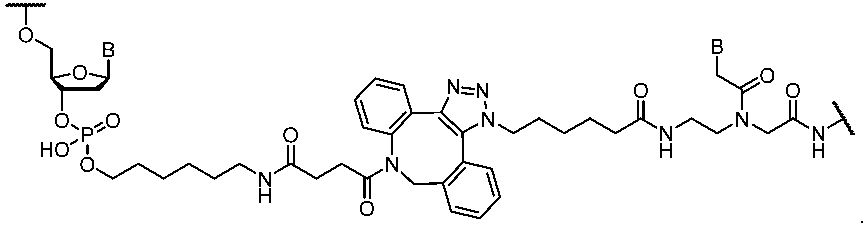 wherein: R¹ is alkylene, cycloalkyl, or 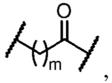 wherein m is 0 to 10 wherein each R is independently an alkyl, alkenyl, alkynyl, carbocyclyl, or heterocyclyl.

Embodiment 18. The compound of any one of Embodiment 14-17, wherein M is

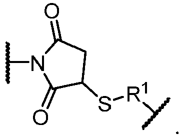

Embodiment 19. The compound of Embodiment 18, wherein R¹ is 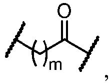 and m is 2.

Embodiment 20. The compound of Embodiment 19, wherein L-M has the following structure 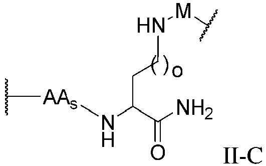 wherein:
AAₛ is a side chain or terminus of an amino acid on the cCPP;
o is an integer from 0 to 10.

Embodiment 21. The compound of any of the preceding Embodiments having the following structure 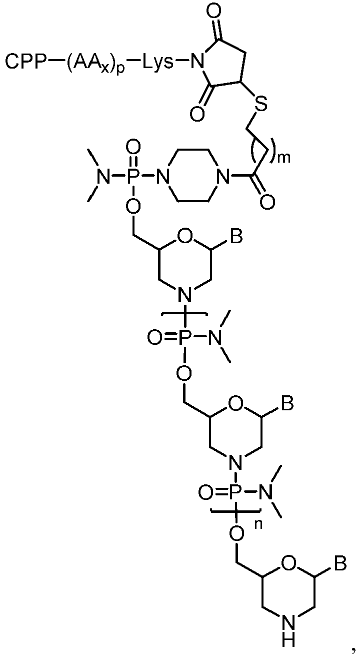 wherein
each B is independently a nucleobase; and
n is 1 to 50.

Embodiment 22. The compound of any one of Embodiments 12-21, wherein the cCPP has a sequence comprising Formula III: 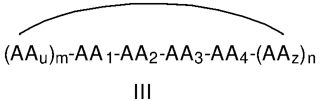 wherein:
each of AA₁, AA₂, AA₃, and AA₄, are independently selected from a D or L amino acid,
each of AAᵤ and AA_{z}, at each instance and when present, are independently selected from a D or L amino acid, and
m and n are independently selected from a number from 0 to 6; and
wherein:
at least two of AAᵤ, at each instance and when present, AA₁, AA₂, AA₃, AA₄, and AA_{z}, at each instance and when present, are independently arginine, and
at least two of AAᵤ, at each instance and when present, AA₁, AA₂, AA₃, AA₄, and AA_{z}, at each instance and when present, are independently a hydrophobic amino acid.

Embodiment 23. The compound of Embodiment 22, wherein the cCPP has a sequence comprising any one of Formula IV-A-D: 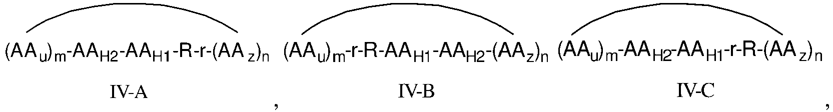 and 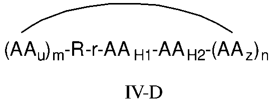 wherein:
each of AA_{H1} and AA_{H2} are independently a D or L hydrophobic amino acid;
at each instance and when present, each of AA_{U} and AA_{Z} are independently a D or L amino acid; and
m and n are independently selected from a number from 0 to 6.

Embodiment 24. The compound of any one of Embodiments 1-23, wherein M is present and covalently bound to the 5' end of the AC or the 3' end of the AC.

Embodiment 25. The compound of any one of Embodiments 1-24, wherein M is present and is covalently bound to the 5' end of the AC.

Embodiment 26. The compound of any one of Embodiments 1-25, wherein M is present and is covalently bound to the backbone of the AC.

Embodiment 27. The compound of any one of Embodiments 114-26, wherein u is 0.

Embodiment 28. The compound of any one of Embodiments 14-27, wherein p is 1.

Embodiment 29. The compound of any one of Embodiments 14-28, wherein q is 12.

Embodiment 30. The compound of any one of Embodiments 1-29, wherein the AC is complementary to a target sequence comprising an intron, comprising by an exon, or bridging an intron/exon junction.

Embodiment 31. The compound of any one of Embodiments 1-30, wherein the AC is complementary to a target sequence comprising an intronic silencer sequence (ISS) or terminal stem loop (TSL) sequence of the target pre-mRNA.

Embodiment 32. The compound of any one of Embodiments 1-31, wherein the AC is complementary to part or all of a splice site.

Embodiment 33. The compound of Embodiment 32, wherein the splice site is a splice donor site, a splice acceptor site, a cryptic splice site, or a mutation-induced aberrant splice site.

Embodiment 34. The compound of any one of Embodiments 1-33, wherein hybridization of the AC with its target sequence results in exon skipping or exon inclusion.

Embodiment 35. The compound of any one of Embodiments 1-34, wherein the AC is 5-50 nucleotides in length.

Embodiment 36. The compound of any one of Embodiments 1-35, wherein the AC comprises one or more modified nucleotides or nucleic acids that affect one or more of nuclease resistance, pharmacokinetics, and affinity.

Embodiment 37. The compound of any one of Embodiments 1-36, wherein the AC comprises one or more phosphorodiamidate morpholino nucleosides, 2'-O-methylated nucleosides, and/or locked nucleic acids (LNAs).

Embodiment 38. The compound of any one of Embodiments 1-37, wherein the target gene is involved in the pathogenesis of a disease.

Embodiment 39. The compound of any one of Embodiments 1-38, wherein the target gene is involved in the pathogenesis of a genetic disease.

Embodiment 40. The compound of any one of Embodiments 1-39, wherein the target gene is involved in the pathogenesis of a cancer, an autoimmune disease, an inflammatory disease, or an infection.

Embodiment 41. The compound of any one of Embodiments 1-40, wherein the target sequence in the pre-mRNA comprises a mutation-induced aberrant splice site.

Embodiment 42. The compound of any one of Embodiments 1-41, wherein hybridization of the AC with the target sequence suppresses aberrant splicing of the target pre-mRNA.

Embodiment 43. The compound of any one of Embodiments 1-42, wherein hybridization of the AC with the target sequence induces preferential expression of one or more protein isomers encoded by the target gene.

Embodiment 44. The compound of any one of claims 1-43, wherein hybridization of the AC with the target sequence suppresses expression of one or more protein isomers encoded by the target gene.

Embodiment 45. The compound of any one of Embodiments 1-44, wherein the target protein produced by splicing and translation of the target pre-mRNA is not functional or is less functional than a wild type target protein in the absence of AC hybridization to the target sequence.

Embodiment 46. The compound of any one of Embodiments 1-45, wherein hybridization of the AC with the target sequence suppresses expression of the target protein that would have been expressed from the target pre-mRNA in the absence of AC hybridization.

Embodiment 47. The compound of any one of Embodiments 1-46, wherein hybridization of the AC with the target sequence results in expression of a re-spliced target protein having one or more improved functions or characteristics compared to the expressed target protein in the absence of AC hybridization.

Embodiment 48. The compound of Embodiment 47, wherein the one or more improved characteristics comprise function and/or activity.

Embodiment 49. The compound of Embodiment 47 or 48, wherein the re-spliced target protein comprises an active fragment of a wild type target protein.

Embodiment 50. The compound of any one of Embodiments 1-49, wherein hybridization of the AC with the target sequence results in expression of a re-spliced target protein that ameliorates or rescues aspects of a disease phenotype associated with the target gene.

Embodiment 51. A pharmaceutical composition comprising the compound of any one of Embodiments 1-50.

Embodiment 52. A cell comprising a compound of any one of Embodiments 1-50.

Embodiment 53. A method of modulating the splicing of a target pre-mRNA in a subject in need thereof, comprising administering a compound of any one of Embodiments 1-50.

Embodiment 54. The method of Embodiment 53, wherein the compound suppresses expression of the target protein translated from the target pre-mRNA in the absence of the compound.

Embodiment 55. The method of Embodiment 53 or 54, wherein administration of the compound results in the expression of a re-spliced target protein.

Embodiment 56. The method of Embodiment 55, wherein the re-spliced target protein has one or more improved characteristics compared to the target protein.

Embodiment 57. The method of Embodiment 56, wherein the one or more improved characteristics are selected from the list consisting of: function, activity, binding, and enzymatic activity.

Embodiment 58. The method of Embodiment 53, wherein administration of the compound results in the increased expression of one or more protein isomers encoded by the target gene.

Embodiment 59. The method of Embodiment 53, wherein administration of the compound results in the decreased expression of one or more protein isomers encoded by the target gene.

Embodiment 60. A method of treating a genetic disease in a subject in need thereof, comprising administering a compound of any one of Embodiments 1-50.

Embodiment 61. The method of Embodiment 60, wherein administration of the compound modulates splicing or expression of a target gene.

Embodiment 62. The method of Embodiment 60 or 61, wherein administration of the compound modulates splicing of the target pre-mRNA.

Embodiment 63. The method of any one of Embodiments 60-62, wherein administration of the compound results in an increase in the expression of a wild type target protein or an active fragment thereof.

Embodiment 64. The method of any one of Embodiments 60-63, wherein administration of the compound results in expression of a re-spliced target protein that is more highly expressed, functional, and/or active than the target protein expressed in the absence of the compound.

Embodiment 65. The method of Embodiment 64, wherein the re-spliced target protein comprises an active fragment of a wild type target protein.

Embodiment 66. The method of any one of Embodiments 60-65, wherein the genetic disease is a central nervous system disorder, a neuromuscular disorder, or a musculoskeletal disorder.

Embodiment 67. The method of any one of Embodiments 60-66, wherein the genetic disease is Duchenne muscular dystrophy, β thalassemia, dystrophin Kobe, osteogenesis imperfect, cystic fibrosis, Merosin-deficient congenital muscular dystrophy type 1A, or spinal muscular atrophy.

Embodiment 68. The method of any one of Embodiments 60-67, wherein the disease is Duchenne muscular dystrophy.

Embodiment 69. A composition comprising:
(a) a cell penetrating peptide (CPP) sequence; and
(b) CRISPR gene-editing machinery,
wherein the CPP is conjugated to the CRISPR gene-editing machinery.

Embodiment 70. The composition of Embodiment 69, wherein the CPP is conjugated to the CRISPR gene-editing machinery through a linker.

Embodiment 71. The composition of Embodiment 69, wherein the CRISPR gene-editing machinery comprises a gRNA, nuclease, or combination thereof.

Embodiment 72. The composition of Embodiment 71, wherein the CRISPR gene-editing machinery comprises a nuclease, wherein the nuclease is Cas9.

Embodiment 73. The method of Embodiment 68, wherein the AC has a nucleic acid sequence of 5'- GCTATTACCTTAACCCA-3' (SEQ ID NO: 152).

Embodiment 74. The method of Embodiment 73, wherein the AC is a phosphorodiamidate morpholino oligomer (PMO).

Embodiment 75. The method of any one of Embodiments 68, 73, and 74, wherein the CPP is cCPP12

Embodiment 76. A method of treating Alzheimer's Disease in a subject in need thereof, comprising administering a compound of any one of Embodiments 1-50.

Embodiment 77. The method of Embodiment 68, wherein the AC has a nucleic acid sequence of 5'- GTAACTGTATTTGGTACTTCC-3' (SEQ ID NO: 153).

Embodiment 78. The method of Embodiment 77, wherein the AC is a phosphorodiamidate morpholino oligomer (PMO).

Embodiment 79. The method of any one of Embodiments 76-78, wherein the CPP is cCPP12.

Embodiment 80. The compound of any one of any of the preceding Embodiments, comprising a nuclear localization sequence.

Embodiment 81. The compound or method of preceding Embodiments, wherein the CPP is cyclic.
The present application also includes the subject matter of the following numbered paragraphs which corresponds to the subject matter as originally claimed in PCT Application No. PCT/US2020/066459 (WO2021/127650) from which this application is ultimately derived:
1. A compound comprising:
   (a) a cyclic cell penetrating peptide (cCPP) sequence and
   (b) an antisense compound (AC) that is complementary to a target sequence in a pre-mRNA sequence.
2. The compound of paragraph 1, wherein the AC comprises at least one modified nucleotide or nucleic acid selected from a phosphorothioate (PS) nucleotide, a phosphorodiamidate morpholino nucleotide, a locked nucleic acid (LNA), a peptide nucleic acid (PNA), a nucleotide comprising a 2'-O-methyl (2'-OMe) modified backbone, a 2'O-methoxyethyl (2'-MOE) nucleotide, a 2',4' constrained ethyl (cEt) nucleotide, and a 2'-deoxy-2'-fluoro-beta-D-arabinonucleic acid (2'F-ANA), and
   wherein hybridization of the AC with the target sequence reduces or prevents splicing, inhibits or regulates translation, mediates degradation, or blocks expansions of nucleotide repeats.
3. The compound of paragraph 1, wherein the AC comprises small interfering RNA (siRNA), microRNA (miRNA), ribozymes, immune stimulating nucleic acids, antisense, antagomir, antimir, microRNA mimic, supermir, Ul adaptor, aptamer, or a CRISPR gene-editing machinery.
4. The compound of any one of the preceding paragraphs, wherein the cCPP is conjugated to the 5' end or the 3' end of the AC.
5. The compound of any one of the preceding paragraphs, further comprising a linker (L), which conjugates the cCPP to the AC.
6. The compound of any one of preceding paragraphs, wherein the L is covalently bound to the side chain of an amino acid on the CPP.
7. The compound of any one of the preceding paragraphs, having a structure according to Formula I-A or Formula I-B:

   cCPP-L-AC (I-A)

   or

   AC-L-cCPP (I-B),

   wherein L of Formula I-A is covalently bound to the side chain of an amino acid on the CPP and to the 5' end of the AC, and L of Formula I-B is covalently bound to the side chain of an amino acid on the CPP and the 3' end of the AC.
8. The compound of any one of paragraphs 7-12, wherein L comprises one or more D or L amino acids, each of which is optionally substituted; alkylene, alkenylene, alkynylene, carbocyclyl, or heterocyclyl, each of which is optionally substituted; or -(R¹⁻X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is independently selected from H, alkyl, alkenyl, alkynyl, carbocyclyl, and heterocyclyl, each of which is optionally substituted, and z is an integer from 1 to 20; or combinations thereof.
9. The compound of any one of paragraphs 5-8, wherein L comprises one or more D or L amino acids; -(R¹⁻X-R²)z-, wherein each of R¹ and R², at each instance, are independently alkylene, each X is independently NR³, -NR³C(O)-, S, and O, wherein R³ is independently selected from H and alkyl, and z is an integer from 1 to 20; or combinations thereof.
10. The compound of the preceding paragraphs, wherein the linker is conjugated to the AC through a bonding group (M).
11. The compound of paragraph 10, wherein M is selected from the group consisting of: 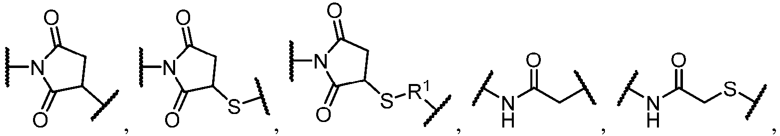 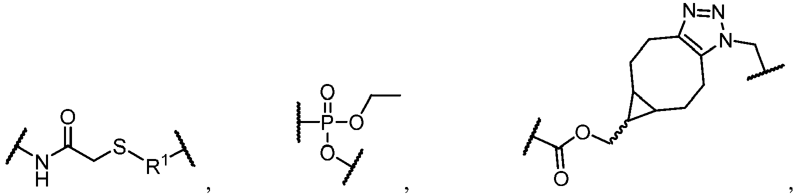 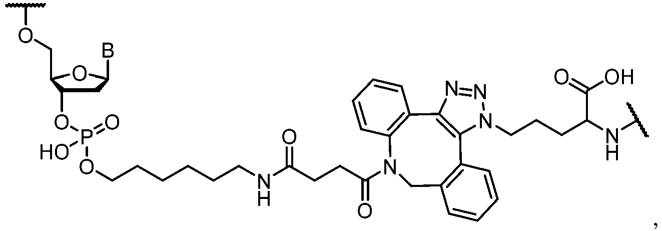 and 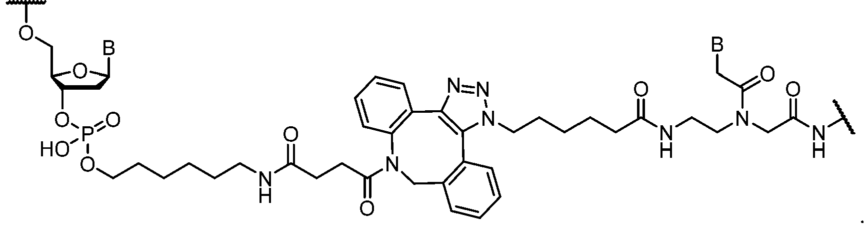 wherein: R¹ is alkylene, cycloalkyl, or 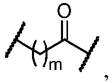 wherein m is 0 to 10 wherein each R is independently an alkyl, alkenyl, alkynyl, carbocyclyl, or heterocyclyl.
12. The compound of any one of paragraphs 10 or 11, wherein M is 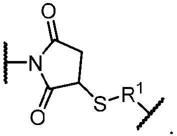
13. The compound of paragraph 12, wherein R¹ is 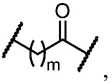 and m is 2.
14. The compound of any of the preceding paragraphs, wherein L has the following structure: 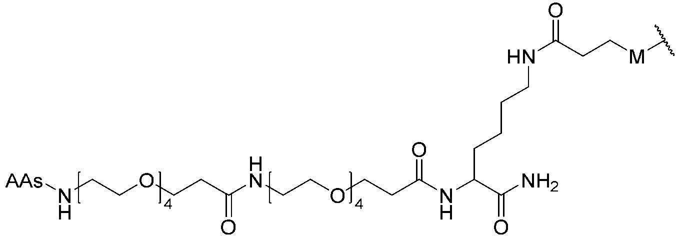 wherein AAₛ is a side chain or terminus of an amino acid on the CPP.
15. The compound of any of the preceding paragraphs having the following structure 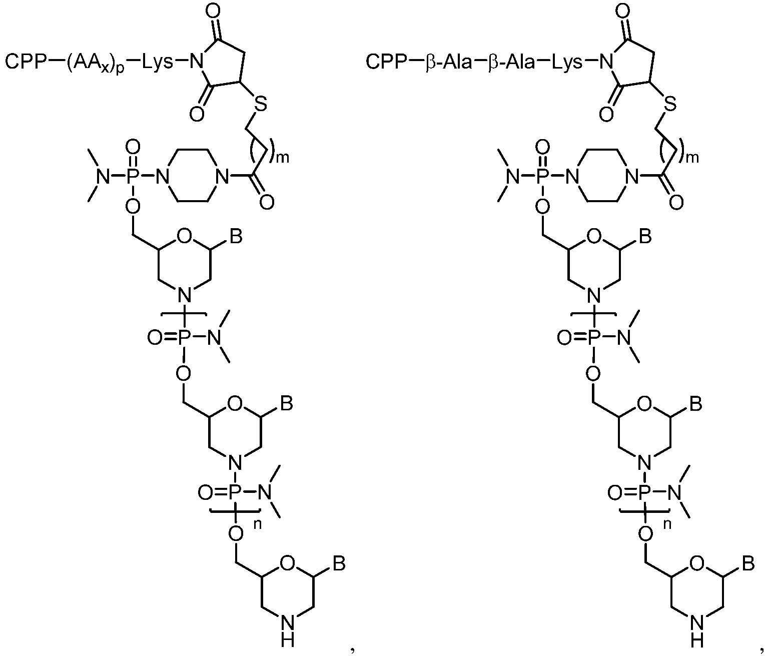 or 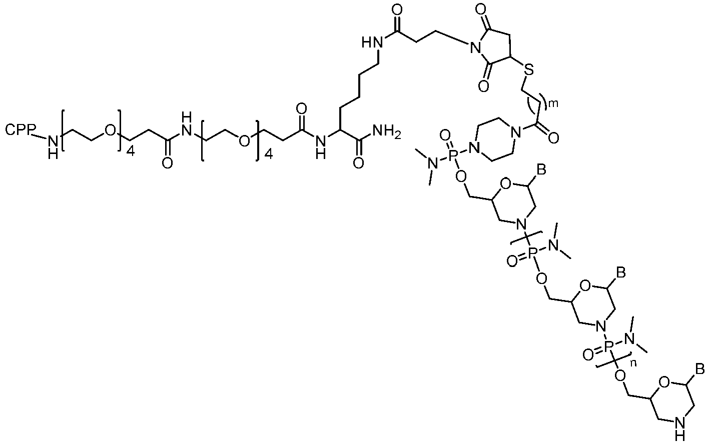 wherein
   each B is independently a nucleobase;
   each AAₓ is independently an amino acid;
   m is 1 to 10; and
   n is an integer from 1 to 50.
16. The compound of any one of the preceding paragraphs, wherein the cCPP has a sequence comprising Formula III: 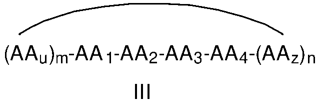 wherein:
   each of AA₁, AA₂, AA₃, and AA₄, are independently selected from a D or L amino acid, each of AAᵤ and AA_{z}, at each instance and when present, are independently selected from a D or L amino acid, and
   m and n are independently selected from a number from 0 to 6; and
   wherein:
   at least two amino acids are independently arginine, and
   at least two amino acids are independently a hydrophobic amino acid.
17. The compound of paragraph 16, wherein the cCPP has a sequence comprising any one of Formula IV-A-D: 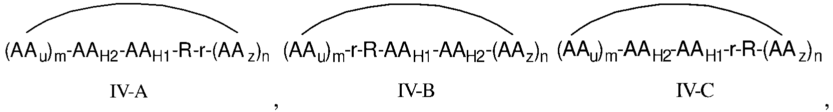 and 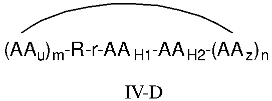 wherein:
   each of AA_{H1} and AA_{H2} are independently a D or L hydrophobic amino acid;
   at each instance and when present, each of AA_{U} and AA_{Z} are independently a D or L amino acid; and
   m and n are independently selected from a number from 0 to 6.
18. The compound of any one of the preceding paragraphs, wherein M is present and covalently bound to the 5' end of the AC or the 3' end of the AC.
19. The compound of any one of the preceding paragraphs, wherein the AC is complementary to a target sequence comprising an intron, comprising by an exon, or bridging an intron/exon junction.
20. The compound of any one of the preceding paragraphs, wherein the AC is complementary to a target sequence comprising an intronic silencer sequence (ISS) or terminal stem loop (TSL) sequence of the target pre-mRNA.
21. The compound of any one of the preceding paragraphs, wherein the AC is complementary to part or all of a splice site.
22. The compound of paragraph 19, wherein the splice site is a splice donor site, a splice acceptor site, a cryptic splice site, or a mutation-induced aberrant splice site.
23. The compound of any one of paragraphs 18-22, wherein hybridization of the AC with its target sequence results in exon skipping or exon inclusion.
24. The compound of any one of paragraphs 1-16, wherein the AC is complementary to at least a portion of expended nucleotide repeats in target mRNA.
25. The compound of paragraph 17, wherein hybridization of the AC with the target sequence blocks transcription of at least a portion of the nucleotide repeats.
26. The compound of any one of any one of the preceding paragraphs, wherein the AC is 5-50 nucleotides in length.
27. The compound of any one of the preceding paragraphs, wherein the AC comprises one or more modified nucleotides or nucleic acids that affect one or more of nuclease resistance, pharmacokinetics, and affinity.
28. The compound of any one of the preceding paragraphs, wherein the AC comprises one or more phosphorodiamidate morpholino nucleosides, 2'-O-methylated nucleosides, and/or locked nucleic acids (LNAs).
29. The compound of any one of the preceding paragraphs, wherein the target gene is involved in the pathogenesis of a disease.
30. The compound of any one of the preceding paragraphs, wherein the target gene is involved in the pathogenesis of a genetic disease.
31. The compound of any one of the preceding paragraphs, wherein the target gene is involved in the pathogenesis of a cancer, an autoimmune disease, an inflammatory disease, or an infection.
32. The compound of any one of the preceding paragraphs, wherein the target sequence in the pre-mRNA comprises a mutation-induced aberrant splice site.
33. The compound of any one of the preceding paragraphs, wherein hybridization of the AC with the target sequence suppresses aberrant splicing of the target pre-mRNA.
34. The compound of any one of the preceding paragraphs, wherein hybridization of the AC with the target sequence induces expression of one or more protein isomers encoded by the target gene.
35. The compound of any one of the preceding paragraphs, wherein hybridization of the AC with the target sequence suppresses expression of one or more protein isomers encoded by the target gene.
36. The compound of any one of the preceding paragraphs, wherein the target protein produced by splicing and translation of the target pre-mRNA is not functional or is less functional than a wild type target protein in the absence of AC hybridization to the target sequence.
37. The compound of any one of the preceding paragraphs, wherein hybridization of the AC with the target sequence suppresses expression of the target protein that would have been expressed from the target pre-mRNA in the absence of AC hybridization.
38. The compound of any one of the preceding paragraphs, wherein hybridization of the AC with the target sequence results in expression of a re-spliced target protein having one or more improved functions or characteristics compared to the expressed target protein in the absence of AC hybridization.
39. The compound of paragraph 38, wherein the one or more improved characteristics comprise function and/or activity.
40. The compound of paragraph 38 or 39, wherein the re-spliced target protein comprises an active fragment of a wild type target protein.
41. The compound of any one of paragraphs 1-40, wherein hybridization of the AC with the target sequence results in expression of a re-spliced target protein that ameliorates or rescues aspects of a disease phenotype associated with the target gene.
42. The compound of any of the preceding paragraphs, further comprising a nuclear localization signal (NLS).
43. The compound of paragraph 42, wherein the C-terminus of the NLS sequence is conjugated to the CPP.
44. A pharmaceutical composition comprising the compound of any one of paragraphs 1-43.
45. A cell comprising a compound of any one of paragraphs 1-43.
46. A method of modulating the splicing of a target pre-mRNA in a subject in need thereof, comprising administering a compound of any one of paragraphs 1-44.
47. The method of paragraph 46, wherein the compound suppresses expression of the target protein translated from the target pre-mRNA in the absence of the compound.
48. The method of paragraph 46 or 47, wherein administration of the compound results in the expression of a re-spliced target protein.
49. The method of paragraph 48, wherein the re-spliced target protein has one or more improved characteristics compared to the target protein.
50. The method of paragraph 49, wherein the one or more improved characteristics are selected from the list consisting of: function, activity, binding, and enzymatic activity.
51. The method of paragraph 46wherein administration of the compound results in the increased expression of one or more protein isomers encoded by the target gene.
52. The method of paragraph 46, wherein administration of the compound results in the decreased expression of one or more protein isomers encoded by the target gene.
53. The method of paragraph 56, wherein administration of the compound results in increased expression of a wild type target protein or an active fragment of a wild type target protein.
54. The method of any one of paragraphs 46-53, wherein the compound modulates splicing of exon 2, 8, 11, 17, 19, 23, 29, 40, 41, 42, 43, 44, 45, 46, 48, 49, 50, 51, 52, 53, 55, and 59 of DMD.
55. The method of any one of paragraphs 46-53, wherein the compound modulates splicing of exon 2, 8, 11, 23 43, 44, 45, 50, 51, 53, and 55 of DMD.
56. The method of any one of paragraphs 46-53, wherein the compound modulates splicing of exon 2, 23, 44, or 51 of DMD.
57. The method of paragraph 56, wherein the AC is selected from Table B1-B3.
58. The method of any one of paragraphs 46-53, wherein the compound modulates splicing of exon 1, exon 2, exon 3, exon 4, exon 5, exon 6, exon 7a, and exon 7b of CD33.
59. The method of paragraph 56, wherein the AC is selected from Table C.
60. A method of treating a genetic disease in a subject in need thereof, comprising administering a compound of any one of paragraphs 1-43.
61. The method of paragraph 60, wherein administration of the compound modulates splicing or expression of a target gene, degrades mRNA, stabilizes mRNA, or sterically blocks mRNA.
62. The method of paragraph 61, wherein administration of the compound modulates splicing of the target pre-mRNA.
63. The method of any one of paragraphs 60-63, wherein administration of the compound results in an increase in the expression of a wild type target protein or an active fragment thereof.
64. The method of any one of paragraphs 65, wherein administration of the compound results in expression of a re-spliced target protein that is more highly expressed, functional, and/or active than the target protein expressed in the absence of the compound.
65. The method of paragraph 64, wherein the re-spliced target protein comprises an active fragment of a wild type target protein.
66. The method of any one of paragraphs 62-65, wherein the genetic disease is a central nervous system disorder, a neuromuscular disorder, or a musculoskeletal disorder.
67. The method of any one of paragraphs 62-66, wherein the genetic disease is Duchenne muscular dystrophy, β thalassemia, dystrophin Kobe, osteogenesis imperfect, cystic fibrosis, Merosin-deficient congenital muscular dystrophy type 1A, or spinal muscular atrophy.
68. The method of any one of paragraphs 62-67, wherein the disease is Duchenne muscular dystrophy.
69. The method of paragraph 68, wherein the AC is selected from Table B1-B3.
70. The method of paragraph 69, wherein the AC has a nucleic acid sequence of 5'-GCTATTACCTTAACCCA-3' (SEQ ID NO: 152).
71. The method of paragraph 70, wherein the AC is a phosphorodiamidate morpholino oligomer (PMO).
72. The method of paragraph 71, wherein the AC has a nucleic acid sequence of 5'-GTAACTGTATTTGGTACTTCC-3' (SEQ ID NO: 153).
73. The method of paragraph 72, wherein the AC is a phosphorodiamidate morpholino oligomer (PMO).
74. The method of paragraph 46-73, wherein the splicing results in an increase in the expression of a wild type target protein or an active fragment thereof in muscle tissue, diaphragm tissue, quadricep, and/or heart tissue.
75. The method of paragraph 46-74, wherein the splicing increase in the expression of a wild type target protein or an active fragment thereof in heart tissue.
76. The method of paragraph 61, wherein administration of the compound blocks transcription of at least a portion of the trinucleotide repeats.
77. The method of paragraph 76, wherein administration of the compound reduces the length and/or number of expanded nucleotide repeats an expressed protein.
78. The method of paragraph 76 or 77, wherein the genetic disease is Fragile X, Friedreich's ataxia (FRDA), Huntington's Disease (HD), Myotonic dystrophy type 1 (DM1), Myotonic dystrophy type 2 (DM2), Spinal and bulbar muscular atrophy (SBMA), Spinal cerebellar ataxia type 1 (SCA1), Spinal cerebellar ataxia type 2 (SCA2), or Spinal cerebellar ataxia type 3 (SCA3).
79. The method of paragraph 76-78, wherein the genetic disease is Fragile X, Friedreich's ataxia (FRDA), Huntington's Disease (HD), Myotonic dystrophy type 1 (DM1).
80. The method of paragraph 79, wherein the genetic disease is FRDA, and the AC is selected from Table 8.
81. The method of paragraph 79, wherein the disease is DM1, and the AC is selected from Table 7.
82. The method of paragraph 61, wherein the AC comprises a gapmer, which hybridizes with the target RNA and catalyzes degradation of the target.
83. The method of paragraph 82, wherein the AC is selected from Table 8.
84. A method of treating Fragile X, Friedreich's ataxia (FRDA), Huntington's Disease (HD), Myotonic dystrophy type 1 (DM1), Myotonic dystrophy type 2 (DM2), Spinal and bulbar muscular atrophy (SBMA), Spinal cerebellar ataxia type 1 (SCA1), Spinal cerebellar ataxia type 2 (SCA2), or Spinal cerebellar ataxia type 3 (SCA3), comprising administering a compound of any one of paragraphs 1-41, wherein the AC comprises a sequence that is complementary to a trinucleotide repeat in a target mRNA sequence, and the AC hybridizes with the target mRNA sequence to blocks transcription of the trinucleotide repeat.
85. The method of paragraph 84, wherein the genetic disease is FRDA, and the AC is selected from Table 8.
86. The method of paragraph 84, wherein the disease is DM1, and the AC is selected from Table 7.
87. A method of blocking or degrading nucleotide repeat expansions in RNA comprising administering a compound of paragraphs 1-43, wherein the AC comprises an antisense oligonucleotide sequence that is complementary to at least a portion of the nucleotide repeats in the target RNA.
88. The method of paragraph 87, wherein the administration prevents or reduces translation of at least a portion of the nucleotide repeats.
89. The method of paragraphs 87 or 88, wherein the nucleotide repeats expansions are trinucleotide repeat expansions, pentanucleotide repeat expansions, or hexanucleotide repeat expansions.
90. The method of paragraphs 87-89, wherein the nucleotide repeat expansions comprises expansions of CAG repeats, CGG repeats, GCC repeats, GAA repeats, or CUG repeats.
91. The method of paragraphs 87-90, wherein the nucleotide repeat expansions are located in the *FMR1* gene, *HTT* gene, *ATP7B* gene, *DMPK* gene, or *FXN* gene.
92. The method of paragraphs 87-90, wherein the nucleotide repeat expansions are located in the *DMPK* gene or *FXN* gene.
93. The method of paragraphs 87-92, wherein the gene is *FXN,* and the AC is selected from Table 8.
94. The method of paragraphs 87-92, wherein the gene is *DMPK,* and the AC is selected from Table 9.
95. The compound or method of any of the preceding paragraphs, wherein the cCPP is selected from Table 4.
96. The compound or method of any of the preceding paragraphs, wherein the cCPP is cCPP12.

## Claims

1. A compound comprising:
(a) a cyclic cell penetrating peptide (cCPP) sequence having a sequence comprising Formula III: 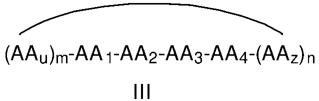 wherein:
each of AA₁, AA₂, AA₃, and AA₄, is independently selected from a D or L amino acid,
each of AAᵤ and AA_{z}, at each instance and when present, is independently selected from a D or L amino acid, and
m and n are independently selected from a number from 0 to 6;
at least two amino acids are independently arginine, and
at least two amino acids are independently a hydrophobic amino acid;
(b) an antisense compound (AC) that is 5-50 nucleotides in length and is complementary to a target sequence in a pre-mRNA sequence, wherein the cCPP is conjugated to the 5' end or the 3' end of the AC;
(c) a linker (L), which conjugates the cCPP to the AC, wherein the L is covalently bound to the side chain of an amino acid on the cCPP;
wherein the compound has a structure according to Formula I-A or Formula I-B:
cCPP-L-AC (I-A)
or
AC-L-cCPP (I-B),
wherein L of Formula I-A is covalently bound to the side chain of an amino acid on the CPP and to the 5' end of the AC, and L of Formula I-B is covalently bound to the side chain of an amino acid on the CPP and the 3' end of the AC; and
(d) a nuclear localization signal (NLS) conjugated to the cCPP, wherein the C-terminus of the NLS sequence is conjugated to the CPP.

2. The compound of claim 1, wherein the AC comprises small interfering RNA (siRNA), microRNA (miRNA), ribozymes, immune stimulating nucleic acids, antisense, antagomir, antimir, microRNA mimic, supermir, Ul adaptor, aptamer, or a CRISPR gene-editing machinery.

3. The compound of claim 1, wherein the AC is a phosphorodiamidate morpholino oligomer (PMO).

4. The compound of any one of claims 1-3, wherein L comprises:
one or more D or L amino acids, each of which is optionally substituted;
alkylene, alkenylene, alkynylene, carbocyclyl, or heterocyclyl, each of which is optionally substituted; or
-(R¹⁻X-R²)z-, wherein each of R¹ and R², at each instance, are independently selected from alkylene, alkenylene, alkynylene, carbocyclyl, and heterocyclyl, wherein
each X is independently NR³, -NR³C(O)-, S, and O, wherein
R³ is independently selected from H, alkyl, alkenyl, alkynyl, carbocyclyl, and heterocyclyl, each of which is optionally substituted, and
z is an integer from 1 to 20;
or combinations thereof.

5. The compound of any one of claims 1-4, wherein L comprises:
one or more D or L amino acids;
-(R¹⁻X-R²)z-, wherein each of R¹ and R², at each instance, are independently alkylene,
wherein
each X is independently NR³, -NR³C(O)-, S, and O,
R³ is independently selected from H and alkyl, and
z is an integer from 1 to 20;
or combinations thereof.

6. The compound of any of claims 1-5, wherein the linker is conjugated to the AC through a bonding group (M) selected from the group consisting of: 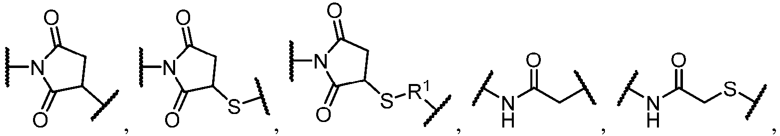 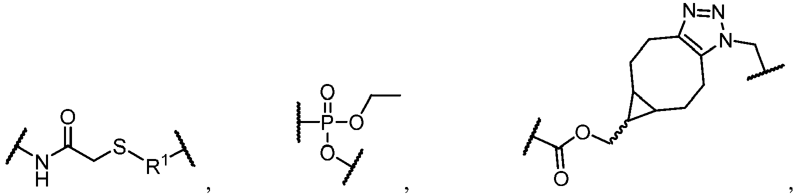 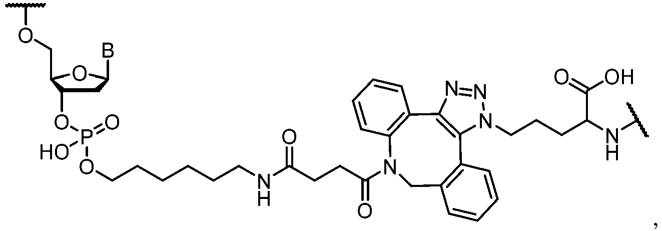 and 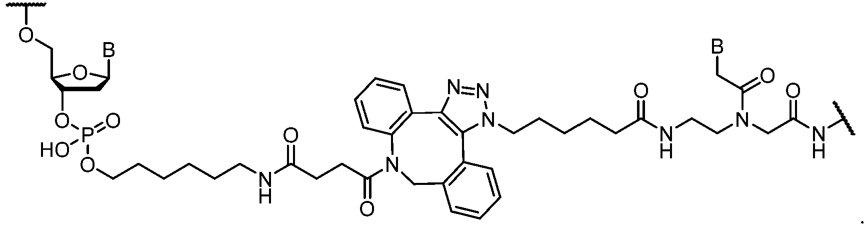 wherein R¹ is alkylene, cycloalkyl, or 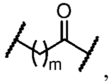 wherein m is 0 to 10 wherein each R is independently an alkyl, alkenyl, alkynyl, carbocyclyl, or heterocyclyl.

7. The compound of claim 6, wherein M is

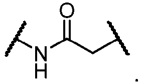

8. The compound of claim 1, wherein the NLS comprises the seven amino acid sequence PKKKRKV.

9. A compound comprising:
(a) a cyclic cell penetrating peptide (cCPP) sequence comprising any one of Formula IV-A-D: 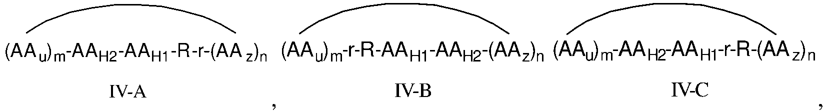 and 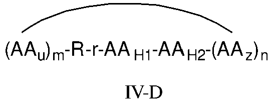 wherein:
each of AA_{H1} and AA_{H2} are independently a D or L hydrophobic amino acid;
at each instance and when present, each of AA_{U} and AA_{Z} are independently a D or L amino acid; and
m and n are independently selected from a number from 0 to 6; and
wherein:
at least two amino acids are independently arginine, and
at least two amino acids are independently a hydrophobic amino acid;
(b) a nuclear localization sequence (NLS) comprising the seven amino acid sequence PKKKRKV;
(c) a linker (L) wherein L comprises one or more D or L amino acids, each of which is optionally substituted; alkylene, alkenylene, alkynylene, carbocyclyl, or heterocyclyl, each of which is optionally substituted; or -(R¹⁻X-R²)z-, wherein each of R¹ and R², at each instance, are independently selected from alkylene, alkenylene, alkynylene, carbocyclyl, and heterocyclyl, each X is independently NR³, -NR³C(O)-, S, and O, wherein R³ is independently selected from H, alkyl, alkenyl, alkynyl, carbocyclyl, and heterocyclyl, each of which is optionally substituted, and z is an integer from 1 to 20; or combinations thereof; and
(d) an antisense compound (AC) that is complementary to a target sequence in a pre-mRNA sequence, wherein hybridization of the AC with its target sequence results in exon skipping or exon inclusion, wherein the compound has a structure according to Formula I-A or Formula I-B:
cCPP-L-AC (I-A)
or
AC-L-cCPP (I-B),
wherein L of Formula I-A is covalently bound to the side chain of an amino acid on the cCPP and to the 5' end of the AC, and L of Formula I-B is covalently bound to the side chain of an amino acid on the cCPP and the 3' end of the AC, and wherein the NLS is coupled to the AC, the cCPP or the linker (L).

10. The compound of any of the preceding claims, wherein the cCPP is selected from Table 4.

11. The compound of any of the preceding claims, wherein the cCPP is cCPP12.

12. A pharmaceutical composition comprising the compound of any one of claims 1-11.

13. The pharmaceutical composition of claim 12 for use in a method of modulating the splicing of a target pre-mRNA in a subject in need thereof.

14. The pharmaceutical composition for use of claim 13, wherein the compound modulates splicing of exon 2, 8, 11, 17, 19, 23, 29, 40, 41, 42, 43, 44, 45, 46, 48, 49, 50, 51, 52, 53, 55, and 59 of DMD.

15. The pharmaceutical composition for use of claim 13, wherein the compound modulates splicing of exon 2, 8, 11, 23, 43, 44, 45, 50, 51, 53, and 55 of DMD.

16. The pharmaceutical composition for use of claim 13, wherein the compound modulates splicing of exon 2, 23, 44, or 51 of DMD.

17. The compound of any of claims 1-11 for use in a method of treating a genetic disease in a subject in need thereof.

18. The compound for use of claim 17, wherein the genetic disease is a central nervous system disorder, a neuromuscular disorder, or a musculoskeletal disorder.

19. The compound for use of claim 17 or 18, wherein the disease is Duchenne muscular dystrophy.

20. The pharmaceutical composition for use of any of claims 13-16, wherein the splicing results in an increase in the expression of a wild type target protein or an active fragment thereof in muscle tissue, diaphragm tissue, quadricep, and/or heart tissue.
